# EUROPÄISCHE PATENTSCHRIFT

(11) **EP 2 393 812 B1**
(45) Veröffentlichungstag und Bekanntmachung des Hinweises auf die Patenterteilung: **03.07.2013**
(21) Anmeldenummer: 10703021.5
(22) Anmeldetag: 05.02.2010
(51) Int. Cl.: C07D 471/10, C07D 487/10, C07D 498/10, C07D 401/14, A61K 31/438, A61P 25/04

(54) **SUBSTITUIERTE SPIROAMIDE ALS B1R-MODULATOREN**
SUBSTITUTED SPIROAMIDES AS B1R-MODULATORS
SPIROAMIDES SUBSTITUÉS EN TANT QUE MODULATEURS DU B1R

(30) Priorität: 06.02.2009 EP 09001659
(43) Veröffentlichungstag der Anmeldung: 14.12.2011
(73) Patentinhaber: Grünenthal GmbH, 52078 Aachen (DE)
(72) Erfinder: SCHUNK, Stefan, 52070 Aachen (DE); REICH, Melanie, 52072 Aachen (DE); ENGELS, Michael Franz-Martin, B-2300 Turnhout (BE); GERMANN, Tieno, 52080 Aachen (DE); JOSTOCK, Ruth, 52223 Stolberg (DE); HEES, Sabine, 52076 Aachen (DE)
(86) Internationale Anmeldenummer: PCT/EP2010/000714
(87) Internationale Veröffentlichungsnummer: WO 2010/089127

(56) Entgegenhaltungen:
- WO-A2-2007/140383

## Beschreibung

Die vorliegende Erfindung betrifft substituierte Spiroamide, Verfahren zu deren Herstellung, Arzneimittel enthaltend diese Verbindungen und die Verwendung von substituierten Spiroamiden zur Herstellung von Arzneimitteln.

Im Gegensatz zur konstitutiven Expression des Bradykinin 2 Rezeptors (B2R) wird der Bradykinin 1 Rezeptor (B1R) in den meisten Geweben nicht oder nur schwach exprimiert. Allerdings ist die Expression des B1R auf verschiedenen Zellen induzierbar. Beispielsweise erfolgt im Verlauf von Entzündungsreaktionen eine rasche und ausgeprägte Induktion des B1R auf neuronalen Zellen aber auch verschiedenen peripheren Zellen wie Fibroblasten, Endothelzellen, Granulozyten, Makrophagen und Lymphozyten. Somit kommt es im Verlauf von Entzündungsreaktionen zu einem Switch von einer B2R zu einer B1R Dominanz auf den beteiligten Zellen. Wesentlich an dieser B1 R Heraufregulation beteiligt sind die Zytokine Interleukin-1 (IL-1) und Tumor Nekrose Faktor alpha (TNFα) (Passos et al. J. Immunol. 2004, 172, 1839-1847). Nach Aktivierung mit spezifischen Liganden können B1 R-exprimierende Zellen anschließend selbst entzündungsfördernde Zytokine wie IL-6 und IL-8 sezernieren (Hayashi et al., Eur. Respir. J. 2000, 16, 452-458). Dies führt zur Einwanderung weiterer Entzündungszellen, z.B. neutrophiler Granulozyten (Pesquero et al., PNAS 2000, 97, 8140-8145). Über diese Mechanismen kann das Bradykinin-B1R-System zur Chronifizierung von Erkrankungen beitragen. Dies belegt eine Vielzahl tierexperimenteller Untersuchungen (Übersichten in Leeb-Lundberg et al., Pharmacol Rev. 2005, 57, 27-77 und Pesquero et al., Biol. Chem. 2006, 387, 119-126). Auch am Menschen zeigt sich eine verstärkte Expression des B1R, z.B. auf Enterozyten und Makrophagen im betroffenen Gewebe von Patienten mit entzündlichen Darmerkrankungen (Stadnicki et al., Am. J. Physiol. Gastrointest. Liver Physiol. 2005, 289, G361-366) bzw. auf T-Lymphozyten von Patienten mit Multipler Sklerose (Pratet al., Neurology. 1999; 53, 2087-2092) oder eine Aktivierung des Bradykinin-B2R-B1R Systems im Verlauf von Infektionen mit Staphyloccocus aureus (Bengtson et al., Blood 2006, 108, 2055-2063). Infektionen mit Staphyloccocus aureus sind verantwortlich für Krankheitsbilder wie oberflächliche Infektionen der Haut bis hin zu septischem Schock.

Aufgrund der dargestellten pathophysiologischen Zusammenhänge ergibt sich für die Anwendung von B1 R-Antagonisten ein großes therapeutisches Potential bei akuten und insbesondere chronisch-entzündlichen Erkrankungen. Hierzu gehören Erkrankungen der Atemwege (Asthma bronchiale, Allergien, COPD/chronic-obstruktive pulmonary disease, zystische Fibrose usw.), entzündliche Darmerkrankungen (Ulcerative Colitis, CD/Crohn's disease usw.), neurologische Erkrankungen (Multiple Sklerose, Neurodegeneration usw.), Entzündungen der Haut (atopische Dermatitis, Psoriasis, bakterielle Infektionen usw.) und Schleimhäute (M. Behcet, Pelvitis, Prostatitis usw.), rheumatische Erkrankungen (rheumatoide Arthritis, Osteoarthritis usw.), septischen Schock und Reperfusionssyndrom (nach Herzinfarkt, Schlaganfall).

Darüber hinaus ist das Bradykinin(Rezeptor)-System auch an der Regulation der Angiogenese beteiligt (Potential als Angiogenese-Inhibitor bei Krebs sowie Makula-Degeneration am Auge) und B1 R-knockout Mäuse sind geschützt vor der Induktion von Übergewicht durch eine besonders fettreiche Ernährung (Pesquero et al., Biol. Chem. 2006, 387, 119-126). B1 R-Antagonisten eignen sich daher auch zur Behandlung von Fettleibigkeit.

B1R-Antagonisten sind insbesondere geeignet zur Behandlung von Schmerz, insbesondere Entzündungsschmerz und neuropathischem Schmerz (Calixto et al., Br. J. Pharmacol 2004, 1-16), hier insbesondere von diabetischer Neuropathie (Gabra et al., Biol. Chem. 2006, 387, 127-143). Weiterhin eignen sie sich zur Behandlung von Migräne.

Bei der Entwicklung von B1R-Modulatoren besteht jedoch das Problem, dass der humane und der Ratten-B1-Rezeptor sich so stark unterscheiden, dass viele Verbindungen, die gute B1R-Modulatoren am humanen Rezeptor sind, nur eine schlechte oder keine Affinität zum Ratten-Rezeptor aufweisen. Dies erschwert die tierpharmakologischen Untersuchungen beträchtlich, da viele Untersuchungen normalerweise an der Ratte durchgeführt werden. Wenn jedoch keine Wirksamkeit am Ratten-Rezeptor vorhanden ist, können weder Wirkung noch Nebenwirkung an der Ratte untersucht werden. Dies hat bereits dazu geführt, dass transgene Tiere mit humanen B1-Rezeptoren für tierpharmakologische Untersuchungen erzeugt wurden (Hess et al., Biol. Chem 2006; 387(2):195-201). Die Arbeit mit transgenen Tieren ist jedoch teurer als die Arbeit mit den unveränderten Tieren.

In den Patentanmeldungen WO 2007/140383 und WO 2007/101007 werden Verbindungen beschrieben, die in *in-vitro*-Assays eine antagonistische Wirkung am Makak-B1-Rezeptor aufweisen. Experimentelle Daten zur Aktivität am humanen B1-Rezeptor oder dem B1-Rezeptor der Ratte sind nicht offenbart.

In den Patentanmeldung WO 2008/040492 und WO 2008/046573 werden Verbindungen beschrieben, die in *in-vitro*-Assays sowohl am humanen B1-Rezeptor als auch am B1-Rezeptor der Ratte antagonistische Wirkung zeigen.

Es besteht weiterhin der Bedarf an neuen B1R-Modulatoren, wobei B1 R-Modulatoren, die sowohl an den Ratten-Rezeptor als auch an den humanen Rezeptor binden, besondere Vorteile bieten.

Eine Aufgabe der vorliegenden Erfindung bestand daher darin, neue Verbindungen zur Verfügung zu stellen, die sich insbesondere als pharmakologische Wirkstoffe in Arzneimitteln eignen, vorzugsweise in Arzneimitteln zur Behandlung von Störungen oder Krankheiten, die zumindest teilweise durch B1R-Rezeptoren vermittelt werden.

Diese Aufgabe wird durch die erfindungsgemäßen substituierten Spiroamide gelöst.

Ein Gegenstand der Erfindung sind daher substituierte Spiroamide der allgemeinen Formel (I) 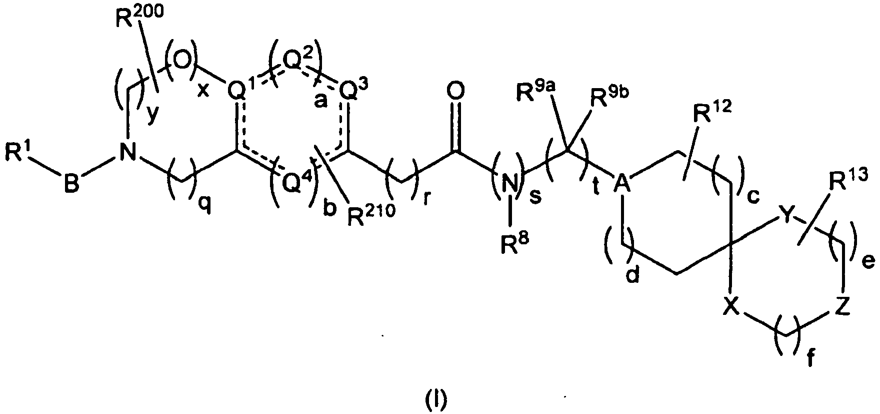 worin
Q¹ für C oder N steht;
Q² für CH, N, O oder S steht;
Q³ für CH, N, O oder S steht;
Q⁴ für CH, N, O oder S steht;
B für C(=O), S(=O)₂ oder für die Gruppe -C(=O)-N(R⁴) steht, wobei deren Stickstoffatom an den Rest R¹ gebunden ist;
a für 0, 1 oder 2 steht;
b für 0 oder 1 steht, mit der Maßgabe, dass a+b = 1 oder 2 ist;
q für 0 oder 1 steht;
x für 0 oder 1 steht;
y für 1, 2 oder 3 steht;
r für 0, 1, 2 oder 3 steht;
R¹ für Aryl, Heteroaryl oder ein über eine C₁₋₃-Alkylengruppe oder C₂₋₆-Alkenylengruppe gebundenes Aryl oder Heteroaryl steht, wobei Aryl und Heteroaryl jeweils mit einem 4-, 5-, 6- oder 7- gliedrigen Cyclus oder Heterocyclus anelliert sein können, wobei der Cyclus und der Heterocyclus jeweils gesättigt oder wenigstens einfach, beispielsweise einfach oder zweifach, ungesättigt, nicht aber aromatisch ist und jeweils an einem oder mehreren seiner Kohlenstoff-Ringglieder mit einem oder mehreren Resten unabhängig voneinander ausgewählt aus der Gruppe bestehend aus F, Cl, Br, I, -CF₃, -O-CF₃, C₁₋₆-Alkyl und O-C₁₋₆-Alkyl substituiert sein kann und wobei der Heterocyclus ein oder mehr, beispielsweise 1, 2 oder 3, Heteroatome oder Heteroatomgruppen unabhängig voneinander ausgewählt aus der Gruppe bestehend aus N, NR⁵⁰, O, S, S=O oder S(=O)₂ enthalten kann; wobei der Rest R⁵⁰ H, C₁₋₆-Alkyl, -C(=O)-R⁵¹, C₃₋₈-Cycloalkyl, Aryl, Heteroaryl oder ein über eine C₁₋₃-Alkylengruppe gebundenes C₃₋₈-Cycloalkyl, Aryl oder Heteroaryl bedeutet, und R⁵¹ C₁₋₆-Alkyl, C₃₋₈-Cycloalkyl, Aryl, Heteroaryl oder ein über eine C₁₋₃-Alkylengruppe gebundenes C₃₋₈-Cycloalkyl, Aryl oder Heteroaryl bedeutet;
R⁴ für H, C₁₋₆Alkyl, Aryl oder ein über eine C₁₋₃-Alkylengruppe gebundenes Aryl steht;
R²⁰⁰ für 0-4 Substituenten steht, die unabhängig voneinander ausgewählt sind aus der Gruppe bestehend aus C₁₋₆-Alkyl, F, Cl, CF₃ und OCF₃;
R²¹⁰ für 0-4 Substituenten steht, die unabhängig voneinander ausgewählt sind aus der Gruppe bestehend aus C₁₋₆-Alkyl, O-C₁₋₆-Alkyl, F, Cl, CF₃ und OCF₃;
s = 0 oder 1 ist,
t = 0, 1, 2 oder 3 ist mit der Maßgabe, dass wenn s für 0 steht t für 0 steht,
R⁸ für H, C₁₋₆-Alkyl, C₃₋₈-Cycloalkyl, Aryl oder Heteroaryl, oder für ein über eine C₁₋₆-Alkylengruppe gebundenes C₃₋₈-Cycloalkyl, Aryl oder Heteroaryl steht;
R^{9a} und R^{9b} jeweils unabhängig voneinander H, F, Cl, OH, C₁₋₆-Alkyl, O-C₁₋₆-Alkyl. C₃₋₈-Cycloalkyl, Aryl oder Heteroaryl, oder ein über eine C₁₋₆-Alkylengruppe gebundenes C₃₋₈-Cycloalkyl, Aryl oder Heteroaryl bedeuten;
- A: für N oder CH steht;
mit der Maßgabe, dass wenn s für 1 und t für 0 steht, A für CH steht; und mit der Maßgabe, dass wenn s und t jeweils für 0 stehen, A für N steht;
- c, d, e und f: jeweils unabhängig voneinander 0, 1 oder 2 bedeuten;
R¹² und R¹³ jeweils unabhängig voneinander für 0 bis 4 Substituenten stehen, die jeweils unabhängig voneinander ausgewählt sind aus der Gruppe bestehend aus F, Cl, OH, =O, C₁₋₆-Alkyl, O-C₁₋₆-Alkyl, C₃₋₈-Cycloalkyl, Aryl, Heteroaryl und über eine C₁₋₆-Alkylengruppe gebundenem C₃₋₈-Cycloalkyl, Aryl oder Heteroaryl;
und/oder zwei benachbarte der 0-4 Substituenten R¹³ ein anelliertes Aryl oder Heteroaryl bilden;
- X: für CR^{14a}R^{14b}, NR¹⁵ oder O steht;
- Y: für CR^{16a}R^{16b}, NR¹⁷ oder O steht;
mit der Maßgabe, dass X nicht NR¹⁵ bedeutet, wenn Y NR¹⁷ bedeutet; und mit der Maßgabe, dass X und Y nicht gleichzeitig O bedeuten;
wobei
R^{14a}, R^{14b}, R^{16a} und R^{16b} jeweils unabhängig voneinander H, F, Cl, OH, C₁₋₆-Alkyl, O-C₁₋₆-Alkyl, C₃₋₈-Cycloalkyl, Aryl oder Heteroaryl bedeuten, oder für ein über eine C₁₋₆-Alkylengruppe gebundenes C₃₋₈-Cycloalkyl, Aryl oder Heteroaryl stehen, und/oder jeweils R^{14a} und R^{14b} gemeinsam für =O und/oder jeweils R^{16a} und R^{16b} gemeinsam für =O stehen können;
R¹⁵ und R¹⁷ jeweils unabhängig voneinander für H, C₁₋₆-Alkyl, C₃₋₈-Cycloalkyl, Aryl oder Heteroaryl stehen, oder ein über eine C₁₋₆-Alkylengruppe gebundenes C₃₋₈-Cycloalkyl, Aryl oder Heteroaryl bedeuten;
Z für CR^{18a}R^{18b}, NR¹⁹ oder O steht;
oder
Z in dem Fall, dass X für O und f für 0 steht, -(C(R¹²⁴)-C(R¹²⁵))-bedeutet, wobei
R¹²⁴ und R¹²⁵ gemeinsam mit den sie verbindenden Kohlenstoffatomen ein ankondensiertes Aryl oder Heteroaryl bilden; oder
Z in dem Fall dass X für O und f für 0 steht, -(N=(CR¹²⁶))-bedeutet, wobei das N-Atom einfach an das O-Atom gebunden ist, und
R¹²⁶ für H, C₁₋₆-Alkyl, C₃₋₈Cycloalkyl, Aryl oder Heteroaryl steht oder ein über eine C₁₋₆-Alkylengruppe gebundenes C₃₋₈-Cycloalkyl, Aryl oder Heteroaryl bedeutet; und
wobei
R^{16a} für H, C₁₋₆-Alkyl, C₃₋₈-Cycloalkyl, Aryl oder Heteroaryl steht, oder ein über eine C₁₋₆-Alkylengruppe gebundenes C₃₋₈-Cycloalkyl, Aryl oder Heteroaryl bedeutet,
oder R^{18a} für eine Gruppe gemäß der allgemeinen Formel (II) steht, 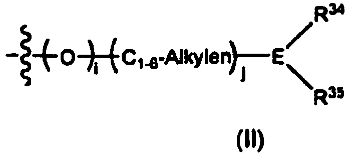 worin
i und j jeweils unabhängig voneinander für 0 oder 1 stehen;
- E: für N oder CH steht, mit der Maßgabe, dass wenn i für 1 und j für 0 steht, E für CH steht
R³⁴ und R³⁵ jeweils unabhängig voneinander H, C₁₋₆-Alkyl, C₃₋₈-Cycloalkyl, Aryl oder Heteroaryl, oder ein über eine C₁₋₃-Alkylengruppe gebundenes Aryl, Heteroaryl oder C₃₋₈-Cycloalkyl bedeuten;
oder R³⁴ und R³⁵ unter Einschluss von E ein 5- oder 6-gliedriges Aryl oder Heteroaryl bilden;
oder R³⁴ und R³⁵ unter Einschluss von E einen gesättigten Heterocyclus gemäß der allgemeinen Formel (III) bilden, 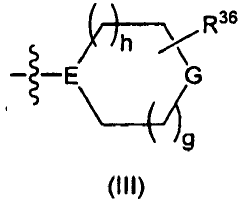 wobei
- h und g: unabhängig voneinander 0, 1 oder 2 bedeuten;
- G: für CR^{37a}R^{37b}, NR³⁸, O, S, S=O oder S(=O)₂ steht, mit der Maßgabe, dass wenn E für CH steht, G nicht für CR^{37a}R^{37b} steht;
R³⁶ für 0 bis 4 Substituenten steht, die jeweils unabhängig voneinander ausgewählt sind aus der Gruppe bestehend aus F, Cl, Br, I, OH, SH, =O, O-C₁₋₆-Alkyl, C₁₋₆-Alkyl, C₃₋₈-Cycloalkyl, Aryl, Heteroaryl und über eine C₁₋₆-Alkylengruppe gebundenem C₃₋₈-Cycloalkyl, Aryl oder Heteroaryl;
und/oder zwei benachbarte Substituenten R³⁶ zusammen ein anelliertes Aryl oder Heteroaryl darstellen;
R^{37a} und R^{37b} jeweils unabhängig voneinander H, F, Cl, Br, I, OH, SH, =O, O-C₁₋₆-Alkyl, C₁₋₆-Alkyl, C₃₋₈-Cycloalkyl, Aryl oder Heteroaryl bedeuten, oder für ein über eine C₁₋₆-Alkylengruppe gebundenes C₃₋₈-Cycloalkyl, Aryl oder Heteroaryl stehen;
R³⁸ für H, C₁₋₆-Alkyl, C₃₋₈-Cycloalkyl, Aryl oder Heteroaryl steht oder ein über eine C₁₋₃-Alkylengruppe gebundenes Aryl, Heteroaryl oder C₃₋₈-Cycloalkyl bedeutet;
R^{18b} für H, OH, C₁₋₆-Alkyl, C₃-₈-Cycloalkyl, O-C₁₋₆-Alkyl, O-(C₃₋₈-Cycloalkyl), (C₁₋₆-Alkylen)-O-C₁₋₆-Alkyl, (C₁₋₆-Alkylen)-O-(C₃₋₈-Cycloalkyl), Aryl, Heteroaryl, O-Aryl oder O-Heteroaryl steht, oder ein über eine C₁₋₆-Alkylengruppe gebundenes Aryl, O-Aryl, Heteroaryl oder O-Heteroaryl bedeutet;
oder R^{18b} für eine Gruppe gemäß der allgemeinen Formel (IV) steht, 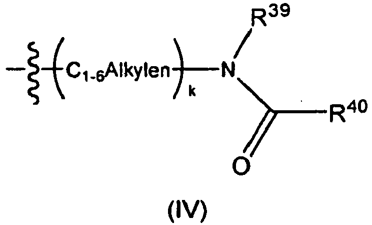 worin
- k: für 0 oder 1 steht;
R³⁹ für H, C₁₋₆-Alkyl, C₃₋₈-Cycloalkyl, Aryl oder Heteroaryl steht, oder ein über eine C₁₋₃-Alkylengruppe gebundenes C₃₋₈-Cycloalkyl, Aryl oder Heteroaryl bedeutet;
R⁴⁰ für C₁₋₆-Alkyl, C₃₋₈-Cycloalkyl, Aryl oder Heteroaryl steht, oder ein über eine C₁₋₆-Alkylengruppe gebundenes C₃₋₈-Cycloalkyl, Aryl oder Heteroaryl bedeutet;
oder
R³⁹ und R⁴⁰ gemeinsam mit der sie verbindenden N-C(=O)-Gruppe einen Ring gemäß der allgemeinen Formel (V) bilden, 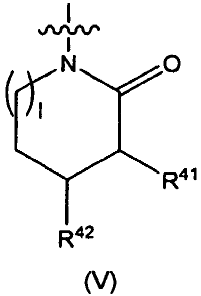 worin
I für 0, 1 oder 2 steht;
und R⁴¹ und R⁴² gemeinsam mit den sie verbindenden Kohlenstoffatomen ein anelliertes Aryl oder Heteroaryl bilden;
R¹⁹ für H; oder (P)_{z}-R²² steht,
worin
- z: für 0 oder 1 steht;
P für (C=O), S(=O)₂ oder C(=O)-N(R²⁴) steht, wobei das N-Atom in der Gruppe C(=O)-N(R²⁴) mit R²² verknüpft ist, wobei
R²⁴ für H, C₁₋₆-Alkyl, C₃₋₈-Cycloalkyl oder für ein über eine C₁₋₃-Alkylengruppe gebundenes Aryl, Heteroaryl oder C₃₋₈-Cycloalkyl steht;
R²² für C₁₋₆-Alkyl, Aryl oder Heteroaryl steht, oder ein über eine C₁₋₆-Alkylengruppe gebundenes Aryl oder Heteroaryl bedeutet; oder
R²²für eine Gruppe gemäß der allgemeinen Formel (VI) steht, worin 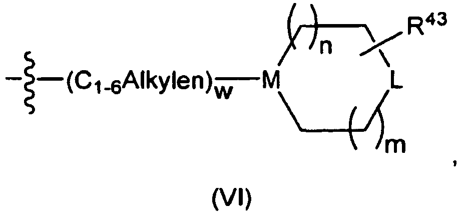
- n: für 0, 1 oder 2 steht;
- m: für 0, 1 oder 2 steht;
- w: für 0 oder 1 steht,
- M: für CH oder N steht;
mit der Maßgabe, dass wenn P für C(=O)-NR²⁴ und w für 0 steht, M für CH steht; und
mit der Maßgabe, dass wenn z und w gleichzeitig für 0 stehen, M für CH steht;
- L: für CR^{44a}R^{44b}, NR⁴⁵, O, S, S=O oder S(=O)₂ steht;
R⁴³ für 0 bis 4 Substituenten steht, die jeweils unabhängig voneinander ausgewählt sind aus der Gruppe bestehend aus F, Cl, OH, =O, C₁₋₆-Alkyl, O-C₁₋₆-Alkyl, C₃₋₈-Cycloalkyl, Aryl, Heteroaryl und über eine C₁₋₆-Alkylengruppe gebundenem C₃₋₈-Cycloalkyl, Aryl oder Heteroaryl;
und/oder zwei benachbarte der 0-4 Reste R⁴³ zusammen ein anelliertes Aryl oder Heteroaryl darstellen;
R^{44a} und R^{44b} jeweils unabhängig voneinander für H, F, Cl, Br, I, OH, C₁₋₆-Alkyl, O-C₁₋₆-Alkyl, C₃₋₈-Cycloalkyl, Aryl oder Heteroaryl stehen, oder ein über eine C₁₋₆-Alkylengruppe gebundenes C₃₋₈-Cycloalkyl, Aryl oder Heteroaryl bedeuten;
oder R^{44a} und R^{44b} gemeinsam für =O stehen können;
R⁴⁵ für H, C₁₋₆-Alkyl, C₃₋₈-Cycloalkyl, Aryl oder Heteroaryl steht, oder ein über eine C₁₋₃-Alkylengruppe gebundenes Aryl, Heteroaryl oder C₃₋₈-Cycloalkyl bedeutet;
wobei die oben genannten Reste C₁₋₆-Alkyl, C₁₋₃-Alkylen, C₁₋₆-Alkylen, C₂₋₆-Alkenylen, C₃₋₈-Cycloalkyl, Aryl und Heteroaryl jeweils unsubstituiert oder einfach oder mehrfach mit gleichen oder verschiedenen Resten substituiert sein können und die oben angegebenen Reste C₁₋₆-Alkyl, C₁₋₃-Alkylen, C₁₋₆-Alkylen und C₂₋₆-Alkenylen jeweils verzweigt oder unverzweigt sein können;
ggf. in Form eines einzelnen Enantiomers oder eines einzelnen Diastereomers, des Razemats, der Enantiomere, der Diastereomere, Mischungen von Enantiomeren und/oder Diastereomeren, sowie jeweils in Form ihrer Basen und/ oder physiologisch verträglichen Salze.

Der Ausdruck "C₁₋₆-Alkyl" umfasst im Sinne dieser Erfindung acyclische gesättigte Kohlenwasserstoffreste mit 1, 2, 3, 4, 5 oder 6 C-Atomen, die verzweigt- oder geradkettig (unverzweigt) sowie unsubstituiert oder ein- oder mehrfach, beispielsweise 2-, 3-, 4- oder 5-fach, mit gleichen oder verschiedenen Resten substituiert sein können. Vorzugsweise können die Alkylreste ausgewählt sein aus der Gruppe bestehend aus Methyl, Ethyl, n-Propyl, iso-Propyl, n-Butyl, iso-Butyl, sec-Butyl, tert-Butyl, n-Pentyl, iso-Pentyl, neo-Pentyl und Hexyl. Besonders bevorzugte Alkylreste können ausgewählt werden aus der Gruppe bestehend aus Methyl, Ethyl, n-Propyl, iso-Propyl, n-Butyl, sec-Butyl, iso-Butyl und tert-Butyl.

Im Sinne dieser Erfindung bedeutet der Ausdruck "C₃₋₈-Cycloalkyl" cyclische gesättigte Kohlenwasserstoffe mit 3, 4, 5, 6, 7 oder 8 Kohlenstoffatomen, die unsubstituiert oder an einem oder mehreren Ringgliedern einfach oder mehrfach, beispielsweise mit 2, 3, 4 oder 5, gleichen oder verschiedenen Resten substituiert sein können. Vorzugsweise kann C₃₋₈-Cycloalkyl ausgewählt sein aus der Gruppe bestehend aus Cyclopropyl, Cyclobutyl, Cyclopentyl, Cyclohexyl, Cycloheptyl und Cyclooctyl.

Der Ausdruck "Aryl" bedeutet im Sinne dieser Erfindung aromatische Kohlenwasserstoffe, insbesondere Phenyle und Naphthyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach, beispielsweise 2-, 3-, 4- oder 5-fach, substituiert vorliegen, wobei die Aryl-Substituenten gleich oder verschieden und in jeder beliebigen und möglichen Position des Aryls sein können. Vorteilhafterweise kann Aryl ausgewählt sein aus der Gruppe bestehend aus Phenyl, 1-Naphthyl und 2-Naphthyl, welche jeweils unsubstituiert oder ein- oder mehrfach, beispielsweise mit 2, 3, 4 oder 5 Resten, substituiert sein können.

Der Ausdruck "Heteroaryl" steht im Sinne der vorliegenden Erfindung für einen 5-, 6-oder 7-gliedrigen zyklischen aromatischen Rest, der mindestens 1, ggf. auch 2, 3, 4 oder 5 Heteroatome, enthält, wobei die Heteroatome gleich oder verschieden sein können und das Heteroaryl unsubstituiert oder ein- oder mehrfach, beispielsweise 2-, 3-, 4- oder 5-fach, mit gleichen oder verschiedenen Resten substituiert sein kann. Die Substituenten können in jeder beliebigen und möglichen Position des Heteroaryls gebunden sein. Der Heterocyclus kann auch Teil eines bi- oder polyzyklischen, insbesondere eines mono-, bi- oder trizyklischen Systems sein, das dann insgesamt mehr als 7-gliedrig sein kann, vorzugsweise bis zu 14-gliedrig. Bevorzugte Heteroatome sind ausgewählt aus der Gruppe bestehend aus N, O und S. Vorzugsweise kann der Heteroarylrest ausgewählt werden aus der Gruppe bestehend aus Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Benzooxazolyl, Benzooxadiazolyl, Imidazothiazolyl, Dibenzofuranyl, Dibenzothienyl, Phtalazinyl, Pyrazolyl, Imidazolyl, Thiazolyl, Oxazolyl, Oxadiazolyl, Triazol, Tetrazol, Isoxazoyl, Pyridinyl (Pyridyl), Pyridazinyl, Pyrimidinyl, Pyrazinyl, Pyranyl, Indazolyl, Purinyl, Indolizinyl, Chinolinyl, Isochinolinyl, Chinazolinyl, Chinoxalinyl, Carbazolyl, Phenazinyl, Phenothiazinyl und Oxadiazolyl, insbesondere aus der Gruppe bestehend aus Thienyl (Thiophenyl), Pyridinyl (Pyridyl), Pyrimidinyl, Thiazolyl, Triazolyl, Imidazolyl, Oxazolyl, Chinazolinyl, Chinolinyl und Isochinolinyl, wobei die Bindung an die allgemeinen Struktur (I) über jedes beliebige und mögliche Ringglied des Heteroaryl-Restes erfolgen kann. Besonders bevorzugt kann der Heteroarylrest ausgewählt werden aus der Gruppe bestehend Chinolinyl, Isochinolinyl, Thienyl, Imidazolyl, Thiazolyl, Triazolyl und Pyridinyl.

Der Ausdruck "C₁₋₃-Alkylengruppe" bzw. "C₁₋₆-Alkylengruppe" umfasst im Sinne der vorliegenden Erfindung acyclische gesättigte Kohlenwasserstoffreste mit 1, 2 oder 3, bzw. mit 1, 2, 3, 4, 5 oder 6 C-Atomen, die verzweigt- oder geradkettig (unverzweigt) sowie unsubstituiert oder ein- oder mehrfach, beispielsweise 2-, 3-, 4- oder 5-fach, mit gleichen oder verschiedenen Resten substituiert sein können und die einen entsprechenden Rest mit der übergeordneten allgemeinen Struktur verknüpfen. Vorzugsweise können die Alkylen-Gruppen ausgewählt sein aus der Gruppe bestehend aus -CH₂-, -CH₂-CH₂-, -CH(CH₃)-, -CH₂-CH₂-CH₂-, -CH(CH₃)-CH₂-, -CH(CH₂CH₃)-, -CH₂-(CH₂)₂-CH₂-, -CH(CH₃)-CH₂-CH₂-, -CH₂-CH(CH₃)-CH₂-, -CH(CH₃)-CH(CH₃)-, -CH(CH₂CH₃)-CH₂-, -C(CH₃)₂-CH₂-, -CH(CH₂CH₂CH₃)-, -C(CH₃)(CH₂CH₃)-. -CH₂-(CH₂)₃-CH₂-, -CH(CH₃)-CH₂-CH₂-CH₂-, -CH₂-CH(CH₃)-CH₂-CH₂-, -CH(CH₃)-CH₂-CH(CH₃)-, -CH(CH₃)-CH(CH₃)-CH₂-, -C(CH₃)₂-CH₂-CH₂-, -CH₂-C(CH₃)₂-CH₂-, -CH(CH₂CH₃)-CH₂-CH₂-, -CH₂-CH(CH₂CH₃)-CH₂-, -C(CH₃)₂-CH(CH₃)-, -CH(CH₂CH₃)-CH(CH₃)-, -C(CH₃)(CH₂CH₃)-CH₂-, -CH(CH₂CH₂CH₃)-CH₂-, -C(CH₂CH₂CH₃)-CH₂-, -CH(CH₂CH₂CH₂CH₃)-, -C(CH₃)(CH₂CH₂CH₃)-, -C(CH₂CH₃)₂- und -CH₂-(CH₂)₄-CH₂-. Besonders bevorzugt können die Alkylen-Gruppen ausgewählt sein aus der Gruppe bestehend aus -CH₂-, -CH₂-CH₂- und -CH₂-CH₂-CH₂-.

Der Ausdruck "C₂₋₆-Alkenylengruppe" umfasst im Sinne der vorliegenden Erfindung acyclische, einfach oder mehrfach, beispielsweise 2-, 3- oder 4-fach, ungesättigte Kohlenwasserstoffreste mit 2, 3, 4, 5 oder 6 C-Atomen, die verzweigt- oder geradkettig (unverzweigt) sowie unsubstituiert oder ein- oder mehrfach, beispielsweise 2-, 3-, 4- oder 5-fach, mit gleichen oder verschiedenen Resten substituiert sein können und die einen entsprechenden Rest mit der übergeordneten allgemeinen Struktur verknüpfen. Dabei weisen die Alkenylengruppen wenigstens eine C=C-Doppelbindung auf. Vorzugsweise können die Alkenylengruppen ausgewählt sein aus der Gruppe bestehend aus -CH=CH-, -CH=CH-CH₂-, -C(CH₃)=CH₂-, -CH=CH-CH₂-CH₂-, -CH₂-CH=CH-CH₂-, -CH=CH-CH=CH-, -C(CH₃)=CH-CH₂-, -CH=C(CH₃)-CH₂-, -C(CH₃)=C(CH₃)-, -C(CH₂CH₃)=CH-, -CH=CH-CH₂-CH₂-CH₂-, -CH₂-CH=CH₂-CH₂-CH₂-, -CH=CH=CH-CH₂-CH₂- und -CH=CH₂-CH-CH=CH₂-.

Der Ausdruck "über eine C₁₋₃-Alkylengruppe, C₁₋₆-Alkylengruppe oder C₂₋₆-Alkenylengruppe gebundenes Aryl oder Heteroaryl" bedeutet im Sinne der vorliegenden Erfindung, dass die C₁₋₃-Alkylengruppen, C₁₋₆-Alkylengruppen bzw. C₂₋₆-Alkenylengruppen sowie Aryl bzw. Heteroaryl die oben definierten Bedeutungen haben und das Aryl bzw. Heteroaryl über eine C₁₋₃-Alkylengruppe, C₁₋₆-Alkylengruppe bzw. C₂₋₆-Alkenylengruppe an die übergeordnete allgemeine Struktur gebunden ist. Beispielhaft seien genannt Benzyl, Phenethyl und Phenylpropyl.

Der Ausdruck "über eine C₁₋₃-Alkylengruppe bzw. C₁₋₆-Alkylengruppe gebundenes C₃₋₆-Cycloalkyl bzw. C₃₋₈-Cycloalkyl " bedeutet im Sinne der vorliegenden Erfindung, dass die C₁₋₃-Alkylengruppe bzw. C₁₋₆-Alkylengruppe, C₃₋₆-Cycloalkyl und C₃₋₈-Cycloalkyl die oben definierten Bedeutungen haben und C₃-₆-Cycloalkyl bzw. C₃₋₈-Cycloalkyl über eine C₁₋₃-Alkylengruppe bzw. C₁₋₆-Alkylengruppe an die übergeordnete allgemeine Struktur gebunden ist.

Im Zusammenhang mit "Alkyl", "Alkylen" und "Cycloalkyl" versteht man unter dem Begriff "substituiert" im Sinne dieser Erfindung die Substitution eines Wasserstoffrestes durch F, Cl, Br, I, CF₃, OCF₃, CN, NH₂, NH-C₁₋₆-Alkyl, NH-C₁₋₆-Alkylen-OH, C₁₋₆-Alkyl, N(C₁₋₆-Alkyl)₂, N(C₁₋₆-Alkylen-OH)₂, NO₂, SH, S-C₁₋₆-Alkyl, S-Benzyl, O-C₁₋₆-Alkyl, OH, O-C₁₋₆-Alkylen-OH, =O, O-Benzyl, C(=O)C₁₋₆-Alkyl, CO₂H, CO₂-C₁₋₆-Alkyl, Phenyl, Phenoxy, Benzyl, Naphthyl, Furyl, Thienyl und Pyridinyl, wobei unter mehrfach substituierten Resten solche Reste zu verstehen sind, die entweder an verschiedenen oder an gleichen Atomen mehrfach, beispielsweise zweifach oder dreifach, substituiert sind, beispielsweise dreifach am gleichen C-Atom wie im Falle von CF₃ oder CH₂CF₃ oder an verschiedenen Stellen wie im Falle von CH(Cl)-CH=CH-CHCl₂. Die Mehrfachsubstitution kann mit gleichen oder verschiedenen Substituenten erfolgen, wie beispielsweise im Falle von CH(OH)-CH=CH-CHCl₂. Insbesondere soll hier darunter die Substitution eines oder mehrerer Wasserstoffreste durch F, Cl, CF₃, NH₂, OH, Phenyl, O-CF₃ oder O-C₁₋₆-Alkyl, insbesondere Methoxy verstanden werden.

In Bezug auf "Aryl" und "Heteroaryl" versteht man im Sinne dieser Erfindung unter "substituiert" die ein- oder mehrfache, beispielsweise 2-, 3-, 4- oder 5-fache, Substitution eines oder mehrerer Wasserstoffatome des entsprechenden Ringsystems durch F, Cl, Br, I, CN, NH₂, NH-C₁₋₆-Alkyl, NH-C₁₋₆-Alkylen-OH, N(C₁₋₆-Alkyl)₂, N(C₁₋₆-Alkylen-OH)₂, NH-Aryl¹, N(Aryl¹)₂, N(C₁₋₆-Alkyl)Aryl¹, Pyrrolinyl, Piperazinyl, Morpholinyl, (C₁₋₃-Alkylen)-Azetidinyl, (C₁₋₃-Alkylen)-Pyrrolinyl oder (C₁₋₃-Alkylen)-Piperidinyl, NO₂, SH, S-C₁₋₆-Alkyl, OH, O-C₁₋₆-Alkyl, O-C₁₋₆-Alkyl-OH, C(=O)C₁₋₆-Alkyl, NHSO₂C₁₋₆-Alkyl, NHCOC₁₋₆-Alkyl, CO₂H, CH₂SO₂-Phenyl, CO₂-C₁₋₆-Alkyl, OCF₃, CF₃, -O-CH₂-O-, -O-CH₂-CH₂-O-, -O-C(CH₃)₂-CH₂-, unsubstituiertes C₁₋₆-Alkyl, Pyrrolidinyl, Imdazolyl, Piperidinyl, Benzyloxy, Phenoxy, Phenyl, Naphthyl, Pyridinyl, -C₁₋₃-Alkylen-Aryl¹, Benzyl, Thienyl, Furyl, wobei Aryl¹ für Phenyl, Furyl, Thienyl oder Pyridinyl steht, an einem oder verschiedenen Atomen, wobei die vorstehend genannten Substituenten - sofern nicht anders angegeben - ggf. ihrerseits mit den genannten Substituenten substituiert sein können. Die Mehrfachsubstitution von Aryl und Heteroaryl kann mit gleichen oder verschiedenen Substituenten erfolgen. Bevorzugte Substituenten für Aryl und Heteroaryl können ausgewählt werden aus der Gruppe bestehend aus -O-C₁₋₃-Alkyl, unsubstituiertem C₁₋₆-Alkyl, F, Cl, Br, I, CN, CF₃, OCF₃, OH, SH, -CH₂-Azetidinyl, Phenyl, Naphthyl, Furyl, Thienyl und Pyridinyl, insbesondere aus der Gruppe bestehend aus F, Cl, Br, CN, CF₃, CH₃; OCH₃ und OCF₃.

In den chemischen Strukturformeln, die zur Beschreibung der erfindungsgemäßen Verbindungen hier verwendet werden, wird zur Beschreibung eines oder mehrerer Substitutionsmuster auch das Symbol " 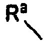 " verwendet, wobei diese Gruppe im Gegensatz zur Darstellung einer Bindung an ein bestimmtes Atom nicht an ein bestimmtes Atom innerhalb der chemischen Strukturformel gebunden ist (R^{a} steht hier beispielhaft für einen Substituenten R mit einer durch die Variable "a" dargestellten Nummerierung). Vielmehr kann der Substituent an jedes mögliche Ringatom gebunden sein.

Dies sei beispielhaft anhand der Gruppe " 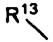 " aus der oben gezeigten allgemeinen Formel (I) erläutert: Die Definition für R¹³ besagt, dass R¹³ für 0 bis 4 Substituenten stehen kann. R¹³ kann also abwesend sein oder es können 1, 2, 3 oder 4 der C-gebundenen Wasserstoffatome innerhalb der durch die allgemeine Formel (I) dargestellten Teilstruktur durch einen in der Definition für den Rest R¹³ vorgesehenen Substituenten ersetzt sein, wobei die jeweiligen Substituenten unabhängig voneinander ausgewählt sein können, also auch unterschiedliche Bedeutungen haben können, und C-gebundene Wasserstoffatome an einem oder mehreren C-Atomen ersetzen können. Wie beispielsweise in der Definition von R¹³ angegeben, können auch jeweils zwei der Substituenten R¹³ zusammen ein anelliertes Aryl oder Heteroaryl (auch ankondensiertes Aryl oder Heteroaryl oder anellierte/ankondensierte Aryl- oder Heteroarylgruppe genannt) darstellen.

Im Rahmen der vorliegenden Erfindung bezeichnet das in Formeln verwendete Symbol 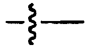 eine Verknüpfung eines entsprechenden Restes an die jeweilige übergeordnete allgemeine Struktur.

Der Fachmann versteht ferner, dass gleiche Reste, die zur Definition unterschiedlicher Substituenten verwendet werden, jeweils unabhänging voneinander sind.

Der Fachmann versteht ferner, dass der rechte Cyclus der nachstehenden Teilstruktur 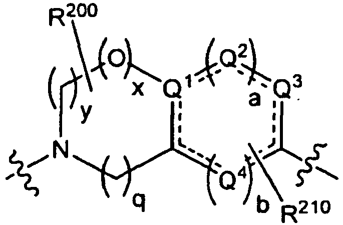 aromatisch ist, was durch die gestrichelte Linie dargestellt ist. Hierbei kann Q² sofern es mehrfach vorhanden ist, d.h. sofern a für 2 steht, bei jedem Auftreten jeweils unabhängig aus der Gruppe bestehend aus CH, N O und S ausgewählt sein.

Unter dem Begriff "physiologisch verträgliches Salz" versteht man im Sinne dieser Erfindung vorzugsweise Salze der erfindungsgemäßen Verbindungen mit anorganischen oder organischen Säuren, die physiologisch - insbesondere bei Anwendung am Menschen und/ oder Säugetier - verträglich sind. Beispiele für geeignete Säuren sind Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Maleinsäure, Milchsäure, Zitronensäure, Glutaminsäure, 1,1-Dioxo-1,2-dihydro1λ⁶-benzo[*d*]isothiazol-3-on (Saccharinsäure), Monomethylsebacinsäure, 5-Oxo-prolin, Hexan-1-sulfonsäure, Nicotinsäure, 2-, 3-oder 4-Aminobenzoesäure, 2,4,6-Trimethyl-benzoesäure, α-Liponsäure, Acetylglycin, Hippursäure, Phosphorsäure und/oder Asparaginsäure. Besonders bevorzugt sind die Salze der Salzsäure (Hydrochloride) sowie der Zitronensäure (Citrate).

R¹ steht in den erfindungsgemäßen Verbindungen vorzugsweise für Phenyl, Naphthyl, Chromanyl, Indolyl, Benzofuranyl, Benzothiophenyl (Benzothienyl), 5,6-Dihydro-4H-cyclopenta[b]thiophenyl, Benzooxazolyl, Benzooxadiazolyl, Pyrrolyl, Furanyl, Thienyl, Pyridinyl, Pyridazinyl, Pyrimidinyl, Pyrazinyl, Imidazothiazolyl, Carbazolyl, Dibenzofuranyl, Dibenzothiophenyl (Dibenzothienyl) oder für ein über eine C₁₋₃-Alkylengruppe oder eine C₂₋₃-Alkenylengruppe gebundenes Phenyl oder Naphthyl, besonders bevorzugt für Phenyl, Naphthyl, Chromanyl, Benzothiophenyl (Benzothienyl), 5,6-Dihydro-4H-cyclopenta[b]thiophenyl, Benzooxadiazolyl, Thienyl, Pyridinyl, Imidazothiazolyl, Dibenzofuranyl oder für ein über eine C₁₋₃-Alkylengruppe oder eine C₂₋₃-Alkenylengruppe gebundenes Phenyl, ganz besonders bevorzugt für Phenyl, Naphthyl, Chromanyl, Benzothiophenyl (Benzothienyl), 5,6-Dihydro-4H-cyclopenta[b]thiophenyl, Thienyl oder für ein über eine C_{1 oder 2}-Alkylengruppe oder -CH=CH-Gruppe gebundenes Phenyl, wobei die vorstehend genannten Aryl oder Heteroaryl-Reste jeweils unsubstituiert oder einfach oder mehrfach, gleich oder verschieden, substituiert sind, wobei die Substituenten unabängig voneinander insbesondere ausgewählt sind aus der Gruppe bestehend aus -O-C₁₋₃-Alkyl, C₁₋₆-Alkyl, F, Cl, Br, I, CF₃, OCF₃, OH, SH, Phenyl, Phenoxy, Naphthyl, Furyl, Thienyl und Pyridinyl und wobei die vorstehend genannten Alkylen- und Alkenylengruppen jeweils unsubstituiert oder einfach oder mehrfach, gleich oder verschieden, substituiert sind, wobei die Substituenten unabängig voneinander insbesondere ausgewählt sind aus der Gruppe bestehend aus -O-C₁₋₃-Alkyl, F, Cl, Br, I, CF₃, OCF₃, OH, SH, Phenyl, Phenoxy, Naphthyl, Furyl, Thienyl und Pyridinyl.

Der Rest R¹ kann insbesondere für Phenyl oder Naphthyl stehen, wobei das Phenyl oder Naphthyl unsubstituiert oder einfach oder mehrfach, beispielsweise 2-, 3-, 4-oder 5-fach, mit gleichen oder verschiedenen Resten ausgewählt aus Methyl, Methoxy, CF₃, OCF₃, F, und Cl substituiert ist.

In ebenfalls bevorzugten Ausführungsformen der erfindungsgemäßen Verbindungen ist der Rest R¹ ausgewählt aus der Gruppe bestehend aus 4-Methoxy-2,3,6-trimethylphenyl, 4-Methoxy-2,6-dimethylphenyl, 2,4,6-Trimethylphenyl, 2,6-Dichlor-4-(trifluormethyl)phenyl, 2-Chlor-6-methylphenyl, 2,4,6-Trichlorphenyl, 2,4,5-Trichlorphenyl, 4-Fluor-2,6-dimethylphenyl, 2,6-Dichlor-4-methoxyphenyl, 2,6-Dichlorphenyl, 2,6-Dichlor-3-methylphenyl, 6-Methoxy-2-naphthyl, 2-Methyl-1-naphthyl, 2-Chlor-1-naphthyl, 2-Fluor-1-naphthyl, 2-Chlor-4-(trifluormethoxy)phenyl, 4-Chlor-2,5-dimethylphenyl, 2-Chlorphenyl, 3-Chlorphenyl, 4-Chlorphenyl, 2,3-Dichlorphenyl, 3,4-Dichlorphenyl, 2,4-Dichlorphenyl, 2-(Trifluormethyl)phenyl, 3-(Trifluormethyl)phenyl, 4-(Trifluormethyl)phenyl, 4-Methyl-1-naphthyl, 5-Chlor-1-naphthyl, 4-Chlor-1-naphthyl, 4-Fluor-1-naphthyl, 4-Methoxy-1-naphthyl, 1-Naphthyl, 2-Naphthyl, Benzothiophenyl, 2,2-Diphenylethanyl und 2,2-Dimethylchroman-6-yl.

Insbesondere kann der Rest R¹ für 4-Methoxy-2,6-dimethylphenyl stehen.

In weiterhin bevorzugten Ausführungsformen der erfindungsgemäßen Verbindungen steht B für S(=O)₂, so dass die allgemeine Formel (I) die folgende Form (I') annimmt:

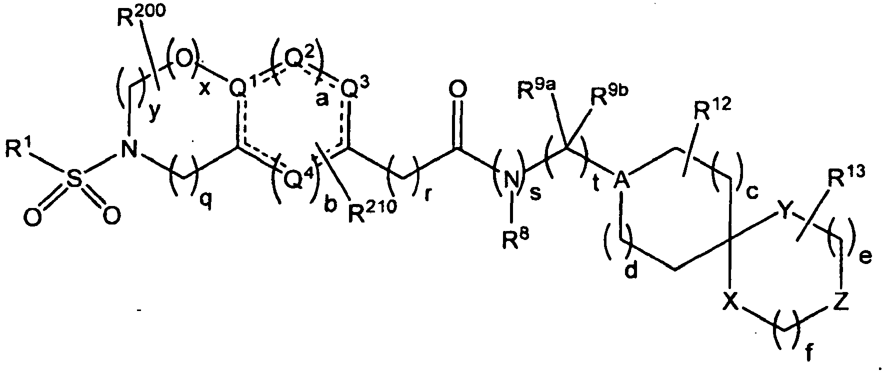

In ebenfalls bevorzugten Ausführungsformen der erfindungsgemäßen Verbindungen steht R⁴ für H oder C₁₋₄-Alkyl.

In weiterhin bevorzugten Ausführungsformen der erfindungsgemäßen Verbindungen können die Substituenten R²⁰⁰ und/oder R²¹⁰ abwesend sein.

In ebenfalls bevorzugten Ausführungsformen der erfindungsgemäßen Verbindungen gemäß der allgemeinen Formel (I) steht die nachfolgend dargestellte Teilstruktur (Ac) 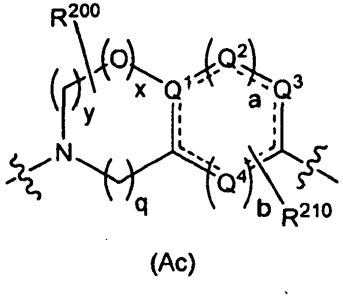 für eine Teilstruktur, die ausgewählt ist aus der Gruppe bestehend aus

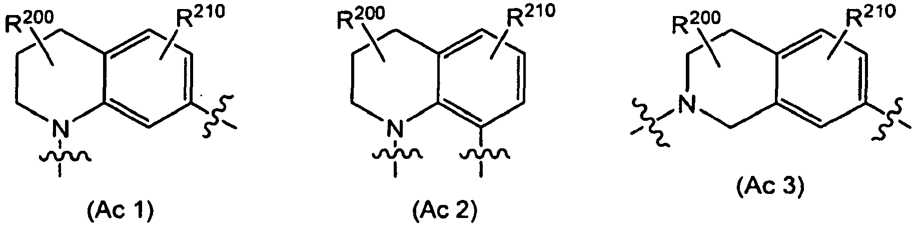

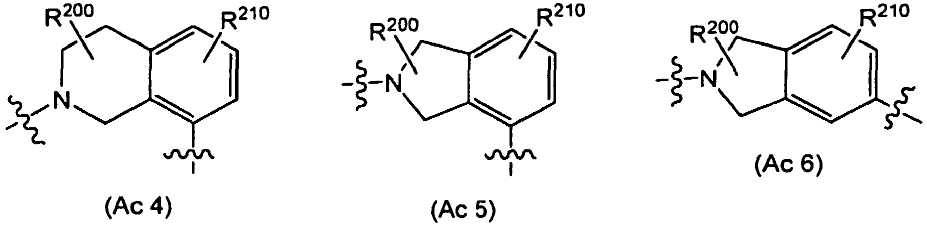

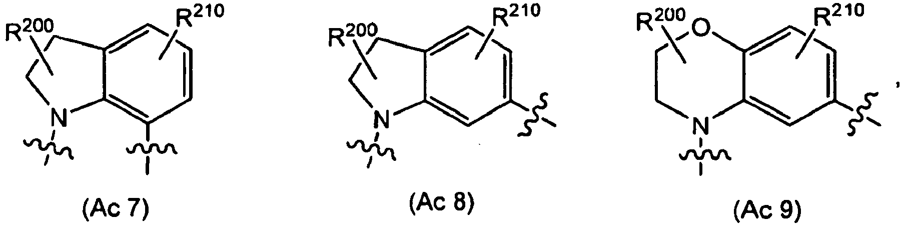

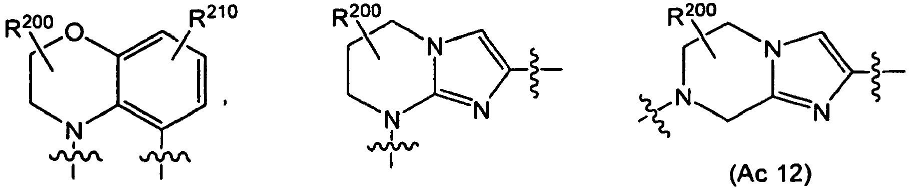

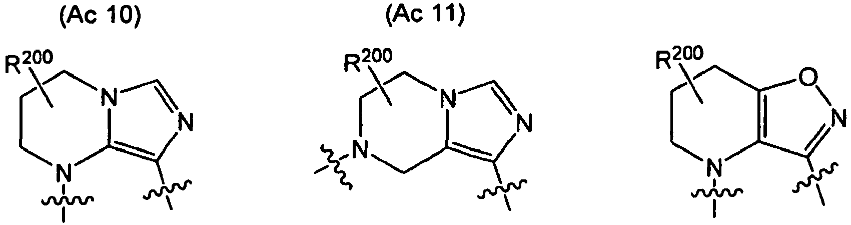

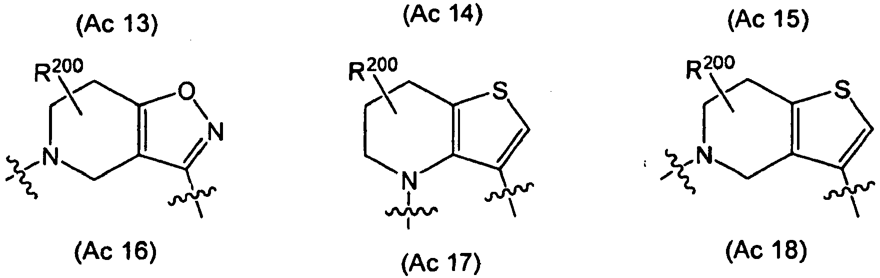

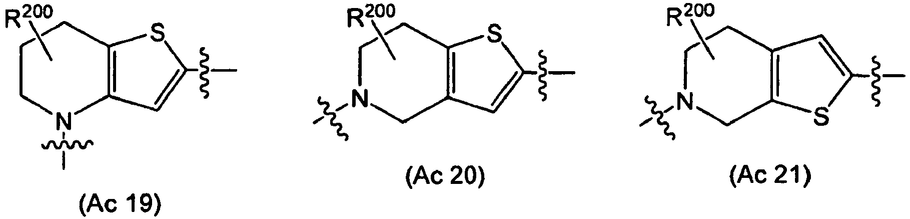

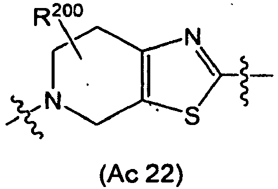

Insbesondere kann die Teilstruktur Ac ausgewählt sein aus der Gruppe bestehend aus den Teilstrukturen Ac 1, Ac 3, Ac 6, Ac 8, Ac 9, Ac 16 und Ac 20.

In ebenfalls bevorzugten Ausführungsformen der erfindungsgemäßen Verbindungen steht r für 0 oder 1, insbesondere jeweils im Zusammenhang mit den vorstehend angegebenen Teilstrukturen Ac1-Ac22.

Ebenfalls bevorzugte Ausführungsformen der erfindungsgemäßen Verbindungen sind solche, in denen R⁸ für H; C₁₋₆-Alkyl, insbesondere Methyl, Ethyl, n-Propyl, iso-Propyl, n-Butyl, iso-Butyl, sec-Butyl oder tert-Butyl; Cyclopropyl, Cyclobutyl, Cyclopentyl, Cyclohexyl, -CH₂CF₃, Phenyl, Benzyl, Phenylethyl, Phenylpropyl, oder ein über eine C₁₋₃-Alkylengruppe gebundenes Cyclopropyl, Cyclobutyl, Cyclopentyl, Cyclohexyl, jeweils unsubstituiert oder einfach oder mehrfach mit gleichen oder verschiedenen Substituenten substituiert, steht.

Weiterhin bevorzugte Ausführungsformen der erfindungsgemäßen Verbindungen sind solche, in denen R^{9a} und R^{9b} jeweils unabhängig voneinander für H; F; Methyl; Ethyl, iso-Propyl, CF₃, Methoxy; Cyclopropyl; Phenyl; Benzyl, Phenylethyl, C₁₋₃-Alkylen-Cyclopropyl, C₁₋₃-Alkylen-Cyclobutyl, C₁₋₃-Alkylen-Cyclopentyl, C₁₋₃-Alkylen-Cyclohexyl, C₁₋₃-Alkylen-CF₃, jeweils unsubstituiert unsubsitituiert oder einfach oder mehrfach mit gleichen oder verschiedenen Substituenten substituiert, stehen, vorzugsweise R^{9a} und R^{9b} beide gleichzeitig für H stehen.

In weiteren bevorzugten Ausführungsformen der erfindungsgemäßen Verbindungen steht A für N steht.

In ebenfalls bevorzugten Ausführungsformen der erfindungsgemäßen Verbindungen stehen s und t jeweils für 0 und A steht für N.

Ferner sind bevorzugte Ausführungsformen der erfindungsgemäßen Verbindungen solche, in denen
- X: für CR^{14a}R^{14b}, NR¹⁵ oder O steht;
- Y: für CR^{16a}R^{16b} steht;

R^{14a}, R^{14b}, R^{16a} und R^{16b} jeweils unabhängig voneinander H, F, Cl, OH, C₁₋₆-Alkyl, O-C₁₋₆-Alkyl, C₃₋₈-Cycloalkyl, Aryl oder Heteroaryl bedeuten, oder für ein über eine C₁₋₆-Alkylengruppe gebundenes C₃₋₈-Cycloalkyl, Aryl oder Heteroaryl stehen,
und/oder jeweils R^{14a} und R^{14b} gemeinsam für =O und/oder jeweils R^{16a} und R^{16b} gemeinsam für =O stehen können;
R¹⁵ für H, C₁₋₆-Alkyl, C₃₋₈-Cycloalkyl, Aryl oder Heteroaryl stehen, oder ein über eine C₁₋₆-Alkylengruppe gebundenes C₃₋₈Cycloalkyl, Aryl oder Heteroaryl bedeuten;
z für CR^{18a}R^{18b} oder NR¹⁹ steht; oder
Z in dem Fall dass X für O und f für 0 steht, -(N=(CR¹²⁶))- bedeutet, wobei das N-Atom einfach an das O-Atom gebunden ist, und
R¹²⁶ für H, C₁₋₆-Alkyl, C₃₋₈-Cycloalkyl, Aryl oder Heteroaryl steht oder ein über eine C₁₋₆Alkylengruppe gebundenes C₃₋₈-Cycloalkyl, Aryl oder Heteroaryl bedeutet;
R^{18a} für H, C₁₋₆-Alkyl, C₃₋₈-Cycloalkyl, -NH(C₁₋₆-Alkyl), -N(C₁₋₆-Alkyl)₂, Phenyl, Pyridyl, Imidazolyl, Triazolyl, Pyrimidyl, Thiazolyl oder Thienyl, jeweils unsubstituiert oder einfach oder mehrfach substituiert; oder für über eine -(O)₀₋₁-C₁₋₆-Alkylengruppe gebundenes Phenyl, Pyridyl, Imidazolyl, Triazolyl, Pyrimidyl, Thiazolyl oder Thienyl, jeweils unsubstituiert oder einfach oder mehrfach substituiert, steht; oder
R^{18a} für den Rest gemäß der allgemeinen Formel (VII) 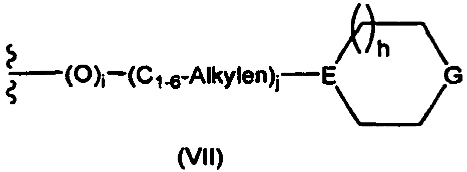 steht, worin
- i: für 0 oder 1 steht;
- j: für 0 oder 1 steht;
- h: für 0 oder 1 steht;
- E: für N oder CH steht; mit der Maßgabe, dass wenn i für 1 und j für 0 steht, E für CH steht;
- G: für CR^{37a}R^{37b} oder NR³⁸ steht; wobei
R^{37a} und R^{37b} unabhängig voneinander für H; F oder C₁₋₆-Alkyl, jeweils unsubstituiert oder einfach oder mehrfach substituiert mit gleichen oder verschiedenen Substituenten, stehen;
R³⁸ für H; C₁₋₆-Alkyl, C₃₋₆-Alkyl oder Pyridyl steht;
R^{18b} für H, OH, C₁₋₆-Alkyl, Phenyl, Pyridyl, Imidazolyl, Triazolyl, Pyrimidyl, Thiazolyl oder Thienyl, jeweils unsubstituiert oder einfach oder mehrfach substituiert mit gleichen oder verschiedenen Substituenten, über eine C₁₋₆-Alkylengruppe gebundenes Phenyl, Pyridyl, Imidazolyl, Triazolyl, Pyrimidyl, Thiazolyl oder Thienyl; jeweils unsubstituiert oder einfach oder mehrfach substituiert mit gleichen oder verschiedenen Substituenten; O-Phenyl oder O-Pyridyl, jeweils unsubstituiert oder einfach oder mehrfach substituiert mit gleichen oder verschiedenen Substituenten; über C₁₋₆Alkylen-NH(C=O) verbrücktes Phenyl, Pyridyl oder Thienyl, jeweils unsubstituiert oder einfach oder mehrfach substituiert mit gleichen oder verschiedenen Substituenten, steht
R¹⁹ für H, C₁₋₆-Alkyl, C₃₋₈-Cycloalkyl, -(C=O)-C₁₋₆-Alkyl, Phenyl, Pyridyl, Thienyl, Thiazolyl, Triazolyl, Pyrimidinyl oder Imidazolyl; jeweils unsubstituiert oder einfach oder mehrfach substituiert mit gleichen oder verschiedenen Substituenten; oder für über eine C₁₋₆-Alkylengruppe gebundenes Phenyl, Pyridyl, Thienyl, Thiazolyl, Pyrimidinyl, Triazolyl oder Imidazolyl; jeweils unsubstituiert oder einfach oder mehrfach substituiert mit gleichen oder verschiedenen Substituenten, steht;
oder für den Rest gemäß der allgemeinen Formel (VIII) 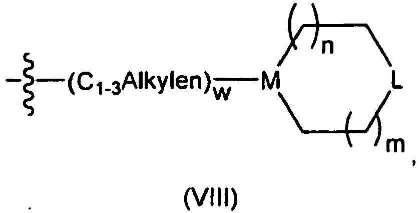 steht, worin
- w: für 0 oder 1 steht;
- n: für 0 oder 1 steht;
- m: für 0 oder 1 steht;
- M: für CH oder N steht, mit der Maßgabe, dass wenn w für 0 steht, M für CH steht;
- L: für CR^{44a}R^{44b} oder NR⁴⁵ steht;
wobei R^{44a} und R^{44b} unabhängig voneinander für H; F oder C₁₋₆-Alkyl, jeweils unsubstituiert oder einfach oder mehrfach substituiert mit gleichen oder verschiedenen Substituenten, stehen; und
R⁴⁵ für H, C₁₋₆-Alkyl, C₃₋₆-Alkyl oder Pyridyl steht.

Weitere bevorzugte Ausführungsformen der erfindungsgemäßen Verbindungen sind solche, in denen s und t jeweils für 0 stehen und die folgende Teilstruktur (SP) 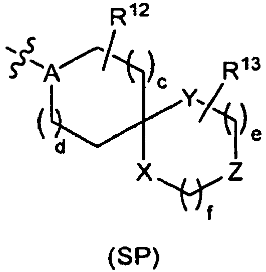 ausgewählt ist aus der Gruppe bestehend aus 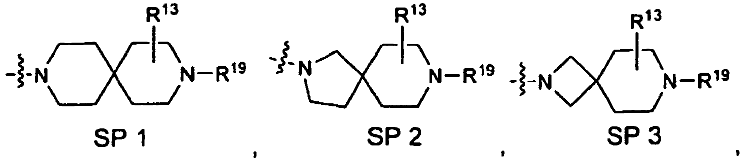 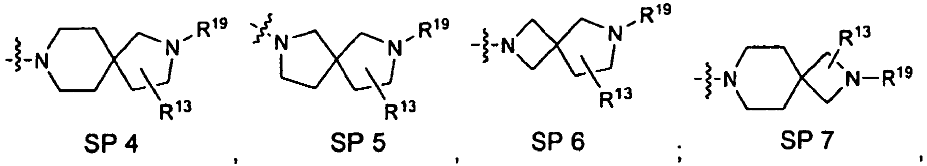 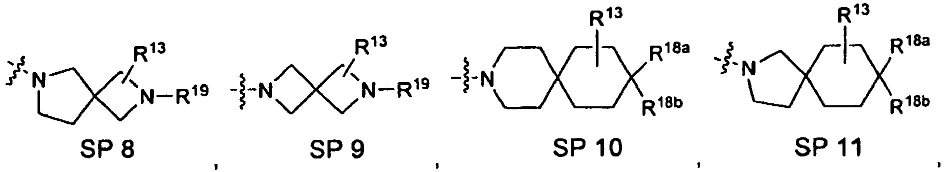 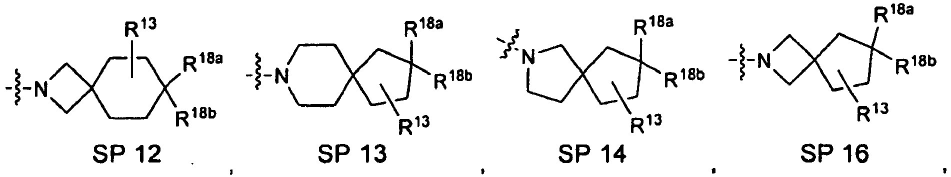 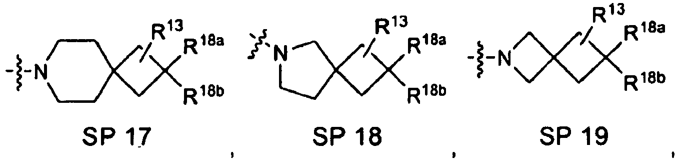 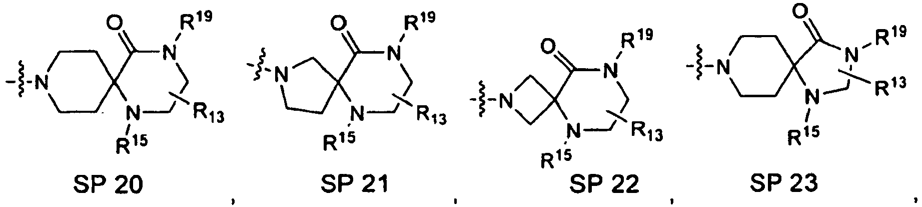 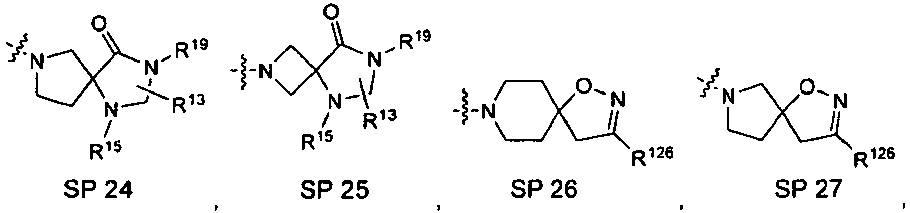 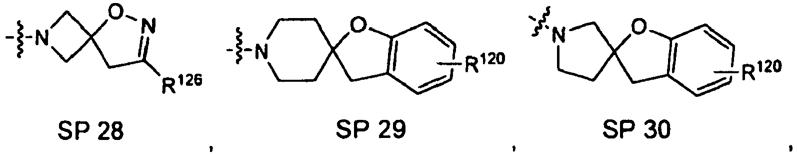 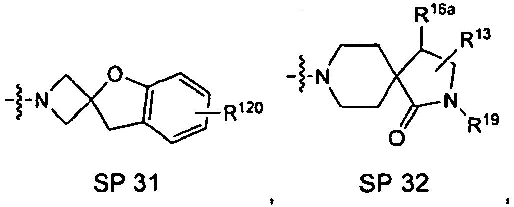 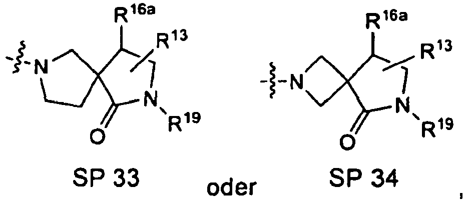 wobei
R¹³ für 1-2 Substituenten steht, die ausgewählt sind aus der Gruppe bestehend aus H und Phenyl, unsubstituiert oder einfach oder mehrfach substituiert mit gleichen oder verschiedenen Substituenten, steht; und/oder zwei der Substituenten R¹³ zusammen =O bilden und/oder zwei benachbarte Substituenten R¹³ zusammen ein anelliertes Aryl oder Heteroaryl, insbesondere eine Benzogruppe, jeweils unsubstituiert oder einfach oder mehrfach substituiert mit gleichen oder verschiedenen Substituenten, bilden,
R¹⁵ für H; C₁₋₆-Alkyl; C₃₋₈-Cycloalkyl, Phenyl, Pyridyl, jeweils unsubstituiert oder einfach oder mehrfach substituiert mit gleichen oder verschiedenen Substituenten; oder für über eine C₁₋₆-Alkylengruppe gebundenes Phenyl oder Pyridyl, jeweils unsubstituiert oder einfach oder mehrfach substituiert mit gleichen oder verschiedenen Substituenten, steht;
R^{16a} für H, C₁₋₆-Alkyl, Phenyl oder Pyridyl, jeweils unsubstituiert oder einfach oder mehrfach substituiert mit gleichen oder verschiedenen Substituenten, steht;
R^{18a} für H; C₁₋₆-Alkyl; C₃₋₈-Cycloalkyl, N(C₁₋₆-Alkyl)₂; NH(C₁₋₆-Alkyl), Azetidinyl; Pyrrolidinyl, Piperidinyl, 4-(C₁₋₆-Alkyl)-Piperazinyl; Phenyl oder Pyridyl, jeweils unsubstituiert oder einfach oder mehrfach substituiert mit gleichen oder verschiedenen Substituenten; oder für über eine -(O)_{0/1}-C₁₋₆-Alkylengruppe gebundenes N(C₁₋₆-Alkyl)₂; NH(C₁₋₆-Alkyl), Azetidinyl; Pyrrolidinyl, Piperidinyl, 4-(C₁₋₆-Alkyl)-Piperazinyl; Phenyl, Imidazolyl, Triazolyl, oder Pyridyl, jeweils unsubstituiert oder einfach oder mehrfach substituiert mit gleichen oder verschiedenen Substituenten, steht;
R^{18b} für H; OH; C₁₋₆-Alkyl; Phenyl oder Pyridyl, jeweils unsubstituiert oder einfach oder mehrfach substituiert mit gleichen oder verschiedenen Substituenten; für O-Phenyl oder O-Pyridyl, jeweils unsubstituiert oder einfach oder mehrfach substituiert mit gleichen oder verschiedenen Substituenten oder für über eine C₁₋₆Alkylengruppe gebundenes Phenyl oder Pyridyl, jeweils unsubstituiert oder einfach oder mehrfach substituiert mit gleichen oder verschiedenen Substituenten; steht;
R¹⁹ für H; C₁₋₆-Alkyl; C₃₋₈-Cycloalkyl, Phenyl, Pyridyl, Thienly, Imidazolyl, Thiazolyl, oder Triazolyl, jeweils unsubstituiert oder einfach oder mehrfach substituiert mit gleichen oder verschiedenen Substituenten; oder für über eine C₁₋₆-Alkylengruppe oder (C=O)-Gruppe gebundenes Phenyl oder Pyridyl, jeweils unsubstituiert oder einfach oder mehrfach substituiert mit gleichen oder verschiedenen Substituenten, steht;
R¹²⁰ für H; F; Cl; OH; OCH₃, O-CF₃, C₁₋₆-Alkyl; CF₃ oder Phenyl, unsubstituiert oder einfach oder mehrfach substituiert, steht;
R¹²⁶ für H; C₁₋₆-Alkyl; C₃₋₆-Cycloalkyl; Phenyl oder Pyridyl; oder für über eine C₁₋₃-Alkylengruppe gebundenes C₃₋₆-Cycloalkyl, Phenyl oder Pyridyl, jeweils unsubstituiert oder einfach oder mehrfach substituiert mit gleichen oder verschiedenen Substituenten, steht.

Die oben gezeigten Teilstrukturen SP1, SP2, SP4, SP5, SP 10, SP23, SP 26 und SP32 können bevorzugt sein.

Weitere bevorzugte Ausführungsformen der erfindungsgemäßen Verbindungen sind solche, in denen s und t jeweils für 0 stehen und die folgende Teilstruktur (SP) 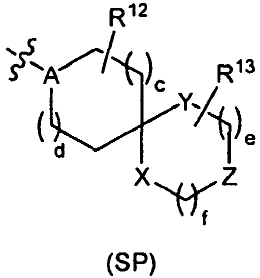 ausgewählt ist aus der Gruppe bestehend aus 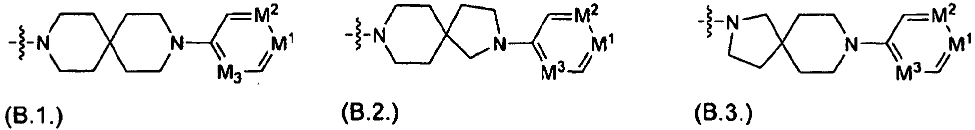 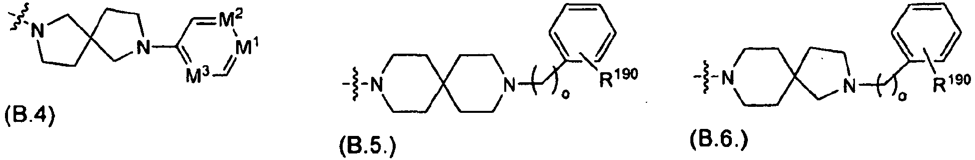 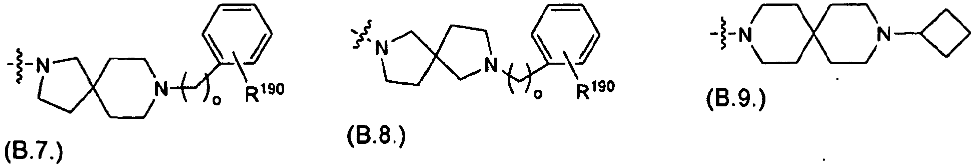 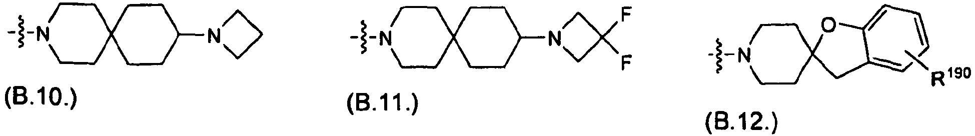 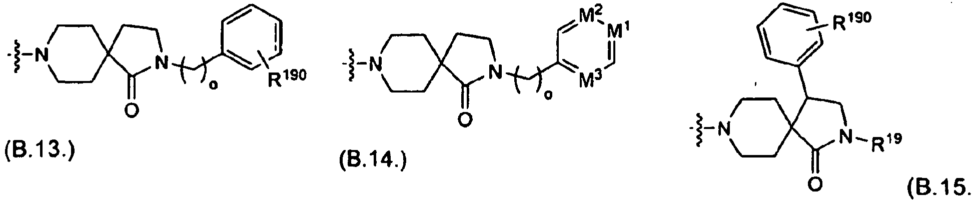 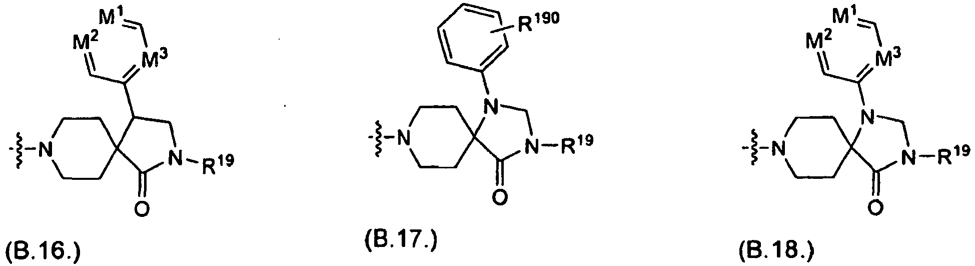 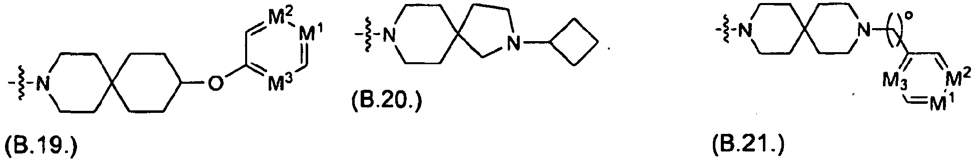 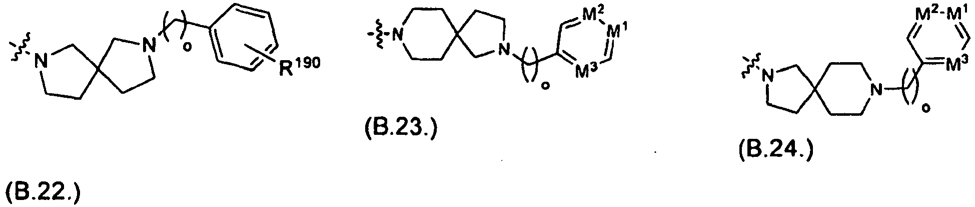 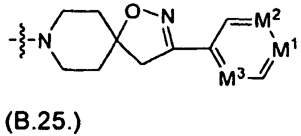 o = 0, 1, 2 oder 3 ist;
M¹, M² und M³ unabhängig voneinander jeweils für N oder CH stehen können, wobei eine Variabel aus M¹, M² und M³ für N und die anderen beiden für CH stehen;
R¹⁹ ausgewählt ist aus der Gruppe bestehend aus H; C₁₋₆Alkyl, insbesondere Methyl, Ethyl, n-Propyl, iso-Propyl, n-Butyl, sec-Butyl, iso-Butyl und tert-Butyl; C₃₋₆Cycloalkyl, jeweils unsubstituiert oder einfach oder mehrfach substituiert mit gleichen oder verschiedenen Substituenten; und
R¹⁹⁰ 0-4 Substituenten darstellt, die unabhängig voneinander ausgesucht sind aus F, Cl, O-CF₃, CF₃ oder CN.
Die oben gezeigten Teilstrukturen B.1., B.2., B.3., B.13., B.15., B.17., B.19., B.20., B.21., B.22. und B.25. können bevorzugt sein. In den Teilstrukturen B.1., B.2., B.3., B.19., B.21., B.25. können bevorzugt M¹ für N und jeweils M² und M³ für CH stehen. In der Teilstruktur B.13. können bevorzugt o für 1 stehen und R¹⁹⁰ abwesend sein oder für F in 4-Position stehen. In der Teilstruktur B.15. können bevorzugt R¹⁹ für Methyl stehen und R¹⁹⁰ abwesend sein. In der Teilstruktur B.17. können bevorzugt R¹⁹ für H stehen und R¹⁹⁰ abwesend sein. In der Teilstruktur B.22. können bevorzugt o für 1 stehen und R¹⁹⁰ abwesend sein.

Weitere Ausführungsformen der erfindungsgemäßen Verbindungen sind solche, die durch die im Folgenden gezeigten allgemeinen Formeln C1-C14 dargestellt werden: 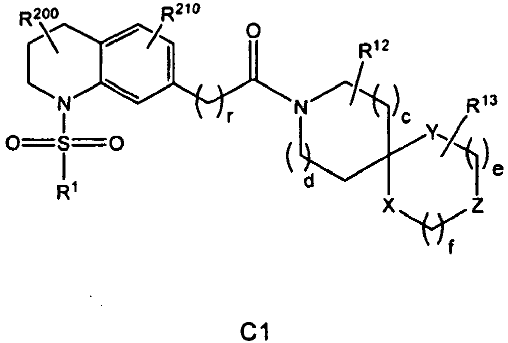 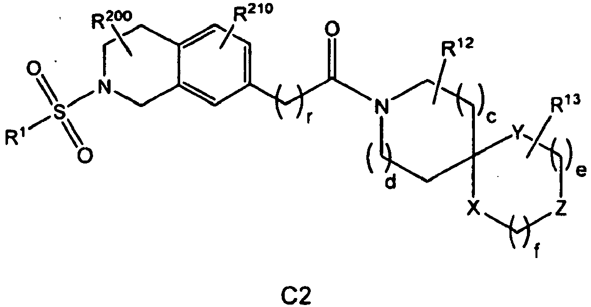 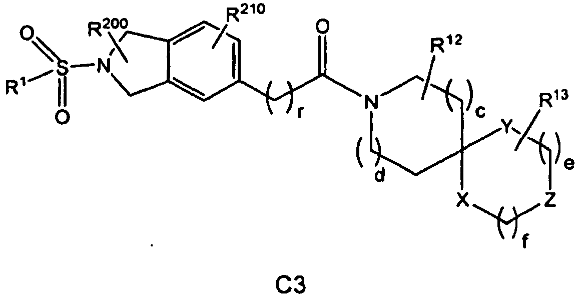 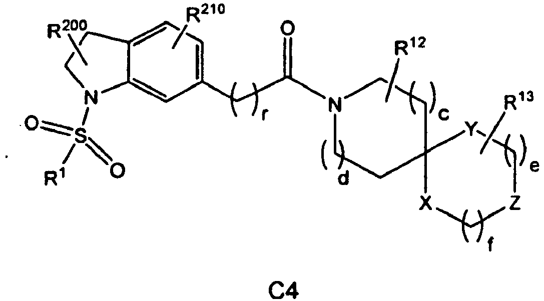 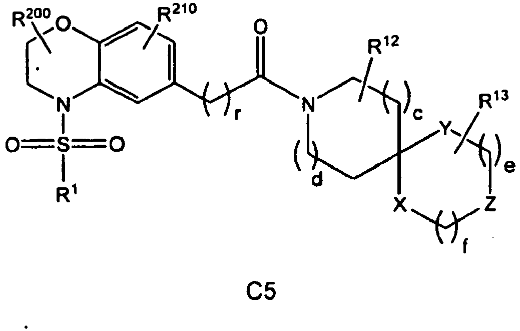 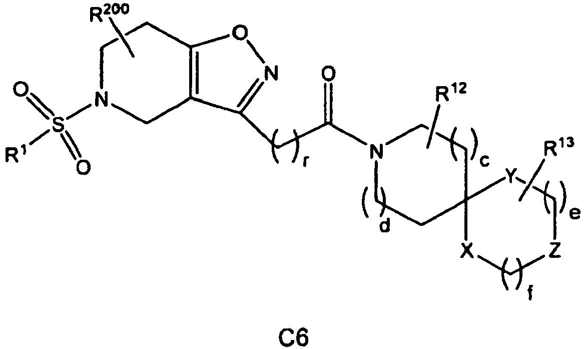 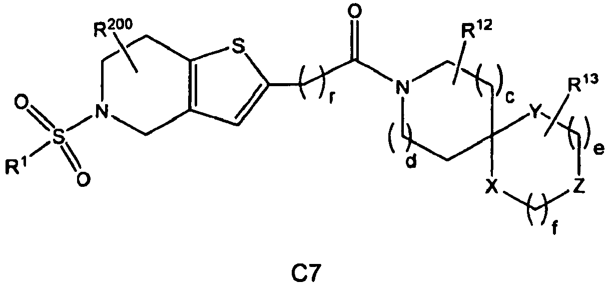 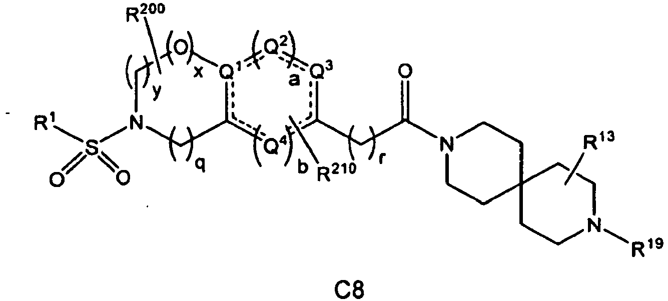 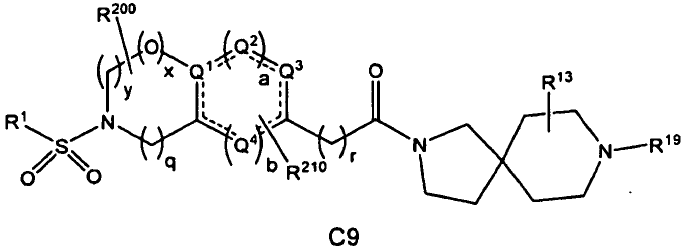 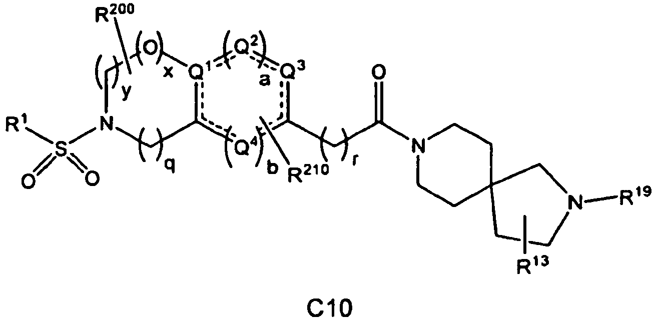 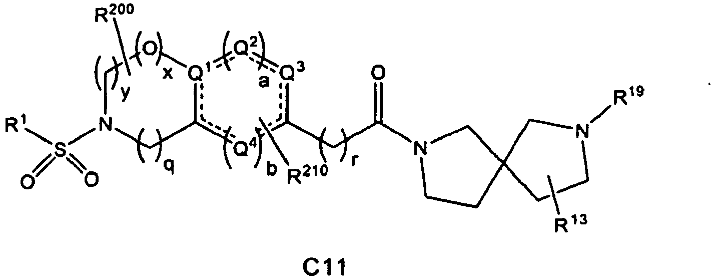 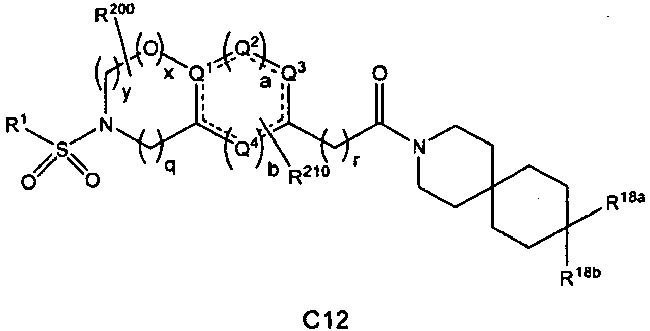 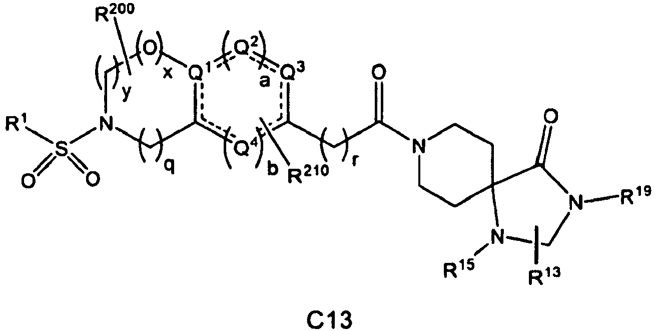 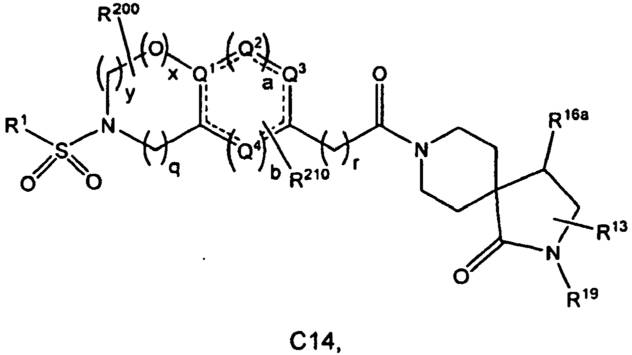 worin die jeweiligen Reste, Variablen und Indices die hierin im Zusammenhang mit den erfindungsgemäßen Verbindungen und deren bevorzugten Ausführungsformen beschriebenen Bedeutungen haben.

In einer weiteren bevorzugten Ausführungsform der vorliegenden Erfindung können die erfindungsgemäßen substituierten Verbindungen ausgewählt sein aus der Gruppe bestehend aus

| **Verbindung** | **Name** |
|---|---|
| G-04 | (2-(4-Methoxy-2,6-dimethylphenylsulfonyl)-1,2,3,4-tetrahydroisochinolin-7-yl)(9-(pyridin-4-yl)-3,9-diazaspiro[5.5]undecan-3-yl)methanon |
| G-09 | 2-(1-(4-Methoxy-2,6-dimethylphenylsulfonyl)-1,2,3,4-tetrahydrochinolin-7-yl)-1-(2-(pyridin-4-yl)-2,8-diazaspiro[4.5]decan-8-yl)ethanon |
| G-10 | 1-(2-Cyclobutyl-2,8-diazaspiro[4.5]decan-8-yl)-2-(1-(4-methoxy-2,6-dimethylphenylsulfonyl)-1,2,3,4-tetrahydrochinolin-7-yl)ethanon, |
| G-11 | 2-(1-(4-Methoxy-2,6-dimethylphenylsulfonyl)-1,2,3,4-tetrahydrochinolin-7-yl)-1-(8-(pyridin-4-yl)-2,8-diazaspiro[4.5]decan-2-yl)ethanon |
| G-12 | 2-(1-(4-Methoxy-2,6-dimethylphenylsullonyl)-1,2,3,4-tetrahydrochinolin-7-yl)-1-(7-(pyridin-4-yl)-2,7-diazaspiro[4.4]nonan-2-yl)ethanon |
| G-13 | (5-(2-Chlorbenzoyl)-4,5,6,7-tetrahydroisoxazolo[4,5-c]pyridin-3-yl)(9-(pyridin-4-yl)-3,9-diazaspiro[5.5]undecan-3-yl)methanon |
| G-14 | (5-(4-Methoxy-2,6-dimethylphenylsulfonyl)-4,5,6,7-tetrahydrothieno[3,2-c]pyridin-2-yl)(9-(pyridin-4-yl)-3,9-diazaspiro[5.5]undecan-3-yl)methanon |
| G-15 | (5-(2-Chlorbenzoyl)-4,5,6,7-tetrahydrothieno[3,2-c]pyridin-2-yl)(9-(pyridin-4-yl)-3,9-diazaspiro[5.5]undecan-3-yl)methanon |
| G-16 | 1-(9-(Pyridin-4-yl)-3,9-diazaspiro[5.5]undecan-3-yl)-2-(1-(2-(trifluormethyl)phenylsulfonyl)-1,2,3,4-tetrahydrochinolin-7-yl)ethanon |
| G-17 | 2-(1-(Naphthalin-1-ylsulfonyl)-1,2,3,4-tetrahydrochinolin-7-yl)-1-(9-(pyridin-4-yl)-3,9-diazaspiro[5.5]undecan-3-yl)ethanon |
| G-18 | 2-(1-(2-Chlor-6-methylphenylsulfonyl)-1,2,3,4-tetrahydrochinolin-7-yl)-1-(9-(pyridin-4-yl)-3,9-diazaspiro[5.5]undecan-3-yl)ethanon |
| G-19 | 2-(4-(4-Methoxy-2,6-dimethylphenylsulfonyl)-3,4-dihydro-2H-benzo[b][1,4]oxazin-6-yl)-1-(9-(pyridin-4-yl)-3,9-diazaspiro[5.5]undecan-3-yl)ethanon |
| G-20 | 2-(1-(4-Methoxy-2,6-dimethylphenylsulfonyl)indolin-6-yl)-1-(9-(pyridin-4-yl)-3,9-diazaspiro[5.5]undecan-3-yl)ethanon |
| G-21 | (1-(2-Chlorbenzoyl)-1,2,3,4-tetrahydrochinolin-7-yl)(9-(pyridin-4-yl)-3,9-diazaspiro[5.5]undecan-3-yl)methanon |
| G-24 | (1-(4-Methoxy-2,6-dimethylphenylsulfonyl)-1,2,3,4-tetrahydrochinolin-7-yl)(9-(pyridin-4-yl)-3,9-diazaspiro[5.5]undecan-3-yl)methanon |
| G-25 | 2-(1-(4-Methoxy-2,6-dimethylphenylsulfonyl)-1,2,3,4-tetrahydrochinolin-7-yl)-1-(9-(pyridin-4-yl)-3,9-diazaspiro[5.5]undecan-3-yl)ethanon |
| G-26 | (5-(4-Methoxy-2,6-dimethylphenylsulfonyl)-4,5,6,7-tetrahydroisoxazolo[4,5-c]pyridin-3-yl)(9-(pyridin-4-yl)-3,9-diazaspiro[5.5]undecan-3-yl)methanon |
| G-30 | 2-(1-(4-Methoxy-2,6-dimethylphenylsulfonyl)-1,2,3,4-tetrahydrochinolin-7-yl)-1-(9-(pyridin-4-yloxy)-3-azaspiro[5.5]undecan-3-yl)ethanon |
| G-32 | 1-(9-(Azetidin-1-yl)-3-azaspiro[5.5]undecan-3-yl)-2-(1-(4-methoxy-2,6-dimethylphenylsulfonyl)-1,2,3,4-tetrahydrochinolin-7-yl)ethanon |
| G-33 | 2-(1-(4-Methoxy-2,6-dimethylphenylsulfonyl)-1,2,3,4-tetrahydrochinolin-7-yl)-1-(3-(pyridin-4-yl)-1-oxa-2,8-diazaspiro[4.5]dec-2-en-8-yl)ethanon |
| G-34 | 1-(9-(3,3-Difluorazetidin-1-yl)-3-azaspiro[5.5]undecan-3-yl)-2-(1-(4-methoxy-2,6-dimethylphenylsulfonyl)-1,2,3,4-tetrahydrochinolin-7-yl)ethanon |
| G-35 | 2-(1-(2-Chlor-4-(trifluormethoxy)phenylsulfonyl)-1,2,3,4-tetrahydrochinolin-7-yl)-1-(9-(pyridin-4-yl)-3,9-diazaspiro[5.5]undecan-3-yl)ethanon |
| G-36 | (2-(4-Methoxy-2,6-dimethylphenylsulfonyl)-1,2,3,4-tetrahydroisochinolin-8-yl)(9-(pyridin-4-yl)-3,9-diazaspiro[5.5]undecan-3-yl)methanon |
| G-37 | (2-(2-Chlor-6-methylphenylsulfonyl)-1,2,3,4-tetrahydroisochinolin-8-yl)(9-(pyridin-4-yl)-3,9-diazaspiro[5.5]undecan-3-yl)methanon |
| G-38 | (2-(2,3-Dichlorphenylsulfonyl)-1,2,3,4-tetrahydroisochinolin-8-yl)(9-(pyridin-4-yl)-3,9-diazaspiro[5.5]undecan-3-yl)methanon |
| G-39 | 2-(2-(4-Methoxy-2,6-dimethylphenylsulfonyl)-1,2,3,4-tetrahydroisochinolin-7-yl)-1-(9-(pyridin-4-yl)-3,9-diazaspiro[5.5]undecan-3-yl)ethanon |
| G-40 | (7-(4-Methoxy-2,6-dimethylphenylsulfonyl)-5,6,7,8-tetrahydroimidazo[1,2-a]pyrazin-2-yl)(9-(pyridin-4-yl)-3,9-diazaspiro[5.5]undecan-3-yl)methanon |
| G-41 | 3-(2-Ch lorphenyl)-1-(7-(9-(pyridin-4-yl)-3, 9-diazaspiro[5.5]undecan-3-carbonyl)-3,4-dihydroisochinolin-2(1H)-yl)prop-2-en-1-on |
| G-42 | (5-Chlorthiophen-2-yl)(7-(9-(pyridin-4-yl)-3,9-diazaspiro[5.5]undecan-3-carbonyl)-3,4-dihydroisochinolin-2(1H)-yl)methanon |
| G-43 | 2-(2-Chlorphenyl)-1-(7-(9-(pyridin-4-yl)-3,9-diazaspiro[5.5]undecan-3-carbonyl)-3,4-dihydroisochinolin-2(1H)-yl)ethanon |
| G-44 | (5-Chlorthiophen-2-yl)(8-(9-(pyridin-4-yl)-3,9-diazaspiro[5.5]undecan-3-carbonyl)-3,4-dihydroisochinolin-2(1H)-yl)methanon |
| G-45 | N-(2-Chlorbenzyl)-7-(9-(pyridin-4-yl)-3,9-diazaspiro[5.5]undecan-3-carbonyl)-3,4-dihydroisochinolin-2(1H)-carboxamid |
| G-46 | N-(3,4-Dichlorphenyl)-3-(9-(pyridin-4-yl)-3,9-diazaspiro[5.5]undecan-3-carbonyl)-6,7-dihydroisoxazolo[4,5-c]pyridin-5(4H)-carboxamid |
| G-47 | (2-(4-Methylnaphthalin-1-ylsulfonyl)-1,2,3,4-tetrahydroisochinolin-7-yl)(9-(pyridin-4-yl)-3,9-diazaspiro[5.5]undecan-3-yl)methanon |
| G-48 | (2-(4-Methoxynaphthalin-1-ylsulfonyl)-1,2,3,4-tetrahydroisochinolin-7-yl)(9-(pyridin-4-yl)-3,9-diazaspiro[5.5]undecan-3-yl)methanon |
| G-49 | 2,2-Diphenyl-1-(7-(9-(pyridin-4-yl)-3,9-diazaspiro[5,5]undecan-3-carbonyl)-3,4-dihydroisochinolin-2(1H)-yl)ethanon |
| G-50 | (2-(4-Chlomaphthalin-1-ylsulfonyl)-1,2,3,4-tetrahydroisochinolin-7-yl)(9-(pyridin-4-yl)-3,9-diazaspiro(5.5]undecan-3-yl)methanon |
| G-51 | 2-(1-(2-Chlor-6-methylphenylsulfonyl)-1,2,3,4-tetrahydrochinolin-7-yl)-1-(8-(pyridin-4-yl)-2,8-diazaspiro[4.5]decan-2-yl)ethanon |
| G-52 | 2-(1-(Naphthalin-1-ylsulfonyl)-1,2,3,4-tetrahydrochinolin-7-yl)-1-(8-(pyridin-4-yl)-2,8-diazaspiro[4.5]decan-2-yl)ethanon |
| G-53 | 2-(1-(Naphthalin-1-ylsulfonyl)-1,2,3,4-tetrahydrochinolin-7-yl)-1-(7-(pyridin-4-yl)-2,7-diazaspiro[4.4]nonan-2-yl)ethanon |
| G-54 | 1-(9-(Azetidin-1-yl)-3-azaspiro[5.5]undecan-3-yl)-2-(1-(naphthalin-1-ylsulfonyl)-1,2,3,4-tetrahydrochinolin-7-yl)ethanon |
| G-55 | N-(3,4-dichlorphenyl)-7-(9-(pyridin-4-yl)-3,9-diazaspiro[5.5]undecan-3-arbonyl)-3,4-dihydroisochinolin-2(1H)-carboxamid |
| G-56 | (2-(4-Fluomaphthalin-1-ylsulfonyl)-1,2,3,4-tetrahydroisochinolin-7-yl)(9-(pyridin-4-yl)-3,9-diazaspiro[5.5]undecan-3-yl)methanon |
| G-57 | 1-(8-(Pyridin-4-yl)-2,8-diazaspiro(4.5]decan-2-yl)-2-(1-(2-(trifluormethyl)phenylsulfonyl)-1,2,3,4-tetrahydrochinolin-7-yl)ethanon |
| G-58 | (5-Methylthiophen-2-yl)(7-(9-(pyridin-4-yl)-3,9-diazaspiro[5.5]undecan-3-carbonyl)-3,4-dihydroisochinolin-2(1H)-yl)methanon |
| G-59 | Benzo[b]thiophen-2-yl(7-(9-(pyridin-4-yl)-3,9-diazaspiro[5.5]undecan-3-carbonyl)-3,4-dihydroisochinolin-2(1H)-yl)methanon |
| G-60 | (5,6-Dihydro-4H-cyclopenta[b]thiophen-2-yl)(7-(9-(pyridin-4-yl)-3,9-diazaspiro[5.5]undecan-3-carbonyl)-3,4-dihydroisochinolin-2(1H)-yl)methanon |
| G-61 | (3-Chlor-6-methoxybenzo[b]thiophen-2-yl)(7-(9-(pyridin-4-yl)-3,9-diazaspiro[5.5]undecan-3-carbonyl)-3,4-dihydroisochinolin-2(1H)-yl)methanon |
| G-62 | (2-(5-Chlornaphthalin-1-ylsulfonyl)-1,2, 3,4-tetrahydroisochinolin-7-yl)(9-(pyridin-4-yl)-3,9-diazaspiro[5.5]undecan-3-yl)methanon |
| G-63 | (5-tert-Butylthiophen-2-yl)(7-(9-(pyridin-4-yl)-3,9-diazaspiro[5.5]undecan-3-carbonyl)-3,4-dihydroisochinolin-2(1H)-yl)methanon |
| G-64 | 2-[1-(2-Chlor-benzoyl)-1,2,3,4-tetrahydro-chinolin-7-yl]-1-(9-pyridin-4-yl-3,9-diazaspiro[5.5]undecan-3-yl)-ethanon |
| G-66 | 2-[1-[(2,6-Dichlor-3-methyl-phenyl)sulfonyl]-1,2,3,4-tetrahydrochinolin-7-yl]-1-(9-pyridin-4-yl-3,9-diazaspiro[5.5]undecan-3-yl)-ethanon |
| G-67 | [2-[(4-Methoxy-2,6-dimethyl-phenyl)sulfonyl]-2,3-dihydro-1H-isoindol-5-yl]-(9-pyridin-4-yl-3,9-diazaspiro[5.5]undecan-3-yl)-methanon |
| G-68 | [2-[(2-Chlor-6-methyl-phenyl)sulfonyl]-2,3-dihydro-1H-isoindol-5-yl]-(9-pyridin-4-yl-3,9-diazaspiro[5.5]undecan-3-yl)-methanon |
| G-69 | [2-(5-Chlor-thiophen-2-carbonyl)-2,3-dihydro-1H-isoindol-5-yl]-(9-pyridin-4-yl-3,9-diazaspiro[5.5]undecan-3-yl)-methanon |
| G-70 | 2-[8-[(4-Methoxy-2,6-dimethyl-phenyl)sulfonyl]-5,6,7,8-tetrahydro-[1,8]naphthyridin-2-yl]-1-(9-pyridin-4-yl-3,9-diazaspiro[5.5]undecan-3-yl)-ethanon |
| G-71 | [7-(5-Chlor-thiophen-2-carbonyl)-5,6,7,8-tetrahydro-imidazo[1,2-a]pyrazin-2-yl]-(9-pyridin-4-yl-3,9-diazaspiro[5.5]undecan-3-yl)-methanon |
| G-72 | [7-[(4-Methoxy-2,6-dimethyl-phenyl)sulfonyl]-5,6,7,8-tetrahydro-imidazo[1,5-a]pyramin-1-yl]-(9-pyridin-4-yl-3,9-diazaspiro[5.5]undecan-3-yl)-methanon |
| G-73 | [2-(4-Methoxy-2,6-dimethyl-benzoyl)-2,3-dihydro-1H-isoindol-5-yl]-(9-pyridin-4-yl-3,9-diazaspiro[5.5]undecan-3-yl)-methanon |
| G_CC-006 | 2-(4-Fluorbenzyl)-8-(1-(4-methoxy-2,6-dimethylphenylsulfonyl)indolin-6-carbonyl)-2,8-diazaspiro[4.5]decan-1-on |
| G_CC-007 | (7-Benzyl-2,7-diazaspiro[4.4]nonan-2-yl)(1-(4-methoxy-2,6-dimethylphenylsulfonyl)indolin-6-yl)methanon |
| G_CC-008 | (1-(4-Methoxy-2,6-dimethylphenylsulfonyl)indolin-6-yl)(9-(pyridin-4-yl)-3,9-diazaspiro[5.5]undecan-3-yl)methanon |
| G_CC-009 | (1-(2-Chlorbenzoyl)indolin-6-yl)(9-(pyridin-4-yl)-3,9-diazaspiro[5.5]undecan-3-yl)methanon |
| G_CC-013 | (1-(4-Chlor-2,5-dimethylphenylsulfonyl)indolin-6-yl)(9-(pyridin-4-yl)-3,9-diazaspiro(5.5)undecan-3-yl)methanon |
| G_CC-018 | 8-(2-(2-Chlorbenzoyl)isoindolin-5-carbonyl)-2-(4-fluorbenzyl)-2,8-diazaspiro[4.5]decan-1-on |
| G_CC-025 | 4-(4-Fluorphenyl)-8-(1-(4-methoxy-2,6-dimethylphenylsulfonyl)-1,2,3,4-tetrahydrochinolin-7-carbonyl)-2-methyl-2,8-diazaspiro[4.5]decan-1-on |
| G_CC-026 | 2-Benzyl-8-(1-(4-methoxy-2,6-dimethylphenylsulfonyl)-1,2,3,4-tetrahydrochinolin-7-carbonyl)-2,8-diazaspiro[4.5]decan-1-on |
| G_CC-027 | (7-Benzyl-2,7-diazaspiro[4.4]nonan-2-yl)(1-(4-methoxy-2,6-dimethylphenylsulfonyl)-1,2,3,4-tetrahydrochinolin-7-yl)methanon |
| G_CC-039 | 2-(4-Fluorbenzyl)-8-(2-(4-methoxy-2,6-dimethylphenylsulfonyl)-1,2,3,4-tetrahydroisochinolin-7-carbonyl)-2,8-diazaspiro[4.5]decan-1-on |
| G_CC-040 | 2-Benzyl-8-(2-(4-methoxy-2,6-dimethylphenylsulfonyl)-1,2,3,4-tetrahydroisochinolin-7-carbonyl)-2,8-diazaspiro[4.5]decan-1-on |
| G_CC-041 | (7-Benzyl-2,7-diazaspiro[4.4]nonan-2-yl)(2-(4-methoxy-2,6-dimethylphenylsulfonyl)-1,2,3,4-tetrahydroisochinolin-7-yl)methanon |
| G_CC-043 | 8-(2-(4-Methoxy-2,6-dimethylphenylsulfonyl)-1,2,3,4-tetrahydroisochinolin-7-carbonyl)-1-phenyl-1,3,8-triazaspiro[4.5]decan-4-on |
| G_CC-045 | (2-(2-Chlorbenzoyl)-1,2,3,4-tetrahydroisochinolin-7-yl)(9-(pyridin-4-yl)-3,9-diazaspiro[5.5]undecan-3-yl)methanon |
| G_CC-053 | 2-Benzyl-8-(2-(1-(4-methoxy-2,6-dimethylphenylsulfonyl)-1,2,3,4-tetrahydrochinolin-7-yl)acetyl)-2,8-diazaspiro[4.5]decan-1-on |
| G_CC-054 | 1-(7-Benzyl-2,7-diazaspiro[4.4]nonan-2-yl)-2-(1-(4-methoxy-2,6-dimethylphenylsulfonyl)-1,2,3,4-tetrahydrochinolin-7-yl)ethanon |
| G_CC-055 | 2-[1-[(4-Methoxy-2,6-dimethyl-phenyl)sulfonyl]-1,2,3,4-tetrahydrochinolin-7-yl]-1-(3-pyridin-4-yl-3,9-diazaspiro[5.5]undecan-9-yl)-ethanon |
| G_CC-056 | [1-[(4-Methoxy-2,6-dimethyl-phenyl)sulfonyl]-1,2,3,4-tetrahydrochinolin-7-yl]-(3-pyridin-4-yl-3,9-diazaspiro[5.5]undecan-9-yl)-methanon |

ggf. in Form eines einzelnen Enantiomers oder eines einzelnen Diastereomers, des Razemats, der Enantiomere, der Diastereomere, Mischungen von Enantiomere oder Diastereomeren, jeweils in Form ihrer Basen und/oder physiologisch verträglichen Salze, insbesondere der Hydrochlorid-Salze.

Die oben verwendete Nummerierung der einzelnen Ausführungsformen der erfindungsgemäßen Verbindungen wird in den nachfolgenden Erläuterungen der vorliegenden Erfindung, insbesondere in der Beschreibung der Beispiele, beibehalten. Gemäß einem Aspekt der vorliegenden Erfindung weisen die erfindungsgemäßen Verbindungen vorzugweise eine antagonistische Wirkung am humanen B1 R-Rezeptor oder dem B1 R-Rezeptor der Ratte auf. In einer bevorzugten Ausführungsform der Erfindung weisen die erfindungsgemäßen Verbindungen eine antagonistische Wirkung sowohl am humanen B1 R- Rezeptor (hB1 R) als auch am B1 R-Rezeptor der Ratte (rB1 R) auf.

In einer bevorzugten Ausführungsform der vorliegenden Erfindung weisen die erfindungsgemäßen Verbindungen im FLIPR-Assay bei einer Konzentration von 10 µM am humanen B1 R-Rezeptor und/ oder am B1 R-Rezeptor der Ratte eine Inhibition von mindestens 15%, 25%, 50%, 70%, 80% oder 90 % auf. Ganz besonders bevorzugt sind Verbindungen, die eine Inhibition am humanen B1R-Rezeptor und am B1 R-Rezeptor der Ratte von mindestens 70%, insbesondere von mindestens 80% und insbesondere bevorzugt von mindestens 90% bei einer Konzentration von 10 µM aufweisen.

Die agonistische bzw. antagonistische Wirkung von Substanzen kann am Bradykinin Rezeptor 1 (B1R) der Spezies Mensch und Ratte mit ektopisch exprimierenden Zelllinien (CHO K1 Zellen) und mit Hilfe eines Ca²⁺-sensitiven Farbstoffs (Fluo-4) im Fluorescent Imaging Plate Reader (FLIPR) quantifiziert werden. Die Angabe in % Aktivierung wird bezogen auf das Ca²⁺-Signal nach Zugabe von Lys-Des-Arg⁹-Bradykinin (0,5 nM), bzw. Des-Arg⁹-Bradykinin (100 nM). Antagonisten führen zu einer Unterdrückung des Ca²⁺-Einstroms nach der Zugabe des Agonisten. Angegeben werden % Inhibition im Vergleich zu der maximal erreichbaren Inhibition. Vorzugsweise wirken die erfindungsgemäßen Substanzen beispielsweise auf den im Zusammenhang mit verschiedenen Erkrankungen relevanten B1R, sodass sie sich als pharmazeutischer Wirkstoff in Arzneimitteln eignen. Ein weiterer Gegenstand der Erfindung sind daher Arzneimittel enthaltend wenigstens ein erfindungsgemäßes Spiroamid, sowie gegebenenfalls geeignete Zusatz- und/oder Hilfsstoffe und/ oder gegebenenfalls weitere Wirkstoffe.

Die erfindungsgemäßen Arzneimittel eignen sich vorzugsweise zur Bekämpfung von Schmerz, insbesondere von Schmerz ausgewählt aus der Gruppe bestehend aus akutem Schmerz, neuropathischem Schmerz, viszeralem Schmerz, chronischem Schmerz und Entzündungsschmerz; oder zur Behandlung von Migräne; Diabetes; Erkrankungen der Atemwege; entzündlichen Darmerkrankungen; neurologischen Erkrankungen; septischem Schock; Reperfusionssyndrom; Fettleibigkeit sowie als Angiogenese-Inhibitor.

Die erfindungsgemäßen Arzneimittel enthalten neben mindestens einem erfindungsgemäßen substituierten Spiroamid gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/ Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, nasal, buccal, rektal oder topisch, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte Spiroamide in einem Depot, in gelöster Form oder in einem Pflaster, gegebenenfalls unter Zusatz von die Haupenetration fördernden Mitteln, sind geeignete perkutane Applikationszubereitungen. Oral oder perkutan anwendbare Zubereitungsformen können die erfindungsgemäßen substituierten Spiroamide verzögert freisetzen. Die erfindungsgemäßen substituierten Spiroamide können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,01 bis 5 mg/kg wenigstens eines erfindungsgemäßen substituierten Spiroamids appliziert. In einer bevorzugten Form des Arzneimittels kann ein enthaltenes erfindungsgemäßes substituiertes Spiroamid als reines Diastereomer und/ oder Enantiomer, als Razemat oder als nicht-äquimolare oder äquimolare Mischung der Diastereomere und/ oder Enantiomere vorliegen.

B1R ist insbesondere am Schmerzgeschehen beteiligt. Entsprechend können die erfindungsgemäßen substituierten Spiroamide zur Herstellung eines Arzneimittels zur Behandlung von Schmerz, insbesondere von akutem, viszeralem, neuropathischem oder chronischem Schmerz, verwendet werden.

Ein weiterer Gegenstand der Erfindung ist daher die Verwendung eines erfindungsgemäßen substituierten Spiroamids zur Herstellung eines Arzneimittels zur Behandlung von Schmerz, insbesondere von akutem, viszeralem, neuropathischem oder chronischem Schmerz. Eine bestimmte Ausführungsform der vorliegenden Erfindung ist die Verwendung zumindest eines der erfindungsgemäßen substituierten Spiroamide zur Herstellung eines Arzneimittels zur Behandlung von Entzündungsschmerz.

Ein weiterer Gegenstand der Erfindung ist die Verwendung eines erfindungsgemäßen substituierten Spiroamids zur Herstellung eines Arzneimittels zur Behandlung von Diabetes, Erkrankungen der Atemwege, zum Beispiel Asthma bronchiale, Allergien, COPD/chronic-obstruktive pulmonary disease oder zystische Fibrose; entzündlichen Darmerkrankungen, beispielsweise Ulcerative Colitis oder CD/Crohn's disease; neurologischen Erkrankungen, beispielsweise Multiple Sklerose oder Neurodegeneration; Entzündungen der Haut, beispielsweise atopische Dermatitis, Psoriasis oder bakterielle Infektionen; rheumatischen Erkrankungen, beispielsweise rheumatoide Arthritis oder Osteoarthritis; septischem Schock; Reperfusionssyndrom, zum Beispiel nach Herzinfarkt oder Schlaganfall, Fettleibigkeit; und als Angiognese-Inhibitor.

Dabei kann es in einer der vorstehenden Verwendungen bevorzugt sein, wenn ein verwendetes substituiertes Spiroamid als reines Diastereomer und/ oder Enantiomer, als Razemat oder als nicht-äquimolare oder äquimolare Mischung der Diastereomere und/ oder Enantiomere vorliegt.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung benötigt, durch Verabreichung einer therapeutisch wirksamen Dosis eines erfindungsgemäßen substituierten Spiroamids, oder eines erfindungsgemäßen Arzneimittels.

Ein weiterer Gegenstand der vorliegenden Erfindung ist ein Verfahren zur Herstellung der erfindungsgemäßen substituierten Spiroamide, insbesondere wie in der folgenden Beschreibung, Beispielen und Ansprüchen ausgeführt. Das erfindungsgemäße Verfahren ist in nachstehendem Schema 1 dargestellt.

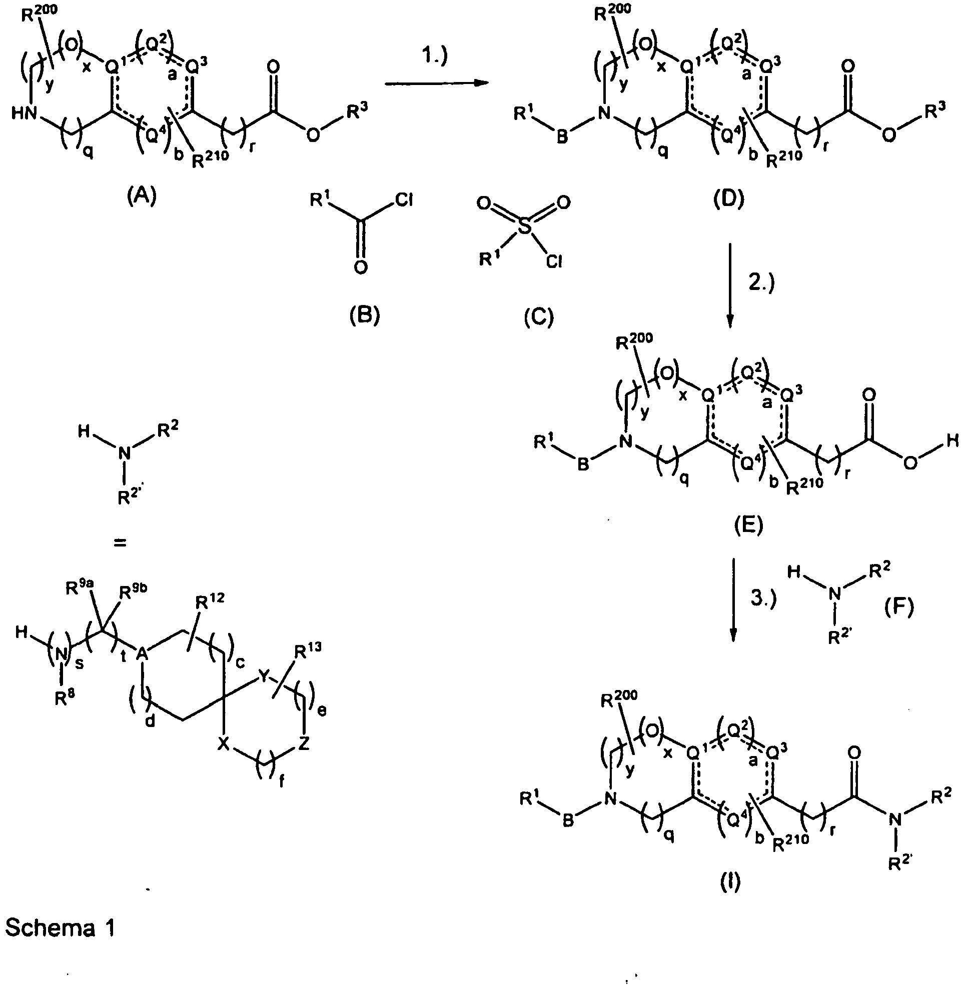

In Stufe 1 werden Carbonsäurechlorid der allgemeinen Formel (B), worin R¹ die vorstehend genannte Bedeutung hat, bzw. Sulfonylchlorid der allgemeinen Formel (C), worin R¹ die vorstehend genannte Bedeutung hat, in wenigstens einem Lösungsmittel, vorzugsweise ausgewählt aus der Gruppe bestehend aus Dichlormethan, Acetonitril, Dimethylformamid, Diethylether, Dioxan, Tetrahydrofuran, Methanol, Ethanol und Isopropanol mit Aminosäureestern (A), in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, Diisopropylethylamin und Pyridin und ggf. unter Zusatz von 4-(Dimethylamino)pyridin oder 1-Hydroxybenzotriazol, bei Temperaturen von vorzugsweise -15°C bis 50°C zu Verbindungen mit der allgemeinen Formel (D) umgesetzt.

Gegebenenfalls können in Stufe 1 anstatt der Carbonsäurechlorid (B) auch die entsprechenden Carbonsäuren eingesetzt werden. Diese Säuren der allgemeinen Formel R¹CO₂H, worin R¹ die vorstehend genannte Bedeutung hat, werden in wenigstens einem Lösungsmittel, vorzugsweise ausgewählt aus der Gruppe bestehend aus Dichlormethan, Acetonitril, Dimethylformamid, Diethylether, Dioxan und Tetrahydrofuran mit Aminosäureestern (A) unter Zusatz wenigstens eines Kupplungsreagenzes, vorzugsweise ausgewählt aus der Gruppe bestehend aus Carbonyldiimidazol (CDI), 2-Chlor-1-methylpyridinium iodid (Mukaiyama Reagenz), N-(3-Dimethylaminopropyl)-N'-ethylcarbodiimid (EDCI), O-(Benzotriazol-1-yl)-N,N,N',N'-tetramethyluronium tetrafluoroborat (TBTU), N,N'-Dicyclohexylcarbodiimid (DCC) und 1-Benzotriazolyloxy-tris-(dimethylamino)-phosphonium hexafluorophosphat (BOP) ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, Diisopropylethylamin und Pyridin und ggf. unter Zusatz von 4-(Dimethylamino)pyridin oder 1-Hydroxybenzotriazol zu Verbindungen mit der allgemeinen Formel (D) umgesetzt.

In Stufe 2 werden Verbindungen der allgemeinen Formen (D) in wenigstens einem Lösungsmittel, vorzugsweise ausgewählt aus der Gruppe bestehend aus Wasser, Methanol, Ethanol, Isopropanol, Diethylether, Tetrahydrofuran, Toluol, Acetonitril, Dimetylformamid, Dioxan und Dimethylsulfoxid mit einer anorganischen Base, vorzugsweise ausgewählt aus der Gruppe bestehend aus Lithiumhydroxid, Natriumhydroxid, Kaliumhydroxid, Kaliumtertbutanolat, Lithium propanthiolat und Natrium phenylselenolat, ggf. unter Zusatz von HMPA oder Lithiumchlorid oder mit einer Lewis Säure, vorzugsweise ausgewählt aus der Gruppe bestehend aus Trimethylsilylchlorid, Bortribromid und Aluminiumtrichlorid, ggf. unter Zusatz von Thiolen, Natriumiodid oder Lithiumchlorid, bei Temperaturen von vorzugsweise 0°C bis 100°C zu Verbindungen der allgemeinen Formel (E) umgesetzt.

In Stufe 3 werden Verbindungen der allgemeinen Formel (E) in wenigstens einem Lösungsmittel, vorzugsweise ausgewählt aus der Gruppe bestehend aus Dichlormethan, Acetonitril, Dimethylformamid, Diethylether, Dioxan und Tetrahydrofuran mit Amin (F), unter Zusatz wenigstens eines Kupplungsreagenzes, vorzugsweise ausgewählt aus der Gruppe bestehend aus Carbonyldiimidazol (CDI), 2-Chlor-1-methylpyridinium iodid (Mukaiyama Reagenz), N-(3-Dimethylaminopropyl)-*N*'-ethylcarbodiimid (EDCI), *O*-(Benzotriazol-1-yl)-*N*,*N*,*N*',*N*'-tetramethyluronium tetrafluoroborat (TBTU), *N*,*N*'-Dicyclohexylcarbodiimid (DCC) und 1-Benzotriazolyloxy-tris-(dimethylamino)-phosphonium hexafluorophosphat (BOP) ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, Diisopropylethylamin und Pyridin und ggf. unter Zusatz von 4-(Dimethylamino)pyridin oder 1-Hydroxybenzotriazol zu Verbindungen mit der allgemeinen Formel (G) umgesetzt.

### Pharmakologische Methoden

### 1. Funktionelle Untersuchung am Bradykinin Rezeptor 1 (B1R)

Die agonistische bzw. antagonistische Wirkung von Substanzen kann am Bradykinin Rezeptor 1 (B1R) der Spezies Mensch und Ratte mit folgendem Assay bestimmt werden. Gemäß diesem Assay wird der Ca²⁺-Einstrom durch den Kanal mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert.

### 2. Methode:

Es werden Chinese Hamster Ovary Zellen (CHO K1 Zellen) verwendet, die stabil mit dem humanen B1 R-Gen (hB1 R-Zellen) bzw. dem B1 R-Gen der Ratte (rB1 R-Zellen), transfiziert sind. Für funktionelle Untersuchungen werden diese Zellen auf schwarze 96-Loch-Platten mit klarem Boden (BD Biosciences, Heidelberg, Deutschland oder Greiner, Frickenhausen, Deutschland) in einer Dichte von 20.000 - 35.000 Zellen/Loch ausplattiert. Über Nacht werden die Zellen bei 37 °C und 5 % CO₂ in Kulturmedium (hB1 R-Zellen: Nutrient Mixture Ham's F12, Gibco Invitrogen GmbH, Karlsruhe, Deutschland oder DMEM, Sigma-Aldrich, Taufkirchen, Deutschland; rB1 R-Zellen: D-MEM/F12, Gibco Invitrogen GmbH, Karlsruhe, Deutschland) mit 10 Vol-% FBS (Fetal bovine serum, Gibco Invitrogen GmbH, Karlsruhe, Deutschland oder PAN Biotech GmbH, Aidenbach, Deutschland) belassen.
Am folgenden Tag werden die Zellen mit 2,13 µM Fluo-4 (Molecular Probes Europe BV, Leiden Niederlande) in HBSS-Puffer (Hank's buffered saline solution, Gibco Invitrogen GmbH, Karlsruhe, Deutschland) mit 2,5 mM Probenecid (Sigma-Aldrich, Taufkirchen, Deutschland) und 10 mM HEPES (Sigma-Aldrich, Taufkirchen, Deutschland) für 60 min bei 37 °C beladen. Anschließend werden die Platten 2 x mit HBSS-Puffer gewaschen und mit HBSS-Puffer versetzt, der zusätzlich 0,1 % BSA (bovines Serumalbumin; Sigma-Aldrich, Taufkirchen, Deutschland), 5,6 mM Glucose und 0,05 % Gelatine (Merck KGaA, Darmstadt, Deutschland) enthält. Nach einer weiteren Inkubation von 20 Minuten bei Raumtemperatur werden die Platten zur Ca²⁺-Messung im FLIPR eingesetzt.
Alternativ wird mit Puffer A (15 mM HEPES, 80 mM NaCl. 5 mM KCI, 1,2 mM CaCl₂, 0,7 mM MgSO₄, 2g/L Glucose, 2,5 mM Probenecid) gewaschen und mit Puffer A versetzt mit 2,5 µM Fluo-4 und 0,025 % Pluronic F127 (Sigma-Aldrich, Taufkirchen, Deutschland) beladen. Danach werden die Zellen 2 x mit Puffer A gewaschen und für 30 Minuten mit Puffer A, der zusätzlich 0,05 % BSA und 0,05 % Gelatine enthält bei Raumtemperatur inkubiert und danach zur Ca²⁺-Messung im FLIPR eingesetzt.

Die Ca²⁺-abhängige Fluoreszenz wird dabei vor und nach Zugabe von Substanzen gemessen (λₑₓ = 488 nm, λₑₘ = 540 nm). Die Quantifizierung erfolgt durch die Messung der höchsten Fluoreszenzintensität (FC, Fluorescence Counts) über die Zeit.

### 3. FLIPR-Assay:

Das FLIPR-Protokoll besteht aus 2 Substanzzugaben. Zunächst werden Testsubstanzen (10 µM) auf die Zellen pipettiert und der Ca²⁺-Einstrom mit der Kontrolle (hB1R: Lys-Des-Arg⁹-Bradykinin >= 50 nM; rB1 R: Des-Arg⁹-Bradykinin 10 µM) verglichen. Daraus ergibt sich die Angabe in % Aktivierung bezogen auf das Ca²⁺-Signal nach Zugabe von Lys-Des-Arg⁹-Bradykinin (>= 50 nM), bzw. Des-Arg⁹-Bradykinin (10 µM).
Nach 10-20 Minuten Inkubation werden Lys-Des-Arg⁹-Bradykinin (hB1 R) bzw. Des-Arg⁹-Bradykinin (rB1 R) in der Konzentration des EC₈₀ appliziert und ebenfalls der Einstrom von Ca²⁺ ermittelt.
Antagonisten führen zu einer Unterdrückung des Ca²⁺-Einstroms. Es werden % Inhibition im Vergleich zu der maximal erreichbaren Inhibition berechnet.

Zur Bestimmung des IC₅₀-Wertes werden die Substanzen in verschiedenen Konzentrationen zugegeben. Es werden Zweifach- oder Dreifach-Bestimmungen (n=2 oder n=3) durchgeführt und diese in mindestens einem weiteren unabhängigen Experiment wiederholt (N>=2).

Vorzugsweise weisen die Verbindungen eine B1R antagonistische Wirkung am humanen Rezeptor und/oder am Ratten-Rezeptor auf. Beispielhaft werden in der nachfolgenden Tabelle die folgenden Daten angegeben: (Dabei steht "% Inh. (rat B1R) 10 µM" für "% Inhibition Ratte B1R bei 10 µM" und "% Inh. (hum. B1R) 10 µM" für "% Inhibition human B1R bei 10 µM".

Im Folgenden wird die Erfindung anhand von Beispielen erläutert. Diese Erläuterungen sind lediglich beispielhaft und schränken den allgemeinen Erfindungsgedanken nicht ein.

### Beispiele

### Abkürzungsverzeichnis:

- DIPEA: Diisopropylethylamin
- EDCI: N-Ethyl-N'-(3-dimethylaminopropyl)-carbodiimid Hydrochlorid
- h: Stunde(n)
- HOBt: 1-Hydroxy-1H-benzotriazol
- konz.: konzentrierte
- min.: Minute(n)
- N: Normal
- RT: Raumtemperatur
- THF: Tetrahydrofuran
- TFA: Trifluoressigsäure
- abs.: absolut
- Äq.: Äquivalent(e)
- Äquiv.: Äquivalent(e)
- Boc: tert-Butylcarbamat
- DCM: Dichlormethan
- M: molare
- EtOAc: Ethylacetat
- Et₃N: Triethylamin
- Cbz: Benzylcarbamat
- DMF: Dimethylformamid
- DBU: 1,8-Diazabicyclo[5.4.0]undec-7-en

Die eingesetzten Chemikalien und Lösungsmittel wurden kommerziell bei den herkömmlichen Anbietern (Acros, Aldrich, Fluka, Lancaster, Maybridge, TCI, Fluorochem, Tyger, ABCR, Fulcrum, FrontierScientific, Milestone etc.) bezogen. Die Ausbeuten der hergestellten Verbindungen sind nicht optimiert.
Die Mischungsverhältnisse von Lösungsmitteln sind stets im Verhältnis Volumen / Volumen angegeben.

Eingesetzte Mengenäquivalente der Reagenzien, sowie Lösungsmittelmengen, Reaktionstemperaturen und -zeiten können bei unterschiedlichen Reaktionen, die nach derselben Methode durchgeführt wurden, leicht variieren.
Aufarbeitungs- und Aufreinigungsmethoden wurden den charakteristischen Eigenschaften der Verbindungen entsprechend angepasst.

Analytik der Verbindungen erfolgte durch HPLC-MS und / oder NMR:
- NMR: Bruker 440 MHz bzw. 600 MHz Gerät
Materialien und Methoden für die LC-MS Analytik: HPLC: Waters Alliance 2795 mit PDA Waters 2998; MS: Micromass Quattro Micro^{™} API ; Säule: Waters Atlantis^{®} T3 , 3 µm, 100 Å, 2.1 x 30 mm; Säulentemperatur: 40 °C, Eluent A: Wasser + 0.1% Ameisensäure; Eluent B: Acetonitril + 0.1% Ameisensäure; Gradient: 0% B zu 100% B in 8.8 min, 100% B für 0.4 min, 100% B zu 0% B in 0.01 min, 0% B für 0.8 min; Fluß: 1.0 ml/min; Ionisation: ES+, 25 V; Make up: 100 µL/min 70% Methanol + 0.2% Ameisensäure; UV: 200 - 400 nm

### Bausteinsynthesen

### 1) Synthese der Aminosäureester A:

### Baustein A-01: Methyl indolin-6-carboxylat

### Stufe 1: 4-Methyl-3-nitrobenzoesäure

Zu 0°C kalter Salpetersäure (69-72%ig; 25 ml; 0.5 Äquiv.) wurde über einen Zeitraum von 10 Minuten konz. Schwefelsäure (25ml; 0.5 Äquiv.) gegeben. Die erhaltene Mischung wurde 30 Minuten bei 0°C gerührt. Konz. Schwefelsäure (75ml; 1.5 Äquiv.) wurde bei Raumtemperatur über einen Zeitraum von 20 Minuten zu 4-Methylbenzoesäure (50 g; 1 Äquiv.) gegeben. Die erhaltene Suspension wurde auf 0°C abgekühlt und über einen Zeitraum von 45 Minuten mit der Nitriersäure versetzt. Das erhaltene Reaktionsgemisch wurde für 1 Stunde bei 10 - 20°C gerührt. Nach vollständigem Umsatz wurde das Reaktionsgemisch auf Eiswasser gegossen, der ausgefallene weiße Feststoff abfiltriert und getrocknet. 4-Methyl-3-nitrobenzoesäure (66.5g; 82.7%) wurde in Form eines weißen Feststoffs erhalten.

### Stufe 2: 4-(2-(Dimethylamino)vinyl)-3-nitrobenzoesäure

Zu einer Lösung von 4-Methyl-3-nitrobenzoesäure (30g; 1 Äquiv.) in Dimethylformamid (150ml) wurde unter Stickstoffatmosphäre DMF-DMA (45.4 g; 2.3 Äquiv.) gegeben. Das erhaltene Reaktionsgemisch wurde für 17 h auf 140°C erhitzt. Nach vollständigem Umsatz wurde das DMF abdestilliert, der Rückstand mit 150ml Methanol versetzt, 2 Stunden bei Raumtemperatur gerührt und anschließend über Nacht bei 0 - 4°C kristallisiert. Der ausgefallene Feststoff wurde abfiltriert, mit eiskaltem Methanol und anschließend mit Hexan gewaschen. Nach dem Trocknen wurde 4-(2-(Dimethylamino)vinyl)-3-nitrobenzoesäure (28 g; 71,79%) in Form eines roten Feststoffs erhalten.

### Stufe 3: Methyl 1H-indol-6-carboxylat

Zu einer Lösung von 4-(2-(Dimethylamino)vinyl)-3-nitrobenzoesäure (14 g; 1 Äquiv.) in einer Mischung aus THF (42 ml), Ethanol (42 ml) und Wasser (140 ml) wurde bei Raumtemperatur Natrium dithionate (164.4g; 16 Äquiv.) über einen Zeitraum von 20 Minuten gegeben. Das Reaktionsgemisch wurde für 90 min. zum Rückfluß erhitzt und anschließend 12 Stunden bei Raumtemperatur gerührt. Nach vollständigem Umsatz wurde Dichlormethan zugegeben, die Phasen getrennt und die wässrige Phase mit Dichlormethan extrahiert. Die vereinigten organischen Phasen wurden über Natriumsulfat getrocknet. Nach Entfernen des Lösungsmittels unter vermindertem Druck wurde säulenchromatographisch (Kieselgel, 10% Ethyl acetat/Hexan) gereinigt. Methyl 1 H-indol-6-carboxylat (8 g; 40%) wurde in Form eines Feststoffs erhalten.

### Stufe 4: Methyl indolin-6-carboxylat

Zu einer Lösung von Methyl 1 H-indol-6-carboxylat (8 g; 1 Äquiv.) in Essigsäure (80 ml) wurde über einen Zeitraum von 10 Minuten, bei 0°C NaCNBH₃ (11.49 g; 0.04 Äquiv.) gegeben. Das Reaktionsgemisch wurde für 20 Minuten bei 0°C gerührt, anschließend auf Raumtemperatur erwärmt und für 1 Stunde bei Raumtemperatur gerührt. Nach vollständigem Umsatz wurde die Essigsäure unter vermindertem Druck abdestilliert und der erhaltene Rückstand in Dichlormethan gelöst. Die entstandenen Phasen wurden getrennt. Die organische Phase wurde mit 1 N Natronlauge gewaschen und über Natriumsulfat getrocknet. Nach Entfernen des Lösungsmittels unter vermindertem Druck wurde säulenchromatographisch (Kieselgel, 10-15% Ethyl acetat/ Hexan) gereinigt. Methyl indolin-6-carboxylat (6 g; 75%) wurde in Form eines Feststoffs erhalten.

### Baustein A-02: Methyl isoindolin-5-carboxylat Hydrochlorid

### Stufe 1: Methyl 3,4-dimethylbenzoat

Zu einer Lösung von 3,4-Dimethylbenzoesäure (35 g; 1 Äquiv.) und Dimethylformamid (1 ml) in 227 ml Methanol wurde bei Raumtemperatur Thionylchlorid (80 ml; 2 Äquiv.) gegeben. Das erhaltene Reaktionsgemisch wurde für 12 h bei Raumtemperatur gerührt und anschließend unter vermindertem Druck konzentriert. Der erhaltene Rückstand wurde in Dichlormethan aufgenommen und mit 5%iger Natriumcarbonat-Lsg. gewaschen. Die organische Phase wurde über Magnesiumsulfat getrocknet und unter vermindertem Druck konzentriert. Das so erhaltene Methyl 3,4-dimethylbenzoat (38,2 g) wurde ohne weitere Aufreinigung in der nächsten Stufe eingesetzt.

### Stufe 2: Methyl 3,4-bis(bromomethyl)benzoat

Zu einer Lösung von Methyl 3,4-dimethylbenzoat (38.2 g; 1 Äquiv.) in 458.4 ml Dichlormethan wurde eine katalytische Menge Benzylperoxid und NBS (82.9 g; 2 Äquiv.) gegeben. Das erhaltene Reaktionsgemisch wurde 15 Minuten bei Raumtemperatur gerührt, danach 18 Stunden zum Rückfluß erhitzt (110 - 120°C). Das Reaktionsgemisch wurde auf Raumtemperatur abgekühlt, filtriert und unter vermindertem Druck konzentriert. Der Rückstand wurde in Dichlormethan aufgenommen, mit 5% Natriumcarbonatlösung gewaschen, über Natriumsulfat getrocknet und abermals unter vermindertem Druck konzentriert. Das so erhaltene Methyl 3,4-bis(brommethyl)benzoat (73.9 g) wurde ohne weitere Aufreinigung in der nächsten Stufe eingesetzt.

### Stufe 3: Methyl 2-benzylisoindolin-5-carboxylat

Eine Lösung von Methyl 3,4-bis(brommethyl)benzoat (9.3g; 1 Äquiv.) und Benzylamin (4.3 g; 1.4 Äquiv.) in 83.7 ml Benzol wurde bei Raumtemperatur innerhalb von 15 Minuten mit TEA versetzt. Das erhaltene Reaktionsgemisch wurde zunächst für 30 Minuten bei Raumtemperatur gerührt, anschließend für 20 Stunden zum Rückfluß (110-120°C) erhitzt. Nach vollständigem Umsatz wurde das Reaktionsgemisch auf Raumtemperatur abgekühlt und filtriert. Das Filtrat wurde in 100 ml Dichlormethan aufgenommen und 2x mit 5%iger Natriumcarbonatlösung gewaschen. Die vereinigten organischen Phasen wurden über Natriumsulfat getrocknet und das Lösungsmittels unter vermindertem Druck entfernt. Nach säulenchromatographischer Aufreinigung (Kieselgel, 5% Essigester / Hexan), wurde Methyl 2-benzylisoindolin-5-carboxylat (2.2 g; 28.3%) in Form eines Feststoffes erhalten.

**Stufe 4: Methyl 2-benzylisoindolin-5-carboxylat Hydrochlorid** (AL/7102/S04) Durch eine Lösung von Methyl 2-benzylisoindolin-5-carboxylat (56 g; 1 Äquiv.) in 560ml Dichlormethan wurde so lange HCl-Gas geleitet, bis sich ein Feststoff bildete. Der Feststoff wurde abfiltriert, mit Hexan gewaschen Und getrocknet. Methyl 2-benzylisoindolin-5-carboxylat Hydrochlorid (43 g) wurde in Form eines weißen Feststoffes erhalten.

### Stufe 5: Methyl isoindolin-5-carboxylat Hydrochlorid

Zu einer Lösung von Methyl 2-benzylisoindolin-5-carboxylat Hydrochlorid (43 g; 1 Äquiv.) in 800 ml Methanol wurde 10% mit Pd/C (4.3g) gegeben. Dieses Gemisch wurde für 90 Minuten bei 60 mm Wasserstoff hydriert. Nach vollständigem Umsatz wurde das Reaktionsgemisch filtriert, das Filtrat unter vermindertem Druck konzentriert und das Rohprodukt aus Chloroform umkristallisiert. So wurde das gewünschte Produkt (25.39g; 84%) in Form eines weissen Feststoffes mit einer Reinheit von 98.63% erhalten.

### Baustein A-03: Methyl 1,2,3,4-tetrahydrochinolin-7-carboxylat

### Stufe 1: 7-(Trifluormethyl)chinolin

Zu einer Mischung aus 3-(Trifluormethyl)aniline (5 g; 1 Äquiv.), Glycerin (5.14 g; 1.8 Äquiv.) und Iod (150 mg) wurde konz. Schwefelsäure (4.56 g; 1.5 Äquiv.) getropft. Das erhaltene Reaktionsgemisch wurde 1 Stunde bei 80-90°C und 3 Stunden bei 160-170°C gerührt. Nach vollständigem Umsatz, wurde die Reaktionsmischung bei Raumtemperatur mit 100 ml Wasser verdünnt, mit Natriumcarbonat neutralisiert und 4x mit 200 ml Dichlormethan extrahiert. Die vereinigten organischen Phasen wurden über Natriumsulfat getrocknet, das Lösungsmittel unter vermindertem Druck entfernt und das Rohprodukt säulenchromatographisch (Kieselgel, 10% EtOAc/Hexan) aufgereinigt. 7-(Trifluormethyl)chinolin (1.35 g; 22.13%) wurde in Form eines weißen, kristallinen Feststoffes erhalten.

### Stufe 2: Methyl chinolin-7-carboxylat

Eine Mischung aus 7-(Trifluormethyl)chinolin (8.9 g; 1 Äquiv.) und 35.6 ml 10% Oleum wurde 3 Stunden auf 150°C erhitzt, anschließend auf Raumtemperatur abgekühlt, mit 90 mlMethanol versetzt und über Nacht auf 80°C erhitzt. Nach vollständigem Umsatz wurde Methanol abdestilliert, der Rückstand in 200 ml Wasser aufgenommen, mit Natriumcarbonat neutralisiert und 3x mit Essigester extrahiert. Die vereinigten organischen Phasen wurden über Natriumsulfat getrocknet, unter vermindertem Druck konzentriert und das Rohprodukt mit Petrolether gewaschen. Methyl chinolin-7-carboxylat (7.4 g; 88%) wurde in Form eines weißen Feststoffe erhalten.

### Stufe 3: Methyl 1,2,3,4-tetrahydrochinolin-T-carboxylat

Zu einer Lösung von Methyl chinolin-7-carboxylat (20.1 g; 1 Äquiv.) in 200 ml Methanol wurde zunächst BF₃-Etherat (30.5 g; 2 Äquiv.) getropft. Anschließend wurde portionsweise Natriumcyanoborhydrid (13.5 g; 2 Äquiv.) zugegeben. Das erhaltene Reaktionsgemisch wurde 20 Minuten bei Raumtemperatur gerührt und über Nacht zum Rückfluß (70-80°C) erhitzt. Nach vollständigem Umsatz wurde das Reaktionsgemisch auf Raumtemperatur abgekühlt, das Lösungsmittel unter vermindertem Druck entfernt, der Rückstand in 300 ml Wasser aufgenommen und 3x mit EtOAc extrahiert. Die vereinigten organischen Phasen wurden über Natriumsulfat getrocknet, unter vermindertem Druck konzentriert und das Rohprodukt aus Isopropanol umkristallisiert. Methyl 1,2,3,4-tetrahydrochinolin-7-carboxylat (14 g; 67.3%) wurde in Form eines farblosen Feststoffes erhalten.

### Baustein A-04: Methyl 1,2,3,4-tetrahydroisochinolin-7-carboxylat

### Stufe 1: 7-Nitro-1,2,3,4-tetrahydroisochinolin Hydrochlorid

Zu 185 ml Schwefelsäure wurde innerhalb von 90 Minuten, bei 0°C, 1,2,3,4-Tetrahydroisochinolin (50 g; 1 Äquiv.) gegeben und das Reaktionsgemisch für 30 Minuten bei 0°C gerührt. Anschließend wurde Kaliumnitrat (40.7 g; 1.2 Äquiv.) portionsweise zugegeben und für 15 Stunden bei Raumtemperatur gerührt. Nach vollständigem Umsatz wurde die Reaktionsmischung auf 500 g Eis geschüttet und mit Ammoniaklösung auf pH 8-9 eingestellt. Danach wurde 3x mit Chloroform extrahiert, die vereinigten organischen Phasen über Natriumsulfat getrocknet und unter vermindertem Druck konzentriert. Der Rückstand wurde in IPA (500 ml) aufgenommen, auf 0°C abgekühlt und mit Salzsäure (2 Äquiv.) versetzt. Der ausgefallene Feststoff wurde abfiltriert und aus Methanol umkristallisiert. 7-Nitro-1,2,3,4-tetrahydroisochinolin (35 g; 52.3%) wurde in Form eines weißen Feststoffes erhalten.

### Stufe 2: 1,2,3,4-Tetrahydroisochinolin-7-amin

Zu einer Lösung von 7-Nitro-1,2,3,4-tetrahydroisochinolin Hydrochlorid (19 g; 1 Äquiv.) in 300 ml Methanol wurde 10% Pd/C (1.9 g) gegeben und das Reaktionsgemisch für 2 Stunden bei 60 PSI hydriert. Nach vollständigem Umsatz wurde über Celite filtriert, der Filterkuchen 4x mit Methanol nachgewaschen, das Filtrat unter vermindertem Druck konzentriert und der Rückstand in 100 ml Wasser aufgenommen. Die wässrige Lösung wurde mit Kaliumhydroxidlösung auf einen pH-Wert von 8-9 eingestellt und 3x mit Chloroform extrahiert. Die vereinigten organischen Phasen wurden über Natriumsulfat getrocknet und unter vermindertem Druck reduziert. 1,2,3,4-Tetrahydroisochinolin-7-amin (9 g; 69.2%) wurde in Form eines blassbraunen Feststoffes erhalten.

### Stufe 3: 1,2,3,4-Tetrahydroisochinolin-7-carbonitril (AL/7103/S04)

Zu einer Lösung von 1,2,3,4-Tetrahydroisochinolin-7-amin (15.3 g; 1 Äquiv.) in 40 ml Wasser wurde bei 0°C Salzsäure (30.6 ml; 2 Äquiv.) gegeben und das Reaktionsgemisch für 10 Minuten bei -5°C gerührt. Natriumnitratlösung, (7.13 g Natriumnitrat in 35 ml Wasser) wurde innerhalb von 30 Minuten bei 0°C zugetropft und das erhaltene Reaktionsgemisch für 30 Minuten bei 0°C gerührt. Abschließend wurde Harnstoff (1.86 g; 0.3 Äquiv.) zugegeben. Eine Mischung aus Natronlauge (10.3 g NaOH in 70 ml H₂O), Kaliumcyanidlösung (33.5 g (5 Äquiv.) KCN in 50 ml H₂O) und 76.5 ml Benzol wurde auf 0°C abgekühlt, langsam mit einer Nickelsulfatlösung (32.6 g (1.2 Äquiv.) NiSO₄ * 6 H₂O in 50ml H₂O) versetzt und für 30 min bei 0°C gerührt. Die Diazoniumlösung wurde langsam bei 0°C zugetropft, das erhaltene Reaktionsgemisch zunächst für 2 h bei Raumtemperatur und anschließend für 1 h bei 50°C gerührt. Nach vollständigem Umsatz wurde auf 0°C abgekühlt, mit Natronlauge auf einen pH-Wert von 8-9 eingestellt und über Celite filtriert und der Filterkuchen mit Dichlormethan nachgewaschen. Die wässrige Lösung wurde 3x mit Dichlormethan extrahiert. Die vereinigten organischen Phasen wurden über Natriumsulfat getrocknet, und unter vermindertem Druck konzentriert. Das Rohprodukt wurde säulenchromatographisch (Kieselgel, 10% Methanol / Chloroform) gereinigt. 1,2,3,4-Tetrahydroisochinolin-7-carbonitril (4 g; 24.5 %) wurde in Form eines Feststoffes erhalten.

### Stufe 4: Methyl 1,2,3,4-Tetrahydroisochinolin-7-carboxylat

Zu einer Lösung von 1,2,3,4-Tetrahydroisochinolin-7-carbonitril (12 g; 1 Äquiv.) wurden 240 ml methanolische Salzsäure (Chlorwasserstoff wurde 4 Stunden über Methanol geleitet) gegeben. Das erhaltene Reaktionsgemisch wurde für 18 Stunden zum Rückfluß erhitzt (80°C). Nach vollständigem Umsatz wurde das Methanol abdestilliert, der Rückstand in 100 ml Wasser aufgenommen und mit Natriumcarbonat auf einen pH-Wert von 8-9 eingestellt. Die Reaktionsmischung wurde 3x mit Dichlormethan extrahiert, die vereinigten organischen Phasen über Natriumsulfat getrocknet und unter vermindertem Druck konzentriert. Das Rohprodukt wurde säulenchromatographisch (Kieselgel, 50% Ethanol/Heptan) gereinigt. Methyl 1,2,3,4-tetrahydroisochinolin-7-carboxylat (8 g; 55%) wurde in Form eines bräunlichen Öls erhalten.

### Baustein A-05: Methyl 2-(1,2,3,4-tehahydrochinolin-7-yl)acetat

### Stufe 1: 1-(3,4-Dihydrochinolin-1(2H)-yl)ethanon

Zu einer Lösung von 1,2,3,4-Tetrahydrochinolin (69 g; (1 Äquiv.) in 690 ml Dichlormethan wurde, bei 0°C, langsam Essigsäureanhydrid (55.5 g; 1.05 Äquiv.) zugetropft. Die Reaktionsmischung wurde für 30 Minuten bei Raumtemperatur gerührt und nach vollständigem Umsatz mit kaltem Wasser und Natriumhydrogencarbonatlösung gewaschen. Die organische Phase wurde über Natriumsulfat getrocknet und unter vermindertem Druck konzentriert. 1-(3,4-Dihydrochinolin-1(2H)-yl)ethanon (85 g; 94.4%) wurde in Form eines farblosen Öls erhalten.

### Stufe 2: 1,1'-(3,4-Dihydrochinolin-1,6(2H)-diyl)diethanon und 1,1'-(3,4-Dihydrochinolin-1,7(2H)-diyl)diethanon

Zu Aluminiumchlorid (194.4 g; 3 Äquiv.) wurde 1-(3,4-Dihydrochinolin-1 (2H)-yl)ethanon (85 g; 1 Äquiv.) getropft, für 30 Minuten gerührt und anschließend innerhalb von 30 Minuten mit destilliertem Acetylchlorid (76.2 g; 2 Äquiv.) versetzt. Die erhaltene Reaktionsmischung wurde für 10 Stunden zum Rückfluß erhitzt (60°C), anschließend bei 0°C mit kaltem Wasser gequencht und mit Essigester extrahiert. Die vereinigten organischen Phasen wurden über Natriumsulfat getrocknet und unter vermindertem Druck konzentriert. Das Rohprodukt wurde säulenchromatografisch (Kieselgel, 75% Essigester/Hexan) gereinigt. Das gewünschte Regioisomerengemisch (54 g; 60.9%) wurde in Form eines gelben Öls erhalten.

### Stufe 3: 1-(1,2,3,4-Tetrahydrochinolin-6-yl)ethanon & 1-(1,2,3,4-Tetrahydrochinolin-7-yl)ethanon

Zu einer Lösung des soeben erhaltenen Regioisomerengemisches (Stufe 2; 54 g; 1 Äquiv.) in 540 ml Methanol wurden innerhalb von 15 Minuten 270 ml konz. Salzsäure getropft. Das Reaktionsgemisch wurde 6 Stunden zum Rückfluß erhitzt. Nach vollständigem Umsatz wurde das Methanol abdestilliert, der Rückstand in 300 ml Wasser gelöst, diese Lösung bei 0°C mit Natriumcarbonat basisch gestellt und abschließend 2x mit 200 ml Essigester extrahiert. Die vereinigten organischen Phasen wurden über Natriumsulfat getrocknet und unter vermindertem Druck konzentriert. Nach säulenchromatographischer Aufreinigung (Kieselgel, 8% Essigester/Hexan) wurden 18g des gewünschten 1-(1,2,3,4-Tetrahydrochinolin-7-yl)ethanon in Form eines blassgelben Feststoffes erhalten.

### Stufe 4: 1-Morpholino-2-(1,2,3,4-tetrahydrochinolin-7-yl)ethanthion

Zu einer Lösung von 1-(1,2,3,4-Tetrahydrochinolin-7-yl)ethanon (12 g; 1 Äquiv.) in Morpholin (16.1 g; 2.5 Äquiv.) wurde portionsweise Schwefel (4.7 g; 0.25 Äquiv.) gegeben und die Mischung für 12 Stunden zum Rückfluß erhitzt (160°C). Nach vollständigem Umsatz wurde das Lösungsmittel unter vermindertem Druck entfernt und das Rohprodukt säulenchromatographisch (Kieselgel, 40% EtOAc/Hexan) aufgereinigt. 1-Morpholino-2-(1,2,3,4-tetrahydrochinolin-7-yl)Ethanthion (11 g; 58.8%) wurde in Form eines gelben Öls erhalten.

### Stufe 5: 2-(1,2,3,4-Tetrahydrochinolin-7-yl)essigsäure

Zu einer Lösung von 1-Morpholino-2-(1,2,3,4-tetrahydrochinolin-7-yl)-ethanthion (11 g; 1 Äquiv.) in 55 ml Ethanol wurden 110 ml einer 20%igen Bariumhydroxidlösung getropft und das erhaltene Reaktionsgemisch für 10 Stunden zum Rückfluß erhitzt. Nach vollständigem Umsatz wurde das Lösungsmittel unter vermindertem Druck entfernt, der Rückstand in 100 ml Wasser aufgenommen und auf 80°C erhitzt. Die Reaktionsmischung wurde nun vorsichtig mit Trockeneis neutralisiert, über Celite filtriert und unter vermindertem Druck konzentriert. Das erhaltene Rohprodukt (14 g) wurde ohne weitere Aufreinigung in der nächsten Stufe eingesetzt.

### Stufe 6: Methyl 2-(1,2,3,4-tetrahydrochinolin-7-yl)acetat

Zu einer Lösung von rohem 2-(1,2,3,4-Tetrahydrochinolin-7-yl)essigsäure (Stufe 5; 14 g; 1 Äquiv.) in 140 ml Methanol wurde bei 0°C innerhalb von 5-10 Minuten Thionylchlorid (13 g; 1.5 Äquiv.) getropft und das erhaltene Reaktionsgemisch anschließend für 6 Stunden zum Rückfluß (70°C) erhitzt. Nach vollständigem Umsatz wurde die Reaktionslösung unter vermindertem Druck konzentriert, der Rückstand in 150 ml Wasser aufgenommen, mit Natriumcarbonat basisch gestellt (pH 7-8) und abschließend 2x mit jeweils 150 ml Chloroform extrahiert. Die vereinigten organischen Phasen wurden über Natriumsulfat getrocknet und unter vermindertem Druck konzentriert. Das Rohprodukt wurde säulenchromatographisch (Kieselgel, 10-12% Essigester/ Hexan) aufgereinigt. Methyl 2-(1,2,3,4-tetrahydrochinolin-7-yl)acetat (4 g) wurde in Form eines farblosen Öls erhalten.

### Baustein A-06: Ethyl 4,5,6,7-tetrahydroisoxazolo[4,5-c]pyridin-3-carboxylat

### Stufe 1: tert-Butyl 4-(pyrrolidin-1-yl)-5,6-dihydropyridin-1(2H)-carboxylat

Zu einer Lösung aus N-Boc-4-piperidon (5 g, 25.12 mmol) und Pyrrolidin (1.96 g, 27.63 mmol) in Toluol (100 ml) wurden katalytische Mengen p-Toluolsulfonsäure (0.47g, 0.27 mmol) gegeben und mit Wasserabscheider 2 Stunden unter Rückfluß gerührt. Die entstandene Lösung wird im Vakuum bis zur Trockne eingeengt und der erhaltene Rückstand direkt in die nachfolgende Stufe eingesetzt.

### Stufe 2: 5-tert-Butyl 3-ethyl 7a-(pyrrolidin-1-yl)-3a,4,7,7a-tetrahydroisoxazolo[4,5-c]pyridin-3,5(6H)-dicarboxylat

Das Rohprodukt der vorherigen Stufe wurde in Dichlormethan (50 ml) gelöst und unter starkem Rühren bei RT (ggf. kühlen) langsam mit einer Lösung aus 2-Chlorhydroxyiminoessigsäure-ethylester (5.3 g, 35.17 mmol) in Dichlormethan (50 ml) versetzt. Anschließend wurde langsam bei 0°C Triethylamin (4.8 ml, 35.17 mmol) hinzugetropft. Das Reaktionsgemisch wurde 16 Stunden bei Raumtemperatur gerührt und durch Zugabe von 10%-iger Zitronensäure beendet. Die Mischung wurde mit Dichlormethan extrahiert und die organischen Phasen mit NaHCO₃-Lösung und NaCl-Lösung gewaschen, über Na₂SO₄ getrocknet und im Vakuum eingeengt. Die Reinigung des Rohproduktes erfolgte durch Säulenchromatographie (Kieselgel 8% Aceton in Hexan).
Ausbeute: 60%

### Stufe 3. Ethyl 4,5,6,7-tetrahydroisoxazolo[4,5-c]pyridin-3-carboxylat

Zu einer Lösung aus 5-tert-Butyl 3-ethyl 7a-(pyrrolidin-1-yl)-3a,4,7,7a-tetrahydroisoxazolo[4,5-c]pyridin-3,5(6H)-dicarboxylat (5.5 g, 14.98 mmol) in DCM (100 ml) wurde bei 0 °C Trifluoressigsäure (6.67 ml, 89.9 mmol) gegeben und die Mischung 16 Stunden unter Rückfluß gerührt. Danach wurde auf 0°C gekühlt und NaHCO₃-Lösung zugegeben. Die Phasen wurden getrennt und die wässrige Phase mit Dichlormethan extrahiert. Die vereinigten organischen Phasen wurden über Na₂SO₄ getrocknet, filtriert und im Vakuum eingeengt. Die Reinigung des Rohproduktes erfolgte durch Säulenchromatographie (Kieselgel, 16% Methanol in Ethylacetat).
Ausbeute: 62%

[Alternativ kann Ethyl 4,5,6,7-tetrahydroisoxazolo[4,5-c]pyridin-3-carboxylat A-06 wie in WO2006105945 beschrieben synthetisiert werden.]

### Baustein A-08: Methyl 2-(3,4-dihydro-2H-benzo[b][1,4]oxazin-6-yl)acetat

### Stufe-1: Methyl 2-(3-oxo-3,4-dihydro-2H-benzo[b][1,4]oxazin-6-yl)acetat

Chloracetylchlorid (1 Äquiv.) wurde bei Raumtemperatur zu einer Mischung von Methyl-(3-amino-4-hydroxy phenyl)acetat (5.5 mmmol) und Natriumhydrogencarbonat (1.1 Äquiv.) in Ethylacetat (5 ml) / Wasser (5 ml) gegeben. Die Reaktionsmischung wurde 1 h gerührt, dann wurden die Phasen getrennt und die organische Phase wurde mit Wasser und gesättigter Natriumchlorid-Lösung gewaschen, über Natriumsulfat getrocknet und unter Vakuum eingeengt. Das Rohprodukt wurde in DMF (11 ml) gelöst, aktiviertes Kaliumcarbonat wurde zugegeben und das Gemisch über Nacht bei Raumtemperatur gerührt. Anschließend wurde die Reaktionsmischung mit Wasser verdünnt und mit Ethylacetat extrahiert. Die organische Phase wurde wiederum mit Wasser und gesättigter Natriumchlorid-Lösung gewaschen, über Natriumsulfat getrocknet und unter Vakuum eingeengt. Das Rohprodukt wurde säulenchromatographisch (Kieselgel, Ethylacetat / Hexan) gereinigt.
Ausbeute: 90%

### Stufe-2: Methyl 2-(3,4-dihydro-2H-benzo[b][1,4]oxazin-6-yl)acetat

Zu einer Lösung des Produktes aus Stufe-1 (2.7 mmol) in trockenem THF (14 ml) wurde bei Raumtemperatur Borandimethylsulfid-Lösung (0.3 ml, 94% in THF) gegeben und die Mischung wurde 2 h refluxiert. Es wurde mit Methanol (1 ml) hydrolysiert und nochmal 15 min refluxiert. Dann wurde die Reaktionsmischung auf Raumtemperatur gekühlt und unter Vakuum eingeengt. Das Rohprodukt wurde säulenchromatographisch (Kieselgel, Ethylacetat / Hexan) gereinigt.
Ausbeute: 62%

### Baustein A-09: Ethyl 5,6,7,8-tetrahydroimidazo[1,2-a]pyrazin-2-carboxylat Hydrochlorid kommerziell erhältlich bei z.B. Activate Scientific.

### Baustein A-10: Methyl 2-(5,6,7,8-tetrahydro-1,8-naphthyridin-2-yl)acetat Hydrochlorid

tert-Butyl-7-(2-methoxy-2-oxoethyl)-3,4-dihydro-1,8-naphthyridin-1(2H)-carboxylat (1 Äq) (CAS [925889-81-2], kommerziell erhältlich bei z.B. Fluorochem) wurde in Methanol (1,6 ml) gelöst, Chlorwasserstoff in Methanol (1,25 M, 5 Äquiv.) zugegeben und das Gemisch auf Siedetemperatur erhitzt. Nach 1 h wurde das Lösungsmittel im Vakuum entfernt und der so erhaltene Rückstand direkt in die nachfolgende Stufe eingesetzt.
Ausbeute: >99%

### 2) Synthese der Säurechloride (B) und Sulfonsäurechloride (C)

Die erfindungsgemäß eingesetzten Sulfonsäurechloride sind käuflich erhältlich oder können nach üblichen, dem Fachmann bekannten Methoden hergestellt werden. Bei den nachfolgend in den eckigen Klammern angegebenen Nummern handelt es sich um die CAS-Nummern.

**Säurechlorid B-01:** 2-Chlorbenzoyl chlorid [609-65-4] kommerziell erhältlich bei z.B. Aldrich.

### Sulfonylchlorid C-01: 4-Methoxy-2,6-dimethylphenyl-1-sulfonyl chlorid

Zu einer Lösung 3,5-Dimethylanisol (1.632 g, 11.982 mmol) in Dichlormethan (15 ml) wurde Chlorsulfonsäure (1.83 ml, 2.3 Äquiv.) in Dichlormethan (10 ml) über 20 min bei 0 °C hinzugetropft. Anschließend wurde das Reaktionsgemisch 10 min bei Raumtemperatur gerührt. Das Reaktionsgemisch wurde auf Eiswasser (3 ml, 5 Äquiv. bezüglich Chlorsulfonsäure) gegeben und die wässrige Phase mit Dichlormethan (3 x 100 ml) extrahiert. Die organische Phase wurde getrocknet (Na₂SO₄) und unter Vakuum eingeengt.
Ausbeute: 2.6 g (92%)

**Sulfonylchlorid C-02: 4-Chlor-2,5-dimethylphenyl-1-sulfonyl chlorid** [88-49-3] kommerziell erhältlich bei z.B. ABCR. **Sulfonylchlorid C-03: 2-(Trifluormethyl)phenyl-1-sulfonyl chlorid** [776-04-5] kommerziell erhältlich bei z.B. Aldrich. **Sulfonylchlorid C-04: Naphthalin-1-sulfonyl chlorid** [85-46-1] kommerziell erhältlich bei z.B. ACROS. **Sulfonylchlorid C-05: 2-Chlor-6-methylphenyl-1-sulfonyl chlorid** [25300-37-2] kommerziell erhältlich bei z.B. Fluorochem. **Sulfonylchlorid C-06: 4-Methoxy-2,3,6-trimethylphenyl-1-sulfonyl chlorid** [80745-07-9] kommerziell erhältlich bei z.B. ABCR. **Sulfonylchlorid C-07:** 2-Chlor-**4-(trifluormethoxy)benzol-1-sulfonyl chlorid** kommerziell erhältlich bei z.B. ABCR **Sulfonylchlorid C-08: 2,6-Dichlor-3-methylphenyl-1-sulfonyl chlorid** kommerziell erhältlich bei z.B. Akos.

### 3) Synthese der acylierten bzw. sulfonylierten Aminosäureester D:

### Allgemeine Methode zur Synthese der sulfonylierten Aminosäureester D

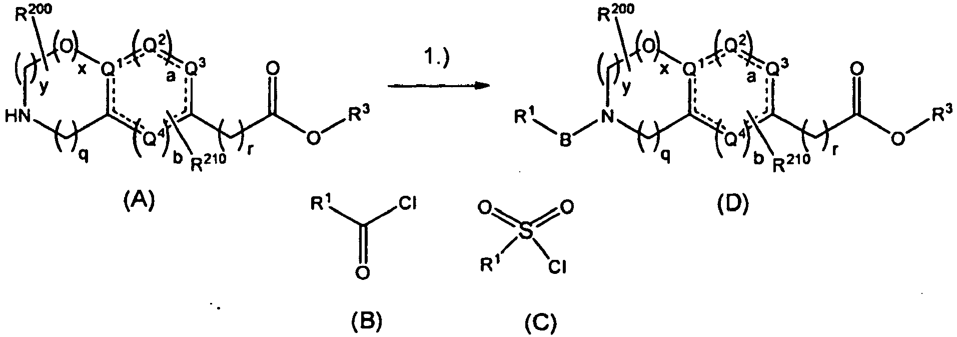

### Synthese der sulfonylierten Aminosäureester D

Allgemeine Arbeitsvorschrift AAV I - Sulfonylierung: Zu einer Lösung des Aminosäureester A (1.2 Äquiv.) und Diisopropylethylamin (1 bis 3 Äquiv.) in Dichlormethan wurde bei Raumtemperatur eine Lösung des Sulfonylchlorids C (1 Äquiv.) in Dichlormethan gegeben. Das Reaktionsgemisch wurde für 12 h bei Raumtemperatur gerührt, anschließend 3x mit einer 1 N HCl-Lösung gewaschen, über Natriumsulfat getrocknet und unter vermindertem Druck konzentriert. Nach säulenchromatographischer Aufreinigung (Silika; Ethylacetat / Hexan) wurde das gewünschte Produkt erhalten.

Allgemeine Arbeitsvorschrift AAV II - Acylierung: Zu einer Lösung des Aminosäureesters A (1 Äquiv.) und Diisopropylethylamin (1 bis 3 Äquiv.) in Dichlormethan wurde bei Raumtemperatur eine Lösung des Säurechlorids B (2 Äquiv.) gegeben. Das Reaktionsgemisch wurde für 12 h bei Raumtemperatur gerührt, mit N,N-Dimethylethan-1,2-diamin (1 bis 3 Äquiv.) versetzt und für 1 h bei Raumtemperatur gerührt. Anschließend wird 3x mit einer 1N HCl-Lösung gewaschen, über Natriumsulfat getrocknet und unter vermindertem Druck konzentriert.

Nach säulenchromatographischer Aufreinigung (Silika; Ethylacetat / Hexan) wurde das gewünschte Produkt erhalten.

Allgemeine Arbeitsvorschrift AAV III- Sulfonylierung: Aminosäureester A (1 Äquiv.) wurde in Dichlormethan und Triethylamin (1 - 2 Äquiv.) gelöst und mit einem Eisbad gekühlt. Das Sulfonylchlorid B (1 - 2 Äquiv.), gelöst in Dichlormethan, wurde bei 0°C langsam zugegeben. Das Kühlbad wurde entfernt und das Reaktionsgemisch 15 h gerührt. Anschließend wurde mit gesättigter Natriumhydrogencarbonatlösung versetzt und die Phasen getrennt. Die wässrige Phase wurde mit Dichlormethan extrahiert, die vereinigten organischen Phasen mit Wasser oder HCl-Lösung (0,05 mol/l) und gesättigter Natriumchlorid-Lösung gewaschen, über Natriumsulfat getrocknet und unter Vakuum eingeengt. Das Rohprodukt wurde säulenchromatographisch (Kieselgel) aufgereinigt.

Allgemeine Arbeitsvorschrift AAV IV - Sulfonylierung: Zu einer eisgekühlten Lösung des Aminosäureesters A (1 Äquiv.) in DCM wurde Triethylamin (1 - 3 Äquiv.), ggf. DMAP (cat.), und anschließend Sulfonylchlorid B (1.2 - 2 Äquiv.), ggf. gelöst in Dichlormethan, hinzugegeben. Das Reaktionsgemisch wurde 1 - 15 h bei Raumtemperatur gerührt, mit Wasser oder gesättigter Natriumhydrogencarbonatlösung verdünnt und mit Dichlormethan extrahiert. Die vereinigten organischen Phasen wurden mit Wasser oder Salzsäure (0,05 mol/l) und mit gesättigter Natriumchlorid-Lösung gewaschen, über Natriumsulfat getrocknet und unter Vakuum eingeengt. Das Rohprodukt wurde säulenchromatographisch (Kieselgel) gereinigt.

Allgemeine Arbeitsvorschrift AAV V- Acylierung: Aminosäureester A (1 Äquiv.) wurde in Dichlormethan und Triethylamin (1.5 Äquiv.) gelöst, mit einem Eisbad gekühlt und 15 min gerührt. Das Carbonsäurechlorid B (1 Äquiv.), gelöst in Dichlormethan, wurde bei 0°C langsam zugegeben. Das Kühlbad wurde entfernt und das Reaktionsgemisch 15 h bei RT gerührt. Anschließend wurde mit gesättigter Natriumhydrogencarbonat-lösung versetzt und die Phasen getrennt. Die wässrige Phase wurde mit Dichlormethan extrahiert, die vereinigten organischen Phasen mit gesättigter Natriumchlorid-Lösung gewaschen, über Natriumsulfat getrocknet und unter Vakuum eingeengt. Das Rohprodukt wurde säulenchromatographisch (Kieselgel) aufgereinigt.

Allgemeine Arbeitsvorschrift AAV Va- Acylierung: Aminosäureester **A** (1 Äquiv.) wurde in Dichlormethan und Triethylamin (2 Äquiv.) gelöst und mit einem Eisbad gekühlt. Das Carbonsäurechlorid **B** (1,2 Äquiv.), gelöst in Dichlormethan, wurde bei 0°C langsam zugegeben. Das Kühlbad wurde entfernt und das Reaktionsgemisch 1 h bei RT gerührt. Anschließend wurde Pyridin katalytisch zugegeben, 3 h auf Siedetemperatur erhitzt und 48 h bei RT gerührt. Pyridin (ca. 1 ml / mmol) wurde zugegeben und 6 h auf Siedetemperatur erhitzt. Anschließend wurde die Reaktionsmischung mit Kupfersulfatlösung (aq) (20 ml) und ges. NaCl-Lösung (20 ml) gewaschen, über Natriumsulfat getrocknet und im Vakuum eingeengt. Das so erhaltene Rohprodukt wurde säulenchromatographisch (Kieselgel) aufgereinigt.

**Tabelle 1: Synthese der acylierten bzw. sulfonylierten Aminosäureester D**

| Ester Nr. | Struktur | Name | Aminosäureester (A) | Carbonsäurechlorid (B) bzw. Sulfonsäurechlorid (C) | Synthese nach: | Ausbeute |
|---|---|---|---|---|---|---|
| D-01 | 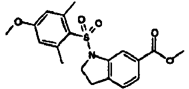 | Methyl 1-(4-methoxy-2,6-dimethylphenylsulfonyl)indolin -6-carboxylat (D-01) | Methyl indolin-6-carboxylat (A-01) | 4-Methoxy-2,6-dimethylphenyl-1-sulfonyl chlorid (C-01) | AAV I | 99% (21 mmol) |
| D-02 | 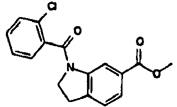 | Methyl 1-(2-chlorbenzoyl)indolin-6-carboxylat (D-02) | Methyl indolin-6-carboxylat (A-01) | 2-Chlorbenzoyl chlorid (8-01) | AAV II | 71% (20 mmol) |
| D-03 | 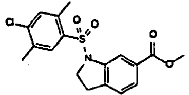 | Methyl 1-(4-chlor-2,5-dimethylphenylsulfonyl)indolin -6-carboxylat (D-03) | Indolin-6-carbonsäure (A-01) | 4-Chlor-2,5-dimethylphenyl-1-sulfonyl chlorid (C-02) | AAV I | 94% (17.6 mmol) |
| D-04 | 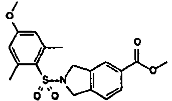 | Methyl 2-(4-methoxy-2,6-dimethylphenylsulfonyl)isoindo lin-5-carboxylat (D-04) | Methyl isoindolin-5-carboxylat (A-02) | 4-Methoxy-2,6-dimethylphenyl-1-sulfonyl chlorid (C-01) | AAV I | 78% (16.6 mmol) |
| D-05 | 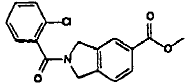 | Methyl 2-(2-chlorbenzoyl)isoindolin-5-carboxylat (D-05) | Methyl isoindolin-5-carboxylat (A-02) | 2-Chlorbenzoyl chlorid (B-01) | AAV II | 68% (16 mmol) |
| D-06 | 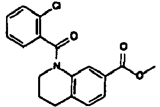 | Methyl 1-(2-chlordenzoyl)-1,2,3,4-tetrahydrochinolin-7-carboxylat (D-06) | Methyl 1,2,3,4-tetrahydrochinolin-7-carboxylat (A-03) | 2-Chlorbenzoyl chlorid (B-01) | AAV II | 62% (13.6 mmol) |
| D-07 | 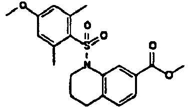 | Methyl 1-(4-methoxy-2,6-dimethylphenylsulfonyl)-1,2,3,4-tetrahydrochinolin-7-carboxylat (D-07) | Methyl 1,2,3,4-tetrahydrochinolin-7-carboxylat (A-03) | 4-Methoxy-2,6-dimethylphenyl-1-sulfonyl chlorid (C-01) | AAV I | 72% (15.3 mmol) |
| D-08 | 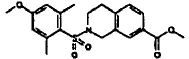 | Methyl 2-(4-methoxy-2,6-dimethylphenylsulfonyl)-1,2,3,4-tetrahydroisochinolin-7-carboxylat (D-08) | Methyl 1,2,3,4-tetrahydroisochinolin-7-carboxylat (A-04) | 4-Methoxy-2,6-dimethylphenyl-1-sulfonyl chlorid (C-01) | AAV I | 64% (11 mmol) |
| D-09 | 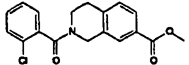 | Methyl 2-(2-chlorbenzoyl)-1,2,3,4-tetrahydroisochinolin-7-carboxylat (D-09) | Methyl 1,2,3,4-tetrahydroisachinolin-7-carboxylat (A-04) | 2-Chlorbenzoyl chlorid (B-01) | AAV II | 89% (18.6 mmol) |
| D-10 | 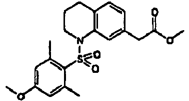 | Methyl 2-(1-(4-methoxy-2,6-dimethylphenylsulfonyl)-1,2,3,4-tetrahydrochinolin-7-yl)acetat (D-10) | Methyl 2-(1,2,3,4-tetrahydrochinolin-7-yl)acetat (A-05) | 4-Methoxy-2,6-dimethylphenyl-1-sulfonyl chlorid (C-01) | AAV I | 40% (8.5 mmol) |
| D-11 | 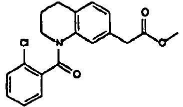 | Methyl 2-(1-(2-chlorbenzoyl)-1,2,3,4-tetrahydrochinolin-7-yl)acetat (D-11) | Methyl 2-(1,2,3,4-tetrahydrochinolin-7-yl)acetat (A-05) | 2-Chlorbenzoyl chlorid (B-01) | AAV II | 100% (19.5 mmol) |
| D-12 | 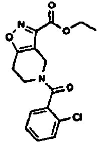 | Ethyl 5-(2-chlorbenzoyl)-4,5,6,7-tetrahydroisoxazolo[4,5-c]pyridin-3-carboxytat (D-12) | Ethyl 4,5,6,7-tetrahydroisoxazolo[4 ,5-c]pyridin-3-carboxylat (A-06) | 2-Chlorbenzoyl chlorid (B-01) | ADV V | 94% (0.40 g) |
| D-15 | 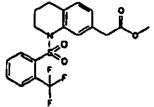 | Methyl 2-(1-(2-(trifluormethyl)phenylsulfonyl)-1,2,3,4-tetrahydrochinolin-7-yl)acetat (D-15) | Methyl 2-(1,2,3,4-tetrahydrochinolin-7-yl)acetat (A-05) | 2-(Trifluofmethyl)phenyl-1 - sulfonyl chlorid (C-03) | AAV III | 34% (0.34 g) |
| D-16 | 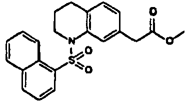 | Methyl 2-(1-(naphthalin-1-ylsulfonyl)-1,2,3,4-tetrahydrochinolin-7-yl)acetat (D-16) | Methyl 2-(1,2,3,4-tetrahydrochinolin-7-yl)acetat (A-05) | Naphthalin-1-sulfonyl chlorid (C-04) | AAV III | >99% (1.03 g) |
| D-17 | 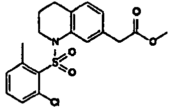 | Methyl 2-(1-(2-chlor-6-methylphenylsulfonyl)-1,2,3,4-tetrahydrochinolin-7-yl)acetat (D-17) | Methyl 2-(1,2,3,4-tetrahydrochinolin-7-yl)acetat (A-05) | 2-Chlor-6-methylphenyl-1-sulfonyl chlorid (C-05) | AAV III | 53% (0.51 g) |
| D-18 | 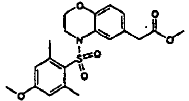 | Methyl 2-(4-(4-methoxy-2,6-dimethylphenylsulfonyl)-3,4-dihydro-2H-benzo[b][1,4]oxazin-6-yl)acetat(D-18) | Methyl 2-(3,4-dihydro-2H-benzo[b][1,4]oxazin-6-yl)acetat (A-08) | 4-Methoxy-2,6-dimethylphenyl-1-sulfonyl chlorid (C-01) | AAV IV | 48% |
| D-20 | 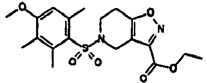 | Ethyl 5-(4-methoxy-2,3,6-trimethylphenylsulfonyl)-4,5,6,7-tetrahydroisoxazolo[4,5-c]pyridin-3-carboxylat (D-20) | Ethyl 4,5,6,7-tetrahydroisoxazolo[4 ,5-c]pyridin-3-carboxylat (A-06) | 4-Methoxy-2,3,6-trimethylphenyl-1-sulfonyl chlorid (C-06) | AAV IV | 47% |
| D-21 | 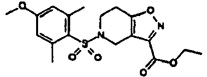 | Ethyl 5-(4-methoxy-2,6-dimethylphenylsulfonyl)-4,5,6,7-tetrahydroisoxazolo[4,5-c]pyridin-3-carboxylat (D-21) | Ethyl 4,5,6,7-tetrahydroisoxazolo[4 ,5-c]pyridin-3-carboxylat (A-06) | 4-Methoxy-2,6-dimethylphenyl-1-sulfonyl chlorid (C-01) | AAV IV | 92% |
| D-22 | 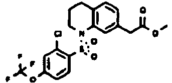 | Methyl 2-(1-(2-chlor-4-(trifluormethoxy)phenylsulfonyl )-1,2,3,4-tetrahydrochinolin-7-yl)acetat (D-22) | Methyl 2-(1,2,3,4-tetrahydrochinolin-7-yl)acetat (A-05) | 2-Chlor-4-(trifluormethoxy)benzol-1-sulfonyl chlorid (C-07) | AAV III | 78% |
| D-27 | 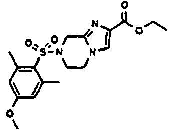 | Ethyl 7-(4-methoxy-2,6-dimethylphenylsulfonyl)-5,6,7,8-tetrahydroimidazo[1,2-a]pyrazin-2-carboxylet (D-27) | Ethyl 5,6,7,8-tetrahydroimidazo[1, 2-a]pyrazin-2-carboxylat Hydrochlorid (A-09) | 4-Methoxy-2,6-dimethylphenyl-1-sulfonyl chlorid (C-01) | AAV V | 65% |
| D-28 | 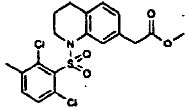 | Methyl 2-(1-(2,6-dichlor-3-methylphenylsulfonylrt,2,3,4-tetrahydrochinolin-7-yl)acetat (D-28) | Methyl 2-(1,2,3,4-tetrahydrochinolin-7-yl)acetat (A-05) | 2,6-Dichlor-3-methylphenyl-1-sulfonyl | AAV V | 26% (0,11 g) |
| D-29 | 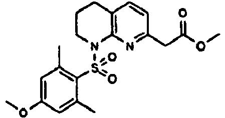 | Methyl 2-(8-(4-methoxy-2,6-dimethylphenylsulfonyl)-5,6,7,8-tetrahydro-1,8-naphthyridin-2-yl)acetat (D-29) | Methyl 2-(5,6,7,8-tetrahydro-1,8-naphthyridin-2-yl)acetat Hydrochlorid (A-10) | 4-Methoxy-2,6-dimethylphenyl-1-sulfonyl chlorid (C-01) | AAV Va | 15% (0,1 g) |

### 4) Synthese der acylierten bzw. sulfonylierten Aminosäuren E

### Allgemeine Methode zur Synthese der acylierten bzw. sulfonylierten Aminosäuren E

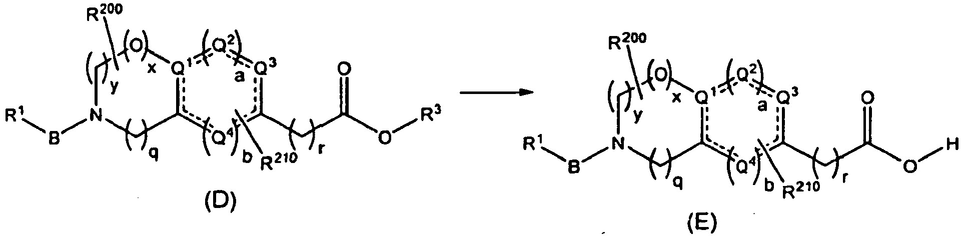

Allgemeine Arbeitsvorschrift AAV VI: Eine Lösung von Ester D (1 Äquiv.) in Methanol / Wasser (1.5 / 1) wurde mit Lithiumhydroxid (2 bis 5 Äquiv.) versetzt und für 12 bis 24 Stunden zum Rückfluß erhitzt. Anschließend wurde dieses Reaktionsgemisch unter vermindertem Druck konzentriert, der Rückstand in Wasser aufgenommen und die wässrige Phase mit 1 N HCl-Lösung angesäuert. Der ausgefallene Feststoff wurde abfiltriert und getrocknet.

Allgemeine Arbeitsvorschrift AAV VII: Der Carbonsäureester D (1 Äquiv.) wurde in Wasser / Methanol gelöst und mit Lithiumhydroxid Monohydrat (1,5 - 4 Äquiv.) versetzt. Das Reaktionsgemisch wurde 1 - 15 h (DC-Kontrolle) bei Raumtemperatur gerührt, dann wurde das Lösungsmittel unter Vakuum abdestilliert. Der Rückstand wurde mit Ethylacetat und mit 1 N Salzsäure oder 10%iger Citronensäure versetzt, die Phasen getrennt und die wässrige Phase mit Ethylacetat (2 - 3 x) extrahiert. Die vereinigten organischen Phasen wurden über Natriumsulfat oder Magnesiumsulfat getrocknet und unter Vakuum eingeengt.

Allgemeine Arbeitsvorschrift AAV VIII: Zu einer Lösung des Carbonsäureesters D (1 Äquiv.) in Tetrahydrofuran / Wasser (1:1) wurde Lithiumhydroxid Monohydrat (5 Äquiv.) gegeben und die Mischung 2 h gerührt. Unter Vakuum wurde das Lösungsmittel entfernt, der Rückstand wurde in verdünnter HCl-Lösung aufgenommen und mit Ethylacetat extrahiert. Die organische Phase wurde mit Wasser und gesättigter Natriumchlorid-Lösung gewaschen, über Natriumsulfat getrocknet und unter Vakuum eingeengt.

**Tabelle 2: Synthese der acylierten bzw. sulfonylierten Aminosäuren E**

| Aminosäure Nr. | Struktur | Name | Aminosäureester (D) | Synthese nach / Kommentar: | Ausbeute |
|---|---|---|---|---|---|
| E-01 | 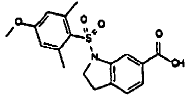 | 1-(4-Methoxy-2,6-dimethylphenylsulfonyl)indolin-6-carbonsäure (E-01) | Methyl 1-(4-methoxy-2,6-dimethylphenylsulfonyl)indolin-6-carboxylat (D-01) | AAV VI | 97% (20.5 mmol) |
| E-02 | 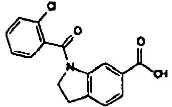 | 1-(2-Chlorbenzoyl)indolin-6-carbonsäure (E-02) | Methyl 1-(2-chlorbenzoyl)indolin-6-carboxylat (D-02) | AAV VI | 99% (22 mmol) |
| E-03 | 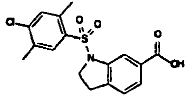 | 1-(4-Chlor-2,5-dimethylphenylsulfonyl)indofin-6-carbonsäure (E-03) | Methyl 1-(4-chlor-2,5-dimethylphenylsulfonyl)indolin-6-carboxylat (D-03) | AAV VI | 92% (17.4 mmol) |
| E-04 | 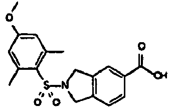 | 2-(4-Methoxy-2,6-dimethylphenylsulfonyl)isoindolin-5-carbonsäure (E-04) | Methyl 2-(4-methoxy-2,6-dimethylphenylsulfonyl)isoindolin-5-carboxylat (D-04) | AAV VI | 97% (16 mmol) |
| E-05 | 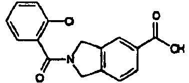 | 2-(2-Chlorbenzoyl)isoindolin-5-carbonsäure (E-05) | Methyl 2-(2-chlorbenzoyl)isoindolin-5-carboxylat (D-05) | AAV VI | >100% (>15.9 mmol) |
| E-06 | 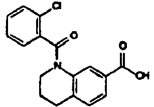 | 1-(2-Chlorbenzoyl)-1,2,3,4-tetrahydrochinolin-7-carbonsäure (E-06) | Methyl 1-(2-chlorbenzoyl)-1,2,3,4-tetrahydrochinolin-7-carboxylat (D-06) | AAV VI | >100% (>13.7 mmol) |
| E-07 | 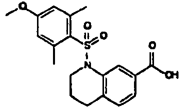 | 1-(4-Methoxy-2,6-dimethylphenylsulfonyl)-1,2,3,4-tetrahydrochinolin-7-carbonsäure (E-07) | Methyl 1-(4-methoxy-2,6-dimethylphenylsulfonyl)-1,2,3,4-tetrahydrochinolin-7-carboxylat (D-07) | AAV VI | 100% (15.4 mmol) |
| E-08 | 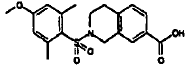 | 2-(4-Methoxy-2,6-dimethylphenylsulfonyl)-1,2,3,4-tetrahydroisochinolin-7-carbonsäure (E-08) | Methyl 2-(4-methoxy-2,6-dimethylphenylsulfonyl)-1,2,3,4-tetrahydroisochinolin-7-carboxylat (D-08) | AAV VI | 91% (10 mmol) |
| E-09 | 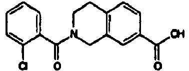 | 2-(2-Chlorbenzoyl)-1,2,3,4-tetrahydroisochinolin-7-carbonsäure (E-09) | Methyl 2-(2-chlorbenzoyl)-1,2,3,4-tetrahydroisochinolin-7-carboxylat (D-09) | AAV VI | 86% (16 mmol) |
| E-10 | 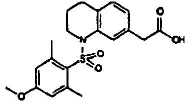 | 2-(1-(4-Methoxy-2,6-dimethylphenylsulfonyl)-1,2,3,4-tetrahydrochinolin-7-yl)essigsäure (E-10) | Methyl 2-(1-(4-methoxy-2,6-dimethylphenylsulfonyl)-1,2,3,4-tetrahydrochinolin-7-yl)acetat (D-10) | AAV VI | 83% (7 mmol) |
| E-11 | 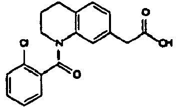 | 2-(1-(2-Chlorbenzoyl)-1,2,3,4-tetrahydrochinolin-7-yl)essigsäure (E-11) | Methyl 2-(1-(2-chlorbenzoyl)-1,2,3,4-tetrahydrochinolin-7-yl)acetat (D-11) | AAV VI | 78% (15.6 mmol) |
| E-12 | 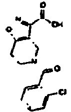 | 5-(2-Chlorbenzoyl)-4,5,6,7-tetrahydroisoxazolo[4,5-c]pyridin-3-carbonsäure (E-12) | Ethyl 5-(2-chlorbenzoyl)-4,5,6,7-tetrahydroisoxazolo[4,5-c]pyridin-3-carboxylat (D-12) | AAV VII | 90% (0.33 g) |
| E-15 | 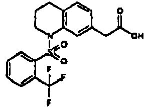 | 2-(1-(2-(Trifluormethyl)phenylsulfonyl)-1,2,3,4-tetrahydrochinolin-7-yl)essigsäure (E-15) | Methyl 2-(1-(2-(trifluormethyl)phenylsulfonyl)-1,2,3,4-tetrahydrochinolin-7-yl)acetat (D-15) | AAV VII | 85% (0.27g) |
| E-16 | 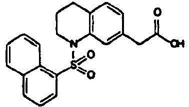 | 2-(1-(Naphthalin-1-ylsulfonyl)-1,2,3,4-tetrahydrochinolin-7-yl)essigsäure (E-16) | Methyl2-(1-(naphthalin-1-ylsulfonyl)-1,2,3,4-tetrahydrochinolin-7-yl)acetat (D-16) | AAV VII | >99% (1.00g) |
| E-17 | 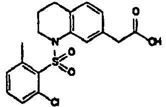 | 2-(1-(2-Chlor-6-methylphenylsulfonyl)-1,2,3,4-tetrahydrochinolin-7-yl)essigsäure (E-17) | Methyl 2-(1-(2-chlor-6-methylphenylsulfonyl)-1,2,3,4-tetrahydrochinolin-7-yl)acetat (D-17) | AAV VII | 89% (0.43 g) |
| E-18 | 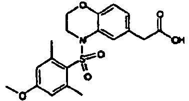 | 2-(4-(4-Methoxy-2,6-dimethylphenylsulfonyl)-3,4-dihydro-2H-benzo[b][1,4]oxazin-6-yl)essigsäure (E-18) | Methyl 2-(4-(4-methoxy-2,6-dimethylphenylsulfonyl)-3,4-dihydro-2H-benzo[b][1,4]oxazin-6-yl)acetat (D-18) | AAV VIII | 77% |
| E-20 | 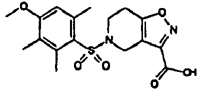 | 5-(4-Methoxy-2,3,6-trimethylphenylsulfonyl)-4,5,6,7-tetrahydroisoxazolo[4,5-c]pyridin-3-carbonsäure (E-20) | Ethyl 5-(4-methoxy-2,3,6-trimethylphenylsulfonyl)-4,5,6,T-tetrahydroisoxazolo[4,5-c]pyridin-3-carboxylat (D-20) | AAV VII | >99% |
| E-21 | 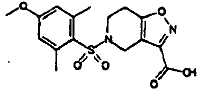 | 5-(4-methoxy-2,6-dimethylphenylsulfonyl)-4,5,6,7-tetrahydroisoxazolo[4,5-c]pyridin-3-carbonsäure (E-21) | Ethyl 5-(4-methoxy-2,6-dimethylphenylsulfonyl)-4,5,6,7-tetrahydroisoxazolo[4,5-c]pyridin-3-carboxylat (D-21) | AAV VII | >99% |
| E-22 | 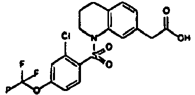 | 2-(1-(2-Chlor-4-(trifluormethoxy)phenylsulfonyl)-1,2,3,4-tetrahydrochinolin-7-yl)essigsäure (E-22) | Methyl 2-(1-(2-chlor-4-(trifluormethoxy)phenylsulfonyl)-1,2,3,4-tetrahydrochinolin-7-yl)acetat (D-22) | AAV VII | 81% |
| E-26 | 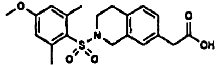 | 2-(2-(4-Methoxy-2,6-dimethylphenylsulfonyl)-1,2,3,4-tetrahydroisochinolin-7-yl)essigsäure (E-26) | | Synthese siehe unten | |
| E-27 | 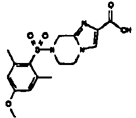 | 7-(4-Methoxy-2,6-dimethylphenylsulfonyl)-5,6,7,8-tetrahydroimidazo[1,2-a]pyrazin-2-carbonsäure (E-27) | Ethyl 7-(4-methoxy-2,6-dimethylphenylsulfonyl)-5,6,7,8-tetrahydroimidazo[1,2-a]pyrazin-2-carboxylat (D-27) | AAV VII | >99% |
| E-28 | 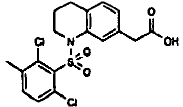 | 2-(1-(2,6-Dichlor-3-methylphenylsulfonyl)-1,2,3,4-tetrahydrochinolin-7-yl)essigsäure (E-28) | Methyl 2-(1-(2,6-dichlor-3-methylphenylsulfonyl)-1,2,3,4-tetrahydrochinolin-7-yl)acetat (D-28) | AAV VII | 89% (0,31 g) |
| E-29 | 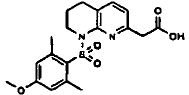 | 2-(8-(4-Methoxy-2,6-dimethylphenylsuHonyl)-5,6,7,& tetrahydro-1,8-naphthyridin-2-yl)essigsäure (E-29) | Methyl 2-(8-(4-methoxy-2,6-dimethylphenylsulfonyl)-5,6,7,8-tetrahydro-1,8-naphthyridin-2-yl)acetat (D-29) | AAV VII | 83% (0.08 g) |
| E-30 | 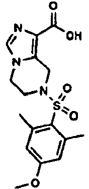 | 7-(4-Methoxy-2,6-dimethylphenylsulfonyl)-5,6,7,8-tetrahydroimidazo[1,5-a]pyrazin-1-carbonsäure (E-30) | | Synthese siehe unten | |

### Baustein E-26:,2-(2-(4-Methoxy-2,6-dimethylphenylsulfonyl)-1,2,3,4-tetrahydroisochinolin-7-yl)essigsäure

### Stufe 1: Methyl 2-(4-methoxy-2,6-dimethylphenylsulfonyl)-1,2,3,4-tetrahydroisochinolin-7-carboxylat

Zu einer Lösung aus Methyl 1,2,3,4-tetrahydroisochinolin-7-carboxylat Hydrochlorid (4.4 mmol, 1 Äquiv.) in DCM wurde bei 25°C TEA (3 Äquiv.) hinzugegeben und das Gemisch 30 min gerührt. Anschließend wurde 4-Methoxy-2,6-dimethylphenyl-1-sulfonyl chlorid (4.4 mmol, 1 Äquiv.) hinzugegeben und das Reaktionsgemisch für 12 h bei 25 °C gerührt. Zu dem Gemisch wurde NH₄Cl-Lösung gegeben und die organische Phase wurde mit Wasser und ges. NaCl-Lösung extrahiert. Die organische Phase wurde über Na₂SO₄ getrocknet und das Lösungsmittel im Vakuum entfernt. Das so erhaltene Rohprodukt wurde ohne weitere Aufreinigung in die nachfolgende Stufe eingesetzt.

### Stufe 2: (2-(4-Methoxy-2,6-dimethylphenylsulfonyl)-1,2,3,4-tetrahydroisochinolin-7-yl)methanol

Zu einer kalten Suspension aus LAH (7.7 mmol, 2 Äquiv.) in THF (30 ml) wurde tropfenweise eine Lösung aus Methyl 2-(4-methoxy-2,6-dimethylphenylsulfonyl)-1,2,3,4-tetrahydroisochinolin-7-carboxylat (3.85 mmol, 1 Äquiv.) in THF (10 ml) hinzugegeben und das resultierende Gemisch 1 h gerührt. Zu dem Reaktionsgemisch wurde THF / Wasser gegeben und es wurde über Celite abfiltriert. Das Filtrat wurde aufkonzentriert und das so erhaltende gelbe Öl wurde ohne weitere Aufreinigung in die nachfolgende Stufe eingesetzt.

### Stufe 3: (2-(4-Methoxy-2,6-dimethylphenylsulfonyl)-1,2,3,4-tetrahydroisochinolin-7-yl)methyl methansulfonat

Zu einer Lösung aus (2-(4-Methoxy-2,6-dimethylphenylsulfonyl)-1,2,3,4-tetrahydroisochinolin-7-yl)methanol (2.22 mmol, 1 Äquiv.) und TEA (2.5 Äquiv.) in DCM (16 ml) wurde bei 0°C MsCl (3.32 mmol, 1.5 Äquiv.) gegeben und das Gemisch 1 h gerührt. Das Reaktionsgemisch wurde mit DCM verdünnt und mit ges. Natriumhydrogencarbonat-Lösung, Wasser und ges. NaCl-Lösung extrahiert und über Na₂SO₄ getrocknet. Das Lösungsmittel wurde im Vakuum entfernt und das Rohprodukt säulenchromatographisch (Kieselgel) aufgereinigt.

### Stufe 4: 2-(2-(4-Methoxy-2,6-dimethylphenylsulfonyl)-1,2,3,4-tetrahydroisochinolin-7-yl)acetonitril

Zu einer Lösung aus (2-(4-Methoxy-2,6-dimethylphenylsulfonyl)-1,2,3,4-tetrahydroisochinolin-7-yl)methyl methansulfonat (2.28 mmol, 1 Äquiv.) in EtOH / Wasser (10 m) wurde KCN (2.73 mmol, 1.2 Äquiv.) gegeben und das Gemisch 24 h refluxiert. Anschließend wurde das Reaktionsgemisch gekühlt und mit Ethylacetat verdünnt. Es wurde mit ges. FeSO₄-Lösung, Wasser und ges. NaCl-Lösung extrahiert, die organische Phase über Na₂SO₄ getrocknet und im Vakuum eingeengt. Das so erhaltene Rohprodukt wurde säulenchromatographisch (Kieselgel) aufgereinigt.
Ausbeute: 71 %

### Stufe 5: 2-(2-(4-Methoxy-2,6-dimethylphenylsulfonyl)-1,2,3,4-tetrahydroisochinolin-7-yl)essigsäure

2-(2-(4-Methoxy-2,6-dimethylphenylsulfonyl)-1,2,3,4-tetrahydroisochinolin-7-yl)acetonitril (2.7 mmol) wurde in 25% wässriger KOH-Lösung (20 ml) für 16 h refluxiert. Das Reaktionsgemisch wurde gekühlt, mit 50% HCl-Lösung auf einen sauren pH-Wert eingestellt und anschließend mit DCM extrahiert. Die organische Phase wurde mit Wasser und ges. NaCl-Lösung extrahiert und über Na₂SO₄ getrocknet. Das Lösungsmittel wurde im Vakuum entfernt und so das gewünschte Produkt als weißer Feststoff erhalten. Dieser wurde ohne weitere Aufreinigung in die nachfolgende Stufe eingesetzt.

### Baustein E-30: 7-(4-Methoxy-2,6-dimethylphenylsulfonyl)-5,6,7,8-tetrahydroimidazo[1,5-a]pyrazin-1-carbonsäure

### Stufe-1: Methyl 4-(diethoxymethyl)-1 H-imidazol-5-carboxylat

Ein Gemisch aus Diethoxyacetonitril (38.72 mmol, 1 Äquiv.) und Methylisocyanoacetat (99.1 mmol, 1.4 Äquiv.) in trockenem Bis(2-methoxyethyl) ether (20 ml) wurde zu einer Suspension aus 30-35% KH (54.2 mmol, 1.4 Äquiv.) in trockenem Bis(2-methoxyethyl) ether (30 ml) gegeben. Das resultierende Gemisch wurde über Nacht auf 70-80 °C erhitzt. Anschließend wurde das Reaktionsgemisch auf 25°C gekühlt und ges. NH₄Cl-Lösung (20 ml) hinzugegeben. Es wurde DCM (100 ml) hinzugegeben und die Phasen getrennt. Die wässrige Phase wurde mit DCM (2 x 75 ml) extrahiert und die vereinigten organischen Phasen über Na₂SO₄ getrocknet, filtriert und das Lösungsmittel im Vakuum entfernt. Zu dem so erhaltenen braunen Öl wurde kalter Ether (10 ml) hinzugegeben und der entstandene Feststoff abfiltriert und mit Ether gewaschen. Das gewünschte Produkt erhielt man so als gelben Feststoff.
Ausbeute: 26 %

### Stufe-2: Methyl 4-formyl-1H-imidazol-5-earboxylat

Zu einer Lösung aus Methyl-4-(diethoxymethyl)-1H-imidazol-5-carboxylat (7.04 mmol, 1 Äquiv.) in Wasser (4.2 ml) wurde Essigsäure (244 mmol, 34.7 Äquiv.) gegeben und das resultierende Gemisch 6 h bei 25 °C unter Stickstoff gerührt. Zu dem Reaktionsgemisch wurde Toluol gegeben und das Lösungsmittel im Vakuum entfernt, um das gewünsche Produkt so als gelben Feststoff zu erhalten. Dieser wurde ohne weitere Aufreinigung in die nachfolgende Stufe eingesetzt.
Ausbeute: quantitativ

### Stufe-3: Methyl 4-((benzyl(2-hydroxyethyl)amino)methyl)-1H-imidazol-5-carboxylat

Methyl 4-formyl-1H-imidazol-5-carboxylat (1.68 mmol, 1 Äquiv.) wurde in trockenem THF (10 ml) aufgenommen. Trockenes Na₂SO₄ (15.53 mmol, 9.2 Äquiv.) und N-Benzylethanolamin (1.99 mmol, 1.18 Äquiv.) wurde hinzugegeben und das Gemisch 1 h bei 25°C unter Stickstoff gerührt. NaBH(OAc)₃ (2.36 mmol, 1.4 Äquiv.) wurde dann portionsweise hinzugegeben und das Reaktionsgemisch 16 h geührt. Anschließend wurde Wasser (5 ml) hinzugegeben und es wurde mit ges. NaHCO₃-Lösung (10 m) neutralisiert. Es wurde mit DCM (2 x 20 ml) extrahiert und die vereinigten organischen Phasen wurden über Na₂SO₄ getrocknet, filtriert und im Vakuum eingeengt. Das so erhaltene Rohprodukt wurde säulenchromatographisch (Kieselgel, 5 -10% MeOH in DCM) aufgereinigt und das gewünschte Produkt so als weißer Feststoff erhalten. Ausbeute: 99 %

### Stufe-4: Methyl 4-((benzyl(2-chloroethyl)amino)methyl)-1H-imidazol-5-carboxylat

Zu einer Lösung aus Methyl 4-((benzyl(2-hydroxyethyl)amino)methyl)-1H-imidazol-5-carboxylat (1.7 mmol, 1 Äquiv.) in DCM (10 ml) wurde Thionyl chlorid (0.5 ml, 4 Äquiv.) hinzugegeben und das Gemisch 16 h bei 45 °C gerührt. Das Reaktionsgemisch wurde auf 25 °C gekühlt und im Vakuum eingeengt. Der Rückstand wurde in Acetonitril aufgenommen, das Lösungsmittel entfernt und der Rückstand für 24 h im Vakuum getrocknet. So erhielt man das gewünschte Produkt als weißen Feststoff. Dieser wurde ohne weitere Aufreinigung in die nachfolgende Stufe eingesetzt.
Ausbeute: quantitativ

### Stufe-5: Methyl 7-benzyl-5,6,7,8-tetrahydroimidazo[1,5-a]pyrazin-1-carboxylat

Zu einer Lösung aus Methyl 4-((benzyl(2-chloroethyl)amino)methyl)-1H-imidazol-5-carboxylat (7.16 mmol, 1 Äquiv.) in Acetonitril (50 ml) wurde bei 0 °C TEA (28.66 mmol, 4 Äquiv.) gegeben. Das resultierende Gemisch wurde 16 h auf 80°C erhitzt. Anschließend wurde es abgekühlt und filtriert, und das Filtrat wurde im Vakuum eingeengt. Das Rohprodukt wurde in DCM und ges. Natriumhydrogencarbonat-Lösung aufgenommen und die organische Phase getrocknet und im Vakuum eingeengt. Das so erhaltene Rohprodukt wurde säulenchromatographisch (Kieselgel, 5% MeOH in DCM) aufgereinigt und das gewünschte Produkt so als braunen Feststoff erhalten.
Ausbeute: 64 %

### Stufe-6: Methyl 5,6,7,8-tetrahydroimidazo[1,5-a]pyrazin-1-carboxylat

Methyl 7-benzyl-5,6,7,8-tetrahydroimidazo[1,5-a]pyrazin-1-carboxylat (3.69 mmol) wurde in MeOH (20 ml) gelöst und die Lösung mit Stickstoff durchspült. Pd(OH)₂ (250 mg) und eine kat. Menge AcOH wurden hinzugegeben und es wurde nochmal 20 min mit Stickstoff durchspült. Das Reaktionsgemisch wurde unter einer Wasserstoffatmosphäre (50 psi) für 5 h ein einer Parr-Hydrierapparatur umgesetzt. Anschließend wurde über Celite filtriert und mit Methanol gewaschen. Das so erhaltene Rohprodukt wurde säulenchromatographisch (Kieselgel, 3-5% MeOH in DCM) aufgereinigt und das gewünschte Produkt so als gelber Feststoff erhalten.
Ausbeute: 99 %

### Stufe-7: Methyl 7-(4-methoxy-2,6-dimethylphenylsulfonyl)-5,6,7,8-tetrahydroimidazo[1,5-a]pyrazin-1-carboxylat

Methyl 5,6,7,8-tetrahydroimidazo[1,5-a]pyrazin-1-carboxylat (0.55 mmol, 1.0 Äquiv.) wurde in DCM (6 ml) gelöst und TEA (7.21 mmol, 2.5 Äquiv.) wurde bei 0 °C hinzugegeben, gefolgt von 4-Methoxy-2,6-dimethylphenyl-1-sulfonyl chlorid (0.66 mmol, 1.2 Äquiv.) in DCM (3 ml). Das Reaktionsgemisch wurde 4 h bei 25°C gerührt und anschließend mit DCM (50 ml) verdünnt und mit Wasser (10 ml) und ges. NaCl-Lösung (10 ml) gewaschen. Die organische Phase wurde über Na₂SO₄ getrocknet und im Vakuum eingeengt. Das so erhaltene Rohprodukt wurde säulenchromatographisch (Kieselgel, 3% MeOH in DCM) aufgereinigt und das gewünschte Produkt so als weißer Feststoff erhalten.
Ausbeute: 31 %

### Stufe 8: 7-(4-Methoxy-2,6-dimethylphenylsulfonyl)-5,6,7,8-tetrahydroimidazo[1,5-a]pyrazin-1-carbonsäure

Ein Gemisch aus Methyl 7-(4-methoxy-2,6-dimethylphenylsulfonyl)-5,6,7,8-tetrahydroimidazo[1,5-a]pyrazin-1-carboxylat (0.13 mmol, 1 equiv), MeOH / H₂O (1:1, 4 ml), und LiOH (0.551 mmol, 6 Äquiv.) wurde 16 h bei 25 °C gerührt. Das organische Lösungsmittel wurde im Vakuum entfernt und die resultierende Suspension mit Wasser (5 ml) verdünnt und mit 1 N HCl bei 0 °C auf einen sauren pH-Wert eingestellt. Anschließend wurde mit Ethylacetat (2 x 15 ml) extrahiert und die vereinigten organischen Phasen mit ges. NaCl-Lösung gewaschen. Die organische Phase wurde über Na₂SO₄ getrocknet und im Vakuum eingeengt, um das Produkt als hellgelben Feststoff zu erhalten. Dieser wurde ohne weitere Aufreinigung in die nachfolgende Stufe eingesetzt.
Ausbeute: 63 %

### 5) Synthese der Aminbausteine F

**Amin F-09: 3-(Pyridin-4-yl)-3,9-diazaspiro[5.5]undecan Dihydrochlorid**

### Stufe (i): tert-Butyl 9-(pyridin-4-yl)-3,9-diazaspiro[5.5]undecan-3-carboxylat

tert-Butyl-3,9-diazaspiro[5.5]undecan-3-carboxylat (1 g, 3.931 mmol), 4-Chlorpyridiniumchlorid (1.765 g, 11.794 mmol) und Triethylamin (2.2 ml, 15.725 mmol) wurden in 1-Butanol (50 ml) 15 h refluxiert. Gesättigte Natriumhydrogencarbonatlösung (30 ml) und Ethylacetat (80 ml) wurden zugegeben, Phasen getrennt und die wässrige Phase mit Ethylacetat (2 x 80 ml) extrahiert. Die vereinigten organischen Phasen wurden über Magnesiumsulfat getrocknet und unter Vakuum eingeengt. Das Rohprodukt wurde säulenchromatographisch (Kieselgel, Ethylacetat / Hexan / Methanol / Ammoniak (25% aq), 400:40:40:1) gereinigt.
Ausbeute: 0.52 g (39%)

### Stufe (ii): 3-(Pyridin-4-yl)-3,9-diazaspiro[5.5]undecan Dihydrochlorid

tert-Butyl-9-(pyridin-4-yl)-3,9-diazaspiro[5.5]undecan-3-carboxylat (0.52 g, 1.569 mmol) wurde mit Chlorwasserstoff in Methanol (1.25 mol/l, 6.3 ml) versetzt und 1 h refluxiert. Das Lösungsmittel wurde unter Vakuum entfernt, der Rückstand in Ethanol (3 ml) aufgenommen und gekühlt. Aceton (80 ml) wurde zugegeben und 30 min im Eisbad gerührt. Der Niederschlag wurde abgesogen, mit Diethylether gewaschen und unter Vakuum getrocknet.
Ausbeute: 0.4 g (83%)
Alternativ kann die Entschützung auch unter Einwirkung von TFA in DCM erfolgen, um das Amin (F-09) als freie Base zu erhalten.

**Amin F-14: 1-Phenyl-1,3,8-triazaspiro[4.5]decan-4-on** [1021-25-6] ist kommerziell erhältlich bei z.B. Acros.

**Amin F-16: 4-(4-Fluorphenyl)-2-methyl-2,8-diazaspiro[4.5]decan-1-on Hydrochlorid** [MDL No.: MFCD08460813] ist kommerziell erhältlich bei z.B. ASWMEDCHEM.

**Amin F-17: 2-(4-Fluorbenzyl)-2,8-diazaspiro[4.5]decan-1-on Hydrochlorid** [MDL No.: MFCD08461093] ist kommerziell erhältlich bei z.B. ASWMEDCHEM.

**Amin F-18: 2-Benzyl-2,8-diazaspiro[4.5]decan-1-on Hydrochlorid** [MDL No.: MFCD02179153] ist kommerziell erhältlich bei z.B. ASWMEDCHEM.

**Amin F-19: 2-Benzyl-2,7-diazaspiro[4.4]nonan** [MDL No.: MFCD04115133] ist kommerziell erhältlich bei z.B. Tyger.

**Amin F-23: 8-(Pyridin-4-yl)-2,8-diazaspiro[4.5]decan Dihydrochlorid** Die Synthese wurde analog der Synthese von Amin **F-09** durchgeführt.

Hierzu wurde in Stufe (i) tert-Butyl 2,8-diazaspiro[4.5]decan-2-carboxylat mit 4-Chlorpyridiniumchlorid umgesetzt (Ausbeute: 22%). Anschließend wurde in Stufe (ii) die Boc-Schutzgruppe abgespalten. Nach Beendigung der Reaktion und Entfernen des Methanols unter Vakuum, der Rückstand in Ethanol aufgenommen, gekühlt und mit Aceton versetzt. Die resultierende Suspension wurde 30 min im Eisbad gerührt, der Niederschlag abgesogen, mit Aceton gewaschen und unter Vakuum getrocknet (Ausbeute Amin F-23: 92%).

**Amin F-24: 2-(Pyridin-4-yl)-2,7-diazaspiro[4.4]nonan Dihydrochlorid**

Die Synthese wurde analog der Synthese von Amin **F-09** durchgeführt.

Hierzu wurde in Stufe (i) tert-butyl 2,7-diazaspiro[4.4]nonan-2-carboxylat mit 4-Chlorpyridiniumchlorid umgesetzt (Ausbeute: 50%). Anschließend wurde in Stufe (ii) die Boc-Schutzgruppe abgespalten. Nach Beendigung der Reaktion und Entfernen des Methanols unter Vakuum wurde der Rückstand in Ethanol aufgenommen, gekühlt und mit Aceton versetzt. Die resultierende Suspension wurde 30 min im Eisbad gerührt, der Niederschlag abgesogen, mit Aceton gewaschen und unter Vakuum getrocknet (Ausbeute Amin F-24: 73%).

**Amin F-26: tert-Butyl 1,8-diazaspiro[4.5]decan-1-carboxylat [336191-17-4]** ist kommerziell erhältlich bei z.B. JW-Pharmalab.

**Amin F-30: 9-(Pyridin-4-yloxy)-3-azaspiro[5.5]undecan Dihydrochlorid Stufe (i): 1-(Benzyloxycarbonyl)piperidin-4-carbonsäure**

Zu Piperidin-4-carbonsäure (25 g) in THF (75 ml) wurde Wasser (75 ml) gegeben, gefolgt von Natriumbicarbonat (30,8 g). Das Gemisch wurde auf to 0°C gekühlt und Cbz chlorid (38,9 ml) hinzugetropft. Anschließend wurde das Reaktionsgemisch 5 h bei Raumtemperatur gerührt (DC-Kontrolle). Nach vollständiger Umsetzung wurde das organische Lösungsmittel abdestilliert und der Rückstand in Wasser (200 ml) aufgenommen, mit Ethylacetat (2 x 150 ml) gewaschen. Die wässrige Phase wurde mit verdünnter wässriger HCl Lösung sauer gestellt und mit Ethylacetat extrahiert. Die organische Phase wurde getrocknet (Na₂SO₄) und unter Vakuum aufkonzentriert.
Ausbeute: 48,5 g (96%)

### Stufe (ii): 1-Benzyl 4-methyl piperidin-1,4-dicarboxylat

1-(Benzyloxycarbonyl)piperidin-4-carbonsäure (48,5 g) in Methanol (485 ml) wurde auf 0°C gekühlt und Thionylchlorid (13,34 ml) hinzugetropft. Das Gemisch wurde anschließend 20 min refluxiert (DC-Kontrolle). Nach vollständiger Umsetzung wurde das Methanol abdestilliert, der Rückstand in Wasser (15 ml) aufgenommen und mit Ethylacetat (2 x 150 ml) extrahiert.. Die vereinigten organischen Phasen wurden mit Wasser und ges. Natriumchloridlösung extrahiert, getrocknet (Na₂SO₄) und unter Vakuum aufkonzentriert.
Ausbeute: 38 g (67%)

### Stufe (iii): Benzyl 4-formylpiperidin-1-carboxylat

Eine Lösung 1-Benzyl 4-methyl piperidin-1,4-dicarboxylat (10 g) in Toluol (100 ml) unter Stickstoff wurde auf -78°C gekühlt. Anschließend wurde DIBAL-H (60,9 ml) bei -78°C hinzugetropft und das Gemisch 1 h bei dieser Temperatur gerührt (DC-Kontrolle). Aufgrund unvollständiger Umsetzung wurde weitere 0.2 eq DIBAL-H hinzugegeben und weitere 30 min gerührt (DC-Kontrolle: etwas Edukt und der entsprechende Alkohol waren zu erkennen). Zu dem Reaktionsgemisch wurden langsam bei -78°C Methanol (40 ml), gefolgt von ges. Natriumchloridlösung (40 ml) hinzugegeben. Das Gemisch wurde über Celite filtriert, das Lösungsmittel unter Vakuum entfernt. Der Rückstand wurde mit Ethylacetat (3 × 75 ml) extrahiert, getrocknet (Na₂SO₄) und unter Vakuum aufkonzentriert. Das so erhaltene Rohprodukt wurde säulenchromatographisch aufgereinigt (Kieselgel, 20% Ethylacetat / Hexan).
Ausbeute: 4,3 g (49%)

### Stufe (iv): Benzyl 9-oxo-3-azaspiro[5.5]undec-7-en-3-carboxylat

Methylvinylketon (1.64 ml), Ethanol (5 ml) and Wasser (5 ml) wurden zu Benzyl 4-formylpiperidin-1-carboxylat (5 g) gegeben. Anschließend wurde das Gemisch zu einer kochenden Lösung Kaliumhydroxid (0,22 g) in Ethanol (10 ml) gegeben und das resultierende Reaktionsgemisch 1 h refluxiert (DC-Kontrolle). Nach vollständiger Umsetzung wurde das Gemisch auf Wasser (25 ml) gegeben und mit Ethylacetat (2 × 50 ml) extrahiert. Die vereinigten organischen Phasen wurden getrocknet (Na₂SO₄) und unter Vakuum aufkonzentriert. Das so erhaltene Rohprodukt wurde säulenchromatographisch aufgereinigt (Kieselgel, 25% Ethylacetat / Hexan).
Ausbeute: 2,8 g (46%)

### Stufe (v): tert-Butyl 9-oxo-3-azaspiro[5.5]undecan-3-carboxylat

Boc-Anhydrid (9,4 ml) und Kaliumcarbonat (7,56 g) wurden zu Benzyl 9-oxo-3-azaspiro[5.5]undec-7-en-3-carboxylat (8,2 g) in EtOH / Wasser (9:1) (200 ml) gegeben. Anschließend wurde Pd/C (1 g) hinzugegeben und 4 h bei 80 psi hydrogenolysiert (DC-Kontrolle). Nach vollständiger Umsetzung wurde das Gemisch über Celite filtriert und mit Ethanol und Ethylacetat nachgespült. Das Filtrat wurde getrocknet (Na₂SO₄) und unter Vakuum aufkonzentriert. Der Rückstand wurde in Ethylacetat und Wasser aufgenommen und die wässrige Phase mit Ethylacetat extrahiert. Die vereinigten organischen Phasen wurden getrocknet (Na₂SO₄) und unter Vakuum aufkonzentriert. Das so erhaltene Rohprodukt wurde säulenchromatographisch aufgereinigt (Kieselgel, 20% Ethylacetat / Hexan). Ausbeute: 2,92 g, 40%

### Stufe (vi): tert-Butyl 9-hydroxy-3-azaspiro[5.5]undecan-3-carboxylat

tert-Butyl 9-oxo-3-azaspiro[5.5]undecan-3-carboxylat (1,5 g) wurde in THF (7,5 ml) gelöst und auf -5°C gekühlt. Anschließend wurde NaBH₄ (0,212 g) hinzugegeben und das Gemisch 1 h bei Raumtemperatur gerührt (DC- Kontrolle). Nach vollständiger Umsetzung wurde zu dem Gemisch Essigsäure gegeben und das Methanol anschließend abdestilliert. Der Rückstand wurde in Wasser (50 ml) aufgenommen und mit Ethylacetat (2 × 50 ml) extrahiert. Die vereinigten organischen Phasen wurden getrocknet (Na₂SO₄) und unter Vakuum aufkonzentriert. Das so erhaltene Rohprodukt wurde säulenchromatographisch aufgereinigt (Kieselgel, 30% Ethylacetat / Hexan).
Ausbeute: 1,2 g (80%)

### Stufe (vii): tert-Butyl 9-(pyridin-4-yloxy)-3-azaspiro[5.5]undecan-3-carboxylat

Zu Natriumhydrid (0,89 g) in DMSO (20 ml) wurde 4-Chlorpyridin Hydrochlorid (1,3 g) gegeben und das Gemisch 10 min gerührt. Anschließend wurde tert-Butyl 9-hydroxy-3-azaspiro[5.5]undecan-3-carboxylat (2,0 g) in DMSO (20 ml) langsam hinzugegeben und das Gemisch über Nacht gerührt (DC-Kontrolle: Umsetzung ca. 30 - 35 %). Eine katalytische Menge Natriumiodid wurde hinzugegeben und das Reaktionsgemisch 8 h bei 80°C gerührt (DC-Kontrolle). Zu dem Reaktionsgemisch wurde Methanol und NaHCO₃ Lösung gegeben und 20 min gerührt. Dann wurde mit Ethylacetat extrahiert und wiederum mit NaHCO₃ Lösung und kaltem Wasser gewaschen. Die organische Phase wurde getrocknet (Na₂SO₄) und unter Vakuum aufkonzentriert. Das so erhaltene Rohprodukt wurde säulenchromatographisch aufgereinigt (Kieselgel, 70% Ethylacetat / Hexan).
Ausbeute: 1,0 g (40%)

### Stufe (viii): 9-(Pyridin-4-yloxy)-3-azaspiro[5.5]undecan Dihydrochlorid

tert-Butyl 9-(pyridin-4-yloxy)-3-azaspiro[5.5]undecan-3-carboxylat (1 g, 2,886 mmol) wurde in Methanol (2 ml) gelöst und mit Chlorwasserstoff in Methanol (1,25 mol/l, 11,5 ml) versetzt und 30 min refluxiert. Das Lösungsmittel wurde unter Vakuum entfernt und der Rückstand in einer geringen Menge Ethanol gelöst. Anschließend wurde Aceton (ca. 25 ml) hinzugegeben, das Gemisch 30 min bei 0°C gerührt und schließlich der entstandene Feststoff abgesogen.
Ausbeute: 0,96 g (>99%)

**Amin F-32: 9-(Azetidin-1-yl)-3-azaspiro[5.5]undecan Dihydrochlorid**

### Stufe (i): tert-Butyl 9-(azetidin-1-yl)-3-azaspiro[5.5]undecan-3-carboxylat

tert-Butyl 9-oxo-3-azaspiro[5.5]undecan-3-carboxylat (Stufe (iv) Amin-F30) (1 g, 3,74 mmol) und Azetidin (0,25 ml, 3,74 mmol) wurden in 1,2-Dichlorethan (15 ml) vorgelegt und mit Natriumtriacetoxyborhydrid (1,1 g, 5,23 mmol) versetzt. Das Reaktionsgemisch wurde 3 d bei Raumtemepartur gerührt und anschließend mit gesättigter Natriumhydrogencarbonatlösung versetzt. Nach Phasentrennung wurde die wässrige Phase mit Dichlormethan (2 x) extrahiert. Die vereinigten organischen Phasen wurden mit gesättigter Natriumchloridlösung (1 x) gewaschen, über Magnesiumsulfat getrocknet und unter Vakuum eingeengt. Das Rohprodukt wurde säulenchromatographisch (Kieselgel, Ethylacetat / Methanol / Ammoniak (25% aq), 100:10:1) gereinigt.
Ausbeute: 1 g (89%)

### Stufe (ii): 9-(Azetidin-1-yl)-3-azaspiro[5.5]undecan

tert-Butyl 9-(azetidin-1-yl)-3-azaspiro[5.5]undecan-3-carboxylat (1 g, 3,24 mmol) wurde mit Chlorwasserstoff in Methanol (1,25 mol/l, 15,5 ml) versetzt und 45 min refluxiert. Das Lösungsmittel wurde unter Vakuum entfernt und der Rückstand in einer geringen Menge Ethanol gelöst. Anschließend wurde durch Zugabe von Aceton ein Feststoff ausgefällt, schließlich Diethylether hinzugegeben und der entstandene Niederschlag abgesogen.
Ausbeute: 0,87 g (95%)

**Amin F-33: 3-(Pyridin-4-yl)-1-oxa-2,8-diazaspiro[4.5]dec-2-en Bis(2,2,2-trifluoracetat)**

### Stufe (i): tert-Butyl 4-methylenpiperidin-1-carboxylat

In einer ausgeheizten, mit Schutzgas gefluteten Apparatur wurde Methyltriphenylphosphoniumbromid (53,82 g, 150 mmol) in Diethylether (300 ml) aufgeschlämmt und auf 0°C gekühlt. Kalium-tert-butylat (15,78 g, 140 mmol) wurde portionsweise zugegeben, die Suspension 30 min gerührt. Boc-4-piperidon (20 g, 100 mmol), gelöst in Diethylether (200 ml), wurde langsam zugetropft, dann auf Raumtemperatur erwärmt und 15 h gerührt. Die Reaktionsmischung wurde gekühlt und mit Ammoniumchlorid-Lösung (300 ml, 10%) versetzt, nach Phasentrennung wurde die wässrige Phase mit Ether (3 × 200 ml) extrahiert, die vereinigten organischen Phasen getrocknet (MgSO₄) und unter Vakuum eingeengt. Das Rohprodukt wurde säulenchromatographisch (Kieselgel) mit Ether / Hexan (1:1) gereinigt.
Ausbeute: 18,57 g (93%)

### Stufe (ii): tert-Butyl 3-(pyridin-4-yl)-1-oxa-2,8-diazaspiro[4.5]dec-2-en-8-carboxylat

(a): (Z)-N-Hydroxyisonicotinimidoylchlorid : Pyridin-4-carbaldoxim (1 g, 8,19 mmol) wurde in DMF (10 ml) gelöst, eine Lösung von N-Chlorsuccinimid (1,31 g, 9,83 mmol) in DMF (5 ml) wurde langsam zugetropft, die Reaktionsmischung wurde bei Raumtemperatur gerührt. Nach vollständiger Umsetzung (dünnschichtchromatographische Kontrolle, hier 6 h) wurden Diethylether (50 ml) und Wasser (20 ml) zugegeben, Phasentrennung, Extraktion der wässrigen Phase mit Diethylether (5 × 30 ml). Die vereinigten organischen Phasen wurden mit Wasser (50 ml) und gesättiger Natriumchloridlösung (50 ml) gewaschen, getrocknet (MgSO₄) und unter Vakuum eingeengt. Die Rohsubstanz wurde ohne weitere Reinigung und Analytik umgesetzt.
   Ausbeute: 0,74 g (100%)
(b): tert-Butyl-4-methylenpiperidin-1-carboxylat (0,7 g, 3,55 mol) wurde in Dichlormethan (10 ml) gelöst und unter Schutzgas auf 0°C gekühlt. (Z)-N-Hydroxyisonicotinimidoylchlorid (1,67 g, 10,64 mmol), gelöst in Dichlormethan (15 ml), wurde zugegeben, gefolgt von Triethylamin (1,2 ml, 8,5 mmol) in Dichlormethan (10 ml). Die Reaktionsmischung wurde langsam auf Raumtemperatur erwärmt und 15 h gerührt. Es wurde mit Dichlormethan (50 ml) verdünnt und mit Wasser, 10%iger Citronensäure und gesättigter Natriumchlorid-Lösung (je 30 ml) gewaschen, getrocknet (MgSO₄) und unter Vakuum eingeengt. Das Rohprodukt wurde säulenchromatographisch (Kieselgel) mit Ethylacetat / Hexan 10 / 1 gereinigt.
   Ausbeute: 0,48 g (42%)

### Stufe (iii): 3-(Pyridin-4-yl)-1-oxa-2,8-diazaspiro(4.5)dec-2-en Bis(2,2,2-trifluoracetat)

tert-Butyl 3-(pyridin-4-yl)-1-oxa-2,8-diazaspiro[4.5]dec-2-en-8-carboxylat (0,48 g, 1,5 mmol) wurde in Dichlormethan (10 ml) gelöst, gekühlt und langsam mit Trifluoressigsäure (1,2 ml, 15 mmol) versetzt. Nach 2 h refluxieren wurde das Lösungsmittel unter Vakuum entfernt und der Rückstand mit je 30 ml Toluol und Methanol co-evaporiert.
Ausbeute: 0,74 g (100%)

**Amin F-34: 9-(3,3-Difluorazetidin-1-yl)-3-azaspiro[5.5]undecan Dihydrochlorid**

### Stufe (i): tert-Butyl 9-(3,3-difluorazetidin-1-yl)-3-azaspiro[5.5]undecan-3-carboxylat

Zu 3,3-Difluorazetidin Hydrochlorid (0,484 g, 3,74 mmol) und Triethylamin (0,52 ml, 3,74 mmol) in 1,2-Dichlorethan (15 ml) wurde tert-Butyl 9-oxo-3-azaspiro[5.5]undecan-3-carboxylat (Stufe (iv) Amin-F30) (1 g, 3,74 mmol) hinzugegeben. Das Gemisch wurde 5 min gerührt und anschließend mit Natriumtriacetoxyborhydrid (1,1 g, 5,23 mmol) versetzt und 3 d bei Raumtemepartur gerührt. Es wurde gesättigte Natriumhydrogencarbonatlösung hinzugegeben und nah Phasentrennung wurde die wässrige Phase mit Dichlormethan (2 x) extrahiert. Die vereinigten organischen Phasen wurden mit gesättigter Natriumchloridlösung (1 x) gewaschen, über Magnesiumsulfat getrocknet und unter Vakuum eingeengt. Ausbeute: 1,26 g (98%)

### Stufe (ii): 9-(3,3-Difluorazetidin-1-yl)-3-azaspiro[5.5]undecan Dihydrochlorid

tert-Butyl 9-(3,3-difluorazetidin-1-yl)-3-azaspiro[5.5]undecan-3-carboxylat (1,26 g, 3,66 mmol) wurde in Chlorwasserstoff in Methanol (1.25 mol/l, 29 ml) gelöst und 45 min refluxiert. Das Lösungsmittel wurde unter Vakuum entfernt und der Rückstand in einer geringen Menge Ethanol gelöst. Anschließend wurde durch Zugabe von Aceton ein Feststoff ausgefällt. Das Gemisch wurde 10 min bei Raumtemperatur gerührt, dann Diethylether hinzugegeben und weitere 30 min bei Raumtemperatur gerührt. Der entstandene Niederschlag wurde abgesogen, mit Diethylether gewaschen und unter Vakuum getrocknet.
Ausbeute: 1,1 g (95%)

| **Amin** | **Struktur** | **Name** |
|---|---|---|
| F-09 | 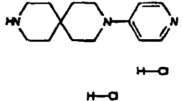 | 3-(Pyridin-4-yl)-3,9-diazaspiro[5.5]undecan dihydrochlorid (F-09) |
| F-14 | 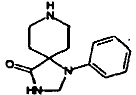 | 1-Phenyl-1,3,8-triazaspiro[4.5]decan-4-on (F-14) |
| F-16 | 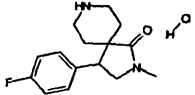 | 4-(4-Fluorphenyl)-2-methyl-2,8-diazaspiro[4.5]decan-1-on hydrochlorid (F-16) |
| F-17 | 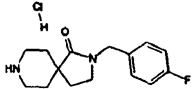 | 2-(4-Fluorbenzyl)-2,8-diazaspiro[4.5]decan-1-on hydrochlorid (F-17) |
| F-18 | 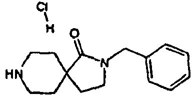 | 2-Benzyl-2,8-diazaspiro[4.5]decan-1-on hydrochlorid (F-18) |
| F-19 | 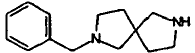 | 2-Benzyl-2,7-diazaspiro[4.4]nonan (F-19) |
| F-23 | 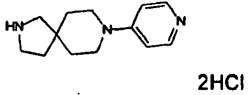 | 8-(Pyridin-4-yl)-2,8-diazaspiro[4.5]decan Dihydrochlorid (F-23) |
| F-24 | 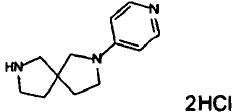 | 2-(Pyridin-4-yl)-2,7-diazaspiro[4.4]nonan Dihydrochlorid (F-24) |
| F-26 | 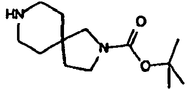 | tert-Butyl 2,8-diazaspiro[4.5]decan-2-carboxylate (F-26) |
| F-30 | 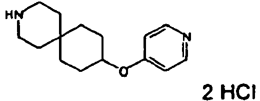 | 9-(Pyridin-4-yloxy)-3-azaspiro[5.5]undecan Dihydrochlorid (F-30) |
| F-32 | 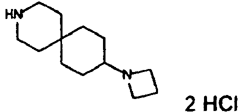 | 9-(Azetidin-1-yl)-3-azaspiro[5.5]undecan Dihydrochlorid (F-32) |
| F-33 | 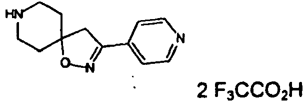 | 3-(Pyridin-4-yl)-1-oxa-2,8-diazaspiro[4.5]dec-2-en Bis(Trifluoracetat) (F-33) |
| F-34 | 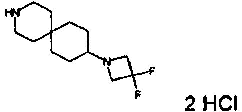 | 9-(3,3-Difluorazetidin-1-yl)-3-azaspiro[5.5]undecan Dihydrochlorid (F-34) |

### Einzelsubstanzsynthesen

### 6) Synthese der erfindungsgemäßen Verbindungen der allgemeinen Formel (I)

Allgemeine Methode zur Synthese der Amide G

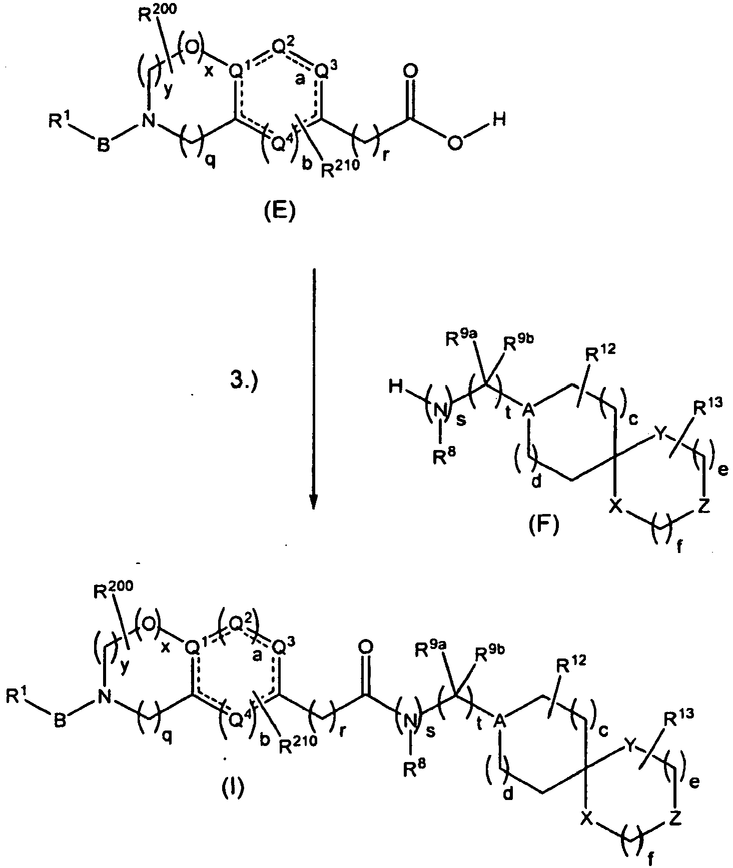

Allgemeine Arbeitsvorschrift AAV IX - CDI-Kupplung: Eine Lösung von Säure E (1 Äquiv.), Diisopropyletylamin (2 Äquiv.) und Carbonyldiimidazol (1.1 Äquiv.) in Dichlormethan wurde für 1 h bei Raumtemperatur gerührt, anschließend mit Amin F (1 Äquiv.; bei der Verwendung von Amin-Hydrochlorid wird die Menge des eingesetzten Diisopropylethylamins entsprechend angepaßt) versetzt und für 12 h bei Raumtemperatur gerührt. Die Reaktionslösung wurde mit Ethylacetat versetzt, 1 x mit ges. Natriumchloridlösung, 1x mit ges. Natriumhydrogencarbonatlösung und 3x mit gesättigter Natriumhydrogencarbonatlösung gewaschen, über Natriumsulfat getrocknet und unter vermindertem Druck konzentriert. Nach säulenchromatographischer Aufreinigung (Alox Neutral, Ethylacetat / Hexan oder Silika, DCM / MeOH ggf. NEt₃) wurde das gewünschte Produkt erhalten.

Allgemeine Arbeitsvorschrift AAV IXa- CDI-Kupplung: Eine Lösung von Säure **E** (1 Äquiv.), Diisopropyletylamin (2 Äquiv.) und Carbonyldiimidazol (1.05 Äquiv.) in Dichlormethan wurde für 1 h bei Raumtemperatur gerührt, anschließend mit Amin **F** (1 Äquiv.; bei der Verwendung von Amin-Hydrochlorid wird die Menge des eingesetzten Diisopropylethylamins entsprechend angepaßt) versetzt und für 12 h bei Raumtemperatur gerührt. Die Reaktionslösung wurde mit DCM verdünnt und mit ges. Natriumhydrogencarbonatlösung versetzt. Die Phasen wurden getrennt, die wässrige Phase 2x mit DCM extrahiert, die vereinigten org. Phasen 1x mit ges. NaCl-Lösung gewaschen, über Natriumsulfat getrocknet und unter vermindertem Druck konzentriert. Nach säulenchromatographischer Aufreinigung (Kieselgel) erhielt man das gewünschte Produkt.

Allgemeine Arbeitsvorschrift AAV X - TBTU-Kupplung: Eine Lösung von Säure **E** (1 Äquiv.), Amin F (1 Äquiv.), HOBt (1.1 Äquiv.) und Diisopropyletylamin (3 Äquiv.; bei der Verwendung von AminHydrochloridn wird die Menge des eingesetzten Diisopropylethylamins entsprechend angepaßt) in THF wurde mit TBTU (1.2 Äquiv.) versetzt und für 12 h bei Raumtemperatur gerührt. Die Reaktionslösung wurde mit Ethylacetat versetzt, 1 x mit ges. Ammoniumchloridlösung, 1 x mit ges. Natriumhydrogencarbonatlösung und 1x mit gesättigter Natriumchloridlösung gewaschen, über Natriumsulfat getrocknet und unter vermindertem Druck konzentriert. Nach säulenchromatographischer Aufreinigung (Alox Neutral, Ethylacetat / Hexan oder Silika, DCM / MeOH ggf. NEt₃) wurde das gewünschte Produkt erhalten.

Allgemeine Arbeitsvorschrift AAV XI - TBTU-Kupplung: Die Carbonsäure **E** (1 Äquiv.), O-(1H-Benzotriazol-1-yl)-N,N,N',N'-tetramethyluronium tetrafluorborat (1 Äquiv.) und 1-Hydroxybenzotriazolhydrat (1 Äquiv.) wurden unter Schutzgas in Tetrahydrofuran vorgelegt und 30 min bei Raumtemperatur gerührt. Eine Lösung des Amins F (ggf. in Form des entsprechenden Hydrochlorids (xHCl)) (1 Äquiv.) und Diisopropylethylamin (DIPEA) (3 - 5 Äquiv.) in Tetrahydrofuran wurde zugegeben und das Reaktionsgemisch 15 h bis 3 d bei Raumtemperatur gerührt. Tetrahydrofuran wurde anschließend unter Vakuum entfernt, der Rückstand in Ethylacetat und gesättigter Natriumhydrogencarbonat-Lösung aufgenommen und die Phasen getrennt. Die wässrige Phase wurde mit Ethylacetat extrahiert und die vereinigten organischen Phasen wiederum mit gesättigter Natriumchloridlösung gewaschen, über Natriumsulfat getrocknet und unter Vakuum eingeengt. Das Rohprodukt wurde säulenchromatographisch (Kieselgel) aufgereinigt.

Allgemeine Arbeitsvorschrift AAV XII - EDCI-HCl-Kupplung: Zu einer eisgekühlten Lösung des Carbonsäure E (1 Äquiv.) in DCM wurde EDCI.HCl (1.5 Äquiv.), HOBt (1 Äquiv.) und DIPEA (4 Äquiv.) gegeben und es wurde 30 min gerührt. Dann wurde eine Lösung des Amins F (1.2 Äquiv.) in DCM zugegeben, das Eisbad wurde entfernt und bei Raumtemperatur über Nacht gerührt. Die Reaktionsmischung wurde mit Dichlormethan verdünnt und mit gesättigter Ammoniumchlorid-Lösung, gesättigter Natriumchlorid-Lösung, gesättigter Natriumcarbonat-Lösung und wieder gesättigter Natriumchlorid-Lösung gewaschen, über Natriumsulfat getrocknet und unter Vakuum eingeengt. Das Rohprodukt wurde säulenchromatographisch (Kieselgel) gereinigt.

Allgemeine Arbeitsvorschrift AAV XIII - CDI-Kupplung: 1,1'-Carbonyldiimidazol (1.05 Äquiv.) und Carbonsäure E (1 Äquiv.) wurden in Dichlormethan oder einem Dichlormethan / N,N-Dimethylformamid (3:2) Gemisch gelöst und 1 h bei Raumtemperatur gerührt. Anschließend wurde das Amin F (ggf. in Form des entsprechenden Hydrochlorids (xHCl)) (1.5 Äquiv.), gelöst in einem Gemisch aus Dichlormethan / N,N-Dimethylformamid (3:2) und Triethylamin (1 - 3 Äquiv.), zugetropft und das Reaktionsgemisch bis zu 3 Tage bei Raumtemperatur gerührt (DC-Kontrolle). Das Gemisch wurde mit ein wenig Wasser versetzt und unter Vakuum eingeengt. Anschließend wurde der Rückstand in Dichlormethan aufgenommen und mit gesättigter Natriumhydrogencarbonat-Lösung sowie gesättigter Natriumchlorid-Lösung gewaschen. Die wässrige Phase wurde mit Dichlormethan extrahiert (2x) und die vereinigten organischen Phasen wiederum mit gesättigter Natriumchlorid-Lösung gewaschen. Dann wurde über Natriumsulfat getrocknet und unter Vakuum eingeengt. Die Reinigung des Rohproduktes erfolgte durch Säulenchromatographie (Kieselgel).

Allgemeine Arbeitsvorschrift AAV XIV - EDCI-HCl-Kunplung: Die Carbonsäure **E** (1 Aquiv.) und N-Ethyl-N'-(3-dimethylamino propyl) carbodiimid Hydrochlorid (EDCI-HCl) (2 Äquiv.) wurden in Dichlormethan gelöst und 30 min gerührt. **N-**Hydroxybenzotriazol (HOBt) (0,05 Äquiv.) wurde zugegeben, gefolgt von einer Lösung des Amins F (ggf. in Form des entsprechenden Hydrochlorids (xHCl)) (1 Äquiv.) und Triethylamin (1 - 4 Aquiv.) in Dichlormethan. Das resultierende Raktionsgemisch wurde 15 h bei Raumtemperatur gerührt. Es wurde mit Dichlormethan verdünnt, gesättigte Natriumhydrogencarbonat-Lösung wurde zugegeben und die Phasen wurden getrennt. Die wässrige Phase wurde mit Dichlormethan extrahiert (2 x), anschließend die vereinigten organischen Phasen mit gesättigter Natriumchlorid-Lösung gewaschen, über Natriumsulfat getrocknet und unter Vakuum eingeengt. Das Rohprodukt wurde säulenchromatographisch (Kieselgel) gereinigt.

Allgemeine Arbeitsvorschrift AAV XV - Castro-Kupplung: Die Carbonsäure **E** (1 Äquiv.) und das Amin F (1 Aquiv.) wurden in DMF gelöst und 4-Methylmorpholin (3 Äquiv.), gefolgt von Benzotriazol-1-yloxytris(dimethylamino)phosphonium hexafluorphosphat (1,3 Äquiv.) hinzugegeben. Das Reaktionsgemisch wurde 4 d bei Raumtemperatur gerührt und anschließend das Lösungsmittel unter Vakuum entfernt. Der Rückstand wurde in Ethylacetat und gesättigter Natriumhydrogencarbonat-Lösung aufgenommen und die Phasen getrennt. Die wässrige Phase wurde mit Ethylacetat extrahiert und die vereinigten organischen Phasen über Magnesiumsulfat getrocknet und unter Vakuum eingeengt. Das Rohprodukt wurde säulenchromatographisch (Kieselgel) aufgereinigt.

Allgemeine Arbeitsvorschrift AAV XVI - HATU Kupplung: Die Carbonsäure E (1 Äquiv.) wurde bei 0 °C in THF gelöst und DIPEA (3.0 Äquiv.) und HATU (2.0 Äquiv.) wurden hinzugegeben. Das Reaktionsgemisch wurde 15 min bei 25°C gerührt und anschließend wieder auf 0 °C gekühlt. Das Amin F (1.0 Äquiv.), gelöst in THF, wurde hinzugegeben und das resultierende Gemisch 16 h bei 25°C gerührt. Das Lösungsmittel wurde im Vakuum entfernt, der Rückstand mit DCM verdünnt und mit ges. Natriumcarbonat-Lösung, ges. Ammoniumchlorid-Lösung, Wasser und ges. Natriumchlorid-Lösung gewaschen, über Natriumsulfat getrocknet und im Vakuum eingeengt. Das Rohprodukt wurde säulenchromatographisch (Kieselgel) gereinigt.

**Tabelle 3: Synthese der Amide G**

| **Beispiel Nr.** | **Struktur** | **Name** | **Aminosäure (E)** | **Amin (F)** | **Ausbeute** | **Analytik (LC/MS)^{[1]}** | **Synthetisiert nach / Kommentar** |
|---|---|---|---|---|---|---|---|
| G-04 | 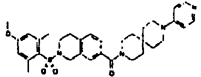 | (2-(4-Methoxy-2,6-dimethylphenylsulfonyly-1,2,3,4-tetrahydroisochinolin-7-yl)(9-(pyridin-4-yl)-3,9-diazaspiro[5.5]undecan-3-yl)methanon (G-04) | 2-(4-Methoxy-2,6-dimethylphenylsulfonyl)-1,2,3,4-tetrahydroisochinolin-7-carbonsäure (E-08) | 3-(Pyridin-4-yl)-3,9-diazaspiro[5.5]undeca n Dihydrochlorid (F-09) | 17% (0.09 mmol) | R₁ = 3.4 min; Purity (UV 200-400 nm) 95%; *m*/*z* = 589.1 [MH]⁺ | AAV X |
| G-11 | 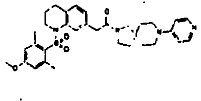 | 2-(1-(4-Methoxy-2,6-dimethylphenylsulfonyl)-1,2,3,4-tetrahydrochinolin-7-yl)-1-(8-(pyridin-4-yl)-2,8-diazaspiro[4.5]decan-2-yl)ethanon (G-11) | 2-(1-(4-Methoxy-2,6-dimethylphenylsulfonyl)-1,2,3,4-tetrahydrochinolin-7-yl)essigsäure (E-10) | 8-(Pyridin-4-yl)-2,8-diazaspiro[4.5]decan (F-23) | 66% (0.15 g) | R₁ = 3.5 min; *m*/*z* = 589.3 [MH]⁺ | AAV XI |
| G-12 | 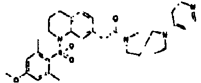 | 2-(1-(4-Methoxy-2,6-dimethylphenylsulfonyl)-1,2,3,4-tetrahydrochinolin-7-yl)-1-(7-(pyridin-4-yl)-2,7-diazaspiro[4.4]nonan-2-yl)ethanon (G-12) | 2-(1-(4-Methoxy-2,6 dimethylphenylsulfonyl)-1,2,3,4-tetrahydrochinolin-7-yl)essigsäure (E-10) | 2-(Pyridin-4-yl)-2,7-diazaspiro[4.4]nonan (F-24) | 90% (0.20 g) | Rₜ = 3.4 min; *m*/*z* = 575.3 [MH]⁺ | AAV XI |
| G-13 | 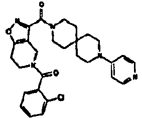 | (5-(2-Chlorbenzoyl)-4,5,6,7-tetrahydroisoxazolo[4,5-c]pyridin-3-yl)(9-(pyridin-4-yl)-3,9-diazaspiro[5.5]undecan-3-yl)methanon (G-13) | 5-(2-Chlorbenzoyl)-4,5,6,7-tetrahydroisoxazolo[4,5-c]pyridin-3-carbonsäure (E-12) | 3-(Pyridin-4-yl)-3,9-diazaspiro[5.5]undeca n Dihydrochlorid (F-09) | 84% (0.17 g) | Rₜ = 2.8 min; *m*/*z* = 520.2 [MH]⁺ | AAV XIII |
| G-16 | 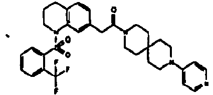 | 1-(9-(Pyridin-4-yl)-3,9-diazaspiro[5.5]undecan-3-yl)-2-(1-(2-(trifluormethyl)phenylsulfonyl)-1,2,3,4-tetrahydrochinolin-7- l yl)ethanon (G-16) | 2-(1-(2-(Trifluormethyl)phenylsulfon yl)-1,2,3,4-tetrahydrochinolin-7-yl)essigsäure (E-15) | 3-(Pyridin-4-yl)-3,9-diazaspiro[5.5]undeca n Dihydrochlorid (F-09) | 87% (0,21 g) | Rₜ = 3.5 min; *m*/*z* = 613.3 [MH]⁺ | AAV XI |
| G-17 | 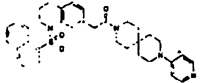 | 2-(1-(Naphthalin-1-ylsulfonyl)-1,2,3,4-tetrahydrochinolin-7-yl)-1-(9-(pyridin-4-yl)-3,9-diazaspiro[5.5]undecan-3-yl)ethanon (G-17) | 2-(1-(Naphthalin-1-ylsulfonyl)-1,2,3,4-tetrahydrochinolin-7-yl)essigsäure (E-16) | 3-(Pyridin-4-yl]-3,9-diazaspiro[5.5]undeca n Dihydrochlorid (F-09) | 73% (0.17 g) | Rₜ = 3.5 min; *m*/*z* = 595.4 [MH]⁺ | AAV XI |
| G-18 | 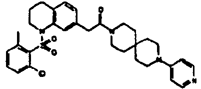 | 2-(1-(2-Chlor-6-methylphenylsulfonyl)-1,2,3,4-tetrahydrochinolin-7-yl)-1-(9-(pyridin-4-yl)-3,9-diazaspiro[5.5]undecan-3-yl)ethanon (G-18) | 2-(1-(2-Chlor-6-methylphenylsulfonyl)-1,2,3,4-tetrahydrochinolin-7-yl)essigsäure (E-17) | 3-(Pyridin-4-yl)-3,9-diazaspiro[5.5]undeca n Dihydrochlorid (F-09) | 85% (0.20 g) | Rₜ = 3.5 min; *m*/*z* = 593.3 [MH]⁺ | AAV XI |
| G-19 | 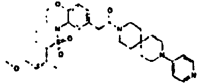 | 2-(4-(4-Methoxy-2,6-dimethylphenysulfonyl)-3,4-dihydro-2H-benzo[b][1,4]oxazin-6-yl)-1-(9-(pyridin-4-yl)-3,9-diazaspiro[5.5]undecan-3-yl)ethanon (G-19) | 2-(4-(4-Methoxy-2,6-dimethylphenylsulfonyl)-3,4-dihydro-2H-benzo[b]1,4]oxazin-6-yl)essigsäure (E-18) | 3-(Pyridin-4-yl)-3,9-diazaspiro[5.5]undeca n Dihydrochlorid (F-09) | 52% | Rₜ = 3.3 min; *m*/*z* = 605.3 [MH]⁺ | AAV XII |
| G-21 | 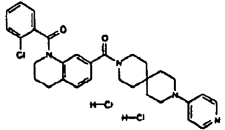 | (1-(2-Chlorbenzoyl)-1,2,3,4-tetrahydrochinolin-7-yl)(9-pyridin-4-yl)-3,9-diazaspiro[5.5]undecan-3 yl)methanon Dihydrochlorid (G-21) | 1-(2-Chlorbenzoyl)-1,2,3,4-tetrahydrochinolin-7-carbonsäure (E-06) | 3-(Pyridin-4-yl)-3,9-diazaspiro[5. 5]undeca n Dihydrochlorid (F-09) | 59% (0,1 g) | Rₜ = 3.0 min; *m*/*z* = 529.1 [MH]⁺ | AAV XI HCl Fällung" |
| G-24 | 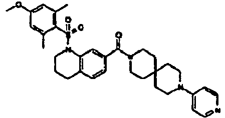 | (1-(4-Methoxy-2,6-dimethylphenylsufonyl)-1,2,3,4-tetrahydrochinolim-7-yl)(9-pyridin-4-yl)-3,9-diazaspiro[5.5 jundecan-3-yl)methanon (G-24) | 1-(4-Methoxy-2,6-dimethylphenylsulfonyl)-1,2,3,4-tetrahydrochinolin-7-carbonsäure (E-07) | 3-(Pyridin-4-yl)-3,9-diazaspiro[5.5]undeca n Dihydrochlorid (F-09) | 89% (0.14 g) | Rₜ = 3.5 min; *m*/*z* = 589.1 [MH]⁺ | AAV XI |
| G.25 | 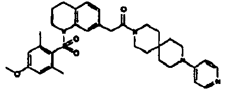 | 2-(1-(4-Methoxy-2,6-dimethylphenylsulfonyl)-1,2,3,4-tetrahydrochinolin-7-yl)-1-(9-(pyridin-4-yl)-3,9-diazaspiro[5.5]undecan-3-yl)ethanon (G-25) | 2-(1-(4-Methoxy-2,6-dimethylphenyl ulfonyl)-1,2,3,4-tetrahydrochinolin-7-yl)essigsäure (E-10) | 3-(Pyridin-4-yl)-3,9-diazaspiro[5.5]undeca n Dihydrochlorid (F-09) | 83% (0,13 g) | Rₜ = 3.5 min; *m*/*z* = 603.1 [MH]˙ | AAV XI |
| G-26 | 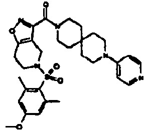 | (5-(4-Methoxy-2,6-dimethylphenylsulfonyl)-4,5,6,7-tetrahydroisoxazolo[4,5-c]pyridin-3-yl)(9-(pyridin-4-yl)3,9-diazaspiro[5.5]undecan-3-yl)methanon (G-26) | 5-(4-Methoxy-2,6-dimethylphenylsulfonyl)-4,5.6,7-tetrahydroisoxazolo[4,5-c]pyridin-3-carbonsäure (E-21) | 3-(Pyridin-4-yl)-3,9-diazaspiro[5.5]undeca n Dihydrochlorid (F-09) | 34% (0,11 g) | Rₜ = 3.2 min; *m*/*z* = 580.0 [MH]⁺ | AAV XIII |
| G-30 | 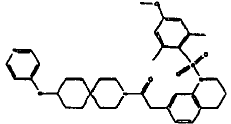 | 2-(1-(4-Methoxy-2,6-dimethylphenylsulfonyl)-1,2,3,4-tetrahydroCHinolin-7-yl)-1-(9-(pyridin-4-yloxy)-3-azaspiro[5.5]undecan-3-yl)ethanon (G-30) | 2-(1-(4-Methoxy-2,6-dimethylphenylsulfonyl)-1,2,3,4-tetrahydrochinolin-7-yl)essigsaure (E-10) | 9-(Pyridin-4-yloxy)-3-azaspiro[5.5]undecan (F-30) | 76% (0,12 g) | Rₜ = 3.7 min; *m*/*z* = 618.4 [MH]˙ | AAV XI |
| G-32 | 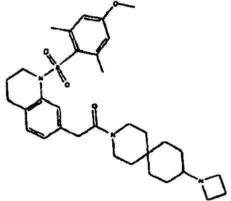 | 1-(9-(Azetidin-1-yl)-3-azaspiro[5.5]undecan-3-yl)-2-(1-(4-methoxy-2,6-dimethylphenylsulfonyl)-1,2,3,4-tetrahydrochinolin-7 -yl)ethanon (G-32) | 2-(1-(4-Methoxy-2,6-dimethylphenylsulfonyl)-1,2,3,4-tetrahydrochinollin-7-yl)essigsäure (E-10) | 9-(Azetidin-1-yl)-3-azaspiro[5.5]undecan (F-32) | 72% (0,16 g) | Rₜ = 3.4 min; *m*/*z* = 580.5[MH]⁺ | AAV XI |
| G-33 | 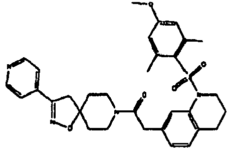 | 2-(1-(4-Methoxy-2,6-dimethylphenylsulfonyl)-1,2,3,4-tetrahydrochinolin-7-yl)-1-(3-(pyridin-4-yl-1-oxa-2,8-diazaspiro[4.5]dec-2-en-8-yl)ethanon (G-33) | 2-(1-(4-Methoxy-2,6-dimethylphenylsulfonyl)-1,2,3,4,tetrahydrochinolin-7-yl)essigsäure (E-10) | 3-(Pyridin-4-yl)-1-oxa-2.8-diazaspiro[4.5]dec-2-en (F-33) | 53% (0,08 g) | Rₜ = 4.2 min; *m*/*z* = 589.3 [MH]⁺ | AAV XI |
| G-34 | 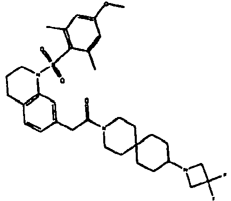 | 1-(9-(3,3-Difluorazetidin-1-yl)-3-azaspiro[5.5]undecan-3-yl)-2-(1-(4-methoxy-2,6-dimethylphenylsulfonyl)-1,2,3,4-tetrahydrochinolin-7-yl)ethanon (G-34) | 2-(1-(4-Methoxy-2,6-dimethylphenylsulfonyl)-1,2,3,4-tetrahydrochinolin-7-yl)essig0saure(E-10) | 9-(3,3-Difluorazedin-1-yl)-3-azaspiro[5.5)undecan (F-34) | 67% (0,16 g) | Rₜ = 3.7 min; *m*/*z* = 616.4 [MH]⁺ | AAV IXa |
| G-35 | 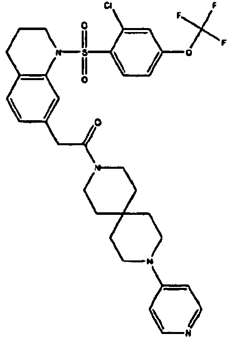 | 2-(1-(2-Chlor-4-(trifluormethoxy)phenylsulfonyl)-1,2,3,4-tetrahydrochinolin-7-yl)-1-(9-(pyridin-4-yl)-3,9-diazaspiro[5.5]undecan-3-yl)ethanon (G-35) | 2-(1-(2-Chlor-4-(trifluormethoxy)phenylsulfo nyl)-1,2,3,4-tetrahydrochinolin-7-yl)essigsäure (E-22) | 3-(Pyridin-4-yl)-3,9-diazaspiro[5.5]undeca n Dihydrochlorid (F-09) | 69% (0.26 g) | Rₜ = 3.9 min; *m*/*z* = 663.4 [MH]⁺ | AAV XI |
| G-36 | 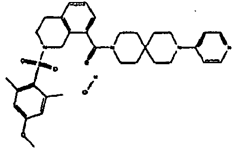 | (2-(4-Methoxy-2,6-dimethylphenylsulfonyl)-1,2,3,4-tetrahydroisochinolin-8-yl)(9-(pyridin-4-yl)-3,9-diazaspiro[5.5]undecan-3-yl)methanon Hydrochlorid (G-36) | 2-(4-Methoxy-2,6-dimethylphenylsulfonyl)-1,2,3,4-tetrahydroisochinolin-8-carbonsaure(E-23) | 3-(Pyridin-4-yl)-3,9-diazaspiro[5.5]undeca n Dihydrochlorid (F-09) | | | |
| G-37 | 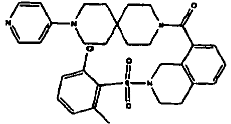 | (2-(2-Chlor-6-methylphenylsulfonyl)-1,2,3,4-tetrahydroisochinolin-8-yl)(9-(pyridin-4-yl)-3,9-diazaspiro[5.5]undecan-3-yl)methanon (G-37) | 2-(2-Chlor-6-methylphenylsulfonyl)-1,2,3,4-tetrahydroisochinolin-8-carbonsäure (E-24) | 3-(Pyridin-4-yl)-3,9-diazaspiro[5.5]undeca n Dihydrochlorid (F-09) | | | |
| G-38 | 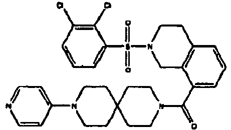 | (2-(2,3-Dichlorphenylsulfonyl)-1,2,3,4-tetrahydroisochinolin-8-yl)(9-(pyridin-4-yl)-3,9-diazaspiro[5.5]undecan-3-yl)methanon (G-38) | 2-(2,3-Dichlorphenylsulfonyl)-1,2,3,4-tetrahydroisochinolin-8-carbonsäure (E-25) | 3-(Pyridin-4-yl)-3,9-diazaspiro[5.5]undeca n Dihydrochlorid (F-09) | | | |
| G-39 | 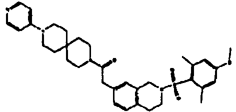 | 2-(2-(4-Methoxy-2,6-dimethylphenylsulfonyl)-1,2,3,4-tetrahydroisochinolin-7-yl)-1-(9-(pyridin-4-yl)-3,9-diazaspiro[5.5]undecan-3-yl)ethanon (G-39) | 2-(2-(4-Methoxy-2,6-dimethylphenylsulfonyl)-1,2,3,4-tetrahydroisochinolin-7-yl)essigsäure (E-26) | 3-(Pyridin-4-yl)-3,9-diazaspiro[5.5]undeca n (F-09) | 66% | Rₜ = 3.6 min; *m*/*z* = 603.5 [MH]⁺ | AAV XII |
| G-40 | 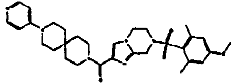 | (7-(4-Methoxy-2,6-dimethylphenylsulfonyl)-5,6,7,8-tetrahydroimidazo[1,2-a]pyrazin-2-yl)(9-(pyridin-4-yl)-3,9-diazaspiro[5.5]undecan-3-yl)methanon (G-40) | 7-(4-Methoxy-2,6-dimethylphenyl)sulfonyl)-5,6,7,8-tetrahydroimidazo[1,2-a]pyrazin-2-carbonsäure(E-27) | 3-(Pyridin-4-yl)-3,9-diazaspiro[5.5]undeca ne Dihydrochlorid (F-09) | 21% (0.04 g) | Rₜ = 2.9 min; *m*/*z* = 579.4 [MH]⁺ | AAV XV |
| G-41 | 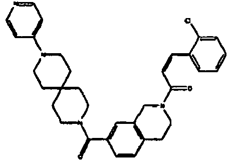 | 3-(2-Chlorphenyl)-1-(7-(9-(pyridin-4-yl)-3,9-diazaspiro[5.5]undecan-3-carbonyl)-3,4-dihydroisochinolin-2(1H)-yl)prop-2-en-1-on (G-41) | 2-(3-(2-Chlorphenyl)acryloyl)-1,2,3,4-tetrahydroisochinolin-7-carbonsäure (E-28) | 3-(Pyridin-4-yl)-3,9-diazaspiro[5.5]undeca n Dihydrochlorid (F-09) | | | |
| G-42 | 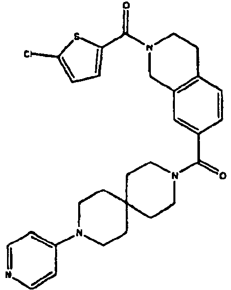 | (5-Chlorthiophen-2-yl)(7-(9-(pyridin-4-yl)-3,9-dizaspiro[5.5]undecan-3-carbonyl)-3,4-dihydroisochinolin-2(1H)-yl)methanon (G-42) | 2-(5-Chlorthiophen-2-carbonyl)-1,2,3,4-tetrahydroisochinolin-7-carbonsäure (E-29) | 3-(Pyridin-4-yl)-3,9-diazaspiro[5.5]undeca n Dihydrochlorid (F-09) | | | |
| G-43 | 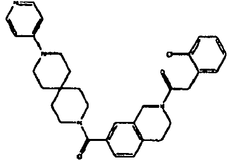 | 2-(2-Chlorphenyl)-1 -(7-(9-(pyridin-4-yl)-3,9-diazaspiro[5.5]undecan-3-Carbonyl)-3,4-dihydroisochinolin-2(1H)-yl)ethanon (G-43) | 2-(2-(2-Chlorphenyl)acetyl)-1,2,3,4-tetrahydroisochinolin-7-carbonsäure (E-30) | 3(Pyridin-4-yl)-3,9-diazaspiro[5.5]undeca n dihydrochlorid (F-09) | | | |
| G-51 | 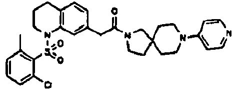 | 2-[1-[(2-Chlor-6-methylphenyl)sulfonyl]-1,2,3,4-tetrahydrochinolin-7-yl]-1-(8-pyridin-4-yl-3,8-diazaspiro[4.5]decan-3-yl)-ethanon (G-51) | 2-(1-(2-Chlor-6-methylphenylsufonyl)-1,2,3,4-tetrahydrochinolin-7-yl)essigsäure (E-17) | 8-(Pyridin-yl-2,8-diazaspiro[4.5]decan (F-23) | 94% (0.23 g) | Rₜ = 3.4 min; *m*/*z* = 579.3 [MH]⁺ | AAV XI |
| G-52 | 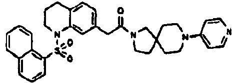 | 2-[1-(Naphthalin-1-yl-sulfonyl)-1,2,3,4-tetrahydro-chinolin-7-yl]-(8-pyridin-4-yl-3,8-diazaspiro[4.5]decan-3-yl)-ethanon (G-52) | 2-(1-(Naphthalin-1-ylsulfonyl)-1,2,3,4-tetrahydrochinolin-7-yl)essigsäure (E-16) | 8-(Pyridin-4-y)-2,8-diazaspiro[4.5]decan (F-23) | 61% (0,14 g) | Rₜ = 3.6 min; m/z = 581.3 [MH]⁺ | AAV XI |
| G.53 | 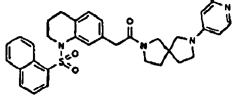 | 2-[1-(Naphthalin-1-ylsulfonyl)-1,2,3,4-tetrahydro-chinolin-7-yl]-1-(8-pyridin-4-yl-3,8-diazaspiro[4.4]nonan-3-yl)-ethanon (G-53) | 2-(1-(Naphthalin-1-ylsulfonyl)-1,2,3,4-tetrahydrochinolin-7-yl)essigsäure (E-16) | 2-(Pyridin-4-yl)-2,7-diazaspir[4.4]nonan (F-24) | 16% (0,17 g) | Rₜ = 3.4 min; *m*/*z* = 567.3 [MH]⁺ | AAV XI |
| G-54 | 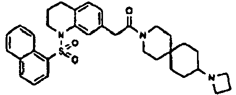 | 1-9-(Azetidin-1-yl)-3-azaspiro[5.5]undecan-3-yl]-2-[1-(nephthalin-1-ylsulfonyl)-1,2,3,4-tetrahydrochinolin-7-yl]-ethanon (G-54) | 2-(1-(Naphthalin-1-ylsulfonyl)-1,2,3,4-tetrahydrochinolin-7-yl)essigsäure (E-16) | 9-(Azetidin-1-yl)-3-azaspiro[5.5]undecan (F-32) | 76% (0.17 g) | Rₜ = 3.5 min; *m*/*z* = 572.3 [MH]⁺ | AAV XI |
| G.57 | 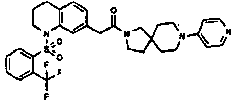 | 1-(8-Pyridin-4-yl-3,8-diazaspiro[4,5]decan-3-yl)-2-[1-[[2-(trifluormethyl)-phenyl[sulfonyl]-1,2,3,4-tetrahydro-chinolin-7-yl]-ethanon(G-57) | 2-(1-(2-(Trifluormethyl)phenylsulfon yl)-1,2,3,4-tetrahydrochinolin-7-yl)essigsäure (E-15) | 8-(Pyridin-4-yl)-2,8-diazaspiro[4.5]decan (F-23) | 93% (0,14 g) | Rₜ = 3.4 min; *m*/*z* = 599.3 [MH]⁺ | AAV XI |
| G-64 | 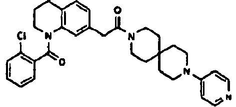 | 2-[1-(2-Chlor-benzoyl)-1,2,3,4-tetrahydro-chinolin-7-yl]-1-(9-pyridin-4-yl-3,9-diazaspiro[5.5]undecan-3-yl)-ethanon (G-64) | 2-(1-(2-Chlorbenzoyl)-1,2,3,4-tetrahydrochinolin-7-yl)essigsäure (E-11) | 3-(Pyridin-4-yl)-3,9-diazaspiro[5.5]undeca n dihydrochlorid (F-09) | 91% (0.15 g) | Rₜ = 3.1 min; *m*/z = 543.1 [MH]⁺ | AAV XI |
| G-66 | 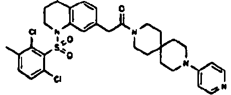 | 2-[1-[(2,6-Dichlor-3-methylphenyl)sulfonyl]-1,2,3,4-tetrahydrochinolin-7-yl]-1-(9-pyridin-4-yl-3,9-diazaspiro[5.5]undecan-3-yl)-ethanon (G-66) | 2-(1-(2,6-Dichlor-3-methylphenylsulfonyl-1,2,3,4-tetrahydrochinolin-7-yl)essigsäure (E-28) | 3-(Pyridin-4-yl)-3,9-diazaspiro[5.5]undeca n dihydrochlorid (F-09) | 88% (0,4 g) | Rₜ = 3.9 min; *m*/*z* = 627.2 [MH]⁺ | AAV XI |
| G-70 | 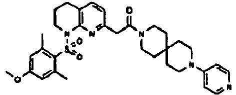 | 2-[8-[(4-Methoxy-2,6-dimethylphenyl)sulfonyl]-5,6,7,8-tetrahydro-[1,8]naphthyridin-2-yl]-1 -(9-pyridin-4-yl-3,9-diazaspiro[5.5]undecan-3-yl)-ethanon (G-70) | 2-(8-(4-Methoxy-2,6 dimethylphenylsulfonyl)-5,6,7,8-tetrahydro-1,8-naphthyridin-2-yl)essigsäure (E-29) | 3-(Pyridin-4-yl)-3,9-diazaspiro[5.5]undeca n dihydrochlorid (F-09) | 32% (0,04 g) | Rₜ = 3.5 min; *m*/*z* = 604.4 [MH]⁺ | AAV XI |
| G-72 | 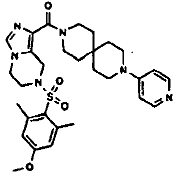 | [7-[(4-Methoxy-2,6-dimethylphenyl)sulfonyl]-5,6,7,9-tetrahydro-imidazo[1,5-a]pyrazin-1-yl]-9-pyridin-4-yl-3,9-diazaspiro[5.5]undecan-3-yl)-methanon (G-72) | 7-(4-Methoxy-2,6-dimethylphenylsulfonyl)-5,6,7,8-tetrahydroimidazo[1,5-a]pyrazin-1-carbonsäure (E-30) | 3-(Pyridin-4-yl)-3,9-diazaspiro[5.5]undeca n (F-09) | 32% | Rₜ = 3.3 min; *m*/*z* = 579.5 [MH]⁺ | AAV XVI |

| | | | | | | | |
|---|---|---|---|---|---|---|---|
| [1] Geräte und Methoden für die HPLC-MS Analytik: HPLC: Waters Alliance 2795 mit PDA Waters 2998; MS: Micromass Quattro Micro^{™} API ; Column: Waters Atlantis^{®} T3 , 3 µm, 100 Å, 2.1 x 30 mm; Säulentemperatur.: 40°C, Eluent A: gereinigtes Wasser +0.1% Ameisensäure; Eluent B: acetonitril (gradient grade) + 0.1% Ameisensäure; Gradient: 0% B to 100% B in 8.8 min, 100% B for 0.4 min, 100% B to 0% B in 0.01 min, 0% B for 0.8 min; Flow: 1.0 mL/min; Ionisation: ES+, 25 V; make up: 100 µL/min 70% methanol + 0.2% Ameisensäure; UV: 200 - 400 nm. | | | | | | | |

(a) Die Hydrochloridfällung erfolgte durch Zugabe von 2 M HCl in Diethylether zu einer Aceton / Diethylether Lösung der entsprechenden freien Base und anschließende Filtration.
   1 H NMR (600 MHz, DMSO-*d*₆) d ppm 2.09 (s, 3 H) 2.44 (s, 3 H) 2.60 (s, 3 H) 2.90 (t, *J*=5.29 Hz, 2 H) 3.52 (t, *J*=5.67 Hz, 2 H) 3.56 - 3.62 (m, 2 H) 3.65 (d, *J*=6.04 Hz, 2 H) 3.74 - 3.83 (m, 2 H) 3.86 (s, 3 H) 3.94 - 4.04 (m, 2 H) 4.22 (s, 2 H) 6.88 (s, 1 H) 6.99 (d, *J*=6.80 Hz, 2 H) 8.24 (d, *J*=5.29 Hz, 2 H)
**Beispielverbindung G-09:** 2-(1-(4-Methoxy-2,6-dimethylphenylsulfonyl)-1,2,3,4-tetrahydrochinolin-7-yl)-1-(2-(pyridin-4-yl)-2,8-diazaspiro[4.5]decan-8-yl)ethanon und
**Beispielverbindung G-10:** 1-(2-cyclobutyl-2,8-diazaspiro[4.5]decan-8-yl)-2-(1-(4-methoxy-2,6-dimethylphenylsulfonyl)-1,2,3,4-tetrahydrochinolin-7-yl)ethanon Hydrochlorid

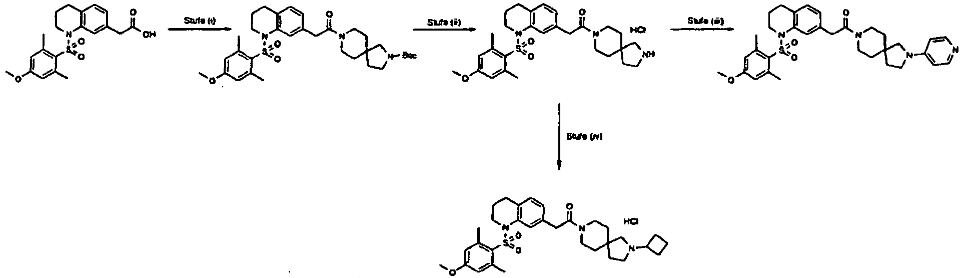

### Stufe (i): tert-Butyl 8-(2-(1-(4-methoxy-2,6-dimethylphenylsulfonyl)-1,2,3,4-tetrahydrochinolin-7-yl)acetyl)-2,8-diazaspiro[4.5]decan-2-carboxylat

Die Zielverbindung wurde nach der allgemeinen Arbeitsvorschrift AAV X (TBTU-Kupplung) aus den Edukten 2-(1-(4-Methoxy-2,6-dimethylphenylsulfonyl)-1,2,3,4-tetrahydrochinolin-7-yl)essigsäure (**E-10**) und tert-Butyl 2,8-diazaspiro[4.5]decan-2-carboxylat (**F-26**) hergestellt.
Ausbeute: 53%

### Stufe (ii): 2-1-(4-Methoxy-2,6-dimethylphenylsulfonyl)-1,2,3,4-tetrahydrochinolin-7-yl)-1-(2,8-diazaspiro[4.5]decan-8-yl)ethanon hydrochlorid

Zu einer Lösung tert-butyl 8-(2-(1-(4-Methoxy-2,6-dimethylphenylsulfonyl)-1,2,3,4-tetrahydrochinolin-7-yl)acetyl)-2,8-diazaspiro[4.5]decan-2-carboxylat (0.5 g, 0.817 mmol) in Methanol (5 ml) wurde bei Raumtemperatur Chlorwasserstoff (1.25 M Lösung in Methanol, 6.5 ml) hinzugegeben und das Reaktionsgemisch 1 h refluxiert. Das Lösungsmittel wurde unter Vakuum entfernt und der Rückstand in ein wenig Aceton aufgenommen und in gekühlten Diethylether getropft. Anschließend wurde 30 min im Eisbad gerührt und der entstandene Feststoff abfiltriert und getrocknet.
Ausbeute: 0.32 g (71%)

### Stufe (iii): 2-(1-(4-Methoxy-2,6-dimethylphenylsulfonyl)-1,2,3,4-tetrahydrochinolin-7-yl)-1-(2-(pyridin-4-yl)-2,8-diazaspiro[4.5]decan-8-yl)ethanon (G-09)

Ein Gemisch aus 2-(1-(4-Methoxy-2,6-dimethylphenylsulfonyl)-1,2,3,4-tetrahydrochinolin-7-yl)-1-(2,8-diazaspiro[4.5]decan-8-yl)ethanon Hydrochlorid (0.15 g, 0.274 mmol), 4-Chlorpyridiniumchlorid (0.12 g, 0.821 mmol) und Triethylamin (0.15 ml, 1.09 mmol) wurden in 1-Butanol (7 ml) 15 h refluxiert. Anschließend wurden gesättigte Natriumhydrogencarbonat-Lösung (20 ml) und Ethylacetat (50 ml) zugegeben, die Phasen getrennt und die wässrige Phase mit Ethylacetat (2 x 50 ml) extrahiert. Die vereinigten organischen Phasen wurden mit gesättigter Natriumchlorid-Lösung (20 ml) gewaschen, über Magnesiumsulfat getrocknet und unter Vakuum eingeengt. Das Rohprodukt wurde säulenchromatographisch (Kieselgel, Ethylacetat / Dichlormethan / Methanol / Ammoniak (25% aq), 300:100:50:1) aufgereinigt und die gewünschte Beispielverbindung **G-09** erhalten.
Ausbeute: 0.1 g (62%)
MS, Rₜ = 3.3 min; *m*/*z* = 589.1 [MH]⁺

### Stufe (iv): 1-(2-Cyclobutyl-2,8-diazaspiro[4.5]decan-8-yl)-2-(1-(4-methoxy-2,6-dimethylphenylsulfonyl)-1,2,3,4-tetrahydrochinolin-7-yl)ethanon Hydrochlorid (G-10)

2-(1-(4-Methoxy-2,6-dimethylphenylsulfonyl)-1,2,3,4-tetrahydrochinolin-7-yl)-1-(2,8-diazaspiro[4.5]decan-8-yl)ethanon Hydrochlorid (aus Stufe (ii)) (140 mg, 0.255 mmol), Triethylamin (0.04 ml, 0.255 mmol) und Cyclobutanon (0.02 ml, 0.255 mmol) wurden in 1,2-Dichlorethan (5 ml) gelöst und mit Natriumtriacetoxyborhydrid (75 mg, 0.358 mmol) und Eisessig (15 mg, 0.255 mmol) versetzt. Das Reaktionsgemisch wurde 15 h gerührt, anschließend mit Dichlormethan verdünnt und mit gesättigter Natriumhydrogencarbonatlösung (10 ml) versetzt. Nach Phasentrennung wurde die wässrige Phase mit Dichlormethan (3 x 30 ml) extrahiert. Die vereinigten organischen Phasen wurden mit gesättigter Natriumchloridlösung (30 ml) gewaschen, über Magnesiumsulfat getrocknet und unter Vakuum eingeengt. Das Rohprodukt wurde säulenchromatographisch (Kieselgel, Ethylacetat / Dichlormethan / Methanol / Ammoniak (25% aq), 300:100:25:1) gereinigt. Schließlich wurde aus etherischer Lösung (plus eine geringe Menge Aceton) mit Chlorwasserstoff in Ether (2 M) das Hydrochlorid gefällt und die gewünschte Beispielverbindung G-10 so erhalten.
Ausbeute: 100 mg (65%)
MS, Rₜ = 3.4 min; *m*/*z* = 566.1 [MH]⁺
**Beispielverbindung G-14:** (5-(4-Methoxy-2,6-dimethylphenylsulfonyl)-4,5,6,7-tetrahydrothieno[3,2-c]pyridin-2-yl)(9-(pyridin-4-yl)-3,9-diazaspiro[5.5]undecan-3-yl)methanon und
**Beispielverbindung G-15:** ((5-(2-Chlorbenzoyl)-4,5,6,7-tetrahydrothieno[3,2-c]pyridin-2-yl)(9-(pyridin-4-yl)-3,9-diazaspiro[5.5]undecan-3-yl)methanon

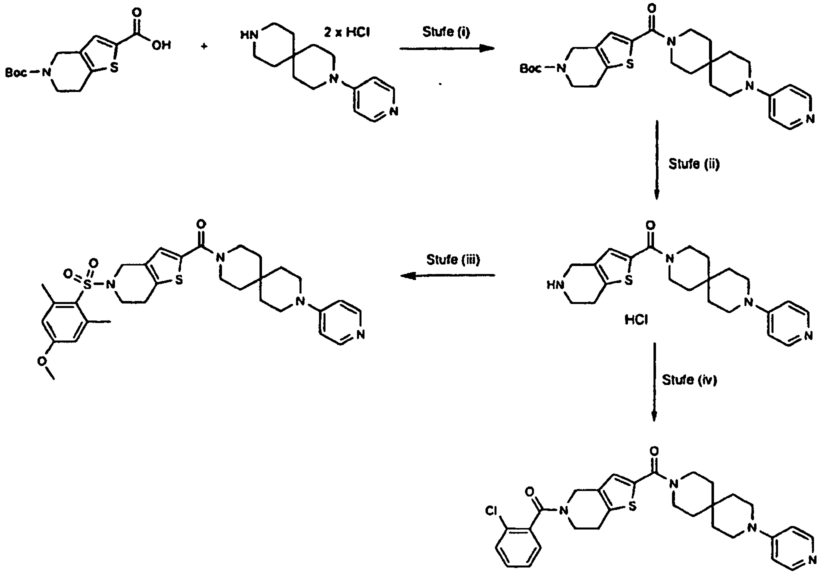

### Stufe (i): tert-Butyl 2-(9-(pyridin-4-yl)-3,9-diazaspiro[5.5]undecan-3-carbonyl)-6,7-dihydrothieno[3,2-c]pyridin-5(4H)-carboxylat

1,1'-Carbonyldiimidazol (119 mg, 0.741 mmol) und 5-(tert-Butoxycarbonyl)-4,5,6,7-tetrahydrothieno[3,2-c]pyridin-2-carbonsäure (200 mg, 0.706 mmol) wurden in Dichlormethan (5 ml) und N,N-Dimethylformamid (3 ml) gelöst und 1 h bei Raumtemperatur gerührt. Anschließend wurde 3-(Pyridin-4-yl)-3,9-diazaspiro[5.5]undecan Dihydrochlorid (322 mg, 1.059 mmol), gelöst in einem Gemisch aus Dichlormethan (5 ml) und Triethylamin (0.293 ml, 2.118 mmol), zugetropft und das Reaktionsgemisch 3 Tage bei Raumtemperatur gerührt. Es wurde mit Dichlormethan (50 ml) verdünnt und mit gesättigter Natriumhydrogencarbonat-Lösung (3 x 10 ml) sowie gesättigter Natriumchlorid-Lösung (10 ml) gewaschen, über Natriumsulfat getrocknet und unter Vakuum eingeengt. Die Reinigung des Rohproduktes erfolgte durch Säuelenchromatographie (Kieselgel, Ethylacetat / Methanol, 5:1).
Ausbeute: 280 mg (80%)

### Stufe (ii): (9-(Pyridin-4-yl)-3,9-diazaspiro[5.5]undecan-3-yl)(4,5,6,7-tetrahydrothieno[3,2-c]pyridin-2-yl)methanon Hydrochlorid

Zu einer Lösung von tert-Butyl 2-(9-(pyridin-4-yl)-3,9-diazaspiro[5.5]undecan-3-carbonyl)-6,7-dihydrothieno[3,2-c]pyridin-5(4H)-carboxylat (280 mg, 0.564 mmol) in Methanol (2 ml) wurde bei Raumtemperatur Chlorwasserstoff in Methanol (2.3 ml, 2.82 mmol, 1.25 mol/l) hinzugegeben und das Reaktionsgemisch 2 h refluxiert. Das Lösungsmittel wurde unter Vakuum entfernt und der Rückstand in ein wenig Ethanol (3 ml) aufgenommen und mit Diethylether (50 ml) versetzt. Anschließend wurde 30 min im Eisbad gekühlt, der entstandene Feststoff abfiltriert, mit Diethylether gewaschen und unter Vakuum getrocknet.
Ausbeute: 180 mg (73%)

### Stufe (iii): (5-(4-Methoxy-2,6-dimethylphenylsulfonyl)-4,5,6,7-tetrahydrothieno[3,2-c]pyridin-2-yl)(9-(pyridin-4-yl)-3,9-diazaspiro[5.5]undecan-3-yl)methanon (G-14)

(9-(Pyridin-4-yl)-3,9-diazaspiro[5.5]undecan-3-yl)(4,5,6,7-tetrahydrothieno[3,2-c]pyridin-2-yl)methanon Hydrochlorid (89 mg, 0.208 mmol) wurde in Dichlormethan (5 ml) gelöst, gekühlt und mit Triethylamin (0.07 ml, 0.52 mmol) versetzt. Bei 0°C wurde eine Lösung aus 4-Methoxy-2,6-dimethylbenzolsulfonylchlorid (50 mg, 0.208 mmol) in Dichlormethan (5 ml) zugetropft, dann 15 h bei Raumtemperatur gerührt. Gesättigte Natriumhydrogencarbonat-Lösung (10 ml) wurde zugegeben, das gemisch 15 min gerührt und die Phasen getrennt. Die wässrige Phase wurde mit Dichlormethan (30 ml) extrahiert, die vereinigten organischen Phasen anschließend mit gesättigter Natriumchlorid-Lösung gewaschen, über Natriumsulfat getrocknet und unter Vakuum eingeengt. Die Reinigung des Rohproduktes erfolgte durch Säulenchromatographie (Kieselgel, Ethylacetat / Methanol / Ammoniak (25% aq), 400:20:1).
Ausbeute: 70 mg (56%)
MS, Rₜ = 3.4 min; *m*/*z* = 595.3 [MH]⁺

### Stufe (iv): ((5-(2-Chlorbenzoyl)-4,5,6,7-tetrahydrothieno[3,2-c]pyridin-2-yl)(9-(pyridin-4-yl)-3,9-diazaspiro[5.5]undecan-3-yl)methanon (G-15)

(9-(Pyridin-4-yl)-3,9-diazaspiro[5.5]undecan-3-yl)(4,5,6,7-tetrahydrothieno[3,2-c]pyridin-2-yl)methanon Hydrochlorid (0.089 g, 0.208 mmol) wurde in Dichlormethan (4 ml) und N,N-Dimethylformamid (1 ml) gelöst und Triethylamin (0.072 ml, 0.52 mmol) hinzugegeben. Das Gemisch wurde mit einem Eisbad gekühlt und 2-Chlorbenzoyl chlorid (0.036 g, 0.208 mmol), gelöst in Dichlormethan (4 ml), wurde bei 0°C langsam zugegeben. Das Kühlbad wurde entfernt und das Reaktionsgemisch 15 h bei RT gerührt. Anschließend wurde mit gesättigter Natriumhydrogencarbonatlösung (15 ml) versetzt und die Phasen getrennt. Die wässrige Phase wurde mit Dichlormethan (25 ml) extrahiert, die vereinigten organischen Phasen mit gesättigter NaCl-Lösung (10 ml) und gesättigter Natriumchlorid-Lösung (10 ml) gewaschen, über Natriumsulfat getrocknet und unter Vakuum eingeengt. Das Rohprodukt wurde säulenchromatographisch (Kieselgel, Ethylacetat / Methanol / Ammoniak (25% aq), 500:100:1) aufgereinigt.
Ausbeute: 80 mg (72%)
MS, Rₜ = 3.0 min; *m*/*z* = 535.2 [MH]⁺

**Beispielverbindung G-20:** 2-(1-(4-Methoxy-2,6-dimethylphenylsulfonyl)indolin-6-yl)-1-(9-(pyridin-4-yl)-3,9-diazaspiro[5.5]undecan-3-yl)ethanon

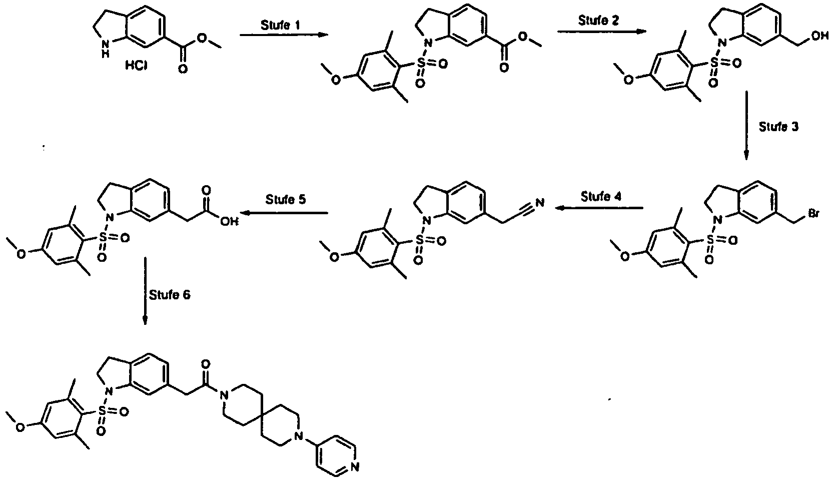

### Stufe-1: Methyl 1-(4-methoxy-2,6-dimethylphenylsulfonyl)indolin-6-carboxylat

Zu einer Lösung Methyl indolin-6-carboxylat hydrochlorid (1.2 g, 0.0056 mol) in trockenem Pyridin (12 ml) wurde 4-Methoxy-2,6-dimethylbenzol-1-sulfonyl chlorid (1.3 g, 0.0056 mol) bei 25°C unter Argon hinzugegeben und das resultierende Reaktionsgemisch über Nacht bei 70 °C gerührt. Das Lösungsmittel unter Vakuum entfernt, der Rückstand in Dichlormethan aufgenommen und mit Wasser (2 x), gesättigter Kupfersulfatlösung (2 x) und gesättigter Natriumchloridlösung gewaschen, anschließend getrocknet (Na₂SO₄) und unter Vakuum eingeengt. Das so erhaltene Rohprodukt wurde säulenchromatographisch (Kieselgel, 10-30% Ethylacetat in Hexan) aufgereinigt.
Ausbeute: 1.9 g (90%)

### Stufe-2: (1-(4-Methoxy-2,6-dimethylphenylsulfonyl)indolin-6-yl)methanol

Zu einer Lösung Methyl 1-(4-methoxy-2,6-dimethylphenylsulfonyl)indolin-6-carboxylat (1 g, 0.0027 mol) in trockenem THF (30 ml) wurde Lithiumborhydrid (0.232 g, 0.0107 mol) bei 0°C unter Argon hinzugegeben und das resultierende Reaktionsgemisch 2 h refluxiert. Das Lösungsmittel unter Vakuum entfernt und der Rückstand mit Wasser versetzt und mit Ethylacetat extrahiert. Die organische Phase wurde mit Wasser und gesättigter Natriumchloridlösung gewaschen, anschließend getrocknet (Na₂SO₄) und unter Vakuum eingeengt. Das so erhaltene Rohprodukt wurde ohne weitere Aufreinigung in die nachfolgende Stufe eingesetzt.
Ausbeute: 0.9 g (97%)

### Stufe-3: 6-(Bromomethyl)-1-(4-methoxy-2,6-dimethylphenylsulfonyl)indolin

Zu einer Lösung (1-(4-Methoxy-2,6-dimethylphenylsulfonyl)indolin-6-yl)methanol (0.9g, 0.0026mol) in trockenem DMF (10 ml) wurde Phosphortribromid (0.9 ml, 0.0090 mol) bei 0°C unter Argon hinzugegeben und das resultierende Reaktionsgemisch 4 h gerührt. Anschließend wurde das Gemisch mit Eis versetzt und mit Ethylacetat extrahiert. Die organische Phase wurde mit Wasser (2 x) und gesättigter Natriumchloridlösung gewaschen, anschließend getrocknet (Na₂SO₄), filtriert und unter Vakuum eingeengt. Das so erhaltene Rohprodukt wurde ohne weitere Aufreinigung in die nachfolgende Stufe eingesetzt.
Ausbeute: 0.7 g (66%)

### Stufe-4: 2-(1-(4-Methoxy-2,6-dimethylphenylsulfonyl)indolin-6-yl)acetonitril

Zu einer Lösung 6-(Brommethyl)-1-(4-methoxy-2,6-dimethylphenylsulfonyl)indolin (0.7 g, 0.0017 mol) in Methanol (14 ml) und Wasser (2 ml) wurde Kaliumcyanid (0.133 g, 0.0020 mol) hinzugegeben und das resultierende Reaktionsgemisch 4 h refluxiert. Anschließend wurde das Gemisch mit Ethylacetat (400 ml) verdünnt und mit gesättigter Natriumchloridlösung (2 x), Wasser (2 x), gesättigter Eisensulfatlösung und schließlich gesättigter Natriumchloridlösung gewaschen. Dann wurde getrocknet (Na₂SO₄), filtriert und unter Vakuum eingeengt. Das so erhaltene Rohprodukt wurde direkt in die nachfolgende Stufe eingesetzt.
Ausbeute: 0.6 g (99%)

### Stufe-5: 2-(1-(4-Methoxy-2,6-dimethylphenylsulfonyl)indolin-6-yl)essigsäure (E-19)

Zu einer Lösung 2-(1-(4-Methoxy-2,6-dimethylphenylsulfonyl)indolin-6-yl)acetonitril (0.6 g, 0.001 6mol) in Ethanol (12 ml) wurde 45% Kaliumhydroxid-Lösung (5 ml) gegeben und das resultierende Reaktionsgemisch 1 h refluxiert. Das Lösungsmittel unter Vakuum entfernt und der Rückstand mit Wasser versetzt und mit Ethylacetat extrahiert. Die wässrige Phase wurde mit Salzsäure tropfenweise in kalter Lösung auf pH ∼2 eingestellt und anschließend mit Ethalacetat extrahiert. Die organische Phase wurde mit gesättigter Natriumchloridlösung gewaschen, getrocknet (Na₂SO₄), filtriert und unter Vakuum eingeengt. Das so erhaltene Rohprodukt wurde direkt in die nachfolgende Stufe eingesetzt.
Ausbeute: 0.3 g (47%)

### Stufe-6: 2-(1-(4-Methoxy-2,6-dimethylphenylsulfonyl)indolin-6-yl)-1-(9-(pyridin-4-yl)-3,9-diazaspiro[5.5]undecan-3-yl)ethanon (G-20)

Zu einer Lösung 2-(1-(4-Methoxy-2,6-dimethylphenylsulfonyl)indolin-6-yl)essigsäure (E-19) (150mg 0.4mmol) in Dichlormethan (10 ml/mmol) wurde Diisopropylethylamin (0.3 ml, 1.6 mmol) bei 0°C hinzugegeben, gefolgt von HOBT (54 mg; 0.48 mmol) und EDCI (115 mg, 0.6 mmol). Das resultierende Reaktionsgemisch wurde 16 h bei 25 °C gerührt, anschließend mit Dichlormethan (30 ml) verdünnt und mit gesättigter Ammoniumchloridlösung, gesättigter Natriumchloridlösung und gesättigter Natriumhydrogencarbonatlösung gewaschen. Anschließend wurde getrocknet (Na₂SO₄ und unter Vakuum eingeengt. Das so erhaltene Rohprodukt wurde säulenchromatographisch (Alox-neutral, 0.5% Methanol in Dichlormethan) aufgereinigt.
Ausbeute: 150 mg (64%)
MS, Rₜ = 3.4 min; *m*/*z* = 589.4 [MH]⁺

**Beispielverbindungen G-41 bis G-43, G-45, G-47 bis G-50, G-55, G-56, G-58 bis G-63:**

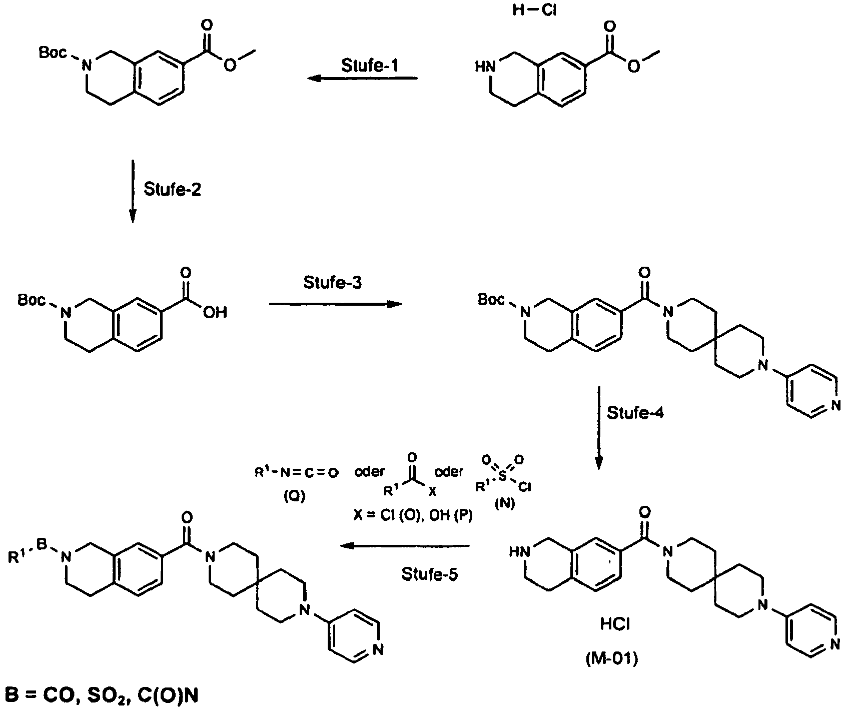

### Stufe-1: 2-tert-Butyl 7-methyl 3,4-dihydroisochinolin-2,7(1H)-dicarboxylat

Zu einer Lösung Methyl 1,2,3,4-tetrahydroisochinolin-7-carboxylat hydrochlorid (5 g, 0,021 mol) in Dichlormethan (42 ml) wurde bei 0°C Triethylamin (6,08 g, 0,0043 mol) und Di-tert-butyldicarbonat (5,6 g, 0,026 mol) gegeben und das resultierende Reaktionsgemisch 16 h gerührt. Das Reaktionsgemisch wurde mit dest. Wasser versetzt und die Phasen wurden getrennt. Die organische Phase wurde je 1x mit 1 M HCl und ges. NaCl-Lösung gewaschen, getrocknet (Na₂SO₄) filtriert und unter Vakuum eingeengt. Das so erhaltene Rohprodukt wurde direkt in die nachfolgende Stufe eingesetzt.
Ausbeute: 7,9 g (>99%)

### Stufe-2: 2-(tert-Butoxycarbonyl)-1,2,3,4-tetrahydroisochinolin-7-carbonsäure

Zu einer Lösung 2-tert-Butyl 7-methyl 3,4-dihydroisochinoline-2,7(1 H)-dicarboxylat (7,9 g, 0,027 mol) in Methanol (110 ml) wurde in Wasser (73 ml) gelöstes Lithiumhydroxid Monohydrat (5,8 g, 0,136 mol) gegeben und die Mischung 4 h gerührt. Das Lösungsmittel wurde im Vakuum entfernt. Der Rückstand wurde mit Wasser und Diethylether versetzt und die Phasen getrennt. Die wässrige Phase wurde mit verdünnter wässriger HCl-Lösung auf einen sauren pH eingestellt und mit Ethylacetat extrahiert. Die organische Phase wurde über Magnesiumsulfat getrocknet, filtriert und im Vakuum eingeengt. Das so erhaltene Rohprodukt wurde direkt in die nachfolgende Stufe eingesetzt.
Ausbeute: 5,8 g (76%)

### Stufe-3: tert-Buty17-(9-(pyridin-4-yl)-3,9-diazaspiro[5.5]undecane-3-carbonyl)-3,4-dihydroisochinolin-2(1H)-carboxylat

2-(tert-Butoxycarbonyl)-1,2,3,4-tetrahydroisochinolin-7-carbonsäure (2 g, 0,0072 mol), O-(1H-Benzotriazol-1-yl)-N,N,N',N'-tetramethyluronium tetrafluorborat (2,3 g, 0,0072 mol).) und 1-Hydroxybenzotriazolhydrat (0,99 g, 0,0072 mol) wurden unter Stickstoff in Tetrahydrofuran gelöst und das Gemisch 30 min bei RT gerührt. Eine Lösung 3-(Pyridin-4-yl)-3,9-diazaspiro[5.5]undecan dihydrochlorid (**F-09**) (2,18 g, 0,0072 mol.) und Diisopropylethylamin (DIPEA) (4,28 g, 0,025 mol) in Tetrahydrofuran wurde zugegeben und das Reaktionsgemisch 15 h bei RT gerührt. Tetrahydrofuran wurde anschließend im Vakuum entfernt, der Rückstand in Ethylacetat und ges. Natriumhydrogencarbonat-Lösung aufgenommen und die Phasen getrennt. Die wässrige Phase wurde mit Ethylacetat extrahiert und die vereinigten organischen Phasen wiederum mit ges. Natriumchloridlösung gewaschen, über Natriumsulfat getrocknet und im Vakuum eingeengt. Das Rohprodukt wurde säulenchromatographisch (Ethylacetat / Methanol 20:1 +1% 25%ige Ammoniak-Lösung (aq)) aufgereinigt.
Ausbeute: 1,91 g (54%)

### Stufe-4: (9-(Pyridin-4-yl)-3,9-diazaspiro[5.5]undecan-3-yl)(1,2,3,4-tetrahydroisochinolin-7-yl)methanon (M-01)

Zu einer Lösung von tert-Butyl 7-(9-(pyridin-4-yl)-3,9-diazaspiro[5.5]undecan-3-carbonyl)-3,4-dihydroisochinolin-2(1 H)-carboxylat (1,9 g, 3,87 mmol) in Methanol (4 ml) wurde bei RT 1.25 M Chlorwasserstoff in Methanol (15,5 ml, 19,36 mmol) hinzugegeben und das Reaktionsgemisch 1,5 h refluxiert. Das Lösungsmittel wurde im Vakuum entfernt und der Rückstand in ein wenig Ethanol (3 ml) aufgenommen und mit Diethylether (50 ml) versetzt. Anschließend wurde 30 min im Eisbad gekühlt, der entstandene Feststoff abfiltriert, mit Diethylether gewaschen und im Vakuum getrocknet.
Ausbeute: 1,56 g (94%)

### Stufe-5:

### Allgemeine Arbeitsvorschrift AAV [A]:

(9-(Pyridin-4-yl)-3,9-diazaspiro[5.5]undecan-3-yl)(1,2,3,4-tetrahydroisochinolin-7-yl)methanon **(M-01)** (1 Äquiv.) wurde in Dichlormethan gelöst, gekühlt und mit Triethylamin (2 Äquiv.) versetzt. Bei 0°C wurde eine Lösung aus dem Säurechlorid (○) (1 Äquiv.) in Dichlormethan zugetropft, dann 15 h bei RT gerührt. Ges. Natriumhydrogencarbonat-Lösung wurde zugegeben und die Phasen getrennt. Die wässrige Phase wurde mit Dichlormethan extrahiert, die vereinigten organischen Phasen anschließend mit ges. Natriumchlorid-Lösung gewaschen, über Natriumsulfat getrocknet und im Vakuum eingeengt. Die Reinigung des Rohproduktes erfolgte durch Säulenchromatographie (Kieselgel).

### Allgemeine Arbeitsvorschrift AAV [B]:

(9-(Pyridin-4-yl)-3,9-diazaspiro[5.5]undecan-3-yl)(1,2,3,4-tetrahydroisochinolin-7-yl)methanon **(M-01)** (1 Äquiv.) wurde in Dichlormethan gelöst, gekühlt und mit Triethylamin (2,5 Äquiv.) versetzt. Bei 0°C wurde eine Lösung aus dem Sulfonylchlorid **(N)** (1,2 Äquiv.) in Dichlormethan zugetropft, dann 15 h bei RT gerührt. Gesättigte Natriumhydrogencarbonat-Lösung wurde zugegeben und die Phasen getrennt. Die wässrige Phase wurde mit Dichlormethan extrahiert, die vereinigten organischen Phasen anschließend mit gesättigter Natriumchlorid-Lösung gewaschen, über Natriumsulfat getrocknet und im Vakuum eingeengt. Die Reinigung des Rohproduktes erfolgte durch Säulenchromatographie (Kieselgel).

### Allgemeine Arbeitsvorschrift AAV [C]:

Die Carbonsäure (**P**) (1 Äquiv.), O-(1H-Benzotriazol-1-yl)-N,N,N',N'-tetramethyluronium tetrafluorborat (1 Äquiv.) und 1-Hydroxybenzotriazolhydrat (1 Äquiv.) wurden unter Schutzgas in Tetrahydrofuran vorgelegt und 30 min bei RT gerührt. Eine Lösung (9-(Pyridin-4-yl)-3,9-diazaspiro[5.5]undecan-3-yl)(1,2,3,4-tetrahydroisochinolin-7-yl)methanon **(M-01)** (1 Äquiv.) und Diisopropylethylamin (DIPEA) (2 Äquiv.) in Tetrahydrofuran wurde zugegeben und das Reaktionsgemisch 15 h bei RT gerührt. Tetrahydrofuran wurde anschließend im Vakuum entfernt, der Rückstand in Dichlormethan und ges. Natriumhydrogencarbonat-Lösung aufgenommen und die Phasen getrennt. Die wässrige Phase wurde mit Dichlormethan extrahiert, die vereinigten organischen Phasen anschließend mit ges. Natriumchlorid-Lösung gewaschen, über Natriumsulfat getrocknet und im Vakuum eingeengt. Die Reinigung des Rohproduktes erfolgte durch Säulenchromatographie (Kieselgel).

### Allgemeine Arbeitsvorschrift AAV [D]:

(9-(Pyridin-4-yl)-3,9-diazaspiro[5.5]undecan-3-yl)(1,2,3,4-tetrahydroisochinolin-7-yl)methanon **(M-01)** (1 Äquiv.) wurde in Toluol und Triethylamin (1,2 Äquiv.) gelöst. Das Isocyanat **(Q)** (1 Äquiv.) wurde zugegeben, das Reaktionsgemisch 4 h refluxiert und 15 h bei Raumtemperatur gerührt. Toluol wurde im Vakuum entfernt, der Rückstand in Dichlormethan und ges. Natriumhydrogencarbonat-Lösung aufgenommen und die Phasen getrennt. Die wässrige Phase wurde mit Dichlormethan extrahiert, die vereinigten organischen Phasen anschließend mit ges. Natriumchlorid-Lösung gewaschen, über Natriumsulfat getrocknet und im Vakuum eingeengt. Die Reinigung des Rohproduktes erfolgte durch Säulenchromatographie (Kieselgel).

| Beispiel Nr. | Struktur | Name | Intermediat (M) | (N), (O), (P) oder (Q) | Ausbeute | Analytik (LC/MS)^{[1]} | Synthetisiert nach/ Kommentar |
|---|---|---|---|---|---|---|---|
| G-41 | 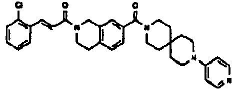 | 3-(2-Chlorphenyl)-1-(7-(9-(pyridin-4-yl)-3,9-diazaspiro[5.5]undecan-3-carbonyl)-3.4-dihydroisochinolin-2(1H)-yl)prop-2-en-1-on (G41) | (9-(Pyridin-4-yl)-3,9-diazaspiro[5.5]undecan-3-yl)(1,2,3,4-tetrahydroisochinolin-7-yl)methanon Hydrochlorid (M-01) | (E)-3-(2-Chlorphenyl)acryloyl chlorid (O-01) | 77% (0.18 mmol) | Rₜ = 3.4 min; *m*/*z* = 555.4 [MH]⁺ | AAV [A] |
| G-42 | 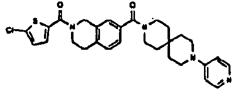 | (5-Chlorthiophen-2-yl)(7-(9-(pyridin-4-yl)-3,9-diazaspiro[5.5]undecan-3-carbonyl-3,4-dihydroisochinolin-2(1H)-yl)methanon (G-42) | (9-(Pyridin-4-yl)-3,9-diazaspiro[5.5]undecan-3-yl)(1,2,3,4-tetrahydroisochinolin-7-yl)methanon Hydrochlorid (M-01) | 5-Chlorthiophen-2-carbonsäure (P-01) | 96% (0,34 mmol) | Rₜ = 3.1 min; *m*/*z* = 535.3 [MH]⁺ | AAV [C] |
| G-43 | 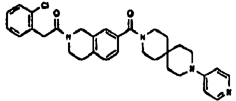 | 2-(2-Chlorphenyl)-1 -(7-(9-(pyridin-4-yl)-3,9-diazaspiro[5.5]undecan-3-carbonyl)-3,4-dihydroisochinolin-2(1H)-yl)ethanon (G-43) | (9-(Pyridin-4-yl)-3,9-diazaspiro(5.5]undecan-3-yl)(1,2,3,4-tetrahydroisochinolin-7-yl)methanon Hydrochlorid (M-01) | 2-(2-Chlorphenyl)acetyl Chlorid (O-02) | 71% (0,17 mmol) | Rₜ = 3.4 min; *m*/*z* = 543.4 [MH]⁺ | AAV [A] |
| G-45 | 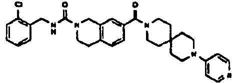 | N-[(2-Chlorphenyl)-methyl]-7-(9-Pyridin-4-yl-3,9-diazaspiro[5.5]undecan-3-carbonyl)-1,2,3,4-tetrahydro-isochinolin-2-carbonsäure amid (G-45) | (9-(Pyridin-4-yl)-3,9-diazaspiro[5. 5]undecan-3-yl)(1,2,3,4-tetrahydroisochinolin-7-yl)methanon Hydrochlorid (M-01) | 1-Chlor-2-(isocyanatomethyl)be nzol (O-01) | 45% (0.13 mmol) | Rₜ = 3.0 min; *m*/*z* = 558.4 [MH]⁺ | AAV [D] |
| G-47 | 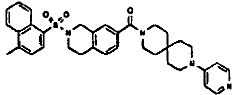 | [2-[(4-Methyl-naphthalin-1-yl)sulfonyl]-1,2,3,4-tetrahydroisochinolin-7-yl-3-(9-pyridin-4-yl-3. 9-diazaspiro[5.5]undecan-3-yl)-methanon (G-47) | (9-(Pyridin-4-yl)-3,9-diazaspiro[5.5]undecan-3-yl)(1,2,3,4-tetrahydroisochinolin-7-yl)methanon Hydrochlorid (M-01) | 4-Methylnaphthalin-1-sulfonyl chlorid (N-01) | 77% (0.26 mmol) | Rₜ = 3.7 min; *m*/*z* = 595.3 [MH]⁺ | AAV [B] |
| G-48 | 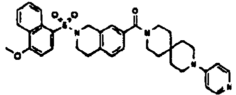 | [2-[(4-Methoxy-naphthalin-1-yl)sulfonyl]-1,2,3,4-tetrahydroisochinolin-7-yl]-(9-pyridin-4-yl-3,9-diazaspiro[5.5]undecan-3-yl)-methanon (G-48) | (9-(Pyridin-4-yl)-3,9-diazaspiro[5.5]undecan-3-yl)(1,2,3,4-tetrahydroisochinolin-7-yl)methanon Hydrochlorid (M-01) | 4-Methoxynaphthalin-1-sulfonyl chlorid (N-02) | 76% (0,21 mmol) | Rₜ = 3.7 min; *m*/z = 611.3 [MH]⁺ | AAV [B] |
| G-49 | 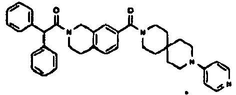 | 2,2-Diphenyl-1-[7-(9-pyridin-4-yl-3,9-diazaspiro[5.5]undecan-3-carbonyl)-1,2,3,4-tetrahydro-isochinolin-2-yl]-ethanon (G-49) | (9-(Pyridin-4-yl)-3,9-diazaspiro[5.5]undecan-3-yl)(1,2,3,4-tetrahydroisochinolin-7-yl)methanon Hydrochlorid (M-01) | 2,2-Diphenylacetyl chlorid (O-03) | 85% (0,24 mmol) | Rₜ = 3.5 min; *m*/*z* = 585.4 [MH]⁺ | AAV [A] |
| G-50 | 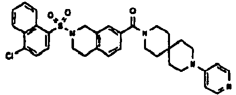 | [2-[(4-Chlor-naphthalin-1-yl)sulfonyl]-1,2,3,4-tetrahydro-isochinolin-7-yl]-(9-pyridin-4-yl-3,9-diazaspiro[5,5]undecan-3-yl)-methanon (G-50) | (9-(Pyridin-4-yl)-3,9-diazaspiro[5.5]undecan-3-yl)(1,2,3,4-tetrahydroisochinolin-7-yl)methanon Hydrochlorid (M-01) | 4-Chlornaphthalin-1-sulfonyl chlorid (N-03)) | 52% (0.15 mmol) | Rₜ =3.9 min; *m*/*z* = 615.3 [MH]⁺ | AAV [B] |
| G-55 | 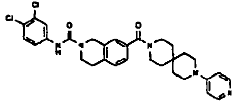 | N-(3,4-Dichlorphenyl)-7-(9-pyridin-4-yl-3,9-diazaspiro[5.5]undecan-3-carbonyl)-1,2,3,4-tetrahydroisochinolin-2-carbonsäure amid (G-55) | (9-(Pyridin-4-yl)-3,9-diazaspiro[5.5]undecan-3-yl)(1,2,3,4-tetrahydroisochinolin-7-yl)methanon Hydrochlorid (M-01) | 1,2-Dichlor-4-Isocyanatobenzol (Q-02) | 49% (0,13 mmol) | Rₜ = 3.5 min; *m*/*z* = 578.3 [MH]⁺ | AAV [D] |
| G-56 | 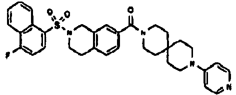 | (2-[(4-Fluor-naphthalin-1-yl)sulfonyl]-1,2,3,4-tetrahydro-isochinolin-7-yl]-(9-pyridin-4-yl-3,9-diazaspiro[5.5]undecan-3-yl)-methanon (G-56) | (9-(Pyridin-4-yl)-3,9-diazaspiro[5.5]undecan-3-yl)(1,2,3,4-tetrahydrisochinolin-7-yl)methanon Hydrochlorid (M-01) | 4-Fluornaphthalin-1-sulfonyl chlorid (N-04) | 53% (0,15 mmol) | Rₜ = 3.6 min; *m*/*z* = 599.3 [MH]⁺ | AAV [B] |
| G-58 | 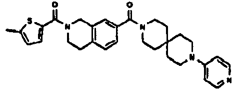 | [2-(5-Methyl-thiophen-2-carbonyl)-1,2,3,4-tetrahydro-isochinolin-7-yl]-(9-pyridin-4-yl-3,9-diazaspiro[5.5]undecan-3-yl)-methanon (G-58) | (9-(Pyridin-4-yl)-3,9-diazaspiro[5.5]undecan-3-yl)(1,2,3,4-tetrahydroisochinolin-7-yl)methanon Hydrochlorid (M-01) | 5-Methylthiophen-2-carbonyl chlorid (O-04) | 83% (0,23 mmol) | Rₜ = 3.0 min; *m*/*z* = 515.3 [MH]⁺ | AAV [A] |
| G-59 | 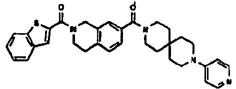 | [2-(Benzo[b]thiophen-2-carbonyl)-1,2,3,4-tetrahydro-isochinolin-7-yl]-(9-pyridin-4-yl-3,9-diazaspiro[5,5]undecan-3-yl)-methanon (G-59) | (9-(Pyridin-4-yl)-3,9-diazaspiro[5.5]undecan-3-yl)(1,2,3,4-tetrahydroisochinolin-7-yl)methanon Hydrochlorid (M-01) | Benzo[b]thiophen-2-carbonyl chlorid (0-05) | 32% (0,09 mmol) | Rₜ = 3.3 min; *m*/*z* = 551.3 [MH]⁺ | AAV [A] |
| G-60 | 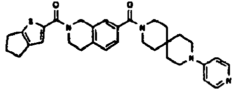 | [2-(5,6-Dihydro-4H-cyclopenta[b]phiophen-2-carbonyl)-1,2,3,4-tetrahydro-Isochinolin-7-yl]-(9-pyridin-4-yl-3,9-diazaspiro[5.5]undecan-3-yl)-methanon (G-60) | (9-(Pyridin-4-yl)-3,9-diazaspiro[5.5]undecan-3-yl)(1,2,3,4-tetrahydroisochinolin-7-yl)methanon Hydrochlorid (M-01) | 5,6-Dihydro-4H-cyclopenta[b]thiophen e-2-carbonyl chlorid (O-06) | 46% (0,13 mmol) | Rₜ = 3.2 min; *m*/*z* = 541,3 [MH]⁺ | AAV [A] |
| G-61 | 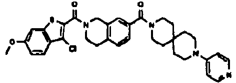 | [2-(3-Chlor-6-methoxy-benzo[b]thiophen-2-carbonyl)-1,2,3,4-tetrahydraisodinolim7.yl]-(9-pyridin-4-yl-3,9-diazaspiro[5.5]undecan-3-yl)-methanon (G-61) | (9-(Pyridin-4-yl)-3,9-diazaspiro[5.5]undecan-3-yl)(1,2,3,4-tetrahydroisochinolin-7-yl)methanon Hydrochlorid (M-01) | 3-Chlor-6-methoxybenzo[b]thiop hen-2-carbony chlorid (O-07) | 46% (0,16 mmol) | Rₜ = 3.7 min; *m*/*z*=6t5.2 [MH]⁺ | AAV [A] |
| G-62 | 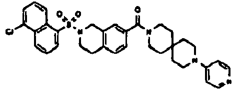 | [2-[(5-Chlor-naphthalin-1-yl)sulfonyl]-1,2,3,4-tetrahydro-isochinolin-1-yl]-(9-pyridin-4-yl-3,9-diazaspiro[5.5]undecan-3-yl)-methanon (G-62) | (9-(Pyridin-4-yl)-3,9-diazaspiro[5.5]undecan-3-yl)(1,2,3,4-tetrahydroisochinolin-7-yl)methanon Hydrochlorid (M-01) | 5-Chlornaphthalin-1-sulfonyl chlorid (N-05)) | 46% (0,13 mmol) | Rₜ = 3.9 min; *m*/*z* = 615.3 [MH]⁺ | AAV [B] |
| G-63 | 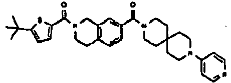 | [2-(5-tert-Butyl-thiophen-2-cerbonyl)-1,2,3,4-tetrahydro-isochinolin-7-yl]-(9-pyridin-4-yl-3,9-diazaspiro[5.5]undecan-3-yl)-methanon (G-63) | (9-(Pyridin-4-yl)-3,9-diazaspiro[5.5]undecan-3-yl)(1,2,3,4-tetrahydroisochinolin-7-yl)methanon Hydrochlorid (M-01) | 5-tert-Butylthiophen-2-carbonyl chlorid (O-08) | 68% (0,27 mmol) | Rₜ = 3.8 min; *m*/*z* = 557.3 [MH]⁺ | AAV [A] |

**Beispielverbindungen G-67 bis G-69 und G-73:**

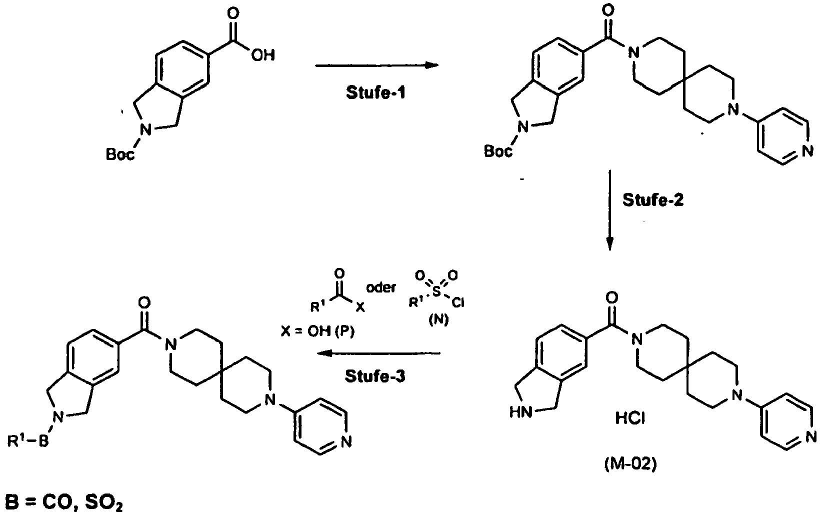

### Stufe-1: tert-Butyl 5-(9-(pyridin-4-yl)-3,9-diazaspiro[5.5]undecan-3-carbonyl)isoindolin-2-carboxylat

2-(tert-Butoxycarbonyl)isoindolin-5-carbonsäure (0,5 g, 0,0019 mol) ([CAS: 149353-71-9] kommerziell erhältlich bei z.B. Milestone), O-(1H-Benzotriazol-1-yl)-N,N,N',N'-tetramethyluronium tetrafluorborat (0,6 g, 0,0019 mol) und 1-Hydroxybenzotriazolhydrat (0,26 g, 0,0019 mol) wurden unter Schutzgas in Tetrahydrofuran gelöst und 30 min bei RT gerührt. Eine Lösung 3-(Pyridin-4-yl)-3,9-diazaspiro[5.5]undecan dihydrochlorid (F-09) (0,5 g, 0,0019 mol) und Diisopropylethylamin (DIPEA) (0,86 g, 0,066 mol) in Tetrahydrofuran wurde zugegeben und das Reaktionsgemisch 15 h bei RT gerührt. Tetrahydrofuran wurde anschließend im Vakuum entfernt, der Rückstand in Ethylacetat und ges. Natriumhydrogencarbonat-Lösung aufgenommen und die Phasen getrennt. Die wässrige Phase wurde mit Ethylacetat extrahiert und die vereinigten organischen Phasen wiederum mit ges. Natriumchloridlösung gewaschen, über Natriumsulfat getrocknet und im Vakuum eingeengt. Das Rohprodukt wurde säulenchromatographisch (Essigester / Methanol 10:1 + 1% Ammoniak-Lösung (25% aq)) aufgereinigt.
Ausbeute: 0,85 g (94%)

### Stufe-2: Isoindolin-5-yl(9-(pyridin-4-yl)-3,9-diazaspiro[5.5]undecan-3-yl)methanon (M-02)

Zu einer Lösung von tert-Butyl 5-(9-(pyridin-4-yl)-3,9-diazaspiro[5.5]undecan-3-carbonyl)isoindolin-2-carboxylat (0,85 g, 1,78 mmol) in Methanol (1 ml) wurde bei RT 1,25 M Chlorwasserstoff in Methanol (7,1 ml, 8,92 mmol) hinzugegeben und das Reaktionsgemisch 1 h refluxiert. Das Lösungsmittel wurde im Vakuum entfernt und der Rückstand in ein wenig Ethanol (2 ml) aufgenommen und mit Diethylether (50 ml) versetzt. Anschließend wurde 30 min im Eisbad gekühlt, der entstandene Feststoff abfiltriert, mit Diethylether gewaschen und im Vakuum getrocknet.
Ausbeute: 0,74 g (92%)

### Stufe-3:

### Allgemeine Arbeitsvorschrift AAV [E]:

Isoindolin-5-yl(9-(pyridin-4-yl)-3,9-diazaspiro[5.5]undecan-3-yl)methanon (**M-02**) (1 Äquiv.) wurde in Dichlormethan gelöst, gekühlt und mit Triethylamin (3 Äquiv.) versetzt. Bei 0°C wurde eine Lösung aus dem Sulfonylchlorid (**N**) (1 Äquiv.) in Dichlormethan zugetropft, dann 1 h bei RT gerührt. Ges. Natriumhydrogencarbonat-Lösung wurde zugegeben und die Phasen getrennt. Die wässrige Phase wurde mit Dichlormethan extrahiert, die vereinigten organischen Phasen über Natriumsulfat getrocknet und unter Vakuum eingeengt. Die Reinigung des Rohproduktes erfolgte durch Säulenchromatographie (Kieselgel)

### Allgemeine Arbeitsvorschrift AAV [F]:

Die Carbonsäure (**P**) (1 Äquiv.), O-(1H-Benzotriazol-1-yl)-N,N,N',N'-tetramethyluronium tetrafluorborat (1 Äquiv.) und 1-Hydroxybenzotriazolhydrat (1 Äquiv.) wurden unter Schutzgas in Tetrahydrofuran vorgelegt und 30 min bei RT gerührt. Eine Lösung Isoindolin-5-yl(9-(pyridin-4-yl)-3,9-diazaspiro[5.5]undecan-3-yl)methanon (**M-02**) (1 Äquiv.) und Diisopropylethylamin (DIPEA) (2,5 Äquiv.) in Tetrahydrofuran wurde zugegeben und das Reaktionsgemisch 15 h bei RT gerührt. Tetrahydrofuran wurde anschließend im Vakuum entfernt, der Rückstand in Dichlormethan und ges. Natriumhydrogencarbonat-Lösung aufgenommen und die Phasen getrennt. Die wässrige Phase wurde mit Dichlormethan extrahiert, die vereinigten organischen Phasen anschließend mit ges. Natriumchlorid-Lösung gewaschen, über Natriumsulfat getrocknet und im Vakuum eingeengt. Die Reinigung des Rohproduktes erfolgte durch Säulenchromatographie (Kieselgel).

### Allgemeine Arbeitsvorschrift AAV [G]:

Isoindolin-5-yl(9-(pyridin-4-yl)-3,9-diazaspiro[5.5]undecan-3-yl)methanon (**M-02**) (1 Äquiv.) wurde in DMF und 4-Methylmorpholin (4 Äquiv.)vorgelegt und die Carbonsäure (**P**) (1 Äquiv.) gefolgt von Benzotriazol-1-yloxytris(dimethylamino)phosphonium hexafluorphosphat (1,3 Äquiv.) hinzugegeben. Das Reaktionsgemisch wurde 15 h bei RT gerührt und anschließend das Lösungsmittel im Vakuum entfernt. Der Rückstand wurde in Dichlormethan und ges. Natriumhydrogencarbonat-Lösung aufgenommen und die Phasen getrennt. Die wässrige Phase wurde mit Dichlormethan extrahiert und die vereinigten organischen Phasen über Magnesiumsulfat getrocknet und im Vakuum eingeengt. Das Rohprodukt wurde säulenchromatographisch (Kieselgel) aufgereinigt.

| Beispiel Nr. | Struktur | Name | Intermediat (M) | (N), (O) oder (P) | Ausbeute | Analytik (LC/MS)^{[1]} | Synthetisiert nach / Kommentar |
|---|---|---|---|---|---|---|---|
| G-67 | 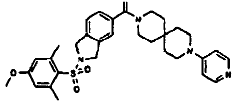 | [2-[(4-Metho)ty-2,6-dimethylphenyl)sulfonyl]2,3-dihydro-1H-isoindol-5-yl]-(9-pyridin-4-yl-3,9-diazaspiro[5.5]undecan-3-yl)-methanon (G-67) | Isoindolin-5-yl(9-(pyridin-4-yl)-3,9-diazaspiro[5.5]undecan-3-yl)methanon Hydrochlorid (M-02) | 4-Methoxy-2,6-dimethylphenyl-1-sulfonyl chlorid (N-06) | 78% (0,34 mmol) | Rₜ = 3.8 min; *m*/*z* = 575.3 [MH]⁺ | AAV [E] |
| G-68 | 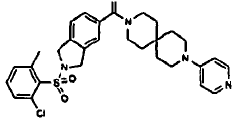 | [2-[(2-Chlor-6-methylphenyl)sulfonyl]-2,3-dihydro-1H-isoindol-5-yl]-(9-pyridin-4-yl-3,9-diazaspiro[5.5]undecan-3-yl)-methanon (G-68) | Isoindolin-5-yl(9-(pyridin-4-yl)-3,9-diazaspiro[5.5]undecan-3-yl)methanon Hydrochlorid (M-02) | 2-Chlor-6-methylphenyl-1-sulfonyl chlorid (N-07) | 72% (0,32 mmol) | Rₜ = 3.8 min; *m*/*z* = 565.2 [MH]⁺ | AAV [E] |
| G-69 | 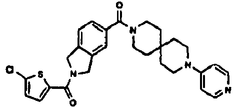 | {2-(5-Chlor-thiophen-2-carbonyl)-2,3-dihydro-1H-isoindol-5-yl]-(9-pyridin-4-yl-3,9-diazaspiro[5.5]undecan-3-yl)-methanon (G-69) | Isoindolin-5-yl(9-(pyridin-4-yl)-3,9-diazaspiro[5.5]undecan-3-yl)methanon Hydrochlorid (M-02) | 5-Chlorthiophen-2-carbonsäure (P-01) | 80% (0,23 mmol) | Rₜ = 3.4 min: *m*/*z* = 521.2 [MH]⁺ | AAV [F] |
| G-73 | 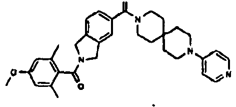 | [2-(4-Methoxy-2,6-dimethyl-benzoyl)-2,3-dihydro-1H-isoindol-5-yl]-(9-Pyridin-4-yl-3,9-diazaspiro[5.5]undecan-3-yl)-methanon (G-73) | Isolndolin-5-yl(9-(pyridin.4-yl)-3,9-diazaspiro[5.5]undecan-3-yl)methanon Hydrochlorid (M-02) | 4-methoxy-2,6-dimethylbenzoic acid (P-02) | 17% (0.07 mmol) | Rₜ = 3.3 min; *m*/*z* = 539.3 [MH]⁺ | AAV [G] |

**Beispielverbindungen G-36 bis G-38 und G-44:**

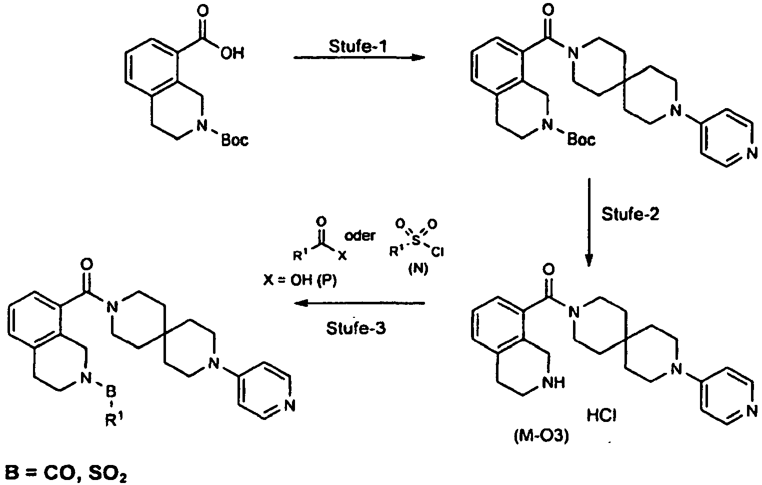

### Stufe-1: tert-Butyl 8-(9-(pyridin-4-yl)-3,9-diazaspiro[5.5]undecan-3-carbonyl)-3,4-dihydroisochinolin-2(1H)-carboxylat

3-(Pyridin-4-yl)-3,9-diazaspiro[5.5]undecan dihydrochlorid (F-09) (0,49 g, 1,62 mmol) wurde in DMF und Triethylamin (0,45 g, 3,25 mmol) gelöst und 2-(tert-Butoxycarbonyl)-1,2,3,4-tetrahydroisochinolin-8-carbonsäure (0,45 g, 1,62 mmol) (kommerziell erhältlich bei z.B. Ennova) gefolgt von Benzotriazol-1-yloxytris (dimethylamino)phosphonium hexafluorphosphat (0,93 g, 2,11 mmol) und 4-Methylmorpholin (0,53 g, 4,87 mmol) hinzugegeben. Das Reaktionsgemisch wurde 15 h bei RT gerührt und anschließend das Lösungsmittel im Vakuum entfernt. Der Rückstand wurde in Ethylacetat und ges. Natriumhydrogencarbonat-Lösung aufgenommen und die Phasen getrennt. Die wässrige Phase wurde mit Ethylacetat extrahiert und die vereinigten organischen Phasen mit ges.
Natriumhydrogencarbonat-Lösung und ges. Natriumchlorid-Lösung gewaschen, über Magnesiumsulfat getrocknet und im Vakuum eingeengt. Das Rohprodukt wurde säulenchromatographisch (Kieselgel, Ethylacetat / Methanol 20:1 + Ammoniak-Lösung (25% aq)) aufgereinigt.
Ausbeute: 0,78 g (98%)

### Stufe-2: (9-(Pyridin-4-yl)-3,9-diazaspiro[5.5]undecan-3-yl)(1,2,3,4-tetrahydroisochinolin-8-yl)methanon (M-03)

Zu einer Lösung von tert-Butyl 8-(9-(pyridin-4-yl)-3,9-diazaspiro[5.5]undecan-3-carbonyl)-3,4-dihydroisochinolin-2(1H)-carboxylat (0,77 g, 1,57 mmol) in Methanol (2 ml) wurde bei RT 1,25 M Chlorwasserstoff in Methanol (9,4 ml, 11,7 mmol) hinzugegeben und das Reaktionsgemisch 1 h refluxiert. Das Lösungsmittel wurde im Vakuum entfernt und der Rückstand in ein wenig Ethanol (2 ml) aufgenommen und mit Diethylether (50 ml) versetzt. Anschließend wurde 30 min im Eisbad gekühlt, der entstandene Feststoff abfiltriert, mit Diethylether gewaschen und im Vakuum getrocknet.
Ausbeute: 0,56 g (83%)

### Stufe-3:

### Allgemeine Arbeitsvorschrift AAV [H]:

Die Carbonsäure (P) (1,2 Äquiv.), O-(1H-Benzotriazol-1-yl)-N,N,N',N'-tetramethyluronium tetrafluorborat (1 Äquiv.) und 1-Hydroxybenzotriazolhydrat (1 Äquiv.) wurden unter Schutzgas in Tetrahydrofuran vorgelegt und 30 min bei RT gerührt. Eine Lösung (9-(Pyridin-4-yl)-3,9-diazaspiro[5.5]undecan-3-yl)(1,2,3,4-tetrahydroisochinolin-8-yl)methanon (**M-03**) (1 Äquiv.) und Diisopropylethylamin (DIPEA) (2 Äquiv.) in Tetrahydrofuran wurde zugegeben und das Reaktionsgemisch 15 h bei RT gerührt. Tetrahydrofuran wurde anschließend im Vakuum entfernt, der Rückstand in Dichlormethan und ges. Natriumhydrogencarbonat-Lösung aufgenommen und die Phasen getrennt. Die wässrige Phase wurde mit Dichlormethan extrahiert, die vereinigten organischen Phasen anschließend mit ges. Natriumchlorid-Lösung gewaschen, über Natriumsulfat getrocknet und im Vakuum eingeengt. Die Reinigung des Rohproduktes erfolgte durch Säulenchromatographie (Kieselgel).

### Allgemeine Arbeitsvorschrift AAV [I]:

(9-(Pyridin-4-yl)-3,9-diazaspiro[5.5]undecan-3-yl)(1,2,3,4-tetrahydroisochinolin-8-yl)methanon (**M-03**) (1 Äquiv.) wurde in Dichlormethan gelöst, gekühlt und mit Triethylamin (2,5 Äquiv.) versetzt. Bei 0°C wurde eine Lösung aus dem Sulfonylchlorid (**N**) (1 Äquiv.) in Dichlormethan zugetropft, dann 15 h bei RT gerührt. Ges. Natriumhydrogencarbonat-Lösung wurde zugegeben und die Phasen getrennt. Die wässrige Phase wurde mit Dichlormethan extrahiert, die vereinigten organischen Phasen über Natriumsulfat getrocknet und im Vakuum eingeengt. Die Reinigung des Rohproduktes erfolgte durch Säulenchromatographie (Kieselgel).

| Beispiel Nr. | Struktur | Name | Intermediat (M) | (N), (O) oder (P) | Ausbeute | Analytik (LC/MS)^{[1]} | Synthetisiert nach / Kommentar |
|---|---|---|---|---|---|---|---|
| G-36 | 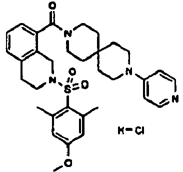 | (2-(4-Methoxy-2,6-dimethylphenylsulfonyl)-1,2,3,4- ' tetrahydroisochinolin-8-ylj(9-(pyridirr 4-yl)-3,9-diazaspiro[5.5jundecan-3-yl)methanon Hydrochlorid (G-36) | (9-(Pyridin-4-yl)-yl)-3,9-diazaspiro[5.5]undecan-3-yl)(1,2,3,4-tetrahydroisochinolin-8-yl)methanon Hydrochlorid (M-03) | 4-Methoxy-2,6-dimethylphenyl-1-sulfonyl chlorid (N-06) | 48% (0,16 mmol) | Rₜ = 3.4 min; *m*/*z* = 589.4 [MH]⁺ | AAV [I] Das Hydrochlorid wurde mit 2 M HCl in Diethytether gefällt. |
| G.37 | 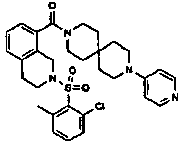 | (2-(2-Chlor-6-methylphenylsulfonyl)-1,2,3,4-tetrahydroisochinolin-8-yl)(9-(pyridin-4-yl)-3,9-diazaspiro[5.5]undecan-3-yl)methanon (G-37) | (9-(pyridin-4-yl)-3,9-diazaspiro[5.5]undecan-3-yl)(1,2,3,4-tetrahydroisochinolin-8-yl)methanon Hydrochlorid (M-03) | 2-Chlor-6-methylphenyl-1-sulfonyl chlorid (N-07) | 85% (0,26 mmol) | Rₜ = 3.5 min: *m*/*z* = 579.3 [MH]⁺ | AAV [I] |
| G-38 | 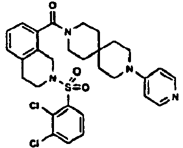 | (2-(2,3-Dichlororphenylsulfonyl)-1,2,3,4-tetrahydr&sochinolW49y#(9. (pyridin-4-yl)-3,9-diazaspiro[5.5]undecan-3-yl)methanon (G-38) | (9-(Pyridin-4-yl)-3,9-diazaspiro[5.5]undecan-3-yl)(1,2,3,4-tetrahydroisochinolin-8-yl)methanon Hydrochlorid (M-03) | 2,3-Dichlorphenyl-l-sulfonyl chlorid (N-10) | 88% (0,27 mmol) | Rₜ = 3,5 min; *m*/*z* = 599.2 [MH]⁺ | AAV [I] |
| G-44 | 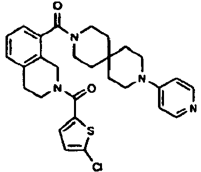 | (2-(5-Chlor-thiophen-2-carbonyl)-1,2,3,4-tetrahydro-isochinolin-8-yl)-(9-pyridin-4-yl-3,9-diazaspiro[5.5]undecan-3-yl)-methanon (G-44) | (9-(Pyridin-4-yl)-3,9-diazaspiro[5.5]undecan-3 yl)(1,2,3,4-tetrahydroisochinolin-8-yl)methanon Hydrochlorid (M-03) | 5-Chlorthiophen-2-carbonsäure (P-01) | 80% (0,28 mmol) | Rₜ = 3.2 min; *m*/*z* = 535.3 [MH]⁺ | AAV [H] |

**Beispielverbindung G-46: N-(3,4-Dichlorphenyl)-3-(9-pyridin-4-yl-3,9-diazaspiro[5.5]undecan-3-carbonyl)-4,5,6,7-tetrahydro-isoxazolo[4,5-c]pyridin-5-carbonsäure amid Hydrochlorid**

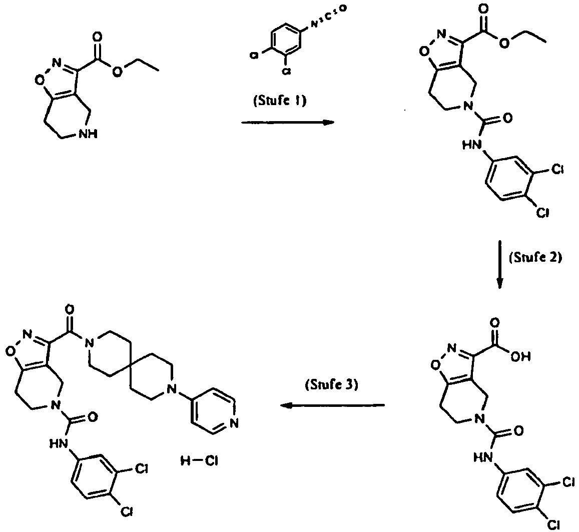

### Stufe-1: Ethyl 5-(3,4-Dichlorphenylcarbamoyl)-4,5,6,7-tetrahydroisoxazolo[4,5-c]pyridin-3-carboxylat

Ethyl 4,5,6,7-tetrahydroisoxazolo[4,5-c]pyridin-3-carboxylat (A-06) (Synthese siehe oben) (0,5 g, 2,55 mmol) wurde in Toluol (25 ml) gelöst und 1,2-Dichlor-4-isocyanatobenzol (0,48 g, 2,55 mmol) wurde zugegeben. Das Reaktionsgemisch wurde 2 h refluxiert. Toluol wurde im Vakuum entfernt, der Rückstand in Ethylacetat und ges. Natriumhydrogencarbonat-Lösung aufgenommen und die Phasen getrennt. Die wässrige Phase wurde mit Ethylacetat extrahiert, die vereinigten organischen Phasen anschließend mit ges. Natriumchlorid-Lösung gewaschen, über Natriumsulfat getrocknet und im Vakuum eingeengt. Die Reinigung des Rohproduktes erfolgte säulenchromatographisch (Kieselgel, Diethylether / Hexan 2:1)
Ausbeute: 1,2 g (>99%)

### Stufe-2: 5-(3,4-Dichlorphenylcarbamoyl)-4,5,6,7-tetrahydroisoxazolo[4,5-c]pyridin-3-carbonsäure

Zu einer Lösung Ethyl 5-(3,4-dichlorphenylcarbamoyl)-4,5,6,7-tetrahydroisoxazolo [4,5-c]pyridin-3-carboxylat (1,2 g, 3,019 mmol) in Methanol (25 ml) wurde in Wasser (8 ml) gelöstes Lithiumhydroxid Monohydrat (56%ig) (0,25 g, 6,038 mmol) gegeben und die Mischung 15 h gerührt. Das Lösungsmittel wurde im Vakuum entfernt. Die wässrige Phase wurde mit verdünnter HCI-Lösung (aq) auf einen sauren pH-Wert eingestellt und mit Ethylacetat extrahiert. Die organische Phase wurde über Natriumsulfat getrocknet, filtriert und im Vakuum eingeengt. Das so erhaltene Rohprodukt wurde direkt in die nachfolgende Stufe eingesetzt.
Ausbeute: 0,94 g (87%)

### Stufe-3: N-(3,4-Dichlorphenyl)-3-(9-(pyridin-4-yl)-3,9-diazaspiro[5.5]undecan-3-carbonyl)-6,7-dihydroisoxazolo[4,5-c]pyridin-5(4H)-carboxamid (G-46)

3-(Pyridin-4-yl)-3,9-diazaspiro[5.5]undecan Dihydrochlorid (0,17 g, 0,562 mmol) wurde in DMF (8,6 ml) und Triethylamin (0,15 ml, 1,123 mmol) gelöst und mit 4-Methylmorpholin (0,17 g, 1,685 mmol), gefolgt von 5-(3,4-Dichlorphenylcarbamoyl)-4,5,6,7-tetrahydroisoxazolo[4,5-c]pyridin-3-carbonsäure (0,2 g, 0,562 mmol) und Benzotriazol-1-yloxytris(dimethylamino)phosphonium hexafluorphosphat (0,32 g, 1,3 mmol).) versetzt. Das Reaktionsgemisch wurde 15 h bei RT gerührt und anschließend das Lösungsmittel im Vakuum entfernt. Der Rückstand wurde in Ethylacetat und ges. Natriumhydrogencarbonat-Lösung aufgenommen und die Phasen getrennt. Die wässrige Phase wurde mit Ethylacetat extrahiert und die vereinigten organischen Phasen anschließend mit ges. Natriumchlorid-Lösung und ges. Natriumhydrogencarbonat-Lösung gewaschen,über Magnesiumsulfat getrocknet und im Vakuum eingeengt. Das Rohprodukt wurde säulenchromatographisch (Kieselgel, Ethylacetat / Methanol 20:1 + 1% Ammoniak-Lösung (25% aq)) aufgereinigt. Das aufgereinigte Produkt wurde in Methylethylketon (2 ml) gelöst, bei 0°C mit 2 M Chlorwasserstoff in Diethylether (4 Äquiv.) versetzt und 1 h im Eisbad gerührt. Der Feststoff wurde abgesaugt, mit Diethylether gewaschen und im Vakuum getrocknet.
Ausbeute: 0,16 g (47%)
MS, Rₜ = 3.4 min; m/z = 569.3 [MH]⁺

**Beispielverbindung G-71: [7-(5-Chlor-thiophen-2-carbonyl)-5,6,7,8-tetrahydro-imidazo[1,2-a]pyrazin-2-yl]-(9-pyridin-4-yl-3,9-diazaspiro[5.5]undecan-3-yl)-methanon**

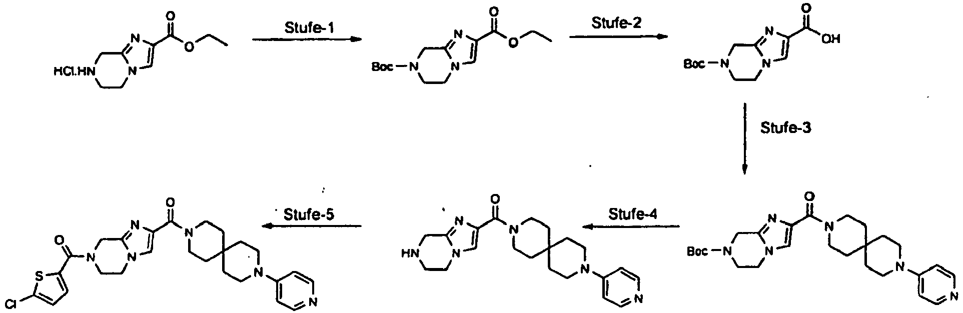

### Stufe-1: 7-tert-Butyl 2-ethyl 5,6-dihydroimidazo[1,2-a]pyrazin-2,7(8H)-dicarboxylat

Zu einer Lösung aus Ethyl 5,6,7,8-tetrahydroimidazo[1,2-a]pyrazin-2-carboxylat Hydrochlorid (3.33 mmol, 1.0 Äquiv.) in DCM (25 m) wurde bei 0 °C Et₃N (8.33 mmol, 2.5 Äquiv.) hinzugegeben und das Gemisch 10 min gerührt. Anschließend wurde Boc-anhydrid (4.99 mmol, 1.5 Äquiv.) bei 0 °C tropfenweise zu dem Gemisch gegeben und dann bei 25 °C 16 h gerührt. Das Reaktionsgemisch wurde mit DCM (100 ml) verdünnt und mit Waser (2 × 50 ml) und ges. NaCl-Lösung (2 × 50 ml) extrahiert. Die organische Phase wurde über Na₂SO₄ getrocknet und im Vakuum eingeengt. Das Rohprodukt wurde säulenchromatographisch (35 % Ethylacetat in Hexan) aufgereinigt und so das gewünschte Produkt als weißer Feststoff erhalten.
Ausbeute: 66 %

### Stufe-2: 7-(tert-Butoxycarbonyl)-5,6,7,8-tetrahydroimidazo[1,2-a]pyrazin-2-carbonsäure

Zu einem Lösung aus 7-tert-Butyl 2-ethyl 5,6-dihydroimidazo[1,2-a]pyrazin-2,7(8H)-dicarboxylat (2.2 mmol, 1.0 Äquiv.) in Methanol / Wasser (2.5:1, 35 ml) wurde bei 0 °C LiOH.H₂O (8.8 mmol, 4.0 Äquiv) gegeben und das Gemisch anschließend 2 h bei 25 °C gerührt. Nach vollständiger Umsetzung (DC-Kontrolle) wurde das Methanol im Vakuum entfernt, der Rückstand mit Wasser (40 ml) verdünnt und mit Ethylacetat (2 x 40 ml) extrahiert. Anschließend wurde die wässrige Phase mit 1 N HCl Lösung auf pH 2-3 eingestellt und der entstandene Feststoff abfiltriert. Der Feststoff wurde in Toluol aufgenommen und das Lösungsmittel anschließend im Vakuum entfernt (2 x), um das gewünschte Produkt als weißen Feststoff zu erhalten.
Ausbeute: 68 %

### Stufe-3: tert-Butyl 2-(9-(pyridin-4-yl)-3,9-diazaspiro[5.5]undecan-3-carbonyl)-5,6-dihydroimidazo[1,2-a]pyrazin-7(8H)-carboxylat

Zu einer Lösung aus 7-(tert-Butoxycarbonyl)-5,6,7,8-tetrahydroimidazo[1,2-a]pyrazin-2-carbonsäure (1.49 mmol, 1.0 Äquiv.) in DCM (20 ml) wurden bei 0 °C DIPEA (5.96 mmol, 4.0 Äquiv.), EDCI (2.24 mmol, 1.5 equiv.) und HOBt (2.24 mmol, 1.5 Äquiv.) gegeben und das Gemisch 30 min gerührt. Eine Lösung aus 3-(Pyridin-4-yl)-3,9-diazaspiro[5.5]undecan (**F-19**) (1.49 mmol, 1.0 Äquiv.) in DCM (5 ml) wurde bei 0 °C zugegeben und das Reaktionsgemisch anschließend 16 h bei 25 °C gerührt. Das Gemisch wurde mit DCM (100 ml) verdünnt und mit ges. Natriumcarbonat-Lösung (65 ml), ges. Ammoniumchlorid-Lösung (65 ml), Wasser (50 ml) und ges. Natriumchlorid-Lösung (50 ml) gewaschen, über Na₂SO₄ getrocknet und im Vakuum eingeengt. Das so erhaltene Rohprodukt wurde direkt in die nachfolgende Stufe eingesetzt.
Ausbeute: 63 %

### Stufe-4: (9-(Pyridin-4-yl)-3,9-diazaspiro[5.5]undecan-3-yl)(5,6,7,8-tetrahydroimidazo[1,2-a]pyrazin-2-yl)methanon

Zu einer gekühlten (0°C) Lösung aus tert-Butyl 2-(9-(pyridin-4-yl)-3,9-diazaspiro[5.5]undecan-3-carbonyl)-5,6-dihydroimidazo[1,2-a]pyrazin-7(8H)-carboxylat (0.86 mmol, 1.0 Äquiv.) in DCM (10 ml) wurde TFA (2.5 ml) gegeben und das Gemisch 2 h bei 25 °C gerührt. Nach vollständiger Umsetzung (DC-Kontrolle) wurde das Lösungsmittel im Vakuum entfernt um so das gewünschte Produkt als gelben Feststoff zu erhalten. Dieser wurde direkt ohne weitere Aufreinigung in die nachfolgende Stufe eingesetzt.

### Stufe-5: [7-(5-Chlor-thiophen-2-carbonyl)-5,6,7,8-tetrahydro-imidazo[1,2-a]pyrazin-2-yl]-(9-pyridin-4-yl-3,9-diazaspiro[5.5]undecan-3-yl)-methanon

Zu einer Lösung aus 5-Chlorthiophen-2-carbonyl chlorid (0.86 mmol, 1.0 Äquiv.) in DCM (20 ml) wurde bei 0 °C EDCI (1.29 mmol, 1.5 equiv.), HOBt (1.29 mmol, 1.5 Äquiv.) und DIPEA (3.44 mmol, 4 Äquiv.) gegeben und das Gemisch 30 min gerührt. Dann wurde eine Lösung aus tert-Butyl 2-(9-(pyridin-4-yl)-3,9-diazaspiro[5.5]undecan-3-carbonyl)-5,6-dihydroimidazo[1,2-a]pyrazin-7(8H)-carboxylat (0.86 mmol, 1.0 Äquiv.) in DCM (5 ml) zugegeben, das Eisbad entfernt und bei 25 °C 16 h gerührt. Die Reaktionsmischung wurde mit Dichlormethan (100 ml) verdünnt und mit ges. Natriumcarbonat-Lösung (65 ml), ges. Ammoniumchlorid-Lösung (65 ml), Wasser (50 ml) und ges. Natriumchlorid-Lösung (50 ml) gewaschen, über Na₂SO₄ getrocknet und im Vakuum eingeengt. Das Rohprodukt wurde säulenchromatographisch (8 % Methanol in DCM) gereinigt und so das gewünschte Produkt als weißer Feststoff erhalten.
Ausbeute: 11 %
MS, Rₜ = 2.7 min; *m*/*z* = 525.2 [MH]⁺

### Library Synthesen

### 7) Parallelsynthese der erfindungsgemäßen Verbindungen der allgemeinen Formel I

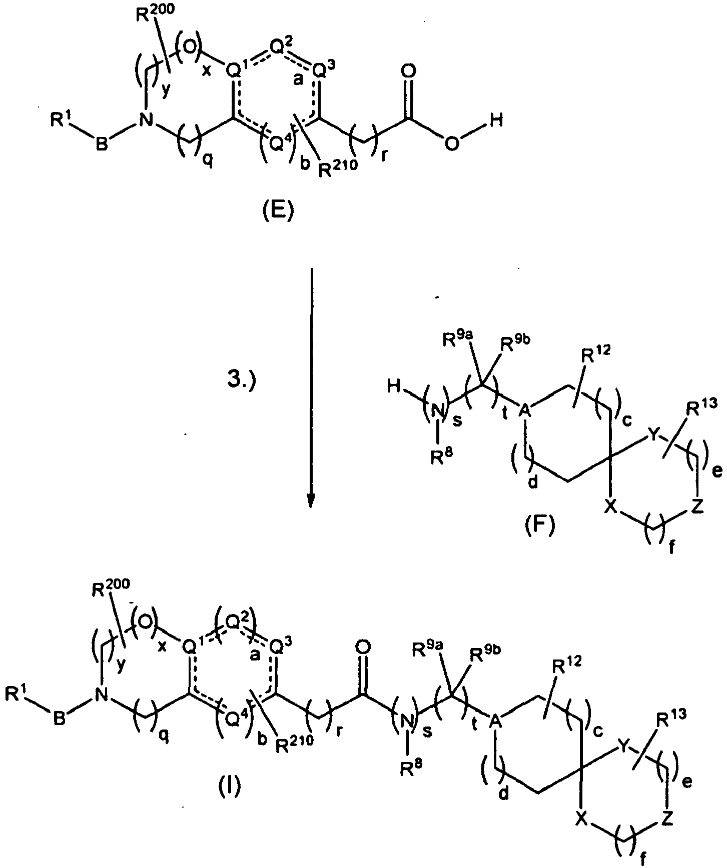

1)
   Gemäß vorstehendem Schema wurden die Säurebausteine E parallelsynthetisch mit den Aminen F zu den erfindungsgemäßen Spiroamiden der allgemeinen Formel (I) umgesetzt. Die Korrelation von Produkt zu Reagenz, Baustein und Methode ist der Synthesematrix zu entnehmen. Die Rohprodukte der Parallelsynthese wurden durch HPLC-MS analysiert^{[1]} und anschließend mittels Reverse Phase HPLC-MS aufgereinigt^{[1]}. Die Identität der Produkte konnte durch analytische HPLC-MS Messungen^{[3]} nachgewiesen werden.
2.)
   Zu einer Lösung von Amin F (100 µM), Hünigs Base (600 µM) und HOBT (5 µM) in 1 ml Tetrahydrofuran wurde zunächst eine Lösung der entsprechenden Säure E (100 µM) in 1 ml Tetrahydrofuran und anschließend eine Lösung von TBTU (125 µM) in 1 ml Acetonitril gegeben. Das Reaktionsgemisch wurde für 18 Stunden bei Raumtemperatur geschüttelt. Zur Aufarbeitung wurden die Ansätze mit 3 ml einer ½ gesättigten Natriumhydrogencarbonat Lösung und mit 3 ml Ethylacetat versetzt. Die weitere Aufarbeitung erfolgte auf einem Myriad-Allex-Aufarbeitungssystem (Firma: Mettler-Toledo). Nach Durchmischung wurde die organische Phase separiert, die wässrige mit 2x mit 3 ml Ethylacetat extrahiert und die organischen Phasen vereinigt. Die Entfernung des Lösungsmittels erfolgte unter Vakuum in Vakuumzentrifugen (Firma GeneVac). Die finale Aufreinigung erfolgte über HPLC-MS^{[3]}. Die finale Analytik wurde mittels LC-MS durchgeführt^{[2]}.
   [1] Geräte und Methoden für die HPLC-MS Analydik:
      Parallelsynthese Methode: HPLC: Waters Alliance 2795 with PDA Waters 2996; MS: ZQ 2000 MassLynx Single Quadrupol MS Detector; Column: Atlantis dC18 30 x 2.1 mm, 3 µm; Säulentemperatur: 40 °C, Eluent A: gereinigtes Wasser + 0.1% Ameisensäure; Eluent B: methanol (gradient grade) + 0.1% Ameisensäure; Gradient: 0% B to 100% B in 2.3 min, 100% B for 0.4 min, 100% B to 0% B in 0.01 min, 0% B for 0.8 min; Flow: 1.0 mL/min; Ionisation: ES+, 25V; make up: 100µl/min 70% methanol + 0.2% Ameisensäure; UV: 200 - 400 nm.
   [2] Geräte und Methoden für die HPLC-MS Aufreinigung: Prep Pump: Waters 2525; Make Up Pump: Waters 515; Auxilary Detektor: Waters DAD 2487; MS Detektor: Waters Micromass ZQ; Injector/Fraction Collector: Waters Sample Manager 2767; Gradient: Initial: 60% Water 40% Methanol -> 12-14.5 min: 0% Water 100% Methanol -> 14.5-15 min: 60% Water 40% Methanol; Flow: 35 ml/min Column: *Macherey-Nagel, C18 Gravity,* 100x21 mm, 5µ.
   [3] Geräte und Methoden für die HPLC-MS Analytik: HPLC: Waters Alliance 2795 with PDA Waters 2998; MS: Micromass Quattro Micro^{™} API ; Column: Waters Atlantis^{®} T3 , 3 µm, 100 Å, 2.1 × 30 mm; Col. temp.: 40 °C, Eluent A: gereinigtes Wasser + 0.1 % Ameisensäure; Eluent B: acetonitril (gradient grade) + 0.1% Ameisensäure; Gradient: 0% B to 100% B in 8.8 min, 100% B for 0.4 min, 100% B to 0% B in 0.01 min, 0% B for 0.8 min; Flow: 1.0 mL/min; Ionisation: ES+, 25 V; make up: 100 µL/min 70% methanol + 0.2% formic acid; UV: 200 - 400 nm.

| Beispiel Nr. | Struktur | Name | Aminosäure (E) | Amin (F) | Ausbeute | Analytik LC/MS)^{[1]} |
|---|---|---|---|---|---|---|
| G_CC-006 | 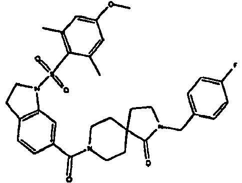 | 2-(4-Fluorbenzyl)-8-(1-(4-methoxy-2,6-dimethylphenylsulfonyl)indolin-6-carbonyl)-2,8-diazaspiro[4.5]decan-1-on | 1-(4-Methoxy-2,6-dimethylphenylsulfonyl)indoli n-6-carbonsäure (E-01) | 2-(4-Fluorbenzyl)-2,8-diazaspiro[4.5]decan-1-on hydrochlorid (F-17) | | Rₜ = 2.5 min; *m*/*z* = 606.1 [MH]⁺ |
| G_CC-007 | 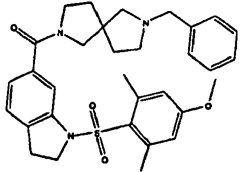 | (7-Benzyl-2,7-diazaspiro(4.4]nonan-2-yl)(1-(4-methoxy-2,6-dimethylphenylsulfonyl)indolin-6-yl)methanon | 1-(4-Methoxy-2,6-dimethylphenylsulfonyl)indoli n-6-carbonsäure (E-01) | 2-Benzyl-2,7-diazaspiro[4.4]nonan (F-19) | | Rₜ = 2.0min; *m*/*z =* 560.1 [MH]⁺ |
| G_CC-008 | 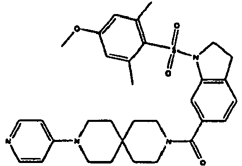 | (1-(4-Methoxy-2,6-dimethylphenylsulfonyl)indolin-6-yl)(9-(pyridin-4-yl)-3,9-diazaspiro[5.5]undecan-3-yl)methanon | 1-(4-Methoxy-2,6-dimethylphenylsulfonyl)indoli n-6-carbonsäure (E-01) | 3-(Pyridin-4-yl)-3,9-diazaspiro[5.5]undeca n dihydrochlorid (F-09) | | Rₜ = 2.0 min; *m*/*z* = 575.1 [MH]⁺ |
| G_CC-009 | 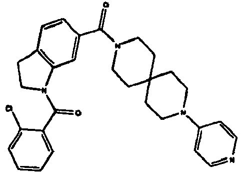 | (1-(2-Chlorbenzoyl)indolin-6-yl)(9-(pyridin-4-yl)-3,9-diazaspiro[5.5]undecan-3-yl)methanon | 1-(2-Chlorbenzoynindolin-Cr carbonsäure (E-02) | 3-(Pyridin-4-yl)-3,9-diazaspiro[5.5]undeca n dihydrochlorid (F-09) | | Rₜ = 1.9 min; *m*/*z* = 515.1 [MH]⁺ |
| G_CC-013 | 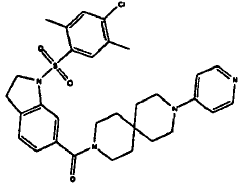 | (1-(4-Chlor-2,5-dimethylphenylsulftonyl)indolin-6-yl)(9-(pyridin-4-yl)-3,9-iazaspiro[5.5]undecan-3-yl)methanon | 1-(4-Chlor-2,5-dimethylphenylsuffonyl)indoli n-6-carbonsäure (E-03) | 3-(Pyridin-4-yl)-3,9-diazaspiro[5.5]undeca n dihydrochlorid (F-09) | | Rₜ = 2.1 min; *m*/*z* = 579.1 [MH]⁺ |
| G_CC-018 | 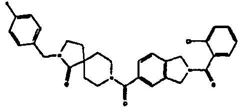 | 8-(2-(2-Chlorbenzoyl)isoindoün-5-carbonyl)-2-(4-fluorbenzyl)-2,8-diazaspiro[4.5]decan-1-on | 2-(2-Chlorbenzoyl)isoindolin-5-carbonsäure (E-05) | 2-(4-Fluorbenzyl)-2,8-diazaspiro[4.5]decan-1-on hydrochlorid (F-17) | | Rₜ = 2.3 min; *m*/*z = 546.1* [MH]⁺ |
| G_CC-025 | 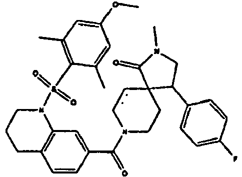 | 4-(4-Fluorphenyl)-8-(1-(4-methoxy-2,6-dimethylphenylsulfonyl)-1,2,3,4-tetrahydrochinolin-7-carbonyl)-2-methyl-2,8-diazaspiro[4.5]decan-1-on | 1-(4-Methoxy-2,6-dimethylphenylsulfonyl)-1,2,3,4-tetrahydrochinolin-7-carbonsäure (E-07) | 4-(4-Fluorphenyl)-2-methyl-2,8-diazaspiro[4.5]decan-1-on hydrochlorid (F-16) | | Rₜ = 2.5 min; *m*/*z* = 620.1 [MH]⁺ |
| G_CC-026 | 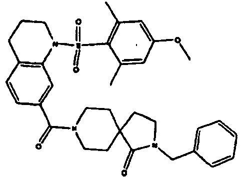 | 2-Benzyl-8-(1-(4-methoxy-2,6-dimethylphenylsulfonyl)-1,2,3,4-tetrahydrochinolin-7-carbonyl)-2,8-diazaspiro[4.5]decan-1-on | 1-(4-Methoxy-2,6-dimethylphenylsulfonyl)-1,2,3,4.tetrahydrochinolin-7-carbonsäure (E-07) | 2-Benzyl-2,8-diazaspiro[4.5]decan-1-on hydrochlorid (F-18) | | Rₜ = 2.5 min; *m*/*z* = 602.1 [MH]⁺ |
| G_CC-027 | 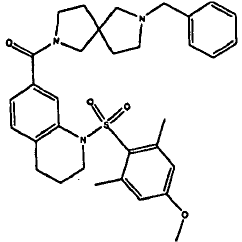 | (7-Benzyl-2,7-diazaspiro[4.4]nonan-2-yl)(1-(4-methoxy-2,6-dimethylphenylsulfonyl)-1,2,3,4-tetrahydrochinalin-7-yl)methanon | 1-(4-Methoxy-2,6-dimethylphenylsulfonyl)-1,2,3,4-tetrahydrochinolin-7-carbonsäure (E-07) | 2-Benzyl-2,7-diazaspiro[4.4]nonan (F-19) | | Rₜ = 2.0 min; *m*/*z* = 574.1 [MHF]⁺ |
| G_CC-039 | 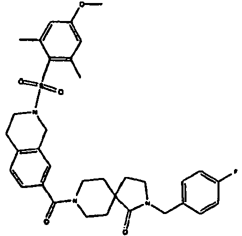 | 2-(4-Fluorbenzyl)-8-(2-(4-methoxy-2,6-dimethylphenylsulfonyl)-1,2,3,4-tetrahydroisochinolin-7-carbonyl)-2,8-diazaspiro]4.5]decan-1-on | 2-(4-Methoxy-2,6-dimethylphenylsulfonyl)-1,2,3,4-tetrahydroisochinolin-7-carbonsäure (E-08) | 2-(4-Fluorbenzyl)-2,8-diazaspiro[4.5]decan-1-on hydrochlorid (F-17) | | Rₜ = 2.5 min; *m*/*z* = 620.1 [MH]⁺ |
| G_CC-040 | 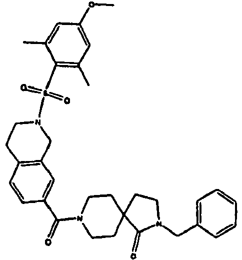 | 2-Benzyl-8-(2-(4-methoxy-2,6-dimethylphenylsulfonyl)-1,2,3,4-tetrahydroisochinolin-7-carbonyl)-2,8-diezaspiro[4.5]decan-1-on | 2-(4-Methoxy-2,6-dimethylphenylsulfenyl)-1,2,3,4-tetrahydroisochinolin-7-carbonsäure (E-08) | 2-Benzyl-2,8-diazaspiro[4.5]decan-1-on hydrochlorid (F-18) | | Rₜ = 2.5 min; *m*/*z* = 602.1 [MH]⁺ |
| G_CC-041 | 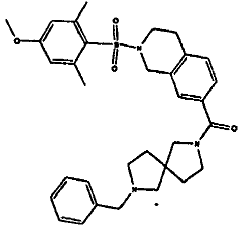 | (7-Benzyl-2,7-diazaspiro[4.4]nonan-2-yl)(2-(4-methoxy-2,6-dimethylphenylsulfonyl)-1,2,3.4-tetrahydroisochinolin-7-yl)methanon | 2-(4-Methoxy-2,6-dimethylphenylsulfonyl)-1,2,3,4-tetrahydroichinolin-7-carbonsäure (E-08) | 2-Benzyl-2,7-diazaspiro[4.4]nonan (F-19) | | Rₜ = 2.0 min; *m*/*z* = 574.2 [MH]⁺ |
| G_CC-043 | 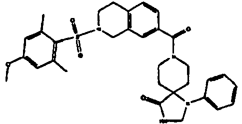 | 8-(2-(4-Methoxy-2,6-dimethylphenylsulfonyl)-1,2,3,4-tetrahydroisochinolin-7-carbonyl)-1-phenyl-1,3,8-triazaspiro[4.5]decan-4-on | 2-(4-Methoxy-2,6-dimethylphenylsulfonyl)-1,2,3,4-tetrahydroisochinolin-7-carbonsäure (E-08) | 1-Phenyl-1,3,8-triazaspiro[4.5]decan-4-on (F-14) | | Rₜ = 2.4 min; *m*/*z* = 589.1 [MH]⁺ |
| G_CC-045 | 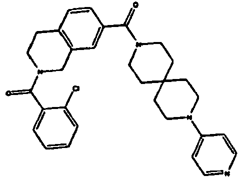 | (2-(2-Chlorbenzoyl)-1,2,3,4-tetrahydroisochinolin-7-yl)(9-(pyridin-4-yl)-3,9-diazaspiro[5.5]undecan-3-yl)methanon | 2-(2-Chlorbenzoylj-1,2,3,4-tetrahydroisochinolin-7-carbonsäure (E-09) | 3-(Pyridin-4-yl)-3,9-diazaspiro[5.5]undeca n dihydrochlorid (F-09) | | Rₜ = 1.9 min; *m*/*z* = 529.1 [MH]⁺ |
| G_CC-053 | 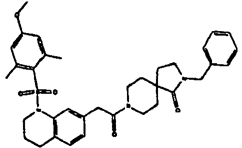 | 2-Benzyl-8-(2-(1-(4-methoxy-2,6-dimethylphenylsulfonyl)-1,2,3,4-tetrahydrochinolin-7-yl)acetyl)-2,8-diazaspiro[4.5]decan-1-on | 2-(1-(4-Methoxy-2,6-dimethylphenylsulfonyl)-1,2,3,4-terahydrochinolin-7-yl)essigsäure (E-10) | 2-Benzyl-2,8-diazaspiro[4.5]decan-1-on hydrochlorid (F-18) | | Rₜ = 2.5 min; *m*/*z* = 616.1 [MH]⁺ |
| G_CC-054 | 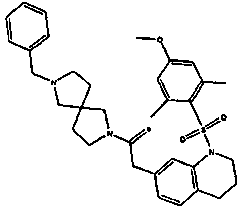 | 1-(7-Benzyl-2,7-diazaspiro[4.4]nonan-2-yl)-2-(1-(4-methoxy-2,6-dimethylphenylsulfonyl)-1.2.3,4-tetrahydrochinolin-7-yl)ethanon | 2-(1-(4-Methoxy-2,6-dimethylphenylsulfonyl)-1,2,3,4-tetrahydrochinolin-7-yl)essigsäure (E-10) | 2-Benzyl-2,7-diazaspiro[4.4]nonan (F-19) | | Rₜ = 2.0 min; *m*/*z* = 588.2 [MH]⁺ |
| G_CC-055 | 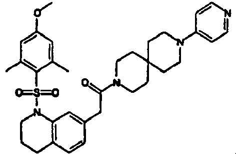 | 2-[1-[(4-Methoxy-2,6-dimethylphenyl)sulfonyl]-1,2,3,4-tetrahydrochinolin-7-yl]-1-(3-pyridin-4-yl-3,9-diazaspiro[5.5]undecan-9-yl)-ethanon | 2-(1-(4-Methoxy-2,6-dimethylphenylsulfonyl)-1,2,3,4-tetrahydrochinolin-7-yl)essigsäure (E-10) | 3-(Pyridin-4-yl)-3,9-diazaspiro[5.5]undeca n dihydrochlorid (F-09) | | Rₜ = 2.0 min; *m*/*z* = 603.2 [MH]⁺ |
| G_CC-056 | 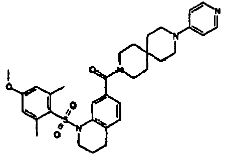 | [1-[(4-Methoxy-2,6-dimethylphenyl)sulfonyl]-1,2,3,4-tetrahydrochinolin-7-yl]-(3-pyridin-4-yl-3,9-diazaspiro[5.5]undecan-9-yl)-methanon | 1-(4-Methoxy-2,6-dimethylphenylsulfonyl)-1,2,3,4-tetrahydrochinolin-7-carbonsäure (E-07) | 3-(Pyridin-4-yl)-3,9-diazaspiro[5.5]undeca n dihydrochlorid (F-09) | | Rₜ = 2.0 min; *m*/*z* = 589.2 [MH]⁺ |

### Pharmakologische Daten

Die pharmakologischen Daten wurden wie obenstehend beschrieben bestimmt. Beispielhaft werden in der untenstehenden Tabelle die folgenden Daten angegeben:

| **Verbindung** | **B1R Antagonismus, Ratte [10 µM] % Inhibition** | **B1R Antagonismus, Human [10 µM] % Inhibition** |
|---|---|---|
| G-04 | 99 | 97 |
| G-09 | 96 | 96 |
| G-10 | 80 | 97 |
| G-11 | 99 | 99 |
| G-12 | 102 | 100 |
| G-13 | 94 | 100 |
| G-14 | 97 | 97 |
| G-15 | 81 | 66 |
| G-16 | 100 | 98 |
| G-17 | 89 | 97 |
| G-18 | 98 | 97 |
| G-19 | 100 | 100 |
| G-20 | 102 | 100 |
| G-21 | 101 | 95 |
| G-24 | 100 | 99 |
| G-25 | 98 | 100 |
| G-26 | 95 | 99 |
| G-30 | 98 | 46 |
| G-32 | 102 | 91 |
| G-33 | 73 | 65 |
| G-34 | 86 | 18 |
| G-35 | 101 | 79 |
| G-36 | 99 | 99 |
| G-37 | 90 | 99 |
| G-38 | 91 | 97 |
| G-39 | 89 | 99 |
| G-40 | 102 | 100 |
| G-41 | 90 | 99 |
| G-42 | 102 | 100 |
| G-43 | 102 | 99 |
| G-44 | 62 | 99 |
| G-45 | 97 | 97 |
| G-46 | 57 | 35 |
| G-47 | 80 | 72 |
| G-48 | 95 | 73 |
| G-49 | 106 | 99 |
| G-50 | 91 | 69 |
| G-51 | 106 | 100 |
| G-52 | 106 | 98 |
| G-53 | 105 | 95 |
| G-54 | 55 | 57 |
| G-55 | 94 | 92 |
| G-56 | 90 | 87 |
| G-57 | 101 | 100 |
| G-58 | 91 | 100 |
| G-59 | 95 | 86 |
| G-60 | 91 | 98 |
| G-61 | 91 | 77 |
| G-62 | 78 | 44 |
| G-63 | 80 | 83 |
| G-64 | 96 | 59 |
| G-66 | 100 | 98 |
| G-67 | 101 | 100 |
| G-68 | 81 | 90 |
| G-69 | 70 | 99 |
| G-70 | 101 | 88 |
| G-71 | 80 | 91 |
| G-72 | 104 | 100 |
| G-73 | 13 | 85 |
| G_CC-006 | 71 | |
| G_CC-007 | 59 | |
| G_CC-008 | 100 | |
| G_CC-009 | 78 | |
| G_CC-013 | 99 | 87 |
| G_CC-018 | 51 | |
| G_CC-025 | 51 | |
| G_CC-026 | 58 | |
| G_CC-027 | 58 | |
| G_CC-039 | 71 | |
| G_CC-040 | 70 | |
| G_CC-041 | 64 | |
| G_CC-043 | 74 | |
| G_CC-045 | 92 | |
| G_CC-053 | 84 | |
| G_CC-054 | 71 | |
| G_CC-055 | 98 | 96 |
| G_CC-056 | 100 | 98 |

## Patentansprüche

1. Substituierte Spiroamid-Verbindung der allgemeinen Formel (I), 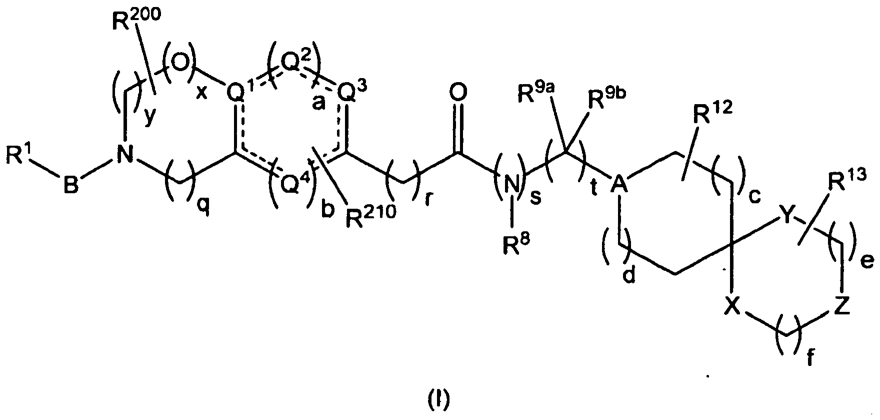 worin
Q¹ für C oder N steht;
Q² für CH, N, O oder S steht;
Q³ für CH, N, O oder S steht;
Q⁴ für CH, N, O oder S steht;
B für C(=O), S(=O)₂ oder für die Gruppe -C(=O)-N(R⁴) steht, wobei deren Stickstoffatom an den Rest R¹ gebunden ist;
a für 0, 1 oder 2 steht;
b für 0 oder 1 steht, mit der Maßgabe, dass a+b = 1 oder 2 ist; q für 0 oder 1 steht;
x für 0 oder 1 steht;
y für 1, 2 oder 3 steht;
r für 0, 1, 2 oder 3 steht;
R¹ für Aryl, Heteroaryl oder ein über eine C₁₋₃-Alkylengruppe oder C₂₋₆-Alkenylengruppe gebundenes Aryl oder Heteroaryl steht, wobei Aryl und Heteroaryl jeweils mit einem 4-, 5-, 6- oder 7- gliedrigen Cyclus oder Heterocyclus anelliert sein können, wobei der Cyclus und der Heterocyclus jeweils gesättigt oder wenigstens einfach ungesättigt, nicht aber aromatisch ist und jeweils an einem oder mehreren seiner Kohlenstoff-Ringglieder mit einem oder mehreren Resten unabhängig voneinander ausgewählt aus der Gruppe bestehend aus F, Cl, Br, I, -CF₃, -O-CF₃, C₁₋₆-Alkyl und O-C₁₋₆-Alkyl substituiert sein kann und wobei der Heterocyclus ein oder mehr Heteroatome oder Heteroatomgruppen unabhängig voneinander ausgewählt aus der Gruppe bestehend aus N, NR⁵⁰, O, S, S=O oder S(=O)₂ enthalten kann; wobei der Rest R⁵⁰ H, C₁₋₆-Alkyl, -C(=O)-R⁵¹, C₃₋₈-Cycloalkyl, Aryl, Heteroaryl oder ein über eine C₁₋₃-Alkylengruppe gebundenes C₃₋₈-Cycloalkyl, Aryl oder Heteroaryl bedeutet, und R⁵¹ C₁₋₆-Alkyl, C₃₋₈-Cycloalkyl, Aryl, Heteroaryl oder ein über eine C₁₋₃-Alkylengruppe gebundenes C₃₋₈-Cycloalkyl, Aryl oder Heteroaryl bedeutet;
R⁴ für H, C₁₋₆₋Alkyl, Aryl oder ein über eine C₁₋₃-Alkylengruppe gebundenes Aryl steht;
R²⁰⁰ für 0-4 Substituenten steht, die unabhängig voneinander ausgewählt sind aus der Gruppe bestehend aus C₁₋₆-Alkyl, F, Cl, CF₃ und OCF₃;
R²¹⁰ für 0-4 Substituenten steht, die unabhängig voneinander ausgewählt sind aus der Gruppe bestehend aus C₁₋₆-Alkyl, O-C₁₋₆-Alkyl, F, Cl, CF₃ und OCF₃;
s = 0 oder 1 ist,
t = 0, 1, 2 oder 3 ist mit der Maßgabe, dass wenn s für 0 steht t für 0 steht,
R⁸ für H, C₁₋₆-Alkyl, C₃₋₈-Cycloalkyl, Aryl oder Heteroaryl, oder für ein über eine C₁₋₆-Alkylengruppe gebundenes C₃₋₈-Cycloalkyl, Aryl oder Heteroaryl steht;
R^{9a} und R^{9b} jeweils unabhängig voneinander H, F, Cl, OH, C₁₋₆-Alkyl, O-C₁₋₆-Alkyl, C₃₋₈-Cycloalkyl, Aryl oder Heteroaryl, oder ein über eine C₁₋₆-Alkylengruppe gebundenes C₃₋₈-Cycloalkyl, Aryl oder Heteroaryl bedeuten;
A für N oder CH steht;
mit der Maßgabe, dass wenn s für 1 und t für 0 steht, A für CH steht; und mit der Maßgabe, dass wenn s und t jeweils für 0 stehen, A für N steht;
c, d, e und f jeweils unabhängig voneinander 0, 1 oder 2 bedeuten;
R¹² und R¹³ jeweils unabhängig voneinander für 0 bis 4 Substituenten stehen, die jeweils unabhängig voneinander ausgewählt sind aus der Gruppe bestehend aus F, Cl, OH, =O, C₁₋₆-Alkyl, O-C₁₋₆-Alkyl, C₃₋₈-Cycloalkyl, Aryl, Heteroaryl und über eine C₁₋₆-Alkylengruppe gebundenem C₃₋₈-Cycloalkyl, Aryl oder Heteroaryl;
und/oder zwei benachbarte der 0-4 Substituenten R¹³ ein anelliertes Aryl oder Heteroaryl bilden;
X für CR^{14a}R^{14b}, NR¹⁵ oder O steht;
Y für CR^{16a}R^{16b}, NR¹⁷ oder O steht;
mit der Maßgabe, dass X nicht NR¹⁵ bedeutet, wenn Y NR¹⁷ bedeutet; und
mit der Maßgabe, dass X und Y nicht gleichzeitig O bedeuten;
wobei
R^{14a}, R^{14b}, R^{16a} und R^{16b} jeweils unabhängig voneinander H, F, Cl, OH, C₁₋₆-Alkyl, O-C₁₋₆-Alkyl, C₃₋₈-Cycloalkyl, Aryl oder Heteroaryl bedeuten, oder für ein über eine C₁₋₆-Alkylengruppe gebundenes C₃₋₈-Cycloalkyl, Aryl oder Heteroaryl stehen,
und/oder jeweils R^{14a} und R^{14b} gemeinsam für =O und/oder jeweils R^{16a} und R^{16b} gemeinsam für =O stehen können;
R¹⁵ und R¹⁷ jeweils unabhängig voneinander für H, C₁₋₆-Alkyl, C₃₋₈Cycloalkyl, Aryl oder Heteroaryl stehen, oder ein über eine C₁₋₆-Alkylengruppe gebundenes C₃₋₈-Cycloalkyl, Aryl oder Heteroaryl bedeuten;
Z für CR^{18a}R^{18b}, NR¹⁹ oder O steht;
oder
Z in dem Fall, dass X für O und f für 0 steht, -(C(R¹²⁴)-C(R¹²⁵))-bedeutet, wobei
R¹²⁴ und R¹²⁵ gemeinsam mit den sie verbindenden Kohlenstoffatomen ein ankondensiertes Aryl oder Heteroaryl bilden; oder
Z in dem Fall dass X für O und f für 0 steht, -(N=CR¹²⁶)- bedeutet, wobei das N-Atom einfach an das O-Atom gebunden ist, und
R¹²⁶ für H, C₁₋₆-Alkyl, C₃₋₈-Cycloalkyl, Aryl oder Heteroaryl steht oder ein über eine C₁₋₆-Alkylengruppe gebundenes C₃₋₈-Cycloalkyl, Aryl oder Heteroaryl bedeutet; und wobei
R^{18a} für H, C₁₋₆-Alkyl, C₃₋₈-Cycloalkyl, Aryl oder Heteroaryl steht, oder ein über eine C₁₋₆-Alkylengruppe gebundenes C₃₋₈-Cycloalkyl, Aryl oder Heteroaryl bedeutet,
oder R^{18a} für eine Gruppe gemäß der allgemeinen Formel (II) steht, 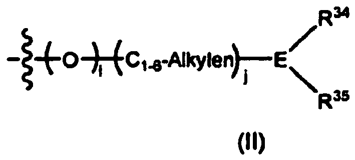 worin i und j jeweils unabhängig voneinander für 0 oder 1 stehen;
E für N oder CH steht, mit der Maßgabe, dass wenn i für 1 und j für 0 steht, E für CH steht
R³⁴ und R³⁵ jeweils unabhängig voneinander H, C₁₋₆-Alkyl, C₃₋₈-Cycloalkyl, Aryl oder Heteroaryl, oder ein über eine C₁₋₃-Alkylengruppe gebundenes Aryl, Heteroaryl oder C₃₋₈-Cycloalkyl bedeuten;
oder R³⁴ und R³⁵ unter Einschluss von E ein 5- oder 6-gliedriges Aryl oder Heteroaryl bilden;
oder R³⁴ und R³⁵ unter Einschluss von E einen gesättigten Heterocyclus gemäß der allgemeinen Formel (III) bilden, 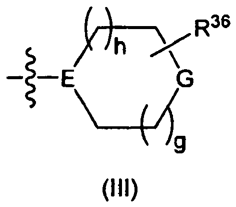 wobei
h und g unabhängig voneinander 0, 1 oder 2 bedeuten;
G für CR^{37a}R^{37b}, NR³⁸, O, S, S=O oder S(=O)₂ steht, mit der Maßgabe, dass wenn E für CH steht, G nicht für CR^{37a}R^{37b} steht;
R³⁶ für 0 bis 4 Substituenten steht, die jeweils unabhängig voneinander ausgewählt sind aus der Gruppe bestehend aus F, Cl, Br, I, OH, SH, =O, O-C₁₋₆-Alkyl, C₁₋₆-Alkyl, C₃₋₈-Cycloalkyl, Aryl, Heteroaryl und über eine C₁₋₆-Alkylengruppe gebundenem C₃₋₈-Cycloalkyl, Aryl oder Heteroaryl;
und/oder zwei benachbarte Substituenten R³⁶ zusammen ein anelliertes Aryl oder Heteroaryl darstellen;
R^{37a} und R^{37b} jeweils unabhängig voneinander H, F, Cl, Br, I, OH, SH, =O, O-C₁₋₆-Alkyl, C₁₋₆-Alkyl, C₃₋₈-Cycloalkyl, Aryl oder Heteroaryl bedeuten, oder für ein über eine C₁₋₆-Alkylengruppe gebundenes C₃₋₈-Cycloalkyl, Aryl oder Heteroaryl stehen;
R³⁸ für H, C₁₋₆-Alkyl, C₃₋₈-Cycloalkyl, Aryl oder Heteroaryl steht oder ein über eine C₁₋₃-Alkylengruppe gebundenes Aryl, Heteroaryl oder C₃₋₈-Cycloalkyl bedeutet;
R^{18b} für H, OH, C₁₋₆-Alkyl, C₃₋₈-Cycloalkyl, O-C₁₋₆-Alkyl, O-(C₃₋₈-Cycloalkyl), (C₁₋₆-Alkylen)-O-C₁₋₆-Alkyl, (C₁₋₆-Alkylen)-O-(C₃₋₈-Cycloalkyl), Aryl, Heteroaryl, O-Aryl oder O-Heteroaryl steht, oder ein über eine C₁₋₆-Alkylengruppe gebundenes Aryl, O-Aryl, Heteroaryl oder O-Heteroaryl bedeutet;
oder R^{18b} für eine Gruppe gemäß der allgemeinen Formel (IV) steht, 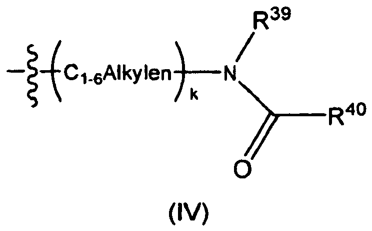 worin
k für 0 oder 1 steht;
R³⁹ für H, C₁₋₆-Alkyl, C₃₋₈-Cycloalkyl, Aryl oder Heteroaryl steht, oder ein über eine C₁₋₃-Alkylengruppe gebundenes C₃₋₈-Cycloalkyl, Aryl oder Heteroaryl bedeutet;
R⁴⁰ für C₁₋₆-Alkyl, C₃₋₈-Cycloalkyl. Aryl oder Heteroaryl steht, oder ein über eine C₁₋₆-Alkylengruppe gebundenes C₃₋₈-Cycloalkyl, Aryl oder Heteroaryl bedeutet;
oder
R³⁹ und R⁴⁰ gemeinsam mit der sie verbindenden N-C(=O)-Gruppe einen Ring gemäß der allgemeinen Formel (V) bilden, 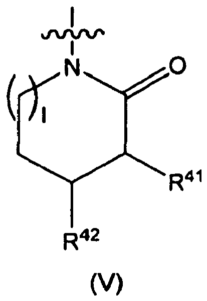 worin
I für 0, 1 oder 2 steht;
und R⁴¹ und R⁴² gemeinsam mit den sie verbindenden Kohlenstoffatomen ein anelliertes Aryl oder Heteroaryl bilden;
R¹⁹ für H; oder (P)_{z}-R²² steht,
worin
z für 0 oder 1 steht;
P für (C=O), S(=O)₂ oder C(=O)-N(R²⁴) steht, wobei das N-Atom in der Gruppe C(=O)-N(R²⁴) mit R²² verknüpft ist, wobei
R²⁴ für H, C₁₋₆-Alkyl, C₃₋₈-Cycloalkyl oder für ein über eine C₁₋₃-Alkylengruppe gebundenes Aryl, Heteroaryl oder C₃₋₈-Cycloalkyl steht;
R²² für C₁₋₆-Alkyl, Aryl oder Heteroaryl steht, oder ein über eine C₁₋₆-Alkylengruppe gebundenes Aryl oder Heteroaryl bedeutet; oder
R²² für eine Gruppe gemäß der allgemeinen Formel (VI) steht, worin 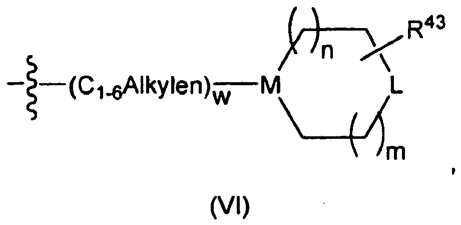
n für 0, 1 oder 2 steht;
m für 0, 1 oder 2 steht;
w für 0 oder 1 steht,
M für CH oder N steht;
mit der Maßgabe, dass wenn P für C(=O)-NR²⁴ und w für 0 steht, M für CH steht; und
mit der Maßgabe, dass wenn z und w gleichzeitig für 0 stehen, M für CH steht;
L für CR^{44a}R^{44b}, NR⁴⁵, O, S, S=O oder S(=O)₂ steht;
R⁴³ für 0 bis 4 Substituenten steht, die jeweils unabhängig voneinander ausgewählt sind aus der Gruppe bestehend aus F, Cl, OH, =O, C₁₋₆-Alkyl, O-C₁₋₆-Alkyl, C₃₋₈-Cycloalkyl,Aryl, Heteroaryl und über eine C₁₋₆-Alkylengruppe gebundenem C₃₋₈-Cycloalkyl, Aryl oder Heteroaryl;
und/oder zwei benachbarte der 0-4 Reste R⁴³ zusammen ein anelliertes Aryl oder Heteroaryl darstellen;
R^{44a} und R^{44b} jeweils unabhängig voneinander für H, F, Cl, Br, I, OH, C₁₋₆-Alkyl, O-C₁₋₆-Alkyl, C₃₋₈-Cycloalkyl, Aryl oder Heteroaryl stehen, oder ein über eine C₁₋₆-Alkylengruppe gebundenes C₃₋₈-Cycloalkyl, Aryl oder Heteroaryl bedeuten;
oder R^{44a} und R^{44b} gemeinsam für =O stehen können;
R⁴⁵ für H, C₁₋₆-Alkyl, C₃₋₈-Cycloalkyl, Aryl oder Heteroaryl steht, oder ein über eine C₁₋₃-Alkylengruppe gebundenes Aryl, Heteroaryl oder C₃₋₈-Cycloalkyl bedeutet;
wobei die oben genannten Reste C₁₋₆-Alkyl, C₁₋₃-Alkylen, C₁₋₆-Alkylen, C₂₋₆-Alkenylen, C₃₋₈-Cycloalkyl, Aryl und Heteroaryl jeweils unsubstituiert oder einfach oder mehrfach mit gleichen oder verschiedenen Resten substituiert sein können und die oben angegebenen Reste C₁₋₆-Alkyl, C₁₋₃-Alkylen, C₁₋₆-Alkylen und C₂₋₆-Alkenylen jeweils verzweigt oder unverzweigt sein können;
ggf. in Form eines einzelnen Enantiomers oder eines einzelnen Diastereomers, des Razemats, der Enantiomere, der Diastereomere, Mischungen von Enantiomeren und/oder Diastereomeren, sowie jeweils in Form ihrer Basen und/ oder physiologisch verträglichen Salze.

2. Substituierte Verbindung gemäß Anspruch 1, worin R¹ für Phenyl, Naphthyl, Chromanyl, Indolyl, Benzofuranyl, Benzothiophenyl (Benzothienyl), 5,6-Dihydro-4H-cyclopenta[b]thiophenyl, Benzooxazolyl, Benzooxadiazolyl, Pyrrolyl, Furanyl, Thienyl, Pyridinyl, Pyridazinyl, Pyrimidinyl, Pyrazinyl, Imidazothiazolyl, Carbazolyl, Dibenzofuranyl, Dibenzothiophenyl (Dibenzothienyl) oder für ein über eine C₁₋₃-Alkylengruppe oder eine C₂₋₃-Alkenylengruppe gebundenes Phenyl oder Naphthyl steht, bevorzugt für Phenyl, Naphthyl, Chromanyl, Benzothiophenyl (Benzothienyl), 5,6-Dihydro-4H-cyclopenta[b]thiophenyl, Benzooxadiazolyl, Thienyl, Pyridinyl, Imidazothiazolyl, Dibenzofuranyl oder für ein über eine C₁₋₃-Alkylengruppe oder eine C₂₋₃-Alkenylengruppe gebundenes Phenyl steht, besonders bevorzugt für Phenyl, Naphthyl, Chromanyl, Benzothiophenyl (Benzothienyl), 5,6-Dihydro-4H-cyclopenta[b]thiophenyl, Thienyl oder für ein über eine C₁ oder ₂-Alkylengruppe oder -CH=CH-Gruppe gebundenes Phenyl steht, wobei die vorstehend genannten Aryl oder Heteroaryl-Reste jeweils unsubstituiert oder einfach oder mehrfach, gleich oder verschieden, substituiert sind, wobei die Substituenten unabängig voneinander insbesondere ausgewählt sind aus der Gruppe bestehend aus -O-C₁₋₃-Alkyl, C₁₋₆-Alkyl, F, Cl, Br, I, CF₃, OCF₃, OH, SH, Phenyl, Phenoxy, Naphthyl, Furyl, Thienyl und Pyridinyl und wobei die vorstehend genannten Alkylen- und Alkenylengruppen jeweils unsubstituiert oder einfach oder mehrfach, gleich oder verschieden, substituiert sind, wobei die Substituenten unabängig voneinander insbesondere ausgewählt sind aus der Gruppe bestehend aus -O-C₁₋₃-Alkyl, F, Cl, Br, I, CF₃, OCF₃, OH, SH, Phenyl, Phenoxy, Naphthyl, Furyl, Thienyl und Pyridinyl.

3. Substituierte Verbindung gemäß Anspruch 1 oder 2, worin B für S(=O)₂ steht.

4. Substituierte Verbindung gemäß einem oder mehreren der Ansprüche 1 bis 3, worin in der allgemeinen Formel (I) die Teilstruktur (Ac) 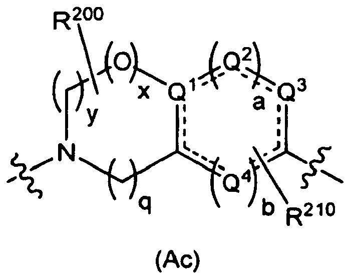 für eine Teilstruktur steht, die ausgewählt ist aus der Gruppe bestehend aus

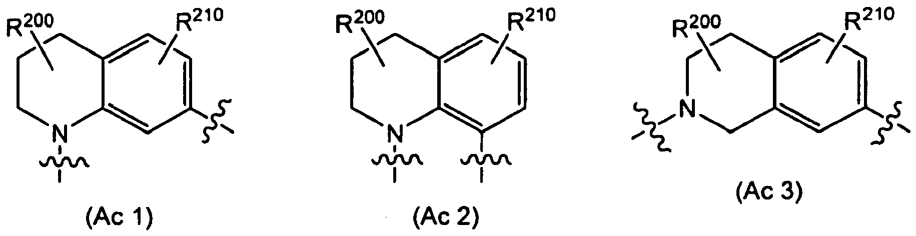

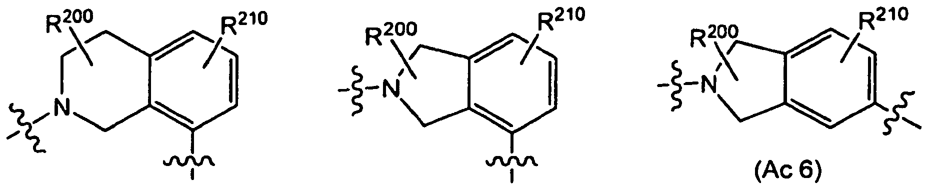

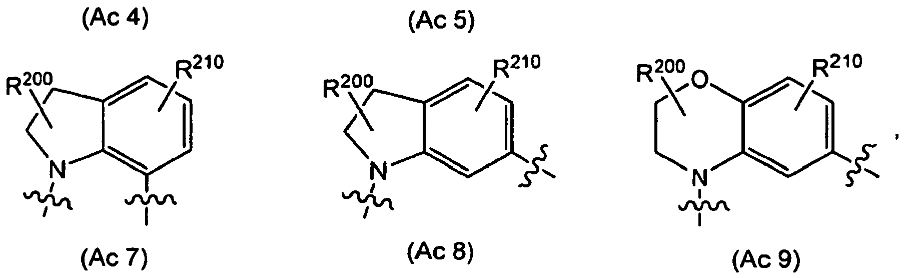

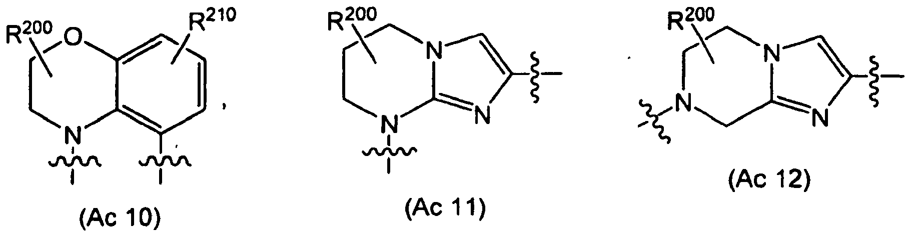

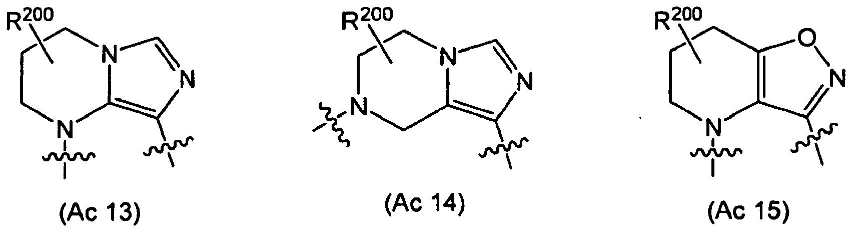

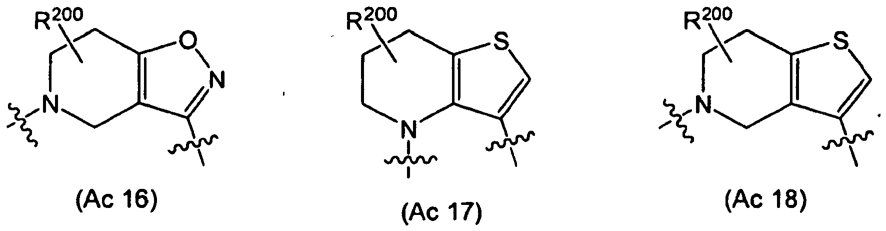

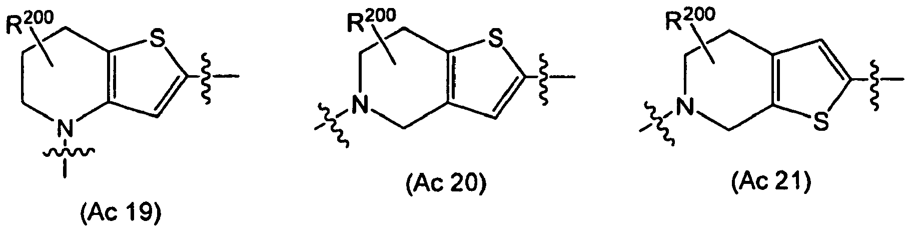

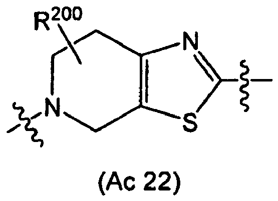

5. Substituierte Verbindung gemäß einem oder mehreren der Ansprüche 1 bis 4, worin R⁸ für H; C₁₋₆-Alkyl, insbesondere Methyl, Ethyl, n-Propyl, iso-Propyl, n-Butyl, iso-Butyl, sec-Butyl oder tert-Butyl; Cyclopropyl, Cyclobutyl, Cyclopentyl, Cyclohexyl, -CH₂CF₃, Phenyl, Benzyl, Phenylethyl, Phenylpropyl, oder ein über eine C₁₋₃-Alkylengruppe gebundenes Cyclopropyl, Cyclobutyl, Cyclopentyl, Cyclohexyl, jeweils unsubstituiert oder einfach oder mehrfach mit gleichen oder verschiedenen Substituenten substituiert, steht.

6. Substituierte Verbindung gemäß einem oder mehreren der Ansprüche 1 bis 5, worin R^{9a} und R^{9b} jeweils unabhängig voneinander für H; F; Methyl; Ethyl, iso-Propyl, CF₃, Methoxy; Cyclopropyl; Phenyl; Benzyl, Phenylethyl, C₁₋₃-Alkylen-Cyclopropyl, C₁₋₃-Alkylen-Cyclobutyl, C₁₋₃-Alkylen-Cyclopentyl, C₁₋₃-Alkylen-Cyclohexyl, C₁₋₃-Alkylen-CF₃, jeweils unsubstituiert unsubsitituiert oder einfach oder mehrfach mit gleichen oder verschiedenen Substituenten substituiert, stehen, vorzugsweise R^{9a} und R^{9b} beide gleichzeitig für H stehen.

7. Substituierte Verbindung gemäß einem oder mehreren der Ansprüche 1 bis 6, worin A für N steht.

8. Substituierte Verbindung gemäß einem oder mehreren der Ansprüche 1 bis 7, worin s und t jeweils für 0 stehen und A für N steht.

9. Substituierte Verbindung gemäß einem oder mehreren der Ansprüche 1 bis 8, worin
X für CR^{14a}R^{14b}, NR¹⁵ oder O steht;
Y für CR^{16a}R^{16b} steht;
R^{14a}, R^{14b}, R^{16a} und R^{16b} jeweils unabhängig voneinander H, F, Cl, OH, C₁₋₆-Alkyl, O-C₁₋₆-Alkyl, C₃₋₈-Cycloalkyl, Aryl oder Heteroaryl bedeuten, oder für ein über eine C₁₋₆-Alkylengruppe gebundenes C₃₋₈-Cycloalkyl, Aryl oder Heteroaryl stehen,
und/oder jeweils R^{14a} und R^{14b} gemeinsam für =O und/oder jeweils R^{16a} und R^{16b} gemeinsam für =O stehen können;
R¹⁵ für H, C₁₋₆-Alkyl, C₃₋₈-Cycloalkyl, Aryl oder Heteroaryl stehen, oder ein über eine C₁₋₆-Alkylengruppe gebundenes C₃₋₈-Cycloalkyl, Aryl oder Heteroaryl bedeuten;
Z für CR^{18a}R^{18b} oder NR¹⁹ steht; oder
Z in dem Fall dass X für O und f für 0 steht, -(N)=(CR¹²⁶))- bedeutet, wobei das N-Atom einfach an das O-Atom gebunden ist, und
R¹²⁶ für H, C₁₋₆-Alkyl, C₃₋₈-Cycloalkyl, Aryl oder Heteroaryl steht oder ein über eine C₁₋₆-Alkylengruppe gebundenes C₃₋₈-Cycloalkyl, Aryl oder Heteroaryl bedeutet;
R^{18a} für H, C₁₋₆-Alkyl, C₃₋₈-Cycloalkyl, -NH(C₁₋₆-Alkyl), -N(C₁₋₆-Alkyl)₂, Phenyl, Pyridyl, Imidazolyl, Triazolyl, Pyrimidyl, Thiazolyl oder Thienyl, jeweils unsubstituiert oder einfach oder mehrfach substituiert; oder für über eine -(O)₀₋₁-C₁₋₆-Alkylengruppe gebundenes Phenyl, Pyridyl, Imidazolyl, Triazolyl, Pyrimidyl, Thiazolyl oder Thienyl, jeweils unsubstituiert oder einfach oder mehrfach substituiert, steht; oder
R^{18a} für den Rest gemäß der allgemeinen Formel (VII) 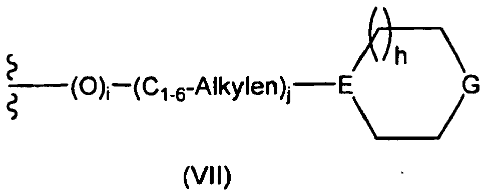 steht, worin
i für 0 oder 1 steht;
j für 0 oder 1 steht;
h für 0 oder 1 steht;
E für N oder CH steht; mit der Maßgabe, dass wenn i für 1 und j für 0 steht, E für CH steht;
G für CR^{37a}R^{37b} oder NR³⁸ steht; wobei
R^{37a} und R^{37b} unabhängig voneinander für H; F oder C₁₋₆-Alkyl, jeweils unsubstituiert oder einfach oder mehrfach substituiert mit gleichen oder verschiedenen Substituenten, stehen;
R³⁸ für H; C₁₋₆-Alkyl, C₃₋₆-Alkyl oder Pyridyl steht;
R^{18b} für H, OH, C₁₋₆-Alkyl, Phenyl, Pyridyl, Imidazolyl, Triazolyl, Pyrimidyl, Thiazolyl oder Thienyl, jeweils unsubstituiert oder einfach oder mehrfach substituiert mit gleichen oder verschiedenen Substituenten, über eine C₁₋₆-Alkylengruppe gebundenes Phenyl, Pyridyl, Imidazolyl, Triazolyl, Pyrimidyl, Thiazolyl oder Thienyl; jeweils unsubstituiert oder einfach oder mehrfach substituiert mit gleichen oder verschiedenen Substituenten; O-Phenyl oder O-Pyridyl, jeweils unsubstituiert oder einfach oder mehrfach substituiert mit gleichen oder verschiedenen Substituenten; über C₁₋₆-Alkylen-NH(C=O) verbrücktes Phenyl, Pyridyl oder Thienyl, jeweils unsubstituiert oder einfach oder mehrfach substituiert mit gleichen oder verschiedenen Substituenten, steht
R¹⁹ für H, C₁₋₆-Alkyl, C₃₋₈-Cycloalkyl, -(C=O)-C₁₋₆-Alkyl, Phenyl, Pyridyl, Thienyl, Thiazolyl, Triazolyl, Pyrimidinyl oder Imidazolyl; jeweils unsubstituiert oder einfach oder mehrfach substituiert mit gleichen oder verschiedenen Substituenten; oder für über eine C₁₋₆-Alkylengruppe gebundenes Phenyl, Pyridyl, Thienyl, Thiazolyl, Pyrimidinyl, Triazolyl oder Imidazolyl; jeweils unsubstituiert oder einfach oder mehrfach substituiert mit gleichen oder verschiedenen Substituenten, steht;
oder für den Rest gemäß der allgemeinen Formel (VIII) 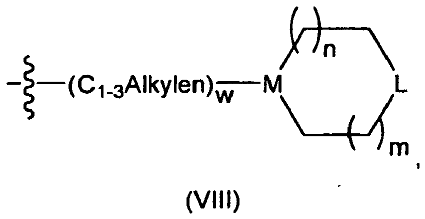 steht, worin
w für 0 oder 1 steht;
n für 0 oder 1 steht;
m für 0 oder 1 steht;
M für CH oder N steht, mit der Maßgabe, dass wenn w für 0 steht, M für CH steht;
L für CR^{44a}R^{44b} oder NR⁴⁵ steht;
wobei R^{44a} und R^{44b} unabhängig voneinander für H; F oder C₁₋₆-Alkyl, jeweils unsubstituiert oder einfach oder mehrfach substituiert mit gleichen oder verschiedenen Substituenten, stehen; und
R⁴⁵ für H, C₁₋₆-Alkyl, C₃₋₆-Alkyl oder Pyridyl steht.

10. Substituierte Verbindung gemäß einem oder mehreren der Ansprüche 1 bis 9, worin s und t jeweils für 0 stehen und die folgende Teilstruktur (SP) 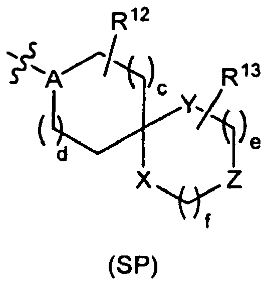 ausgewählt ist aus der Gruppe bestehend aus 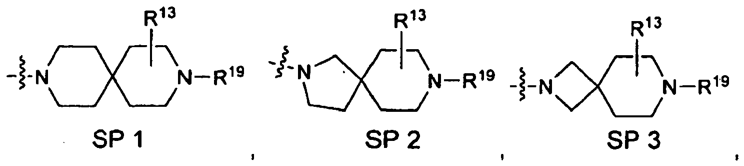 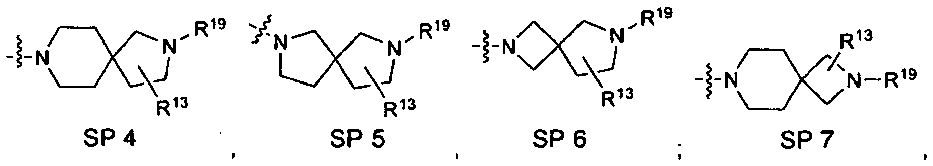 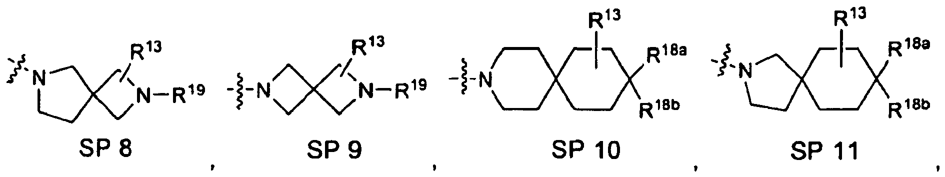 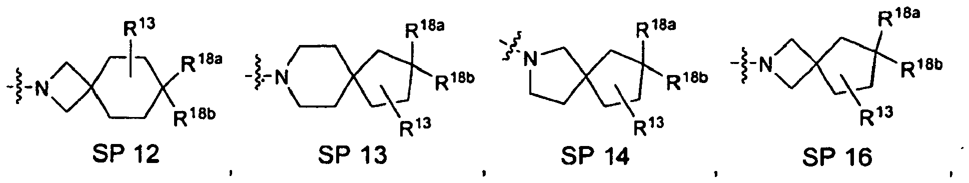 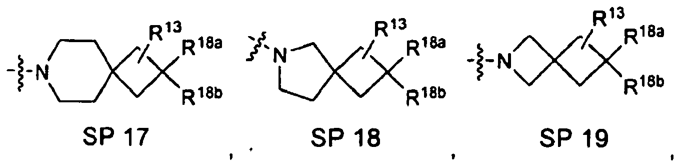 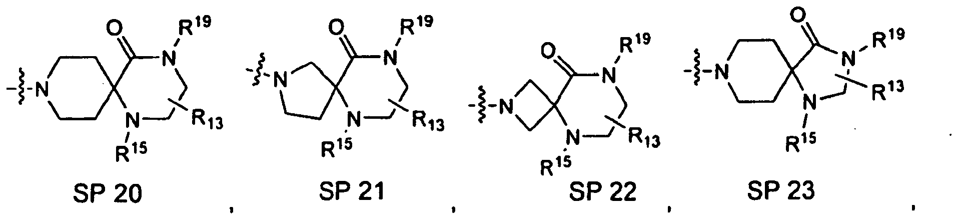 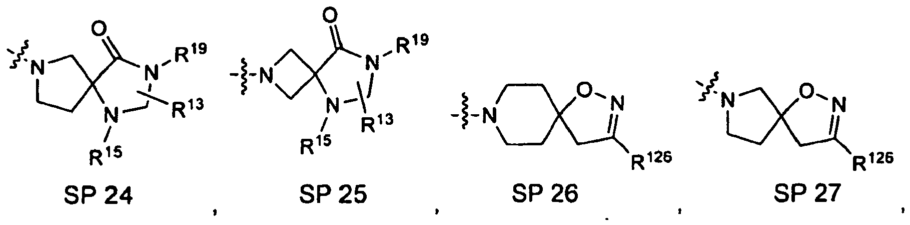 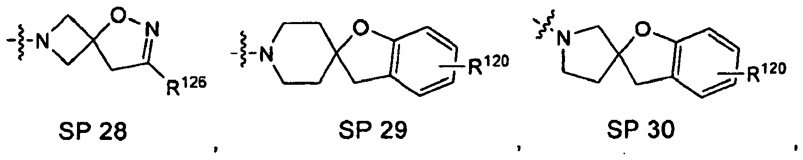 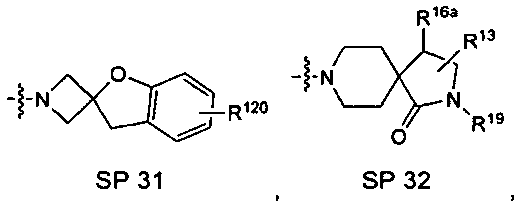 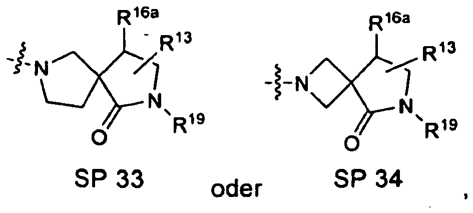 wobei
R¹³ für 1-2 Substituenten steht, die ausgewählt sind aus der Gruppe bestehend aus H und Phenyl, unsubstituiert oder einfach oder mehrfach substituiert mit gleichen oder verschiedenen Substituenten, steht; und/oder zwei der Substituenten R¹³ zusammen =O bilden und/oder zwei benachbarte Substituenten R¹³ zusammen ein anelliertes Aryl oder Heteroaryl, insbesondere eine Benzogruppe, jeweils unsubstituiert oder einfach oder mehrfach substituiert mit gleichen oder verschiedenen Substituenten, bilden,
R¹⁵ für H; C₁₋₆-Alkyl; C₃₋₈-Cycloalkyl, Phenyl, Pyridyl, jeweils unsubstituiert oder einfach oder mehrfach substituiert mit gleichen oder verschiedenen Substituenten; oder für über eine C₁₋₆-Alkylengruppe gebundenes Phenyl oder Pyridyl, jeweils unsubstituiert oder einfach oder mehrfach substituiert mit gleichen oder verschiedenen Substituenten, steht;
R^{16a} für H, C₁₋₆-Alkyl, Phenyl oder Pyridyl, jeweils unsubstituiert oder einfach oder mehrfach substituiert mit gleichen oder verschiedenen Substituenten, steht;
R^{18a} für H; C₁₋₆-Alkyl; C₃₋₈-Cycloalkyl, N(C₁₋₆-Alkyl)₂; NH(C₁₋₆-Alkyl), Azetidinyl; Pyrrolidinyl, Piperidinyl, 4-(C₁₋₆-Alkyl)-Piperazinyl; Phenyl oder Pyridyl, jeweils unsubstituiert oder einfach oder mehrfach substituiert mit gleichen oder verschiedenen Substituenten; oder für über eine -(O)_{0/1}-C₁₋₆-Alkylengruppe gebundenes N(C₁₋₆-Alkyl)₂; NH(C₁₋₆-Alkyl), Azetidinyl; Pyrrolidinyl, Piperidinyl, 4-(C₁₋₆-Alkyl)-Piperazinyl; Phenyl, Imidazolyl, Triazolyl, oder Pyridyl, jeweils unsubstituiert oder einfach oder mehrfach substituiert mit gleichen oder verschiedenen Substituenten, steht;
R^{18b} für H; OH; C₁₋₆-Alkyl; Phenyl oder Pyridyl, jeweils unsubstituiert oder einfach oder mehrfach substituiert mit gleichen oder verschiedenen Substituenten; für O-Phenyl oder O-Pyridyl, jeweils unsubstituiert oder einfach oder mehrfach substituiert mit gleichen oder verschiedenen Substituenten oder für über eine C₁₋₆-Alkylengruppe gebundenes Phenyl oder Pyridyl, jeweils unsubstituiert oder einfach oder mehrfach substituiert mit gleichen oder verschiedenen Substituenten; steht;
R¹⁹ für H; C₁₋₆-Alkyl; C₃₋₈-Cycloalkyl, Phenyl, Pyridyl, Thienly, Imidazolyl, Thiazolyl, oder Triazolyl, jeweils unsubstituiert oder einfach oder mehrfach substituiert mit gleichen oder verschiedenen Substituenten; oder für über eine C₁₋₆-Alkylengruppe oder (C=O)-Gruppe gebundenes Phenyl oder Pyridyl, jeweils unsubstituiert oder einfach oder mehrfach substituiert mit gleichen oder verschiedenen Substituenten, steht;
R¹²⁰ für H; F; Cl; OH; OCH₃, O-CF₃, C₁₋₆-Alkyl; CF₃ oder Phenyl, unsubstituiert oder einfach oder mehrfach substituiert, steht;
R¹²⁶ für H; C₁₋₆-Alkyl; C₃₋₆-Cycloalkyl; Phenyl oder Pyridyl; oder für über eine C₁₋₃-Alkylengruppe gebundenes C₃₋₆-Cycloalkyl, Phenyl oder Pyridyl, jeweils unsubstituiert oder einfach oder mehrfach substituiert mit gleichen oder verschiedenen Substituenten, steht.

11. Substituierte Verbindungen gemäß einem oder mehreren der Ansprüche 1 bis 10, worin s und t jeweils für 0 stehen und die folgende Teilstruktur (SP) 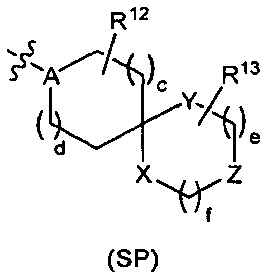 ausgewählt ist aus der Gruppe bestehend aus 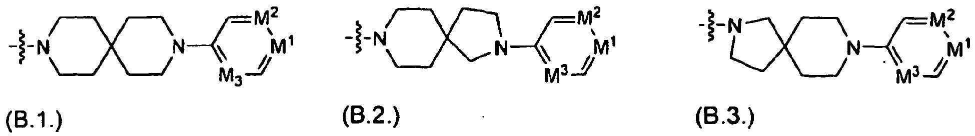 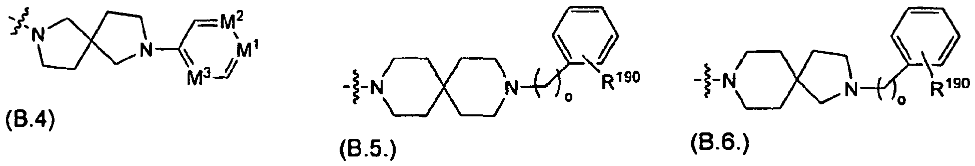 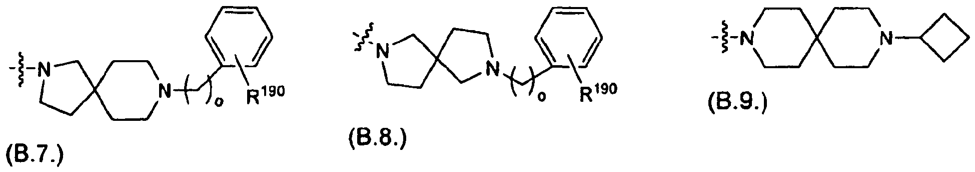 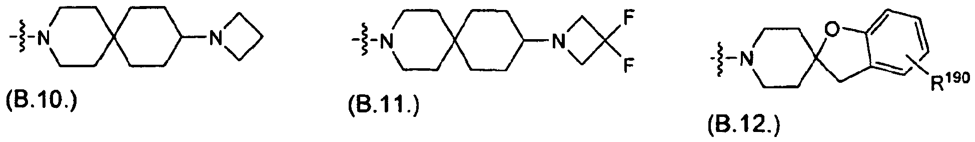 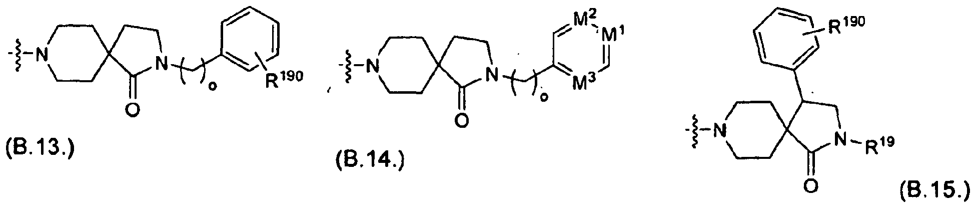 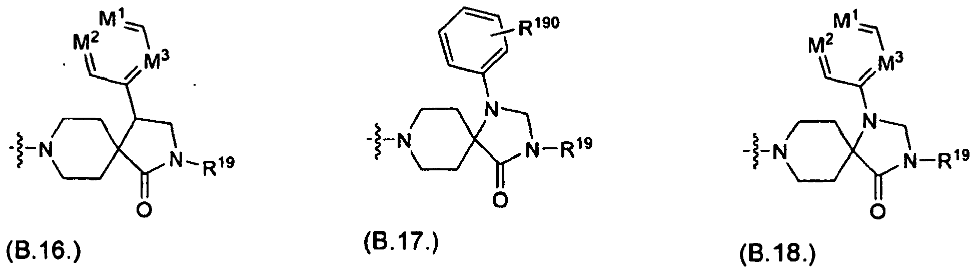 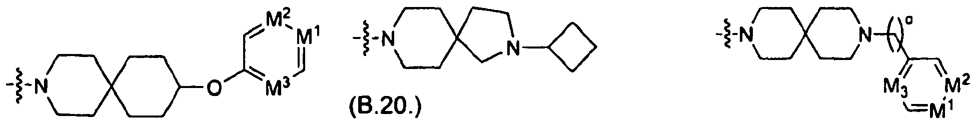 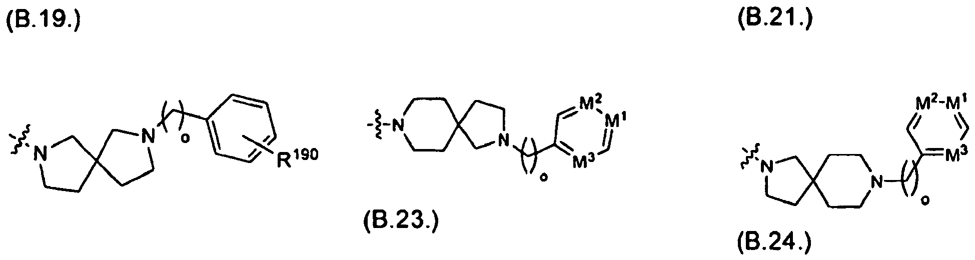 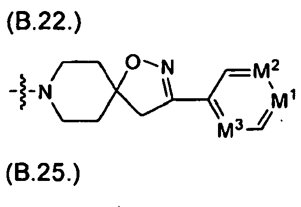 o = 0, 1, 2 oder 3 ist;
M¹, M² und M³ unabhängig voneinander jeweils für N oder CH stehen können, wobei eine Variabel aus M¹, M² und M³ für N und die anderen beiden für CH stehen;
R¹⁹ ausgewählt ist aus der Gruppe bestehend aus H; C₁₋₆Alkyl, insbesondere Methyl, Ethyl, n-Propyl, iso-Propyl, n-Butyl, sec-Butyl, iso-Butyl und tert-Butyl; C₃₋₆Cycloalkyl, jeweils unsubstituiert oder einfach oder mehrfach substituiert mit gleichen oder verschiedenen Substituenten; und
R¹⁹⁰ 0-4 Substituenten darstellt, die unabhängig voneinander ausgesucht sind aus F, Cl, O-CF₃, CF₃ oder CN.

12. Verbindung gemäß einem oder mehreren der Ansprüche 1 bis 11 ausgewählt aus der Gruppe bestehend aus
| **Verbindung** | **Name** |
|---|---|
| G-04 | (2-(4-Methoxy-2,6-dimethylphenylsulfonyl)-1,2,3,4-tetrahydroisochinolin-7-yl)(9-(pyridin-4-yl)-3,9-diazaspiro[5.5]undecan-3-yl)methanon |
| G-09 | 2-(1-(4-Methoxy-2,6-dimethylphenylsulfonyl)-1,2,3,4-tetrahydrochinolin-7-yl)-1-(2-(pyridin-4-yl)-2,8-diazaspiro[4.5]decan-8-yl)ethanon |
| G-10 | 1-(2-Cyclobutyl-2,8-diazaspiro[4.5]decan-8-yl)-2-(1-(4-methoxy-2,6-dimethylphenylsulfonyl)-1,2,3,4-tetrahydrochinolin-7-yl)ethanon |
| G-11 | 2-(1-(4-Methoxy-2,6-imethylphenylsulfonyl)-1,2,3,4-tetrahydrochinolin-7-yl)-1-(8-(pyridin-4-yl)-2,8-diazaspiro[4.5]decan-2-yl)ethanon |
| G-12 | 2-(1-(4-Methoxy-2,6-dimethylphenylsulfonyl)-1,2,3,4-tetrahydrochinolin-7-yl)-1-(7-(pyridin-4-yl)-2,7-diazaspiro[4.4]nonan-2-yl)ethanon |
| G-13 | (5-(2-Chlorbenzoyl)-4,5,6,7-tetrahydroisoxazolo[4,5-c]pyridin-3-yl)(9-(pyridin-4-yl)-3,9-diazaspiro[5.5]undecan-3-yl)methanon |
| G-14 | (5-(4-Methoxy-2,6-dimethylphenylsulfonyl)-4,5,6,7-tetrahydrothieno[3,2-c]pyridin-2-yl)(9-(pyridin-4-yl)-3,9-diazaspiro[5.5]undecan-3-yl)methanon |
| G-15 | (5-(2-Chlorbenzoyl)-,5,6,7-tetrahydrothieno[3,2-c]pyridin-2-yl)(9-(pyridin-4-yl)-3,9-diazaspiro[5.5]undecan-3-yl)methanon |
| G-16 | 1-(9-(Pyridin-4-yl)-3,9-diazaspiro[5.5]undecan-3-yl)-2-(1-(2-(trifluormethyl)phenylsulfonyl)-1,2,3,4-tetrahydrochinolin-7-yl)ethanon |
| G-17 | 2-(1-(Naphthalin-1-ylsulfonyl)-1,2,3,4-tetrahydrochinolin-7-yl)-1-(9-(pyridin-4-yl)-3,9-diazaspiro[5.5]undecan-3-yl)ethanon |
| G-18 | 2-(1-(2-Chtor-6-methylphenylsulfonyl)-1,2,3,4-tetrahydrochinolin-7-yl)-1-(9-(pyridin-4-yl)-3,9-diazaspiro[5.5]undecan-3-yl)ethanon |
| G-19 | 2-(4-(4-Methoxy-2,6-dimethylphenylsulfonyl)-3,4-dihydro-2H-benzo[b][1,4]oxazin-6-yl)-1-(9-(pyridin-4-yl)-3,9-diazaspiro[5.5]undecan-3-yl)ethanon |
| G-20 | 2-(1-(4-Methoxy-2,6-dimethylphenylsulfonyl)indolin-6-yl)-1-(9-(pyridin-4-yl)-3,9-diazaspiro[5.5]undecan-3-yl)ethanon |
| G-21 | (1-(2-Chlorbenzoyl)-1,2,3,4-tetrahydrochinolin-7-yl)(9-(pyridin-4-yl)-3,9-diazaspiro[5.5]undecan-3-yl)methanon |
| G-24 | (1-(4-Methoxy-2,6-dimethylphenylsuffonyl)-1,2,3,4-tetrahydrochinolin-7-yl)(9-(pyridin-4-yl)-3,9-diazaspiro[5.5]undecan-3-yl)methanon |
| G-25 | 2-(1-(4-Methoxy-2,6-dimethylphenylsulfonyl)-1,2,3,4-tetrahydrochinolin-7-yl)-1-(9-(pyridin-4-yl)-3.9-diazaspiro[5.5]undecan-3-yl)ethanon |
| G-26 | (5-(4-Methoxy-2,6-dimethylphenylsulfonyl)-4,5,6,7-tetrahydroisoxazolo[4,5-c]pyridin-3-yl)(9-(pyridin-4-yl)-3,9-diazaspiro[5.5]undecan-3-yl)methanon |
| G-30 | 2-(1-(4-Methoxy-2,6-dimethylphenylsulfonyl)-1,2,3,4-tetrahydrochinolin-7-yl)-1-(9-(pyridin-4-yloxy)-3-azaspiro[5.5]undecan-3-yl)ethanon |
| G-32 | 1-(9-(Azetidin-1-yl)-3-azaspiro[5.5]undecan-3-yl)-2-(1-(4-methoxy-2,6-dimethylphenylsulfonyl)-1,2,3,4-tetrahydrochinolin-7-yl)ethanon |
| G-33 | 2-(1-(4-Methoxy-2,6-dimethylphenylsulfonyl)-1,2,3,4-tetrahydrochinotin-7-yl)-1-(3-(pyridin-4-yl)-1-oxa-2,8-diazaspiro[4.5]dec-2-en-8-yl)ethanon |
| G-34 | 1-(9-(3,3-Difluorazehdin-1-yl)-3-azaspiro[5.5]undecan-3-yl)-2-(1-(4-methoxy-2,6-dimethylphenylsutfonyl)-1,2,3,4-tetrahydrochinolin-7-yl)ethanon |
| G-35 | 2-(1-(2-Chlor-4-(trifluormethoxy)phenylsulfonyl)-1,2,3,4-tetrahydrochinolin-7-yl)-1-(9-(pyridin-4-yl)-3,9-diazaspiro[5.5]undecan-3-yl)ethanon |
| G-36 | (2-(4-Methoxy-2,6-dimethylphenylsulfonyl)-1,2,3,4-tetrahydroisochinolin-8-yl)(9-(pyridin-4-yl)-3,9-diazaspiro[5.5]undecan-3-yl)methanon |
| G-37 | (2-(2-Chlor-6-methylphenylsulfonyl)-1,2,3,4-tetrahydroisochinolin-8-yl)(9-(pyridin-4-yl)-3,9-diazaspiro[5.5]undecan-3-yl)methanon |
| G-38 | (2-(2,3-Dichlorphenylsulfonyl)-1,2,3,4-tetrahydroisochinolin-8-yl)(9-(pyridin-4-yl)-3,9-diazaspiro[5.5]undecan-3-yl)methanon |
| G-39 | 2-(2-(4-Methoxy-2,6-dimethylphenylsulfonyl)-1,2,3,4-tetrahydroisochinolin-7-yl)-1-(9-(pyridin-4-yl)-3,9-diazaspiro[5.5]undecan-3-yl)ethanon |
| G-40 | (7-(4-Methoxy-2,6-dimethylphenylsulfonyl)-5,6,7,8-tetrahydroimidazo[1,2-a]pyrazin-2-yl)(9-(pyridin-4-yl)-3,9-diazaspiro[5.5]undecan-3-yl)methanon |
| G-41 | 3-(2-Chlorphenyl)-1-(7-(9-(pyridin-4-yl)-3,9-diazaspiro[5.5]undecan-3-carbonyl)-3,4-dihydroisochinolin-2(1H)-yl)prop-2-en-1-on |
| G-42 | (5-Chlorthiophen-2-yl)(7-(9-(pyridin-4-yl)-3,9-diazaspiro[5.5]undecan-3-carbonyl)-3,4-dihydroisochinolin-2(1H)-yl)methanon |
| G-43 | 2-(2-Chlorphenyl)-1-(7-(9-(pyridin-4-yl)-3,9-diazaspiro[5.5]undecan-3-carbonyl)-3,4-dihydroisochinolin-2(1H)-yl)ethanon |
| G-44 | (5-Chlorthiophen-2-yl)(8-(9-(pyridin-4-yl)-3,9-diazaspiro[5.5]undecan-3-carbonyl)-3,4-dihydroisochinolin-2(1H)-yl)methanon |
| G-45 | N-(2-Chlorbenzyl)-7-(9-(pyridin-4-yl)-3,9-diazaspiro[5.5]undecan-3-carbonyl)-3,4-dihydroisochinolin-2(1H)-carboxamid |
| G-46 | N-(3,4-Dichlorphenyl)-3-(9-(pyridin-4-yl)-3,9-diazaspiro[5.5]undecan-3-carbonyl)-6,7-dihydroisoxazolo[4,5-c]pyridin-5(4H)-carboxamid |
| G-47 | (2-(4-Methylnaphthalin-1-ylsulfonyl)-1,2,3,4-tetrahydroisochinolin-7-yl)(9-(pyridin-4-yl)-3,9-diazaspiro[5.5]undecan-3-yl)methanon |
| G-48 | (2-(4-Methoxynaphthalin-1-ylsulfonyl)-1,2,3,4-tetrahydroisochinolin-7-yl)(9-(pyridin-4-yl)-3,9-diazaspiro[5.5]undecan-3-yl)methanon |
| G-49 | 2,2-Diphenyl-1-(7-(9-(pyridin-4-yl)-3,9-diazaspiro[5.5]undecan-3-carbonyl)-3,4-dihydroisochinolin-2(1H)-yl)ethanon |
| G-50 | (2-(4-Chlornaphthalin-1-ylsulfonyl)-1,2,3,4-tetrahydroisochinolin-7-yl)(9-(pyridin-4-yl)-3,9-diazaspiro[5.5]undecan-3-yl)methanon |
| G-51 | 2-(1-(2-Chlor-6-methylphenylsulfonyl)-1,2,3,4-tetrahydrochinolin-7-yl)-1-(8-(pyridin-4-yl)-2,8-diazaspiro[4.5]decan-2-yl)ethanon |
| G-52 | 2-(1-(Naphthalin-1-ylsulfonyl)-1,2,3,4-tetrahydrochinolin-7-yl)-1-(8-(pyridin-4-yl)-2,8-diazaspiro[4.5]decan-2-yl)ethanon |
| G-53 | 2-(1-(Naphthalin-1-ylsulfonyl)-1,2,3,4-tetrahydrochinolin-7-yl)-1-(7-(pyridin-4-yl)-2,7-diazaspiro[4.4]nonan-2-yl)ethanon |
| G-54 | 1-(9-(Azetidin-1-yl)-3-azaspiro[5.5]undecan-3-yl)-2-(1-(naphthalin-1-ylsulfonyl)-1,2,3,4-tetrahydrochinolin-7-yl)ethanon |
| G-55 | N-(3,4-dichlorphenyl)-7-(9-(pyridin-4-yl)-3,9-diazaspiro[5.5]undecan-3-carbonyl)-3,4-dihydroisochinolin-2(1H)-carboxamid |
| G-56 | (2-(4-Fluornaphthalin-1-ylsulfonyl)-1,2,3,4-tetrahydroisochinolin-7-yl)(9-(pyridin-4-yl)-3,9-diazaspiro[5.5]undecan-3-yl)methanon |
| G-57 | 1-(8-(Pyridin-4-yl)-2,8-diazaspiro[4.5]decan-2-yl)-2-(1-(2-(trifluormethyl)phenyl)phenylsulfonyl)-1,2,3,4-tetrahydrochinolin-7-yl)ethanon |
| G-58 | (5-Methylthiophen-2-yl)(7-(9-(pyridin-4-yl)-3,9-diazaspiro[5.5]undecan-3-carbonyl)-3,4-dihydroisochinolin-2(1H)-yl)methanon |
| G-59 | Benzo[b]thiophen-2-yl(7-(9-(pyridin-4-yl)-3,9-diazaspiro[5.5]undecan-3-carbonyl)-3,4-dihydroisochinolin-2(1H)-yl)methanon |
| G-60 | (5,6-Dihydro-4H-cyclopenta[b]thiophen-2-yl)(7-(9-(pyridin-4-yl)-3,9-diazaspiro[5.5]undecan-3-carbonyl)-3,4-dihydroisochinolin-2(1 H)-yl)methanon |
| G-61 | (3-Chlor-6-methoxybenzo[b]thiophen-2-yl)(7-(9-(pyridin-4-yl)-3,9-diazaspiro[5.5]undecan-3-carbonyl)-3,4-dihydroisochinolin-2(1H)-yl)methanon |
| G-62 | (2-(5-Chlornaphthalin-1-ylsulfonyl)-1,2,3,4-tetrahydroisochinolin-7-yl)(9-(pyridin-4-yl)-3,9-diazaspiro[5.5]undecan-3-yl)methanon |
| G-63 | (5-tert-Butylthiophen-2-yl)(7-(9-(pyridin-4-yl)-3,9-diazaspiro[5.5]undecan-3-carbonyl)-3,4-dihydroisochinolin-2(1H)-yl)methanon |
| G-64 | 2[1-(2-Chlor-benzoyl)-1,2,3,4-tetrahydro-chinolin-7-yl]-1-(9-pyridin-4-yl-3,9-diazaspiro[5.5]undecan-3-yl)-ethanon |
| G-66 | 2-[1-[(2,6-Dichlor-3-methyl-phenyl)sulfonyl]-1,2,3,4-tetrahydrochinolin-7-yl]-1-(9-pyridin-4-yl-3,9-diazaspiro[5.5]undecan-3-yl)-ethanon |
| G-67 | [2-[(4-Methoxy-2,6-dimethyl-phenyl)sulfonyl]-2,3-dihydro-1H-isoindol-5-yl]-(9-pyridin-4-yl-3,9-diazaspiro[5.5]undecan-3-yl)-methanon |
| G-68 | [2-[(2-Chlor-6-methyl-phenyl)sulfonyl]-2,3-dihydro-1H-isoindol-5-yl]-(9-pyridin-4-yl-3,9-diazaspiro[5.5]undecan-3-yl)-methanon |
| G-69 | [2-(5-Chlor-thiophen-2-carbonyl)-2,3-dihydro-1H-isoindol-5-yl]-(9-pyridin-4-yl-3,9-diazaspiro[5.5]undecan-3-yl)-methanon |
| G-70 | 2-[8-[(4-Methoxy-2,6-dimethyl-phenyl)sulfonyl]-5,6,7,8-tetrahydro-[1,8]naphthyridin-2-yl]-1-(9-pyridin-4-yl-3,9-diazaspiro[5.5]undecan-3-yl)-ethanon |
| G-71 | [7-(5-Chlor-thiophen-2-carbonyl)-5,6,7,8-tetrahydro-imidazo[1,2-a]pyrazin-2-yl]-(9-pyridin-4-yl-3,9-diazaspiro[5.5]undecan-3-yl)-methanon |
| G-72 | [7-[(4-Methoxy-2,6-dimethyl-phenyl)sulfonyl]-5,6,7,8-tetrahydro-imidazo[1,5-a]pyrazin-1-yl]-(9-pyridin-4-yl-3,9-diazaspiro[5.5]undecan-3-yl)-methanon |
| G-73 | [2-(4-Methoxy-2,6-dimethyl-benzoyl)-2,3-dihydro-1H-isoindol-5-yl]-(9-pyridin-4-yl-3,9-diazaspiro[5.5]undecan-3-yl)-methanon |
| G_CC-006 | 2-(4-Fluorbenzyl)-8-(1-(4-methoxy-2,6-dimethylphenylsulfonyl)indolin-6-carbonyl)-2,8-diazaspiro[4.5]decan-1-on |
| G_CC-007 | (7-Benzyl-2,7-diazaspiro[4.4]nonan-2-yl)(1-(4-methoxy-2,6-dimethylphenylsulfonyl)indolin-6-yl)methanon |
| G_CC-008 | (1-(4-Methoxy-2,6-dimethylphenylsulfonyl)indolin-6-yl)(9-(pyridin-4-yl)-3,9-diazaspiro[5.5]undecan-3-yl)methanon |
| G_CC-009 | (1-(2-Chlorbenzoyl)indolin-6-yl)(9-(pyridin-4-yl)-3,9-diazaspiro[5.5]undecan-3-yl)methanon |
| G_CC-013 | (1-(4-Chlor-2,5-dimethytphenylsulfonyl)indolin-6-yl)(9-(pyridin-4-yl)-3,9-diazaspiro[5.5]undecan-3-yl)methanon |
| G_CC-018 | 8-(2-(2-Chlorbenzoyl)isoindolin-5-carbonyl)-2-(4-fluorbenzyl)-2,8-diazaspiro[4.5]decan-1-on |
| G_CC-025 | 4-(4-Fluorphenyl)-8-(1-(4-methoxy-2,6-dimethylphenylsulfonyl) 1,2,3,4-tetrahydrochinolin-7-carbonyl)-2-methyl-2,8-diazaspiro[4.5]decan-1-on |
| G_CC-026 | 2-Benzyl-8-(1-(4-methoxy-2,6-dimethylphenylsulfonyl)-1,2,3,4-tetrahydrochinolin-7-carbonyl)-2,8-diazaspiro[4.5]decan-1-on |
| G_CC-027 | (7-Benzyl-2,7-diazaspiro[4.4]nonan-2-yl)(1-(4-methoxy-2,6-dimethylphenylsulfonyl)-1,2,3,4-tetrahydrochinolin-7-yl)methanon |
| G_CC-039 | 2-(4-Fluorbenzyl)-8-(2-(4-methoxy-2,6-dimethylphenylsulfonyl)-1,2,3,4-tetrahydroisochinolin-7-carbonyl)-2,8-diazaspiro[4.5]decan-1-on |
| G_CC-040 | 2-Benzyl-8-(2-(4-methoxy-2,6-dimethylphenylsulfonyl)-1,2,3,4-tetrahydroisochinolin-7-carbonyl)-2,8-diazaspiro[4.5]decan-1-on |
| G_CC-041 | (7-Benzyl-2,7-diazaspiro[4.4]nonan-2-yl)(2-(4-methoxy-2,6-dimethylphenylsulfonyl)-1,2,3,4-tetrahydroisochinolin-7-yl)methanon |
| G_CC-043 | 8-(2-(4-Methoxy-2,6-dimethylphenylsulfonyl)-1,2,3,4-tetrahydroisochinolin-7-carbonyl)-1-phenyl-1,3,8-triazaspiro[4.5]decan-4-on |
| G_CC-045 | (2-(2-Chlorbenzoyl)-1,2,3,4-tetrahydroisochinolin-7-yl)(9-(pyridin-4-yl)-3,9-diazaspiro[5.5]undecan-3-yl)methanon |
| G_CC-053 | 2-Benzyl-8-(2-(1-(4-methoxy-2,6-dimethylphenylsulfonyl)-1,2,3,4-tetrahydrochinolin-7-yl)acetyl)-2,8-diazaspiro[4.5]decan-1-on |
| G_CC-054 | 1-(7-Benzyl-2,7-diazaspiro[4.4]nonan-2-yl)-2-(1-(4-methoxy-2,6-dimethylphenylsulfonyl)-1,2,3,4-tetrahydrochinolin-7-yl)ethanon |
| G_CC-055 | 2-[1-[(4-Methoxy-2,6-dimethyl-phenyl)sulfonyl]-1,2,3,4-tetrahydrochinolin-7-yl]-1-(3-pyridin-4-yl-3,9-diazaspiro[5.5]undecan-9-yl)-ethanon |
| G_CC-055 | [1-[(4-Methoxy-2,6-dimethyl-phenyl)sulfonyl]-1,2,3,4-tetrahydrochinolin-7-yl]-(3-pyridin-4-yl-3,9-diazaspiro[5.5]undecan-9-yl)-methanon |
ggf. in Form eines einzelnen Enantiomers oder eines einzelnen Diastereomers, des Razemats, der Enantiomere, der Diastereomere, Mischungen von Enantiomere oder Diastereomeren, jeweils in Form ihrer Basen und/oder physiologisch verträglichen Salze, insbesondere der Hydrochlorid-Salze.

13. Arzneimittel enthaltend wenigstens eine Verbindung gemäß einem oder mehreren der Ansprüche 1 bis 12.

14. Verwendung wenigstens einer Verbindung gemäß einem oder mehreren der Ansprüche 1 bis 12 zur Herstellung eines Arzneimittels zur Behandlung von Schmerz, insbesondere von akutem Schmerz, viszeralem Schmerz, neuropathischem Schmerz, chronischem Schmerz und/oder Entzündungsschmerz; Migräne; Diabetes; Erkrankungen der Atemwege; entzündlichen Darmerkrankungen; neurologischen Erkrankungen; Entzündungen der Haut; rheumatischen Erkrankungen; septischem Schock; Reperfusionssyndrom; Fettleibigkeit und/oder als Angiognese-Inhibitor.

## Claims

1. Substituted spiroamide compound of the general formula (I) 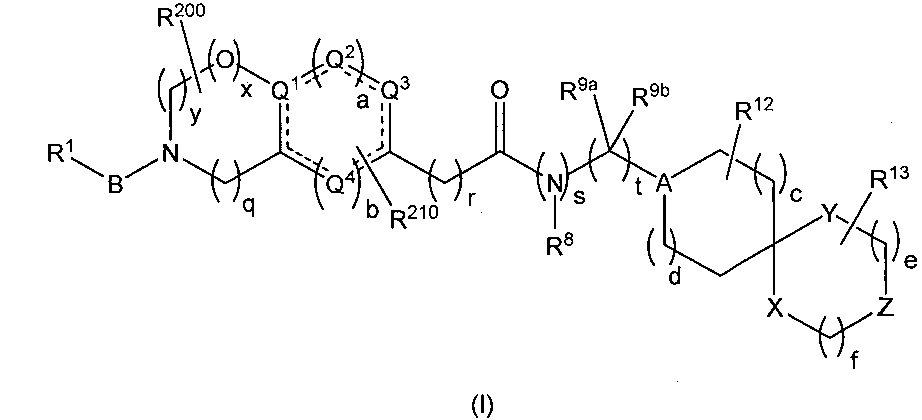 wherein
Q¹ represents C or N;
Q² represents CH, N, O or S;
Q³ represents CH, N, O or S;
Q⁴ represents CH, N, O or S;
B represents C(=O), S(=O)₂ or represents the group -C(=O)-N(R⁴), wherein the nitrogen atom thereof is bonded to the radical R¹;
a represents 0, 1 or 2;
b represents 0 or 1, with the proviso that a+b = 1 or 2;
q represents 0 or 1;
x represents 0 or 1;
y represents 1, 2 or 3;
r represents 0, 1, 2 or 3;
R¹ represents aryl, heteroaryl or an aryl or heteroaryl bonded *via* a C₁₋₃-alkylene group or C₂₋₆-alkenylene group, wherein aryl and heteroaryl in each case can be fused with a 4-, 5-, 6- or 7-membered cycle or heterocycle, wherein the cycle and heterocycle in each case is saturated or at least monounsaturated but is not aromatic and in each case can be substituted on one or more of its carbon ring members by one or more radicals selected independently of one another from the group consisting of F, Cl, Br, I, -CF₃, -O-CF₃, C₁₋₆-alkyl and O-C₁₋₆-alkyl and wherein the heterocycle can contain one or more heteroatoms or heteroatom groups selected independently of one another from the group consisting of N, NR⁵⁰, O, S, S=O and S(=O)₂; wherein the radical R⁵⁰ denotes H, C₁₋₆-alkyl, -C(=O)-R⁵¹, C₃₋₈-cycloalkyl, aryl, heteroaryl or a C₃₋₈-cycloalkyl, aryl or heteroaryl bonded *via* a C₁₋₃-alkylene group, and R⁵¹ denotes C₁₋₆-alkyl, C₃₋₈-cycloalkyl, aryl, heteroaryl or a C₃₋₈-cycloalkyl, aryl or heteroaryl bonded *via* a C₁₋₃-alkylene group;
R⁴ represents H, C₁₋₆-alkyl, aryl or an aryl bonded *via* a C₁₋₃-alkylene group;
R²⁰⁰ represents from 0 to 4 substituents selected independently of one another from the group consisting of C₁₋₆-alkyl, F, Cl, CF₃ and OCF₃;
R²¹⁰ represents from 0 to 4 substituents selected independently of one another from the group consisting of C₁₋₆-alkyl, O-C₁₋₆-alkyl, F, Cl, CF₃ and OCF₃;
s = 0 or 1,
t = 0, 1, 2 or 3, with the proviso that when s represents 0, t represents 0;
R⁸ represents H, C₁₋₆-alkyl, C₃₋₈-cycloalkyl, aryl or heteroaryl, or a C₃₋₈-cycloalkyl, aryl or heteroaryl bonded *via* a C₁₋₆-alkylene group;
R^{9a} and R^{9b} each independently of the other denotes H, F, Cl, OH, C₁₋₆-alkyl, O-C₁₋₆-alkyl, C₃₋₈-cycloalkyl, aryl or heteroaryl, or a C₃₋₈-cycloalkyl, aryl or heteroaryl bonded *via* a C₁₋₆-alkylene group;
A represents N or CH;
with the proviso that when s represents 1 and t represents 0, A represents CH; and
with the proviso that when s and t each represents 0, A represents N;
c, d, e and f each independently of the others denotes 0, 1 or 2;
R¹² and R¹³ each independently of the other represents from 0 to 4 substituents each selected independently of one another from the group consisting of F, Cl, OH, =O, C₁₋₆-alkyl, O-C₁₋₆-alkyl, C₃₋₈-cycloalkyl, aryl, heteroaryl and C₃₋₈-cycloalkyl, aryl or heteroaryl bonded *via* a C₁₋₆-alkylene group;
and/or two of the 0 to 4 substituents R¹³ that are adjacent form a fused aryl or heteroaryl;
X represents CR^{14a}R^{14b}, NR¹⁵ or O;
Y represents CR^{16a}R^{16b}, NR¹⁷ or O;
with the proviso that X does not denote NR¹⁵ when Y denotes NR¹⁷; and
with the proviso that X and Y do not simultaneously denote O;
wherein
R^{14a}, R^{14b}, R^{16a} and R^{16b} each independently of the others denotes H, F, Cl, OH, C₁₋₆-alkyl, O-C₁₋₆-alkyl, C₃₋₈-cycloalkyl, aryl or heteroaryl, or represents a C₃₋₈-cycloalkyl, aryl or heteroaryl bonded *via* a C₁₋₆-alkylene group,
and/or in each case R^{14a} and R^{14b} together can represent =O and/or in each case R^{16a} and R^{16b} together can represent =O;
R¹⁵ and R¹⁷ each independently of the other represents H, C₁₋₆-alkyl, C₃₋₈-cycloalkyl, aryl or heteroaryl, or denotes a C₃₋₈-cycloalkyl, aryl or heteroaryl bonded *via* a C₁₋₆-alkylene group;
Z represents CR^{18a}R^{18b}, NR¹⁹ or O;
or
Z where X represents O and f represents 0, denotes -(C(R¹²⁴)-C(R¹²⁵))-, wherein
R¹²⁴ and R¹²⁵, together with the carbon atoms joining them, form a fused aryl
or heteroaryl; or
Z where X represents O and f represents 0, denotes -(N=(CR¹²⁶))-, wherein the N atom is singly bonded to the O atom, and
R¹²⁶ represents H, C₁₋₆-alkyl, C₃₋₈-cycloalkyl, aryl or heteroaryl, or denotes a C₃₋₈-cycloalkyl, aryl or heteroaryl bonded *via* a C₁₋₆-alkylene group; and
wherein
R^{18a} represents H, C₁₋₆-alkyl, C₃₋₈-cycloalkyl, aryl or heteroaryl, or denotes a C₃₋₈-cycloalkyl, aryl or heteroaryl bonded *via* a C₁₋₆-alkylene group,
or R^{18a} represents a group according to the general formula (II) 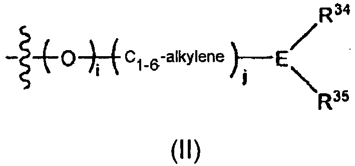 wherein
i and j each independently of the other represents 0 or 1;
E represents N or CH, with the proviso that when i represents 1 and j represents 0, E represents CH,
R³⁴ and R³⁵ each independently of the other denotes H, C₁₋₆-alkyl, C₃₋₈-cycloalkyl, aryl or heteroaryl, or an aryl, heteroaryl or C₃₋₈-cycloalkyl bonded via a C₁₋₃-alkylene group;
or R³⁴ and R³⁵, including E, form a 5- or 6-membered aryl or heteroaryl;
or R³⁴ and R³⁵, including E, form a saturated heterocycle according to the general formula (III) 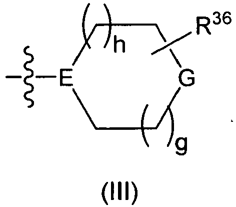 wherein
h and g independently of one another denote 0, 1 or 2;
G represents CR^{37a}R^{37b}, NR³⁸, O, S, S=O or S(=O)₂, with the proviso that when E represents CH, G does not represent CR^{37a}R^{37b};
R³⁶ represents from 0 to 4 substituents each selected independently of one another from the group consisting of F, Cl, Br, I, OH, SH, =O, O-C₁₋₆-alkyl, C₁₋₆-alkyl, C₃₋₈-cycloalkyl, aryl, heteroaryl and C₃₋₈-cycloalkyl, aryl or heteroaryl bonded *via* a C₁₋₆-alkylene group;
and/or two adjacent substituents R³⁶ together represent a fused aryl or heteroaryl;
R^{37a} and R^{37b} each independently of the other denotes H, F, Cl, Br, I, OH, SH, =O, O-C₁₋₆-alkyl, C₁₋₆-alkyl, C₃₋₈-cycloalkyl, aryl or heteroaryl, or represents a C₃₋₈-cycloalkyl, aryl or heteroaryl bonded *via* a C₁₋₆-alkylene group;
R³⁸ represents H, C₁₋₆-alkyl, C₃₋₈-cycloalkyl, aryl or heteroaryl, or denotes an aryl, heteroaryl or C₃₋₈-cycloalkyl bonded *via* a C₁₋₃-alkylene group;
R^{18b} represents H, OH, C₁₋₆-alkyl, C₃₋₈-cycloalkyl, O-C₁₋₆-alkyl, O-(C₃₋₈-cycloalkyl), (C₁₋₈-alkylene)-O-C₁₋₆-alkyl, (C₁₋₆-alkylene)-O-(C₃₋₈-cycloalkyl), aryl, heteroaryl, O-aryl or O-heteroaryl, or denotes an aryl, O-aryl, heteroaryl or O-heteroaryl bonded *via* a C₁₋₆-alkylene group;
or R^{18b} represents a group according to the general formula (IV) 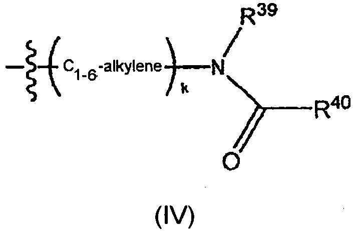 wherein
k represents 0 or 1;
R³⁹ represents H, C₁₋₆-alkyl, C₃₋₈-cycloalkyl, aryl or heteroaryl, or denotes a C₃₋₈-cycloalkyl, aryl or heteroaryl bonded *via* a C₁₋₃-alkylene group;
R⁴⁰ represents C₁₋₆-alkyl, C₃₋₈-cycloalkyl, aryl or heteroaryl, or denotes a C₃₋₈-cycloalkyl, aryl or heteroaryl bonded *via* a C₁₋₆-alkylene group;
or
R³⁹ and R⁴⁰, together with the N-C(=O)- group joining them, form a ring according to the general formula (V) 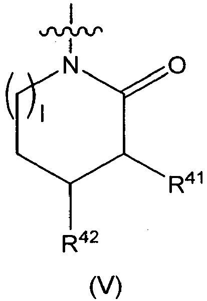 wherein
I represents 0, 1 or 2;
and R⁴¹ and R⁴², together with the carbon atoms joining them, form a fused aryl or heteroaryl;
R¹⁹ represents H; or (P)_{z}-R²²,
wherein
z represents 0 or 1;
P represents (C=O), S(=O)₂ or C(=O)-N(R²⁴), wherein the N atom in the group C(=O)-N(R²⁴) is linked to R²², wherein
R²⁴ represents H, C₁₋₆-alkyl, C₃₋₈-cycloalkyl, or represents an aryl, heteroaryl or C₃₋₈-cycloalkyl bonded *via* a C₁₋₃-alkylene group;
R²² represents C₁₋₆-alkyl, aryl or heteroaryl, or denotes an aryl or heteroaryl bonded *via* a C₁₋₆-alkylene group; or
R²² represents a group according to the general formula (VI) 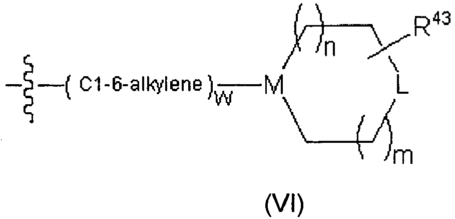 wherein
n represents 0, 1 or 2;
m represents 0, 1 or 2;
w represents 0 or 1,
M represents CH or N;
with the proviso that when P represents C(=O)-NR²⁴ and w represents 0, M represents CH; and
with the proviso that when z and w simultaneously represent 0, M represents CH;
L represents CR^{44a}R^{44b}, NR⁴⁵, O, S, S=O or S(=O)₂;
R⁴³ represents from 0 to 4 substituents each selected independently of one another from the group consisting of F, Cl, OH, =O C₁₋₆-alkyl, O-C₁₋₆-alkyl, C₃₋₈-cycloalkyl, aryl, heteroaryl and C₃₋₈-cycloalkyl, aryl or heteroaryl bonded *via* a C₁₋₆-alkylene group;
and/or two of the 0 to 4 radicals R⁴³ that are adjacent together represent a fused aryl or heteroaryl;
R^{44a} and R^{44b} each independently of the other represents H, F, Cl, Br, I, OH, C₁₋₆-alkyl, O-C₁₋₆-alkyl, C₃₋₈-cycloalkyl, aryl or heteroaryl, or denotes a C₃₋₈-cycloalkyl, aryl or heteroaryl bonded via a C₁₋₆-alkylene group;
or R^{44a} and R^{44b} together can represent =O;
R⁴⁵ represents H, C₁₋₆-alkyl, C₃₋₈-cycloalkyl, aryl or heteroaryl, or denotes an aryl, heteroaryl or C₃₋₈-cycloalkyl bonded *via* a C₁₋₃-alkylene group;
wherein the above-mentioned radicals C₁₋₆-alkyl, C₁₋₃-alkylene, C₁₋₆-alkylene, C₂₋₆-alkenylene, C₃₋₈-cycloalkyl, aryl and heteroaryl in each case can be unsubstituted or mono- or poly-substituted by identical or different radicals, and the above-mentioned radicals C₁₋₆-alkyl, C₁₋₃-alkylene, C₁₋₆-alkylene and C₂₋₆-alkenylene in each case can be branched or unbranched;
optionally in the form of an individual enantiomer or of an individual diastereoisomer, of the racemate, of the enantiomers, of the diastereoisomers, mixtures of enantiomers and/or diastereoisomers, as well as in each case in the form of their bases and/or physiologically acceptable salts.

2. Substituted compound according to claim 1, wherein R¹ represents phenyl, naphthyl, chromanyl, indolyl, benzofuranyl, benzothiophenyl (benzothienyl), 5,6-dihydro-4H-cyclopenta[b]thiophenyl, benzooxazolyl, benzooxadiazolyl, pyrrolyl, furanyl, thienyl, pyridinyl, pyridazinyl, pyrimidinyl, pyrazinyl, imidazothiazolyl, carbazolyl, dibenzofuranyl, dibenzothiophenyl (dibenzothienyl), or a phenyl or naphthyl bonded *via* a C₁₋₃-alkylene group or a C₂₋₃-alkenylene group, preferably phenyl, naphthyl, chromanyl, benzothiophenyl (benzothienyl), 5,6-dihydro-4H-cyclopenta[b]thiophenyl, benzooxadiazolyl, thienyl, pyridinyl, imidazothiazolyl, dibenzofuranyl, or a phenyl bonded *via* a C₁₋₃-alkylene group or a C₂₋₃-alkenylene group, particularly preferably phenyl, naphthyl, chromanyl, benzothiophenyl (benzothienyl), 5,6-dihydro-4H-cyclopenta[b]thiophenyl, thienyl, or a phenyl bonded *via* a C_{1 or 2}-alkylene group or -CH=CH- group, wherein the above-mentioned aryl or heteroaryl radicals are in each case unsubstituted or mono- or poly-substituted by identical or different substituents, the substituents being selected independently of one another in particular from the group consisting of -O-C₁₋₃-alkyl, C₁₋₆-alkyl, F, Cl, Br, I, CF₃, OCF₃, OH, SH, phenyl, phenoxy, naphthyl, furyl, thienyl and pyridinyl, and wherein the above-mentioned alkylene and alkenylene groups are in each case unsubstituted or mono- or poly-substituted by identical or different substituents, the substituents being selected independently of one another in particular from the group consisting of -O-C₁₋₃-alkyl, F, Cl, Br, I, CF₃, OCF₃, OH, SH, phenyl, phenoxy, naphthyl, furyl, thienyl and pyridinyl.

3. Substituted compound according to claim 1 or 2, wherein B represents S(=O)₂.

4. Substituted compound according to one or more of claims 1 to 3, wherein in the general formula (I) the partial structure (Ac) 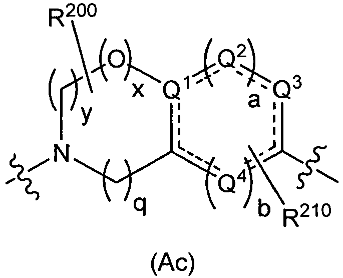 represents a partial structure selected from the group consisting of

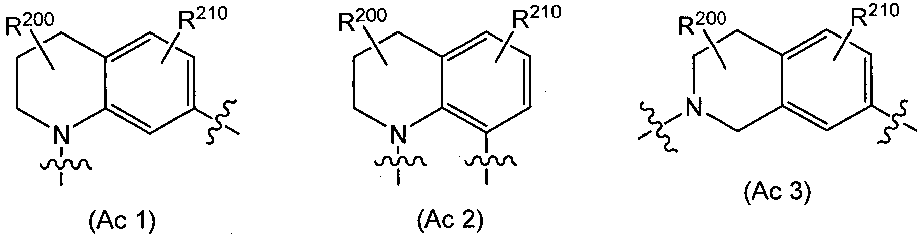

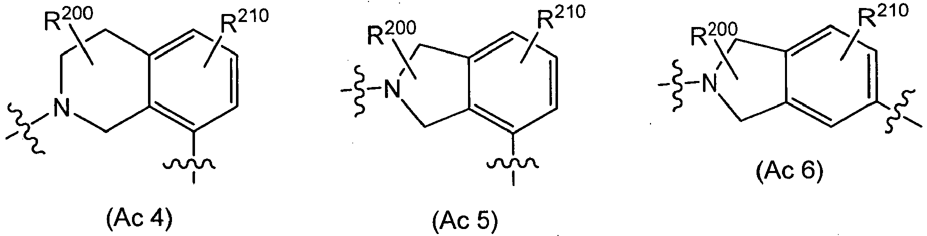

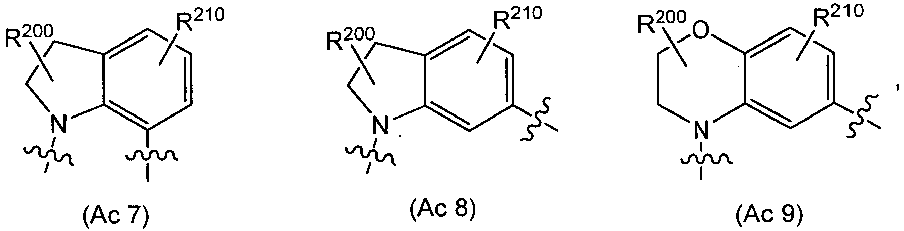

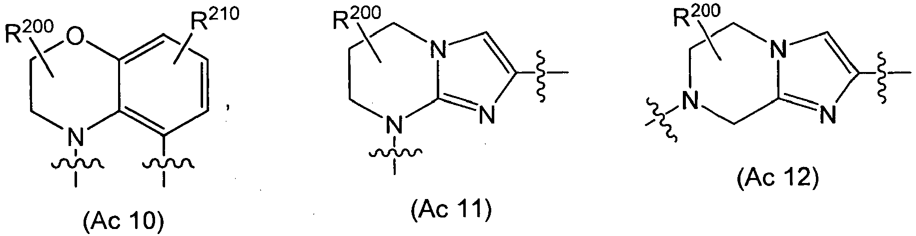

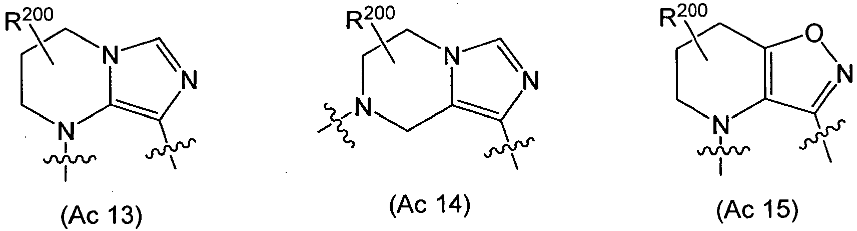

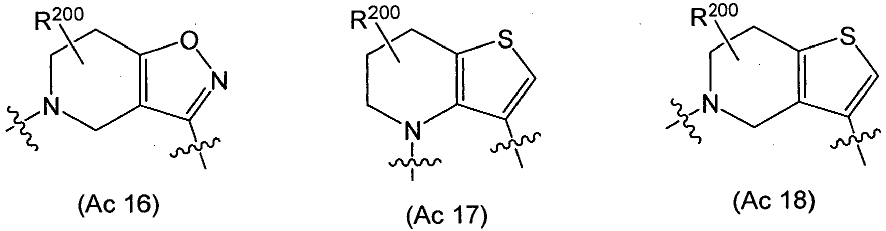

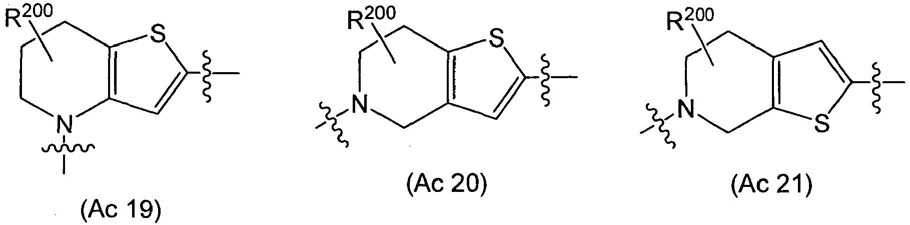

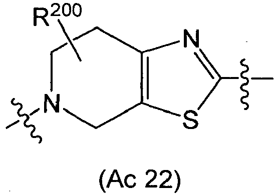

5. Substituted compound according to one or more of claims 1 to 4, wherein R⁸ represents H; C₁₋₆-alkyl, in particular methyl, ethyl, n-propyl, isopropyl, n-butyl, isobutyl, sec-butyl or tert-butyl; cyclopropyl, cyclobutyl, cyclopentyl, cyclohexyl, -CH₂CF₃, phenyl, benzyl, phenylethyl, phenylpropyl, or a cyclopropyl, cyclobutyl, cyclopentyl, cyclohexyl bonded *via* a C₁₋₃-alkylene group, in each case unsubstituted or mono- or poly-substituted by identical or different substituents.

6. Substituted compound according to one or more of claims 1 to 5, wherein R^{9a} and R^{9b} each independently of the other represents H; F; methyl; ethyl, isopropyl, CF₃, methoxy; cyclopropyl; phenyl; benzyl, phenylethyl, C₁₋₃-alkylene-cyclopropyl, C₁₋₃-alkylene-cyclobutyl, C₁₋₃-alkylene-cyclopentyl, C₁₋₃-alkylene-cyclohexyl, C₁₋₃-alkylene-CF₃, in each case unsubstituted or mono- or poly-substituted by identical or different substituents, preferably both R^{9a} and R^{9b} simultaneously represent H.

7. Substituted compound according to one or more of claims 1 to 6, wherein A represents N.

8. Substituted compound according to one or more of claims 1 to 7, wherein s and t each represents 0 and A represents N.

9. Substituted compound according to one or more of claims 1 to 8, wherein
X represents CR^{14a}R^{14b}, NR¹⁵ or O;
Y represents CR^{16a}R^{16b};
R^{14a}, R^{14b}, R^{16a} and R^{16b} each independently of the others denotes H, F, Cl, OH, C₁₋₆-alkyl, O-C₁₋₆-alkyl, C₃₋₈-cycloalkyl, aryl or heteroaryl, or represents a C₃₋₈-cycloalkyl, aryl or heteroaryl bonded *via* a C₁₋₆-alkylene group,
and/or in each case R^{14a} and R^{14b} together can represent =O and/or in each case R^{16a} and R^{16b} together can represent =O;
R¹⁵ represents H, C₁₋₆-alkyl, C₃₋₈-cycloalkyl, aryl or heteroaryl, or denotes a C₃₋₈-cycloalkyl, aryl or heteroaryl bonded *via* a C₁₋₆-alkylene group;
Z represents CR^{18a}R^{18b} or NR¹⁹; or
Z where X represents O and f represents 0, denotes -(N=(CR¹²⁶))-, wherein the N atom is singly bonded to the O atom, and
R¹²⁶ represents H, C₁₋₆-alkyl, C₃₋₈-cycloalkyl, aryl or heteroaryl, or denotes a C₃₋₈-cycloalkyl, aryl or heteroaryl bonded *via* a C₁₋₆-alkylene group;
R^{18a} represents H, C₁₋₆-alkyl, C₃₋₈-cycloalkyl, -NH(C₁₋₆-alkyl), -N(C₁₋₆-alkyl)₂, phenyl, pyridyl, imidazolyl, triazolyl, pyrimidyl, thiazolyl or thienyl, in each case unsubstituted or mono- or poly-substituted; or represents phenyl, pyridyl, imidazolyl, triazolyl, pyrimidyl, thiazolyl or thienyl bonded *via* a -(O)₀₋₁-C₁₋₆-alkylene group, in each case unsubstituted or mono- or poly-substituted; or
R^{18a} represents the radical according to the general formula (VII) 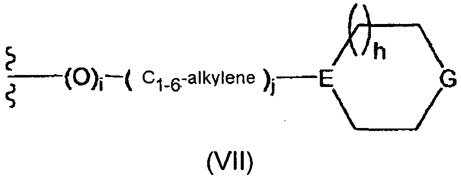 wherein
i represents 0 or 1;
j represents 0 or 1;
h represents 0 or 1;
E represents N or CH; with the proviso that when i represents 1 and j represents 0, E represents CH;
G represents CR^{37a}R^{37b} or NR³⁸; wherein
R^{37a} and R^{37b} independently of one another represent H; F or C₁₋₆-alkyl, in each case unsubstituted or mono- or poly-substituted by identical or different substituents;
R³⁸ represents H; C₁₋₆-alkyl, C₃₋₆-alkyl or pyridyl;
R^{18b} represents H, OH, C₁₋₆-alkyl, phenyl, pyridyl, imidazolyl, triazolyl, pyrimidyl, thiazolyl or thienyl, in each case unsubstituted or mono- or poly-substituted by identical or different substituents, phenyl, pyridyl, imidazolyl, triazolyl, pyrimidyl, thiazolyl or thienyl bonded *via* a C₁₋₆-alkylene group, in each case unsubstituted or mono- or poly-substituted by identical or different substituents; O-phenyl or O-pyridyl, in each case unsubstituted or mono- or poly-substituted by identical or different substituents; C₁₋₆-alkylene-NH(C=O)-bridged phenyl, pyridyl or thienyl, in each case unsubstituted or mono- or poly-substituted by identical or different substituents;
R¹⁹ represents H, C₁₋₆-alkyl, C₃₋₈-cycloalkyl, -(C=O)-C₁₋₆-alkyl, phenyl, pyridyl, thienyl, thiazolyl, triazolyl, pyrimidinyl or imidazolyl; in each case unsubstituted or mono- or poly-substituted by identical or different substituents; or phenyl, pyridyl, thienyl, thiazolyl, pyrimidinyl, triazolyl or imidazolyl bonded via a C₁₋₆-alkylene group, in each case unsubstituted or mono- or poly-substituted by identical or different substituents;
or represents the radical according to the general formula (VIII) 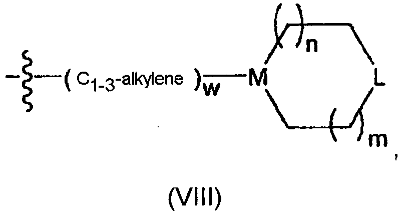 wherein
w represents 0 or 1;
n represents 0 or 1;
m represents 0 or 1;
M represents CH or N, with the proviso that when w represents 0, M represents CH;
L represents CR^{44a}R^{44b} or NR⁴⁵;
wherein R^{44a} and R^{44b} independently of one another represent H; F or C₁₋₆-alkyl, in each case unsubstituted or mono- or poly-substituted by identical or different substituents; and
R⁴⁵ represents H, C₁₋₆-alkyl, C₃₋₆-alkyl or pyridyl.

10. Substituted compound according to one or more of claims 1 to 9, wherein s and t each represents 0 and the following partial structure (SP) 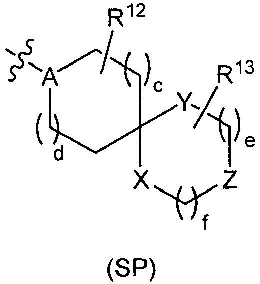 is selected from the group consisting of 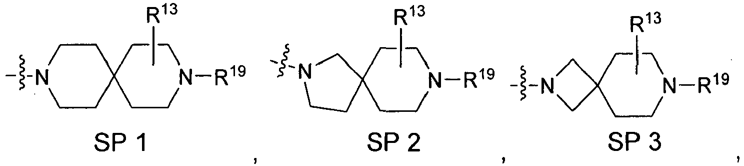 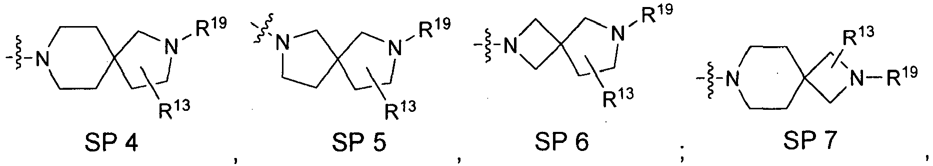 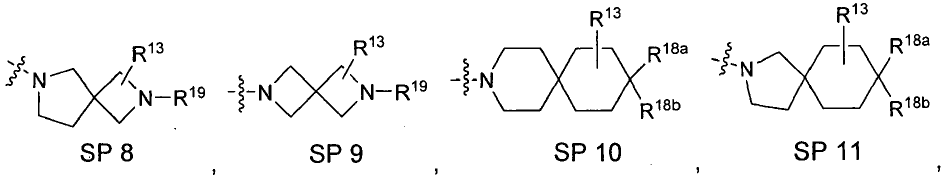 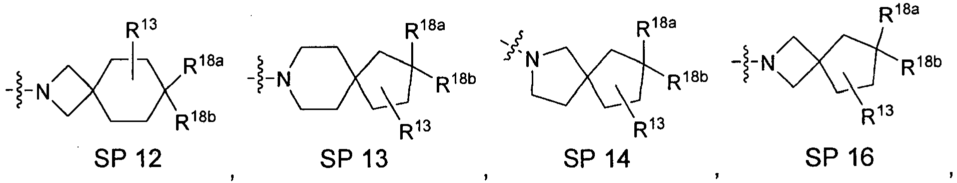 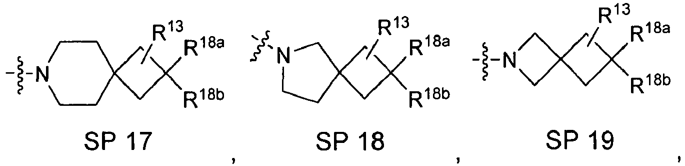 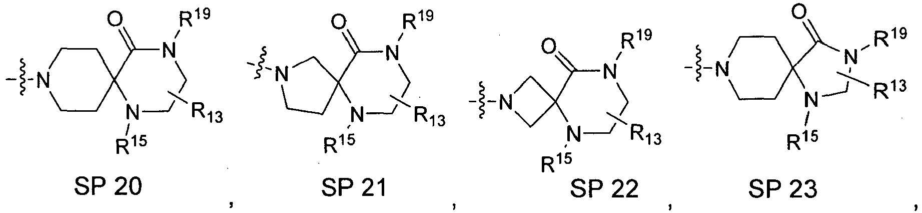 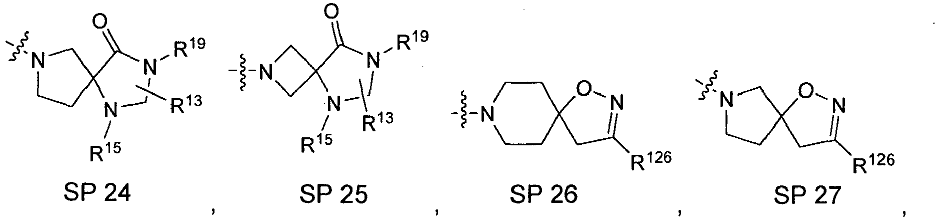 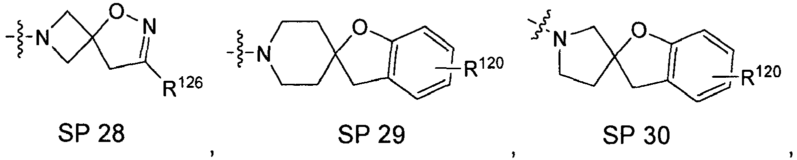 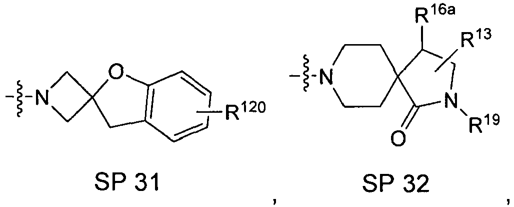 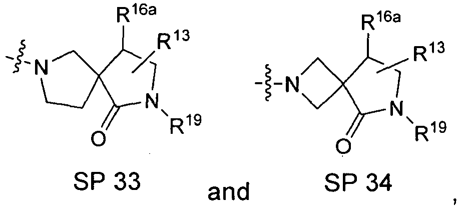 wherein
R¹³ represents 1 or 2 substituents selected from the group consisting of H and phenyl, unsubstituted or mono- or poly-substituted by identical or different substituents; and/or two of the substituents R¹³ together form =O and/or two adjacent substituents R¹³ together form a fused aryl or heteroaryl, in particular a benzo group, in each case unsubstituted or mono- or poly-substituted by identical or different substituents;
R¹⁵ represents H; C₁₋₆-alkyl; C₃₋₈-cycloalkyl, phenyl, pyridyl, in each case unsubstituted or mono- or poly-substituted by identical or different substituents; or phenyl or pyridyl bonded *via* a C₁₋₆-alkylene group and in each case unsubstituted or mono- or poly-substituted by identical or different substituents;
R^{16a} represents H, C₁₋₆-alkyl, phenyl or pyridyl, in each case unsubstituted or mono-or poly-substituted by identical or different substituents;
R^{18a} represents H; C₁₋₆-alkyl; C₃₋₈-cycloalkyl, N(C₁₋₆-alkyl)₂; NH(C₁₋₆-alkyl), azetidinyl; pyrrolidinyl, piperidinyl, 4-(C₁₋₆-alkyl)-piperazinyl; phenyl or pyridyl, in each case unsubstituted or mono- or poly-substituted by identical or different substituents; or N(C₁₋₆-alkyl)₂; NH(C₁₋₆-alkyl), azetidinyl; pyrrolidinyl, piperidinyl, 4-(C₁₋₆-alkyl)-piperazinyl; phenyl, imidazolyl, triazolyl, or pyridyl bonded *via* a -(O)_{0/1}-C₁₋₆-alkylene group and in each case unsubstituted or mono- or poly-substituted by identical or different substituents;
R^{18b} represents H; OH; C₁₋₆-alkyl; phenyl or pyridyl, in each case unsubstituted or mono- or poly-substituted by identical or different substituents; O-phenyl or O-pyridyl, in each case unsubstituted or mono- or poly-substituted by identical or different substituents, or phenyl or pyridyl bonded *via* a C₁₋₆-alkylene group and in each case unsubstituted or mono- or poly-substituted by identical or different substituents;
R¹⁹ represents H; C₁₋₆-alkyl; C₃₋₈-cycloalkyl, phenyl, pyridyl, thienyl, imidazolyl, thiazolyl or triazolyl, in each case unsubstituted or mono- or poly-substituted by identical or different substituents; or phenyl or pyridyl bonded *via* a C₁₋₆-alkylene group or (C=O)- group and in each case unsubstituted or mono- or poly-substituted by identical or different substituents;
R¹²⁰ represents H; F; Cl; OH; OCH₃, O-CF₃, C₁₋₆-alkyl; CF₃ or phenyl, unsubstituted or mono- or poly-substituted;
R¹²⁶ represents H; C₁₋₆-alkyl; C₃₋₆-cycloalkyl; phenyl or pyridyl; or C₃₋₆-cycloalkyl, phenyl or pyridyl bonded *via* a C₁₋₃-alkylene group and in each case unsubstituted or mono- or poly-substituted by identical or different substituents.

11. Substituted compounds according to one or more of claims 1 to 10, wherein s and t each represents 0 and the following partial structure (SP) 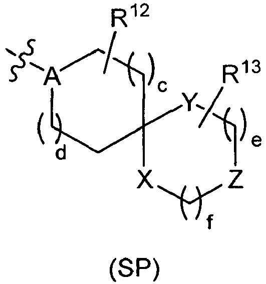 is selected from the group consisting of 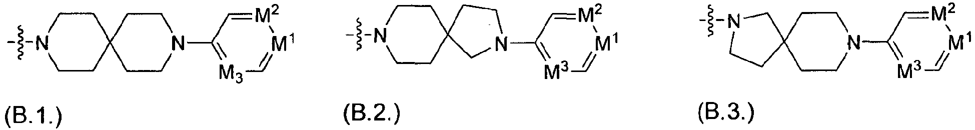 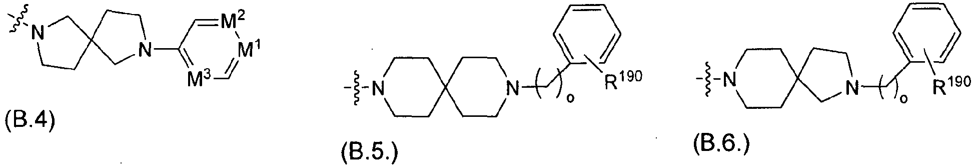 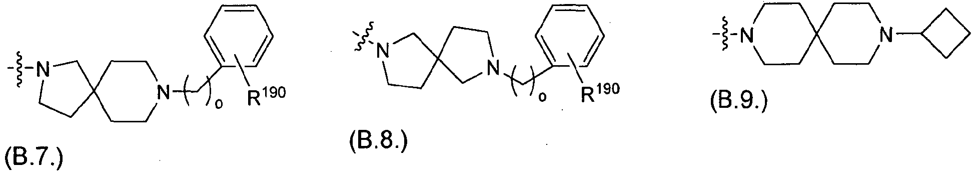 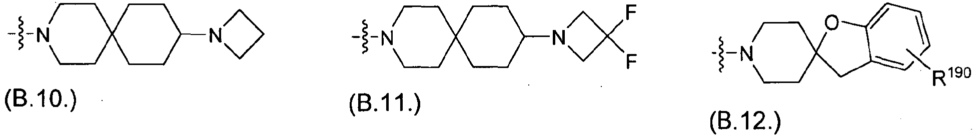 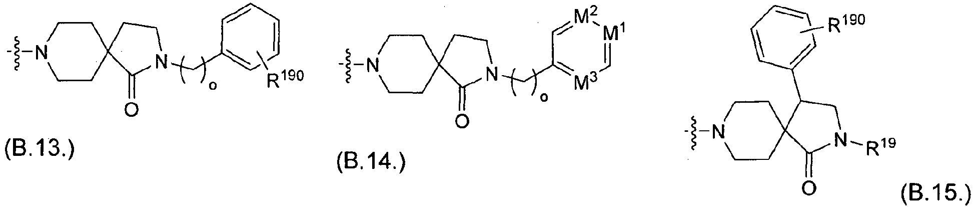 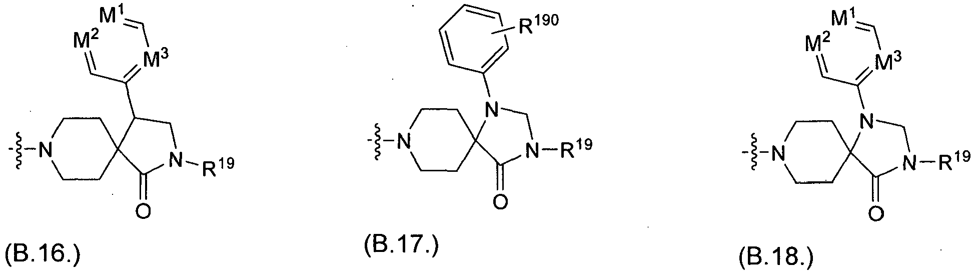 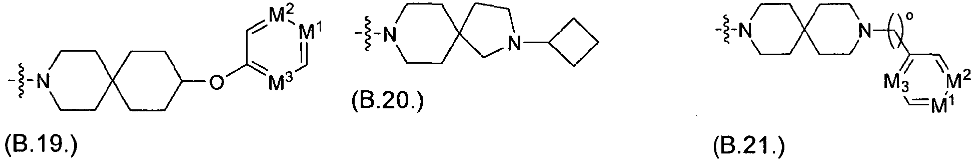 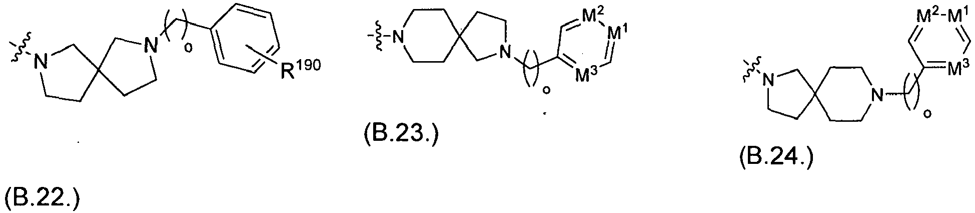 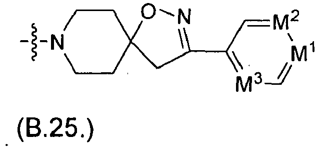 o = 0, 1, 2 or 3;
M¹, M² and M³ each independently of the others can represent N or CH, wherein one of the variables M¹, M² and M³ represents N and the others both represent CH;
R¹⁹ is selected from the group consisting of H; C₁₋₆-alkyl, in particular methyl, ethyl, n-propyl, isopropyl, n-butyl, sec-butyl, isobutyl and tert-butyl; C₃₋₆-cycloalkyl, in each case unsubstituted or mono- or poly-substituted by identical or different substituents; and
R¹⁹⁰ represents from 0 to 4 substituents selected independently of one another from F, Cl, O-CF₃, CF₃ and CN.

12. Compound according to one or more of claims 1 to 11 selected from the group consisting of
| **Compound** | **Name** |
|---|---|
| G-04 | (2-(4-Methoxy-2,6-dimethylphenylsulfonyl)-1,2,3,4-tetrahydroisoquinolin-7-yl)(9-(pyridin-4-yl)-3,9-diazaspiro[5.5]undecan-3-yl)methanone |
| G-09 | 2-(1-(4-Methoxy-2,6-dimethylphenylsulfonyl)-1,2,3,4-tetrahydroquinolin-7-yl)-1-(2-(pyridin-4-yl)-2,8-diazaspiro[4.5]decan-8-yl)ethanone |
| G-10 | 1-(2-Cyclobutyl-2,8-diazaspiro[4.5]decan-8-yl)-2-(1-(4-methoxy-2,6-dimethylphenylsulfonyl)-1,2,3,4-tetrahydroquinolin-7-yl)ethanone |
| G-11 | 2-(1-(4-Methoxy-2,6-dimethylphenylsulfonyl)-1,2,3,4-tetrahydroquinolin-7-yl)-1-(8-(pyridin-4-yl)-2,8-diazaspiro[4.5]decan-2-yl)ethanone |
| G-12 | 2-(1-(4-Methoxy-2,6-dimethylphenylsulfonyl)-1,2,3,4-tetrahydroquinolin-7-yl)-1-(7-(pyridin-4-yl)-2,7-diazaspiro[4.4]nonan-2-yl)ethanone |
| G-13 | (5-(2-Chlorobenzoyl)-4,5,6,7-tetrahydroisoxazolo[4,5-c]pyridin-3-yl)(9-(pyridin-4-yl)-3,9-diazaspiro[5.5]undecan-3-yl)methanone |
| G-14 | (5-(4-Methoxy-2,6-dimethylphenylsulfonyl)-4,5,6,7-tetrahydrothieno[3,2-c]pyridin-2-yl)(9-(pyridin-4-yl)-3,9-diazaspiro[5.5]undecan-3-yl)methanone |
| G-15 | (5-(2-Chlorobenzoyl)-4,5,6,7-tetrahydrothieno[3,2-c]pyridin-2-yl)(9-(pyridin-4-yl)-3,9-diazaspiro[5.5]undecan-3-yl)methanone |
| G-16 | 1-(9-(Pyridin-4-yl)-3,9-diazaspiro[5.5]undecan-3-yl)-2-(1-(2-(trifluoromethyl)phenylsulfonyl)-1,2,3,4-tetrahydroquinolin-7-yl)ethanone |
| G-17 | 2-(1-(Naphthalin-1-ylsulfonyl)-1,2,3,4-tetrahydroquinolin-7-yl)-1-(9-(pyridin-4-yl)-3,9-diazaspiro[5.5]undecan-3-yl)ethanone |
| G-18 | 2-(1-(2-Chloro-6-methylphenylsulfonyl)-1,2,3,4-tetrahydroquinolin-7-yl)-1-(9-(pyridin-4-yl)-3,9-diazaspiro[5.5]undecan-3-yl)ethanone |
| G-19 | 2-(4-(4-Methoxy-2,6-dimethylphenylsulfonyl)-3,4-dihydro-2H-benzo[b][1,4]oxazin-6-yl)-1-(9-(pyridin-4-yl)-3,9-diazaspiro[5.5]undecan-3-yl)ethanone |
| G-20 | 2-(1-(4-Methoxy-2,6-dimethylphenylsulfonyl)indolin-6-yl)-1-(9-(pyridin-4-yl)-3,9-diazaspiro[5.5]undecan-3-yl)ethanone |
| G-21 | (1-(2-Chlorobenzoyl)-1,2,3,4-tetrahydroquinolin-7-yl)(9-(pyridin-4-yl)-3,9-diazaspiro[5.5]undecan-3-yl)methanone |
| G-24 | (1-(4-Methoxy-2,6-dimethylphenylsulfonyl)-1,2,3,4-tetrahydroquinolin-7-yl)(9-(pyridin-4-yl)-3,9-diazaspiro[5.5]undecan-3-yl)methanone |
| G-25 | 2-(1-(4-Methoxy-2,6-dimethylphenylsulfonyl)-1,2,3,4-tetrahydroquínolin-7-yl)-1-(9-(pyridin-4-yl)-3,9-diazaspiro[5.5]undecan-3-yl)ethanone |
| G-26 | (5-(4-Methoxy-2,6-dimethylphenylsulfonyl)-4,5,6,7-tetrahydroisoxazolo[4,5-c]pyridin-3-yl)(9-(pyridin-4-yl)-3,9-diazaspiro[5.5]undecan-3-yl)methanone |
| G-30 | 2-(1-(4-Methoxy-2,6-dimethylphenylsulfonyl)-1,2,3,4-tetrahydroquinolin-7-yl)-1-(9-(pyridin-4-yloxy)-3-azaspiro[5.5]undecan-3-yl)ethanone |
| G-32 | 1-(9-(Azetidin-1-yl)-3-azaspiro[5.5]undecan-3-yl)-2-(1-(4-methoxy-2,6-dimethylphenylsulfonyl)-1,2,3,4-tetrahydroquinolin-7-yl)ethanone |
| G-33 | 2-(1-(4-Methoxy-2,6-dimethylphenylsulfonyl)-1,2,3,4-tetrahydroquinolin-7-yl)-1-(3-(pyridin-4-yl)-1-oxa-2,8-diazaspiro[4.5]dec-2-en-8-yl)ethanone |
| G-34 | 1-(9-(3,3-Difluoroazetidin-1-yl)-3-azaspiro[5,5]undecan-3-yl)-2-(1-(4-methoxy-2,6-dimethylphenylsulfonyl)-1,2,3,4-tetrahydroquinolin-7-yl)ethanone |
| G-35 | 2-(1-(2-Chloro-4-(trifluoromethoxy)phenylsulfonyl)-1,2,3,4-tetrahydroquinolin-7-yl)-1-(9-(pyridin-4-yl)-3,9-diazaspiro[5.5]undecan-3-yl)ethanone |
| G-36 | (2-(4-Methoxy-2,6-dimethylphenylsulfonyl)-1,2,3,4-tetrahydroisoquinolin-8-yl)(9-(pyridin-4-yl)-3,9-diazaspiro[5.5]undecan-3-yl)methanone |
| G-37 | (2-(2-Chloro-6-methylphenylsulfonyl)-1,2,3,4-tetrahydroisoquinolin-8-yl)(9-(pyridin-4-yl)-3,9-diazaspiro[5.5]undecan-3-yl)methanone |
| G-38 | (2-(2,3-Dichlorophenylsulfonyl)-1,2,3,4-tetrahydroisoquinolin-8-yl)(9-(pyridin-4-yl)-3,9-diazaspiro[5.5]undecan-3-yl)methanone |
| G-39 | 2-(2-(4-Methoxy-2,6-dimethylphenylsulfonyl)-1,2,3,4-tetrahydroisoquinolin-7-yl)-1-(9-(pyridin-4-yl)-3,9-diazaspiro[5.5]undecan-3-yl)ethanone |
| G-40 | (7-(4-Methoxy-2,6-dimethylphenylsulfonyl)-5,6,7,8-tetrahydroimidazo[1,2-a]pyrazin-2-yl)(9-(pyridin-4-yl)-3,9-diazaspiro[5.5]undecan-3-yl)methanone |
| G-41 | 3-(2-Chlorophenyl)-1-(7-(9-(pyridin-4-yl)-3,9-diazaspiro[5.5]undecane-3-carbonyl)-3,4-dihydroisoquinolin-2(1H)-yl)prop-2-en-1-one |
| G-42 | (5-Chlorothiophen-2-yl)(7-(9-(pyridin-4-yl)-3, 9-diazaspiro[5.5]undecane-3-carbonyl)-3,4-dihydroisoquinolin-2(1H)-yl)methanone |
| G-43 | 2-(2-Chlorophenyl)-1-(7-(9-(pyridin-4-yl)-3,9-diazaspiro[5.5]undecane-3-carbonyl)-3,4-dihydroisoquinolin-2(1H)-yl)ethanone |
| G-44 | (5-Chlorothiophen-2-yl)(8-(9-(pyridin-4-yl)-3,9-diazaspiro[5.5]undecane-3-carbonyl)-3,4-dihydroisoquinolin-2(1H)-yl)methanone |
| G-45 | N-(2-Chlorobenzyl)-7-(9-(pyridin-4-yl)-3,9-diazaspiro[5.5]-undecane-3-carbonyl)-3,4-dihydroisoquinoline-2(1H)-carboxamide |
| G-46 | N-(3,4-Dichlorophenyl)-3-(9-(pyridin-4-yl)-3,9-diazaspiro[5.5]undecane-3-carbonyl)-6,7-dihydroisoxazolo[4,5-c]-pyridine-5(4H)-carboxamide |
| G-47 | (2-(4-Methylnaphthalin-1-ylsulfonyl)-1,2,3,4-tetrahydroisoquinolin-7-yl)(9-(pyridin-4-yl)-3,9-diazaspiro[5.5]undecan-3-yl)methanone |
| G-48 | (2-(4-Methoxynaphthalin-1-ylsulfonyl)-1,2,3,4-tetrahydroisoquinolin-7-yl)(9-(pyridin-4-yl)-3,9-diazaspiro[5.5]undecan-3-yl)methanone |
| G-49 | 2,2-Diphenyl-1-(7-(9-(pyridin-4-yl)-3,9-diazaspiro[5.5]undecane-3-carbonyl)-3,4-dihydroisoquinolin-2(1H)-yl)ethanone |
| G-50 | (2-(4-Chloronaphthalin-1-ylsulfonyl)-1,2,3,4-tetrahydroisoquinolin-7-yl)(9-(pyridin-4-yl)-3,9-diazaspiro[5.5]undecan-3-yl)methanone |
| G-51 | 2-(1-(2-Chloro-6-methylphenylsulfonyl)-1,2,3,4-tetrahydroquinolin-7-yl)-1-(8-(pyridin-4-yl)-2,8-diazaspiro[4.5]decan-2-yl)ethanone |
| G-52 | 2-(1-(Naphthalin-1-ylsulfonyl)-1,2,3,4-tetrahydroquinolin-7-yl)-1-(8-(pyridin-4-yl)-2,8-diazaspiro[4.5]decan-2-yl)ethanone |
| G-53 | 2-(1-(Naphthalin-1-ylsulfonyl)-1,2,3,4-tetrahydroquinolin-7-yl)-1-(7-(pyridin-4-yl)-2,7-diazaspiro[4.4]nonan-2-yl)ethanone |
| G-54 | 1-(9-(Azetidin-1-yl)-3-azaspiro[5.5]undecan-3-yl)-2-(1-(naphthalin-1-ylsulfonyl)-1,2,3,4-tetrahydroquinolin-7-yl)ethanone |
| G-55 | N-(3,4-dichlorophenyl)-7-(9-(pyridin-4-yl)-3,9-diazaspiro[5.5]undecane-3-carbonyl)-3,4-dihydroisoquinoline-2(1 H)-carboxamide |
| G-56 | (2-(4-Fluoronaphthalin-1-ylsulfonyl)-1,2,3,4-tetrahydroisoquinolin-7-yl)(9-(pyridin-4-yl)-3,9-diazaspiro[5.5]undecan-3-yl)methanone |
| G-57 | 1-(8-(Pyridin-4-yl)-2,8-diazaspiro[4.5]decan-2-yl)-2-(1-(2-(trifluoromethyl)phenylsulfonyl)-1,2,3,4-tetrahydroquinolin-7-yl)ethanone |
| G-58 | (5-Methylthiophen-2-yl)(7-(9-(pyridin-4-yl)-3,9-diazaspiro[5.5]undecane-3-carbonyl)-3,4-dihydroisoquinolin-2(1H)-yl)methanone |
| G-59 | Benzo[b]thiophen-2-yl(7-(9-(pyridin-4-yl)-3,9-diazaspiro[5.5]undecane-3-carbonyl)-3,4-dihydroisoquinolin-2(1H)-yl)methanone |
| G-60 | (5,6-Dihydro-4H-cyclopenta[b]thiophen-2-yl)(7-(9-(pyridin-4-yl)-3,9-diazaspiro[5.5]undecane-3-carbonyl)-3,4-dihydroisoquinolin-2(1H)-yl)methanone |
| G-61 | (3-Chloro-6-methoxybenzo[b]thiophen-2-yl)(7-(9-(pyridin-4-yl)-3,9-diazaspiro[5.5]undecane-3-carbonyl)-3,4-dihydroisoquinolin-2(1H)-yl)methanone |
| G-62 | (2-(5-Chloronaphthalin-1-ylsulfonyl)-1,2,3,4-tetrahydroisoquinolin-7-yl)(9-(pyridin-4-yl)-3,9-diazaspiro[5.5]undecan-3-yl)methanone |
| G-63 | (5-tert-Butylthiophen-2-yl)(7-(9-(pyridin-4-yl)-3,9-diazaspiro[5.5]undecane-3-carbonyl)-3,4-dihydroisoquinolin-2(1H)-yl)methanone |
| G-64 | 2-[1-(2-Chloro-benzoyl)-1,2,3,4-tetrahydro-quinolin-7-yl]-1-(9-pyridin-4-yl-3,9-diazaspiro[5.5]undecan-3-yl)-ethanone |
| G-66 | 2-[1-[(2,6-Dichloro-3-methyl-phenyl)sulfonyl]-1,2,3,4-tetrahydro-quinolin-7-yl]-1-(9-pyridin-4-yl-3,9-diazaspiro[5.5]undecan-3-yl)-ethanone |
| G-67 | [2-[(4-Methoxy-2,6-dimethyl-phenyl)sulfonyl]-2,3-dihydro-1 H-isoindol-5-yl]-(9-pyridin-4-yl-3,9-diazaspiro[5.5]undecan-3-yl)-methanone |
| G-68 | [2-[(2-Chloro-6-methyl-phenyl)sulfonyl]-2,3-dihydro-1H-isoindol-5-yl]-(9-pyridin-4-yl-3,9-diazaspiro[5.5]undecan-3-yl)-methanone |
| G-69 | [2-(5-Chloro-thiophene-2-carbonyl)-2,3-dihydro-1H-isoindol-5-yl]-(9-pyridin-4-yl-3,9-diazaspiro[5.5]undecan-3-yl)-methanone |
| G-70 | 2-[8-[(4-Methoxy-2,6-dimethyl-phenyl)sulfonyl]-5,6,7,8-tetrahydro-[1,8]naphthyridin-2-yl]-1-(9-pyridin-4-yl-3,9-diazaspiro[5.5]-undecan-3-yl)-ethanone |
| G-71 | [7-(5-Chloro-thiophene-2-carbonyl)-5,6,7,8-tetrahydro-imidazo-[1,2-a]pyrazin-2-yl]-(9-pyridin-4-yl-3,9-diazaspiro[5.5]undecan-3-yl)-methanone |
| G-72 | [7-[(4-Methoxy-2,6-dimethyl-phenyl)sulfonyl]-5,6,7,8-tetrahydro-imidazo[1,5-a]pyrazin-1-yl]-(9-pyridin-4-yl-3,9-diazaspiro[5.5]-undecan-3-yl)-methanone |
| G-73 | [2-(4-Methoxy-2,6-dimethyl-benzoyl)-2,3-dihydro-1H-isoindol-5-yl]-(9-pyridin-4-yl-3,9-diazaspiro[5.5]undecan-3-yl)-methanone |
| G_CC-006 | 2-(4-Fluorobenzyl)-8-(1-(4-methoxy-2,6-dimethylphenylsulfonyl)indoline-6-carbonyl)-2,8-diazaspiro[4.5]decan-1-one |
| G_CC-007 | (7-Benzyl-2,7-diazaspiro[4.4]nonan-2-yl)(1-(4-methoxy-2,6-dimethylphenylsulfonyl)indolin-6-yl)methanone |
| G_CC-008 | (1-(4-Methoxy-2,6-dimethylphenylsulfonyl)indolin-6-yl)(9-(pyridin-4-yl)-3,9-diazaspiro[5.5]undecan-3-yl)methanone |
| G_CC-009 | (1-(2-Chlorobenzoyl)indolin-6-yl)(9-(pyridin-4-yl)-3,9-diazaspiro[5.5]undecan-3-yl)methanone |
| G_CC-013 | (1-(4-Chloro-2,5-dimethylphenylsulfonyl)indolin-6-yl)(9-(pyridin-4-yl)-3,9-diazaspiro[5.5]undecan-3-yl)methanone |
| G_CC-018 | 8-(2-(2-Chlorobenzoyl)isoindoline-5-carbonyl)-2-(4-fluorobenzyl)-2,8-diazaspiro[4.5]decan-1-one |
| G_CC-025 | 4-(4-Fluorophenyl)-8-(1-(4-methoxy-2,6-dimethylphenylsulfonyl)-1,2,3,4-tetrahydroquinoline-7-carbonyl)-2-methyl-2,8-diazaspiro[4.5]decan-1-one |
| G_CC-026 | 2-Benzyl-8-(1-(4-methoxy-2,6-dimethylphenylsulfonyl)-1,2,3,4-tetrahydroquinoline-7-carbonyl)-2,8-diazaspiro[4.5]decan-1-one |
| G_CC-027 | (7-Benzyl-2,7-diazaspiro[4.4]nonan-2-yl)(1-(4-methoxy-2,6-dimethylphenylsulfonyl)-1,2,3,4-tetrahydroquinolin-7-yl)methanone |
| G_CC-039 | 2-(4-Fluorobenzyl)-8-(2-(4-methoxy-2,6-dimethylphenylsulfonyl)-1,2,3,4-tetrahydroisoquinoline-7-carbonyl)-2,8-diazaspiro[4.5]decan-1-one |
| G_CC-040 | 2-Benzyl-8-(2-(4-methoxy-2,6-dimethylphenylsulfonyl)-1,2,3,4-tetrahydroisoquinoline-7-carbonyl)-2,8-diazaspiro[4.5]decan-1-one |
| G_CC-041 | (7-Benzyl-2,7-diazaspiro[4.4]nonan-2-yl)(2-(4-methoxy-2,6-dimethylphenylsulfonyl)-1,2,3,4-tetrahydroisoquinolin-7-yl)methanone |
| G_CC-043 | 8-(2-(4-Methoxy-2,6-dimethylphenylsulfonyl)-1,2,3,4-tetrahydroisoquinoline-7-carbonyl)-1-phenyl-1,3,8-triazaspiro[4.5]decan-4-one |
| G_CC-045 | (2-(2-Chlorobenzoyl)-1,2,3,4-tetrahydroisoquinolin-7-yl)(9-(pyridin-4-yl)-3,9-diazaspiro[5.5]undecan-3-yl)methanone |
| G_CC-053 | 2-Benzy)-8-(2-(1-(4-methoxy-2,6-d!methy)phenylsulfonyl)-1,2,3,4-tetrahydroquinolin-7-yl)acetyl)-2,8-diazaspiro[4.5]decan-1-one |
| G_CC-054 | 1-(7-Benzyl-2,7-diazaspiro[4.4]nonan-2-yl)-2-(1-(4-methoxy-2,6-dimethylphenylsulfonyl)-1,2,3,4-tetrahydroquinolin-7-yl)ethanone |
| G_CC-055 | 2-[1-[(4-Methoxy-2,6-dimethyl-phenyl)sulfonyl]-1,2,3,4-tetrahydro-quinolin-7-yl]-1-(3-pyridin-4-yl-3,9-diazaspiro[5.5]undecan-9-yl)-ethanone |
| G_CC-055 | [1-[(4-Methoxy-2,6-dimethyl-phenyl)sulfonyl]-1,2,3,4-tetrahydro-quinolin-7-yl]-(3-pyridin-4-yl-3,9-diazaspiro[5.5]undecan-9-yl)-methanone |
optionally in the form of an individual enantiomer or of an individual diastereoisomer, of the racemate, of the enantiomers, of the diastereoisomers, mixtures of enantiomers or diastereoisomers, in each case in the form of their bases and/or physiologically acceptable salts, in particular the hydrochloride salts.

13. Medicament comprising at least one compound according to one or more of claims 1 to 12.

14. Use of at least one compound according to one or more of claims 1 to 12 in the preparation of a medicament for the treatment of pain, in particular acute pain, visceral pain, neuropathic pain, chronic pain and/or inflammatory pain; migraine; diabetes; respiratory diseases; inflammatory intestinal diseases; neurological diseases; inflammations of the skin; rheumatic diseases; septic shock; reperfusion syndrome; obesity and/or as an angiogenesis inhibitor.

## Revendications

1. Composé spiroamide substitué de formule générale (I), 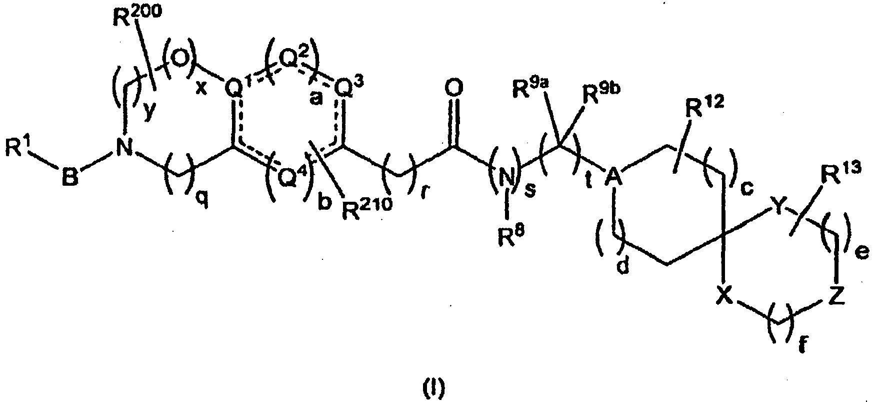 dans laquelle
Q¹ représente C ou N ;
Q² représente CH, N, O ou S ;
Q³ représente CH, N, O ou S ;
Q⁴ représente CH, N, O ou S ;
B représente C(=O), S(=O)₂ ou le groupe -C(=O)-N(R⁴), où son atome d'azote est lié au radical R¹ ;
a représente 0, 1 ou 2 ;
b représente 0 ou 1, à la condition que a+b = 1 ou 2 ; q représente 0 ou 1 ;
x représente 0 ou 1 ;
y représente 1, 2 ou 3 ;
r représente 0, 1, 2 ou 3 ;
R¹ représente un groupe aryle, hétéroaryle ou un groupe aryle ou hétéroaryle lié par un groupe alkylène en C₁ à C₃ ou un groupe alcénylène en C₂ à C₆, le groupe aryle et hétéroaryle pouvant être condensés respectivement par un cycle ou un hétérocycle de 4, 5, 6 ou 7 membres, le cycle et l'hétérocycle étant respectivement saturés ou au moins mono-insaturés, mais pas aromatiques et pouvant être substitués respectivement sur un ou plusieurs de leurs membres du cycle carbone par un ou plusieurs radicaux, choisis indépendamment les uns des autres dans le groupe constitué par F, Cl, Br, I, -CF₃, -O-CF₃, alkyle en C₁ à C₆ et O-alkyle en C₁ à C₆ et l'hétérocycle pouvant contenir un ou plusieurs hétéroatomes ou groupes d'hétéroatomes choisis indépendamment les uns des autres dans le groupe constitué par N, NR⁵⁰, O, S, S=O ou S(=O)₂ ; le radical R⁵⁰ représentant H, alkyle en C₁ à C₆, -C(=O)-R⁵¹, cycloalkyle en C₃ à C₈, aryle, hétéroaryle ou un cycloalkyle en C₃ à C₈, aryle, hétéroaryle lié par un groupe alkylène en C₁ à C₃, et R⁵¹ représentant un groupe alkyle en C₁ à C₆, cycloalkyle en C₃ à C₈, aryle, hétéroaryle ou un groupe cycloalkyle en C₃ à C₈, aryle ou hétéroaryle lié par un groupe alkylène en C₁ à C₃ ;
R⁴ représente H, un groupe alkyle en C₁ à C₆, aryle ou un groupe aryle lié par un groupe alkylène en C₁ à C₃ ;
R²⁰⁰ représente 0 à 4 substituants, qui sont choisis indépendamment les uns des autres dans le groupe constitué par alkyle en C₁ à C₆, F, Cl, CF₃ et OCF₃ ;
R²¹⁰ représente 0 à 4 substituants, qui sont choisis indépendamment les uns des autres dans le groupe constitué par alkyle en C₁ à C₆, O-alkyle en C₁ à C₆, F, Cl, CF₃ et OCF₃ ;
s = 0 ou 1,
t = 0, 1, 2 ou 3 à la condition que si s est égal à 0, t représente 0,
R⁸ représente H, un groupe alkyle en C₁ à C₆, cycloalkyle en C₃ à C₈, aryle ou hétéroaryle ou un groupe cycloalkyle en C₃ à C₈, aryle ou hétéroaryle lié par un groupe alkylène en C₁ à C₆ ;
R^{9a} et R^{9b} représentent, indépendamment les uns des autres, H, F, Cl, OH, un groupe alkyle en C₁ à C₆, O-alkyle en C₁ à C₆, cycloalkyle en C₃ à C₈, aryle ou hétéroaryle, ou un groupe cycloalkyle en C₃ à C₈, aryle ou hétéroaryle lié par un groupe alkylène en C₁ à C₆ ;
A représente N ou CH ;
à la condition que, si s est égal à 1 et t est égal à 0, A représente CH ; et
à la condition que, si s et t représentent chacun 0, A représente N ;
c, d, e et f représentent chacun indépendamment les uns des autres 0, 1 ou 2 ;
R¹² et R¹³ représentent chacun, indépendamment l'un de l'autre, 0 à 4 substituants, qui sont choisis indépendamment les uns des autres dans le groupe constitué par F, Cl, OH, =O, alkyle en C₁ à C₆, O-alkyle en C₁ à C₆, cycloalkyle en C₃ à C₈, aryle, hétéroaryle et le groupe cycloalkyle en C₃ à C₈, aryle ou hétéroaryle lié par un groupe alkylène en C₁ à C₆ ;
et/ou deux des 0 à 4 substituants adjacents R¹³ forment un groupe aryle ou hétéroaryle condensé ;
X représente CR^{14a}R^{14b}, NR¹⁵ ou O ;
Y représente CR^{16a}R^{16b}, NR¹⁷ ou O ;
à la condition que X ne représente pas NR¹⁵ si Y représente NR¹⁷ ; et
à la condition que X et Y ne représentent pas simultanément O ;
où
R^{14a}, R^{14b}, R^{16a} et R^{16b} représentent, chacun indépendamment les uns des autres, H, F, Cl, OH, alkyle en C₁ à C₆, O-alkyle en C₁ à C₆, cycloalkyle en C₃ à C₈, aryle ou hétéroaryle, ou un groupe cycloalkyle en C₃ à C₈, aryle ou hétéroaryle lié par un groupe alkylène en C₁ à C₆ ;
et/ou respectivement R^{14a} et R^{14b} peuvent représenter conjointement =O et/ou R^{16a} et R^{16b} peuvent représenter conjointement =O ;
R¹⁵ et R¹⁷ représentent, chacun indépendamment l'un de l'autre, H, un groupe alkyle en C₁ à C₆, cycloalkyle en C₃ à C₈, aryle ou hétéroaryle, ou un groupe cycloalkyle en C₃ à C₈, aryle ou hétéroaryle lié par un groupe alkylène en C₁ à C₆ ;
Z représente CR¹⁸R^{18b}, NR¹⁹ ou O ;
ou
Z, dans le cas où X représente O et f représente 0, représente -(C(R¹²⁴)-C(R¹²⁵))-, où
R¹²⁴ et R¹²⁵, conjointement avec les atomes de carbone qui les relient, forment un aryle ou hétéroaryle condensé ; ou
Z, dans le cas où X représente O et f représente 0, représente -(N=(CR¹²⁶))-, où l'atome de N est lié à l'atome de O par une simple liaison, et
R¹²⁶ représente H, un groupe alkyle en C₁ à C₆, cycloalkyle en C₃ à C₈, aryle ou hétéroaryle, ou un groupe cycloalkyle en C₃ à C₈, aryle ou hétéroaryle lié par un groupe alkylène en C₁ à C₆ ; et
dans laquelle
R^{18a} représente H, un groupe alkyle en C₁ à C₆, cycloalkyle en C₃ à C₈, aryle ou hétéroaryle, ou un groupe cycloalkyle en C₃ à C₈, aryle ou hétéroaryle lié par un groupe alkylène en C₁ à C₆ ;
ou R^{18a} représente un groupe selon la formule générale (II), 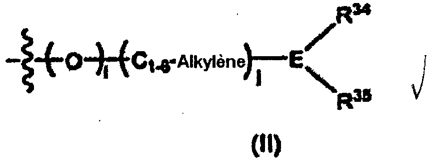 dans laquelle
i et j représentent, chacun indépendamment l'un de l'autre, 0 ou 1 ;
E représente N ou CH, à la condition que si i représente 1 et j représente 0, E représente CH
R³⁴ et R³⁵ représentent, chacun indépendamment l'un de l'autre, H, un groupe alkyle en C₁ à C₆, cycloalkyle en C₃ à C₈, aryle ou hétéroaryle, ou un groupe cycloalkyle en C₃ à C₈, aryle ou hétéroaryle lié par un groupe alkylène en C₁ à C₃ ;
ou R³⁴ et R³⁵ constituent, en incluant E, un groupe aryle ou hétéroaryle de 5 ou 6 membres ; V
ou R³⁴ et R³⁵ constituent, en incluant E, un hétérocycle saturé selon la formule générale (III), 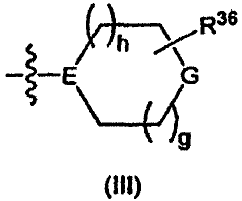 dans laquelle
h et g représentent, indépendamment l'un de l'autre, 0, 1 ou 2 ;
G représente CR^{37a}R^{37b}, NR³⁸, O, S, S=O ou S(=O)₂, à la condition que, si E représente CH, G ne représente pas CR^{37a}R^{37b} ;
R³⁶ représente 0 à 4 substituants, qui sont choisis, chacun indépendamment les uns des autres, dans le groupe constitué par F, Cl, Br, I, OH, SH, =O, O-alkyle en C₁ à C₆, alkyle en C₁ à C₆, cycloalkyle en C₃ à C₈, aryle, hétéroaryle, et cycloalkyle en C₃ à C₈, aryle ou hétéroaryle lié par un groupe alkylène en C₁ à C⁶ ;
et/ou deux des substituants adjacents R³⁶ constituent ensemble un groupe aryle ou hétéroaryle condensé ;
R^{37a} et R^{37b} représentent, chacun indépendamment l'un de l'autre, H, F, Cl, Br, I, OH, SH, =O, un groupe O-alkyle en C₁ à C₆, alkyle en C₁ à C₆, cycloalkyle en C₃ à C₈, aryle ou hétéroaryle, ou un groupe cycloalkyle en C₃ à C₈, aryle ou hétéroaryle lié par un groupe alkylène en C₁ à C₆ ;
R³⁸ représente H, un groupe alkyle en C₁₋₆, cycloalkyle en C₃ à C₈, aryle ou hétéroaryle, ou un groupe cycloalkyle en C₃ à C₈, aryle ou hétéroaryle lié par un groupe alkylène en C₁ à C₃ ;
R^{18b} représente H, OH, un groupe alkyle en C₁ à C₆, cycloalkyle en C₃ à C₈, O-alkyle en C₁ à C₆, O-(cycloalkyle en C₃ à C₈), (alkylène en C₁ à C₆)-O-alkyle en C₁ à C₆, (alkylène en C₁ à C₆)-O-(cycloalkyle en C₃ à C₈), aryle, hétéroaryle, O-aryle ou O-hétéroaryle ou un groupe aryle, O-aryle, hétéroaryle ou O-hétéroaryle lié par un groupe alkylèrie en C₁ à C₆ ;
ou R^{18b} représente un groupe selon la formule générale (IV), 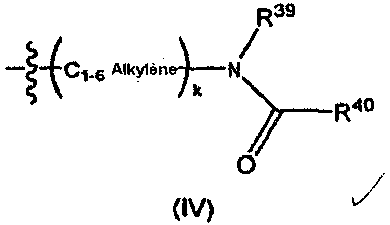 dans laquelle
k représente 0 ou 1 ;
R³⁹ représente H, un groupe alkyle en C₁ à C₆, cycloalkyle en C₃ à C₈, aryle ou hétéroaryle, ou un groupe cycloalkyle en C₃ à C₈, aryle ou hétéroaryle lié par un groupe alkylène en C₁ à C₃ ;
R⁴⁰ représente un groupe alkyle en C₁ à C₆, cycloalkyle en C₃ à C₈, aryle ou hétéroaryle, ou un groupe cycloalkyle en C₃ à C₈, aryle ou hétéroaryle lié par un groupe alkylène en C₁ à C₆ ;
ou
R³⁹ et R⁴⁰ forment, conjointement avec le groupe N-C(=O) auquel ils sont reliés, un cycle selon la formule générale (V), 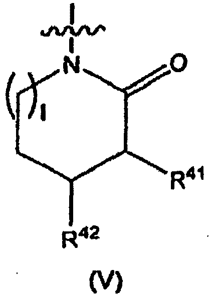 dans laquelle
I représente 0, 1 ou 2 ;
et R⁴¹ et R⁴² forment, conjointement avec les atomes de carbone auxquels ils sont reliés, un aryle ou hétéroaryle condensé ;
R¹⁹ représente H ; ou (P)_{z}-R²²,
où
z représente 0 ou 1 ;
P représente (C=O), S(=O)₂ ou C(=O)-N(R²⁴), où l'atome de N dans le groupe C(=O)-N(R²⁴) est lié à R²², où
R²⁴ représente H, un groupe alkyle en C₁ à C₆, cycloalkyle en C₃ à C₈, ou un groupe cycloalkyle en C₃ à C₈, aryle ou hétéroaryle lié par un groupe alkylène en C₁ à C₃ ;
R²² représente un groupe alkyle en C₁ à C₆, aryle ou hétéroaryle, ou un groupe aryle ou hétéroaryle lié par un groupe alkylène en C₁ à C₆ ; ou
R²² représente un groupe selon la formule générale (VI), dans laquelle 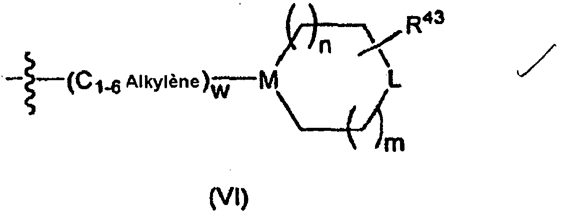 n représente 0, 1 ou 2 ;
m représente 0, 1 ou 2 ;
w représente 0 ou 1 ;
M représente CH ou N ;
à la condition que, si P représente C(=O)-NR²⁴ et w représente 0, M représente CH ; et
à la condition que, si z et w représentent simultanément 0, M représente CH ;
L représente CR^{44a}R^{44b}, NR⁴⁵ , O, S, S=O ou S(=O)₂ ;
R⁴³ représente 0 à 4 substituants, qui sont choisis, respectivement indépendamment les uns des autres, dans le groupe constitué par F, Cl, OH, =O, alkyle en C₁ à C₆, O-alkyle en C₁ à C₆, cycloalkyle en C₃ à C₈, aryle, hétéroaryle, et un groupe cycloalkyle en C₃ à C₈, aryle ou hétéroaryle lié par un groupe alkylène en C₁ à C₆ ;
et/ou deux des 0 à 4 radicaux adjacents R⁴³ constituent ensemble un groupe aryle ou hétéroaryle condensé;
R^{44a} et R^{44b} représentent, chacun indépendamment l'un de l'autre, H, F, Cl, Br, I, OH, un groupe alkyle en C₁ à C₆, O-alkyle en C₁ à C₆, cycloalkyle en C₃ à C₈, aryle ou hétéroaryle, ou un groupe cycloalkyle en C₃ à C₈, aryle ou hétéroaryle lié par un groupe alkylène en C₁ à C₆ ;
ou R^{44a} et R^{44b} peuvent représenter conjointement =O ; R⁴⁵ représente H, un groupe alkyle en C₁ à C₆, cycloalkyle en C₃ à C₈, aryle ou hétéroaryle, ou un groupe cycloalkyle en C₃ à C₈, aryle ou hétéroaryle lié par un groupe alkylène en C₁ à C₃ ;
dans lesquels les radicaux susmentionnés alkyle en C₁ à C₆, alkylène en C₁ à C₃, alkylène en C₁ à C₆, alcénylène en C₂ à C₆, cycloalkyle en C₃ à C₈, aryle et hétéroaryle, peuvent être respectivement non substitués ou mono- ou polysubstitués par des radicaux identiques ou différents et les radicaux susmentionnés alkyle en C₁ à C₆, alkylène en C₁ à C₃, alkylène en C₁ à C₆, et alcénylène en C₂ à C₆ peuvent être respectivement ramifiés ou non ramifiés;
le cas échéant sous la forme d'un énantiomère individuel ou d'un diastéréomère individuel, du racémate, des énantiomères, des diastéréomères, des mélanges des énantiomères et/ou des diastéréomères, ainsi que respectivement sous la forme de leurs bases et/ou de leurs sels physiologiquement acceptables.

2. Composé substitué selon la revendication 1, dans lequel R¹ représente un groupe phényle, naphtyle, chromanyle, indolyle, benzofurannyle, benzothiophényle (benzothiényle), 5,6-dihydro-4H-cyclopenta[b]thiophényle, benzooxazolyle, benzooxadiazolyle, pyrrolyle, furannyle, thiényle, pyridinyle, pyridazinyle, pyrimidinyle, pyrazinyle, imidazothiazolyle, carbazolyle, dibenzofurannyle, dibenzothiophényle (dibenzothiényle) ou un groupe phényle ou naphtyle lié par un groupe alkylène en C₁ à C₃ ou alcénylène en C₂ à C₃, de préférence phényle, naphtyle, chromanyle, benzothiophényle (benzothiényle), 5,6-dihydro-4H-cyclopenta[b]thiophényle, benzooxadiazolyle, thiényle, pyridinyle, imidazothiazolyle, dibenzofurannyle, ou un groupe phényle lié par un groupe alkylène en C₁ à C₃ ou alcénylène en C₂ à C₃, particulièrement préférentiellement phényle, naphtyle, chromanyle, benzothiophényle (benzothiényle), 5,6-dihydro-4H-cyclopenta[b]thiophényle, thiényle ou phényle lié par un groupe alkylène en C₁ ou C₂ ou -CH=CH-, les radicaux aryle ou hétéroaryle mentionnés précédemment pouvant être respectivement non substitués ou mono- ou polysubstitués, de manière identique ou différente, où les substituants sont choisis, indépendamment les uns des autres, en particulier dans le groupe constitué par -O-alkyle en C₁ à C₃, alkyle en C₁ à C₆, F, Cl, Br, I, CF₃, OCF₃, OH, SH, phényle, phénoxy, naphtyle, furyle, thiényle et pyridinyle et où les groupes alkylène et alcénylène susmentionnés sont respectivement non substitués ou mono- ou polysubstitués, de manière identique ou différente, où les substituants sont choisis, indépendamment les uns des autres, en particulier dans le groupe constitué par -O-alkyle en C₁₋₃, F, Cl, Br, I, CF₃, OCF₃, OH, SH, phényle, phénoxy, naphtyle, furyle, thiényle et pyridinyle.

3. Composé substitué selon la revendication 1 ou 2, dans lequel B représente S(=O)₂.

4. Composé substitué selon une ou plusieurs des revendications 1 à 3, dans lequel, dans la formule générale (I), la structure partielle (Ac) 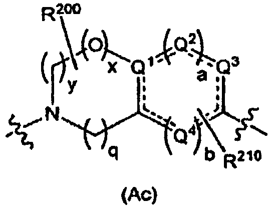 représente une structure partielle qui est choisie dans le groupe constitué par

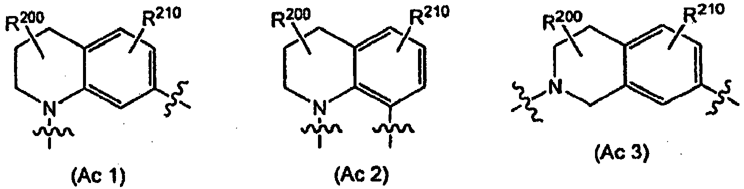

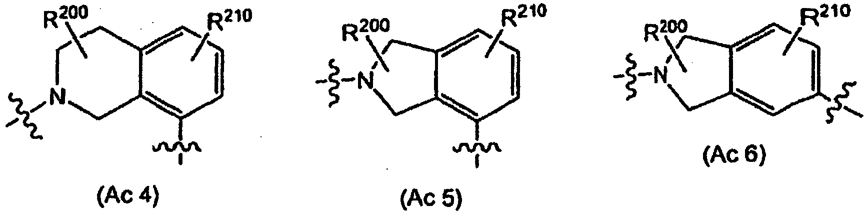

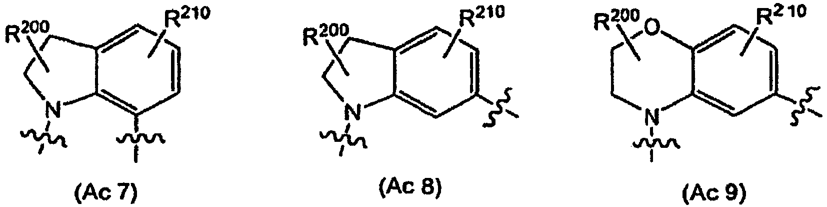

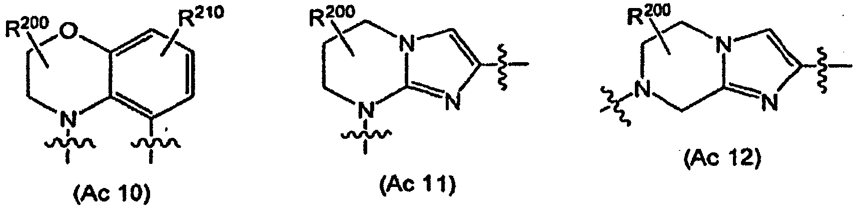

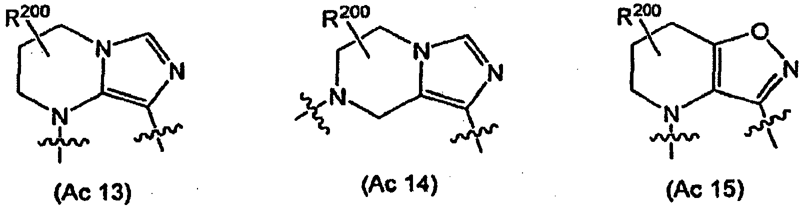

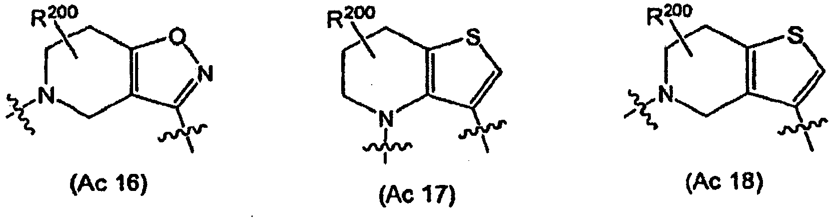

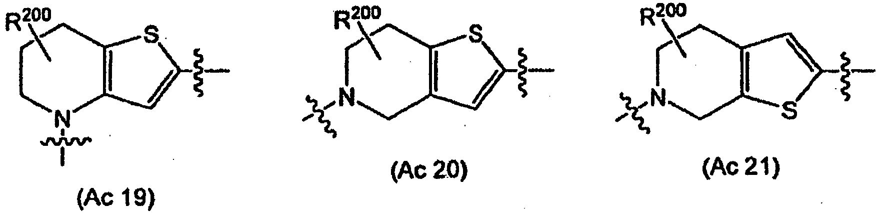

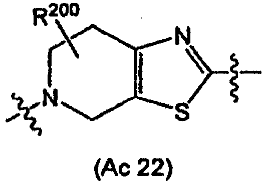

5. Composé substitué selon une ou plusieurs des revendications 1 à 4, dans lequel R⁸ représente H ; un groupe alkyle en C₁ à C₆, en particulier méthyle, éthyle, n-propyle, isopropyle, n-butyle, isobutyle, sec-butyle, ou tert-butyle ; cyclopropyle, cyclobutyle, cyclopentyle, cyclohexyle, -CH₂CF₃, phényle, benzyle, phényléthyle, phénylpropyle, ou un groupe cyclopropyle, cyclobutyle, cyclopentyle, cyclohexyle, lié par un groupe alkylène en C₁₋₃, respectivement non substitué ou mono- ou polysubstitué par des substituants identiques ou différents.

6. Composé substitué selon une ou plusieurs des revendications 1 à 5, dans lequel R^{9a} et R^{9b} représentent, respectivement indépendamment l'un de l'autre, H ; F ; un groupe méthyle ; éthyle, isopropyle, CF₃, méthoxy ; cyclopropyle ; phényle ; benzyle, phényléthyle, alkylène en C₁ à C₃-cyclopropyle, alkylène en C₁ à C₃-cyclobutyle, alkylène en C₁ à C₃-cyclopentyle, alkylène en C₁ à C₃-cyclohexyle, alkylène en C₁ à C₃-CF₃, respectivement non substitués ou mono- ou polysubstitués par des substituants identiques ou différents, de préférence R^{9a} et R^{9b} représentent tous deux simultanément H.

7. Composé substitué selon une ou plusieurs des revendications 1 à 6, dans lequel A représente N.

8. Composé substitué selon une ou plusieurs des revendications 1 à 7, dans lequel s et t représentent chacun 0 et A représente N.

9. Composé substitué selon une ou plusieurs des revendications 1 à 8, dans lequel
X représente CR^{14a}R^{14b}, NR¹⁵ ou O;
Y représente CR^{16a}R^{16b} ;
R^{14a}, R^{14b}, R^{16a} et R^{16b} représentent, respectivement indépendamment les uns des autres, H, F, Cl, OH, un groupe alkyle en C₁ à C₆, O-alkyle en C₁ à C₆, cycloalkyle en C₃ à C₈, aryle ou hétéroaryle, ou un groupe cycloalkyle en C₃ à C₈, aryle ou hétéroaryle lié par un groupe alkylène en C₁ à C₆;
et/ou respectivement R^{14a} et R^{14b} peuvent représenter conjointement =O et/ou respectivement R^{16a} et R^{16b} peuvent représenter conjointement =O ;
R¹⁵ représente H, un groupe alkyle en C₁ à C₆, cycloalkyle en C₃ à C₈, aryle ou hétéroaryle, ou un groupe cycloalkyle en C₃ à C₈, aryle ou hétéroaryle lié par un groupe alkylène en C₁ à C₆ ;
Z représente CR^{18a}R^{18b} ou NR¹⁹ ; ou
Z, dans le cas où X représente O et f représente 0, représente -(N=(CR¹²⁶))-, où l'atome de N est lié à l'atome de O par une simple liaison, et
R¹²⁶ représente H, un groupe alkyle en C₁ à C₆, cycloalkyle en C₃ à C₈, aryle ou hétéroaryle, ou un groupe cycloalkyle en C₃ à C₈, aryle ou hétéroaryle lié par un groupe alkylène en C₁ à C₆ ;
R^{18a} représente H, un groupe alkyle en C₁ à C₆, cycloalkyle en C₃ à C₈, -NH (alkyle en C₁ à C₆), -N(alkyle en C₁ à C₆)₂, phényle, pyridyle, imidazolyle, triazolyle, pyrimidyle, thiazolyle, ou thiényle, respectivement non substitué ou mono- ou polysubstitué ; ou phényle, pyridyle, imidazolyle, triazolyle, pyrimidyle, thiazolyle, ou thiényle, lié par un groupe -(O)₀₋₁-(alkylène en C₁ à C₆), respectivement non substitué ou mono- ou polysubstitué ; ou
R^{18a} représente le radical selon la formule générale (VII), 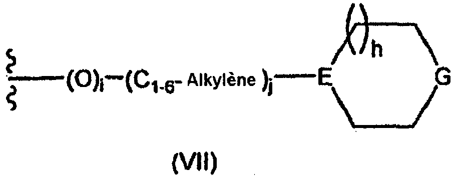 dans laquelle
i représente 0 ou 1 ;
j représente 0 ou 1 ;
h représente 0 ou 1 ;
E représente N ou CH, à la condition que si i représente 1 et j représente 0, E représente CH ;
G représente CR^{37a}R^{37b} ou NR³⁸; où
R^{37a} et R^{37b} représentent, indépendamment l'un de l'autre, H ; F ou un groupe alkyle en C₁ à C₆, respectivement non substitué ou mono- ou polysubstitué par des substituants identiques ou différents ;
R³⁸ représente H ; un groupe alkyle en C₁ à C₆, alkyle en C₃ à C₆ ou pyridyle ;
R^{18b} représente H, OH, un groupe alkyle en C₁ à C₆, phényle, pyridyle, imidazolyle, triazolyle, pyrimidyle, thiazolyle, ou thiényle, respectivement non substitué ou mono- ou polysubstitué par des substituants identiques ou différents ; ou phényle, pyridyle, imidazolyle, triazolyle, pyrimidyle, thiazolyle, ou thiényle, lié par un groupe alkylène en C₁ à C₆; respectivement non substitué ou mono- ou polysubstitué par des substituants identiques ou différents ; O-phényle ou O-pyridyle, respectivement non substitué ou mono- ou polysubstitué par des substituants identiques ou différents ; phényle, pyridyle ou thiényle ponté par un groupe alkylène en C₁₋₆-NH(C=O) respectivement non substitué ou mono- ou polysubstitué par des substituants identiques ou différents ;
R¹⁹ représente H, un groupe alkyle en C₁ à C₆, cycloalkyle en C₃ à C₈, -(C=O)-alkyle en C₁ à C₆, phényle, pyridyle, thiényle, thiazolyle, triazolyle, pyrimidinyle ou imidazolyle ; respectivement non substitué ou mono- ou polysubstitué par des substituants identiques ou différents ; ou phényle, pyridyle, thiényle, thiazolyle, pyrimidinyle, triazolyle, ou imidazolyle lié par un groupe alkylène en C₁ à C₆ ; respectivement non substitué ou mono- ou polysubstitué par des substituants identiques ou différents ;
ou représente le radical selon la formule générale (VIII), 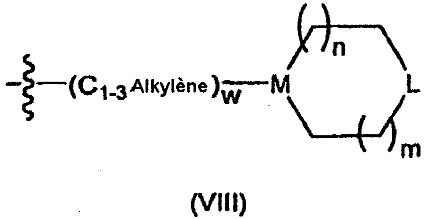 dans laquelle
w représente 0 ou 1 ;
n représente 0 ou 1 ;
m représente 0 ou 1 ;
M représente CH ou N à la condition que, si w représente 0, M représente CH ;
L représente CR^{44a}R^{44b} ou NR⁴⁵ ;
où R^{44a} et R^{44b} représentent, chacun indépendamment l'un de l'autre, H ; F ou un groupe alkyle en C₁ à C₆, respectivement non substitué ou mono- ou polysubstitué par des substituants identiques ou différents ; et
R⁴⁵ représente H, un groupe alkyle en C₁ à C₆, alkyle en C₃ à C₈ ou pyridyle.

10. Composé substitué selon une ou plusieurs des revendications 1 à 9, dans lequel s et t représentent respectivement 0 et ont la structure partielle suivante (SP) 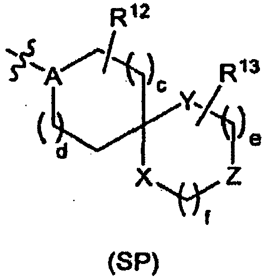 choisie dans le groupe constitué par 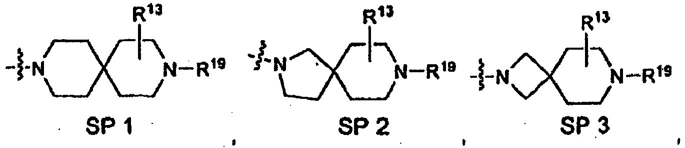 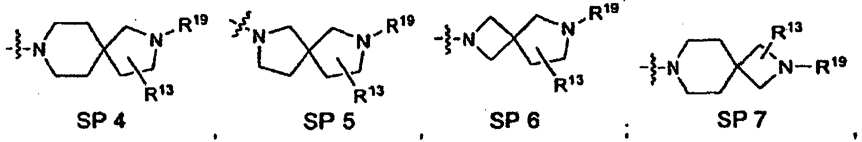 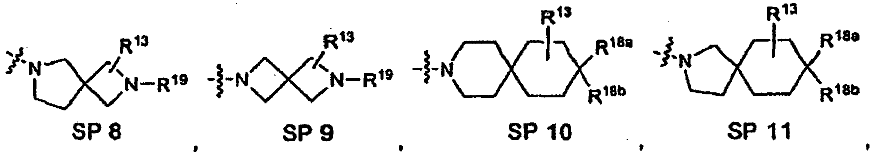 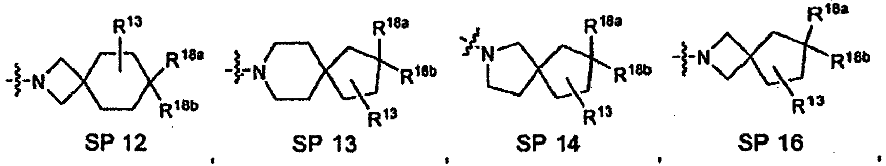 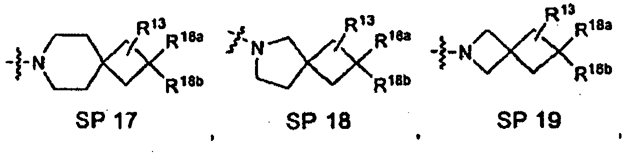 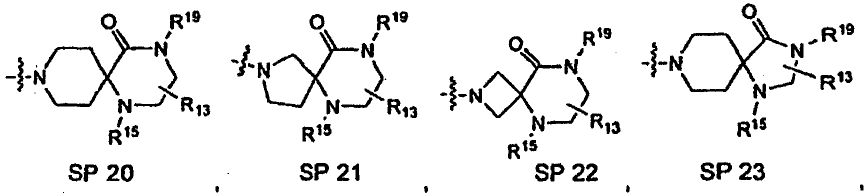 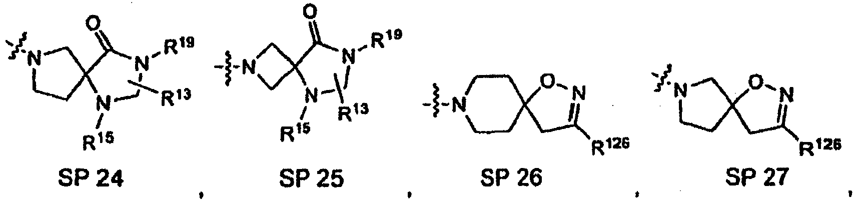 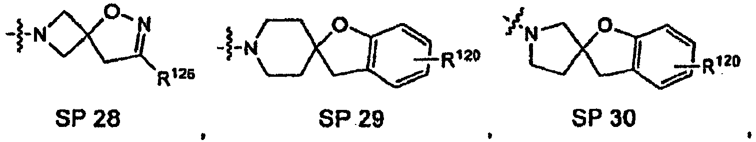 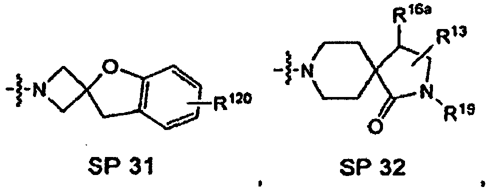 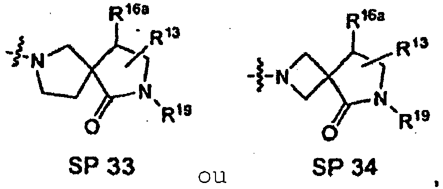 dans lesquelles
R¹³ représente 1 à 2 substituants, qui sont choisis dans le groupe constitué par H et le phényle, respectivement non substitué ou mono- ou polysubstitué par des substituants identiques ou différents ; et/ou deux des substituants R¹³ forment conjointement une liaison =O et/ou deux substituants adjacents R¹³ forment conjointement un aryle ou hétéroaryle condensé, en particulier un groupe benzo, respectivement non substitué ou mono- ou polysubstitué par des substituants identiques ou différents,
R¹⁵ représente H; un groupe alkyle en C₁ à C₆ ; cycloalkyle en C₃ à C₈, phényle, pyridyle, respectivement non substitué ou mono- ou polysubstitué par des substituants identiques ou différents ; ou phényle ou pyridyle, lié par un groupe alkylène en C₁ à C₆, respectivement non substitué ou mono- ou polysubstitué par des substituants identiques ou différents ;
R^{16a} représente H, un groupe alkyle en C₁ à C₆, phényle ou pyridyle, respectivement non substitué ou mono- ou polysubstitué par des substituants identiques ou différents ;
R^{18a} représente H; un groupe alkyle en C₁ à C₆ ; cycloalkyle en C₃ à C₈, N(alkyle en C₁ à C₆)₂ ; NH(alkyle en C₁ à C₆), azétidinyle ; pyrrolidinyle, pipéridinyle, 4-(alkyle en C₁ à C₆)-pipérazinyle ; phényle ou pyridyle, respectivement non substitué ou mono- ou polysubstitué par des substituants identiques ou différents ; ou N(alkyle en C₁ à C₆) ₂ lié par un groupe -(O)_{0/1}alkylène en C₁ à C₆ ; NH(alkyle en C₁ à C₆), azétidinyle ; pyrrolidinyle, pipéridinyle, 4-(alkyle en C₁ à C₆) -pipérazinyle ; phényle, imidazolyle, triazolyle ou pyridyle, respectivement non substitué ou mono- ou polysubstitué par des substituants identiques ou différents ;
R^{18b} représente H; OH; un groupe alkyle en C₁ à C₆ ; phényle ou pyridyle, respectivement non substitué ou mono- ou polysubstitué par des substituants identiques ou différents ; O-phényle ou O-pyridyle, respectivement non substitué ou mono- ou polysubstitué par des substituants identiques ou différents ou phényle ou pyridyle lié par un groupe alkylène en C₁ à C₆, respectivement non substitué ou mono- ou polysubstitué par des substituants identiques ou différents ;
R¹⁹ représente H ; un groupe alkyle en C₁ à C₆, cycloalkyle en C₃ à C₈, phényle, pyridyle, thiényle, imidazolyle, thiazolyle ou triazolyle, respectivement non substitué ou mono- ou polysubstitué par des substituants identiques ou différents ; ou phényle ou pyridyle lié par un groupe alkylène en C₁ à C₆ ou (C=O), respectivement non substitué ou mono- ou polysubstitué par des substituants identiques ou différents ;
R¹²⁰ représente H ; F ; Cl ; OH ; OCH₃, O-CF₃, un groupe alkyle en C₁ à C₆ ; CF₃ ou phényle, non substitué ou mono- ou polysubstitué ;
R¹²⁶ représente H; un groupe alkyle en C₁ à C₆ ; cycloalkyle en C₃ à C₈ ; phényle ou pyridyle ; ou cycloalkyle en C₃ à C₈, phényle ou pyridyle lié par un groupe alkylène en C₁ à C₃, respectivement non substitué ou mono- ou polysubstitué par des substituants identiques ou différents ;

11. Composés substitués selon une ou plusieurs des revendications 1 à 10, dans lesquels s et t représentent respectivement 0 et ont la structure partielle suivante (SP) 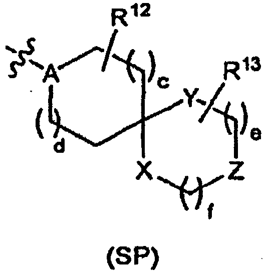 choisie dans le groupe constitué par 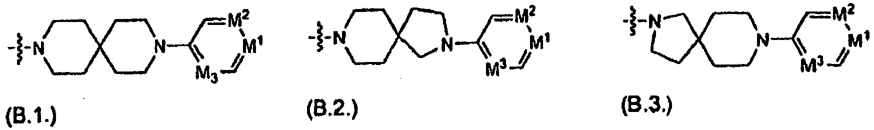 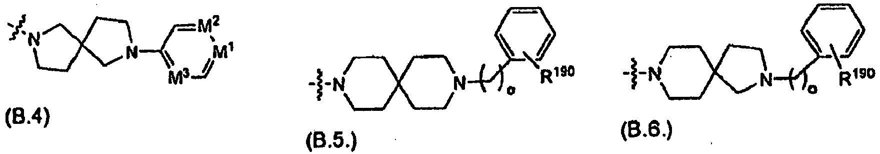 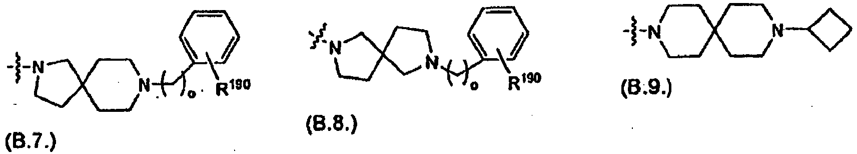 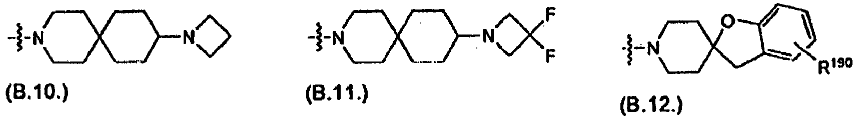 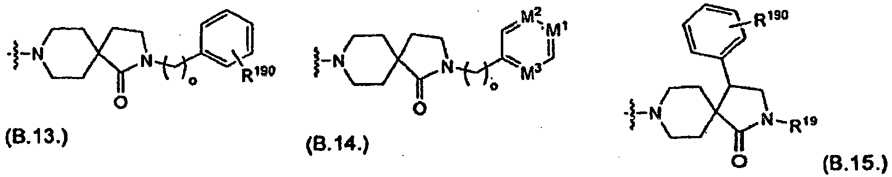 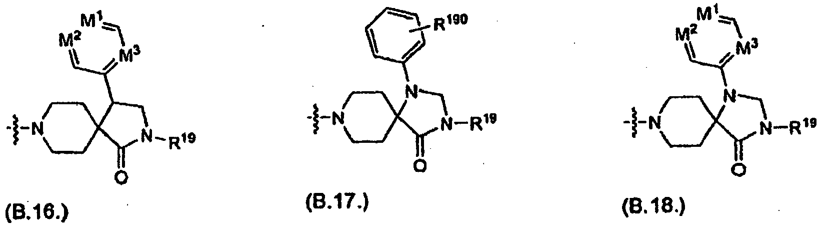 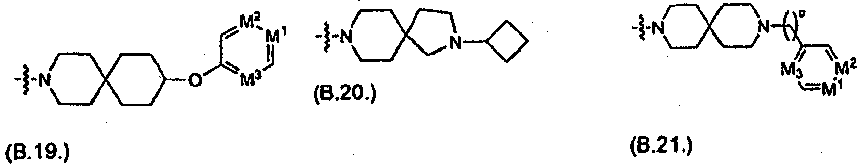 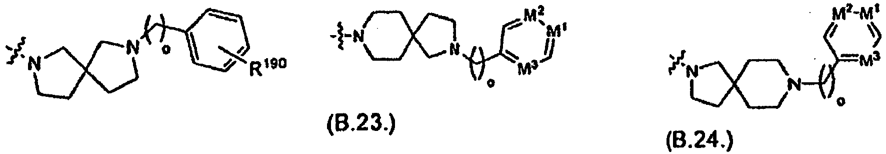 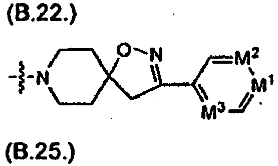 o = 0, 1, 2 ou 3 ;
M¹, M² et M³ peuvent représenter, indépendamment les uns des autres, respectivement N ou CH, où une variable parmi M¹, M² et M³ représente N et les deux autres représentent CH ;
R¹⁹ est choisi dans le groupe constitué par H ; un groupe alkyle en C₁ à C₆, en particulier méthyle, éthyle, n-propyle, isopropyle, n-butyle, sec-butyle, isobutyle et tert-butyle ; cycloalkyle en C₃ à C₆ ; respectivement non substitué ou mono- ou polysubstitué par des substituants identiques ou différents ; et
R¹⁹⁰ représente 0 à 4 substituants qui sont choisis indépendamment les uns des autres parmi F, Cl, O-CF₃, CF₃ ou CN.

12. Composé selon une ou plusieurs des revendications 1 à 11, choisi dans le groupe constitué par
| Composé | Nom |
|---|---|
| G-04 | (2-(4-méthoxy-2,6-diméthylphénylsulfonyl)-1,2,3,4-tétrahydro-isoquinoléine-7-yl)(9-(pyridine-4-yl)-3,9-diazaspiro[5.5]undécane-3-yl)méthanone |
| G-09 | 2-(1-(4-méthoxy-2,6-diméthylphénylsulfonyl)-1,2,3,4-tétrahydroquinoléine-7-yl)-1-(2-(pyridine-4-yl)-2,8-diazaspiro[4.5]décane-8-yl)éthanone |
| G-10 | 1-(2-cyclobutyl-2,8-diazaspiro[4.5]décane-8-yl)2-(1-(4-méthoxy-2,6-diméthylphénylsulfonyl)-1,2,3,4-tétrahydroquinoléine-7-yl) éthanone |
| G-11 | 2-(1-(4-méthoxy-2,6-diméthylphénylsulfonyl)-1,2,3,4-tétrahydroquinoléine-7-yl)-1-(8-(pyridine-4-yl)-2,8-diazaspiro[4.5]décane-2-yl)éthanone |
| G-12 | 2-(1-(4-méthoxy-2,6-diméthylphénylsulfonyl)-1,2,3,4-tétrahydroquinoléine-7-yl)-1-(7-(pyridine-4-yl)-2,7-diazaspiro[4.4]nonane-2-yl)éthanone |
| G-13 | (5-(2-chlorobenzoyl)-4,5,6,7-tétrahydroisoxazolo[4,5-c]pyridine-3-yl)(9-(pyridine-4-yl)-3,9-diazaspiro[5.5]undécane-3-yl)méthanone |
| G-14 | (5-(4-méthoxy-2,6-diméthylphénylsulfonyl)-4,5,6,7-tétrahydrothiéno[3,2-c]pyridine-2-yl)(9-(pyridine-4-yl)-3,9-diazaspiro[5.5]undécane-3-yl)méthanone |
| G-15 | (5-(2-chlorobenzoyl)-4,5,6,7-tétrahydrothiéno[3,2-c]pyridine-2-yl)(9-(pyridine-4-yl)-3,9-diazaspiro[5.5]undécane-3-yl)méthanone |
| G-16 | 1-(9-(pyridine-4-yl)-3,9-diazaspiro[5.5]undécane-3-yl)-2-(1-(2-trifluorométhylphényl-sulfonyl)-1,2,3,4-tétrahydroquinoléine-7-yl)éthanone |
| G-17 | 2-(1-(naphtalène-1-ylsulfonyl)-1,2,3,4-tétrahydroquinoléine-7-yl)-1-(9-(pyridine-4-yl)-3,9-diazaspiro[5.5]undécane-3-yl)éthanone |
| G-18 | 2-(1-(2-chloro-6-méthylphénylsulfonyl)-1,2,3,4-tétrahydroquinoléine-7-yl)-1-(9-(pyridine-4-yl)-3,9-diazaspiro[5.5]undécane-3-yl)éthanone |
| G-19 | 2-(4-(4-méthoxy-2,6-diméthylphénylsulfonyl)-3,4-dihydro-2H-benzo [b] [1,4]oxazine-6-yl)-1-(9-(pyridine-4-yl)-3,9-diazaspiro[5.5]undécane-3-yl)éthanone |
| G-20 | 2-(1-(4-méthoxy-2,6-diméthylphénylsulfonyl)-indoline-6-yl)-1-(9-(pyridine-4-yl)-3,9-diazaspiro[5.5]undécane-3-yl)éthanone |
| G-21 | (1-(2-chlorobenzoyl)-1,2,3,4-tétrahydroquinoléine-7-yl)(9-(pyridine-4-yl)-3,9-diazaspiro-[5.5]undécane-3-yl)méthanone |
| G-24 | (1-(4-méthoxy-2,6-diméthylphénylsulfonyl)-1,2,3,4-tétrahydroquinoléine-7-yl)(9-(pyridine-4-yl)-3,9-diazaspiro[5.5]undécane-3-yl) méthanone |
| G-25 | 2-(1-(4-méthoxy-2,6-diméthylphénylsulfonyl)-1,2,3,4-tétrahydroquinoléine-7-yl)-1-(9-(pyridine-4-yl)-3,9-diazaspiro[5.5]undécane-3-yl)éthanone |
| G-26 | (5-(4-méthoxy-2,6-diméthylphénylsulfonyl)-4,5,6,7-tétrahydro-isoxazolo[4,5-c]pyridine-3-yl)(9-(pyridine-4-yl)-3,9-diazaspiro[5.5]-undécane-3-yl)méthanone |
| G-30 | 2-(1-(4-méthoxy-2,6-diméthylphénylsulfonyl)-1,2,3,4-tétrahydroquinoléine-7-yl)-1-(9-(pyridine-4-yloxy)-3-azaspiro[5.5]undécane-3-yl)éthanone |
| G-32 | 1-(9-(azétidine-1-yl)-3-azaspiro[5.5]undécane-3-yl)-2-(1-(4-méthoxy-2,6-diméthylphénylsulfonyl)-1,2,3,4-tétrahydroquinoléine-7-yl)éthanone |
| G-33 | 2-(1-(4-méthoxy-2,6-diméthylphénylsulfonyl)-1,2,3,4-tétrahydroquinoléine-7-yl)-1-(3-(pyridine-4-yl)-1-oxa-2,8-diazaspiro[4.5]déc-2-ène-8-yl)éthanone |
| G-34 | 1-(9-(3,3-difluoroazétidin-1-yl)-3-azaspiro[5.5]undécane-3-yl)-2-(1-(4-méthoxy-2,6-diméthylphénylsulfonyl)-1,2,3,4-tétrahydroquinoléine-7-yl)éthanone |
| G-35 | 2-(1-(2-chloro-4-(trifluorométhoxy)-phénylsulfonyl)-1,2,3,4-tétrahydroquinoléine-7-yl)-1-(9-(pyridine-4-yl)-3,9-diazaspiro-[5.5]undécane-3-yl)éthanone |
| G-36 | 2-(4-méthoxy-2,6-diméthylphénylsulfonyl)-1,2,3,4-tétrahydro-isoquinoléine-8-yl)(9-(pyridine-4-yl)-3,9-diazaspiro[5.5]undécane-3-yl)méthanone |
| G-37 | 2-(2-chloro-6-méthylphénylsulfonyl)-1,2,3,4-tétrahydro-isoquinoléine-8-yl)(9-(pyridine-4-yl)-3,9-diazaspiro[5.5]undécane-3-yl)méthanone |
| G-38 | 2-(2,3-dichlorophénylsulfonyl)-1,2,3,4-tétrahydro-isoquinoléine-8-yl)(9-(pyridine-4-yl)-3,9-diazaspiro[5.5]undécane-3-yl)méthanone |
| G-39 | 2-(2-(4-méthoxy-2,6-diméthylphénylsulfonyl)-1,2,3,4-tétrahydro-isoquinoléine-7-yl)-1-(9-(pyridine-4-yl)-3,9-diazaspiro[5.5]undécane-3-yl)éthanone |
| G-40 | (7-(4-méthoxy-2,6-diméthylphénylsulfonyl)-5,6,7,8-tétrahydro-imidazo[1,2-a]pyrazine-2-yl)(9-(pyridine-4-yl)-3,9-diazaspiro[5.5]-undécane-3-yl)méthanone |
| G-41 | 3-(2-chlorophényl)-1-(7-(9-(pyridine-4-yl)-3,9-diazaspiro[5.5]undécane-3-carbonyl)-3,4-dihydro-isoquinoléine-2(1H)-yl)prop-2-ène-1-one |
| G-42 | (5-chlorothiophèn-2-yl)(7-(9-(pyridine-4-yl)-3,9-diazaspiro[5.5]undécane-3-carbonyl)-3,4-dihydro-isoquinoléine-2(1H)-yl)méthanone |
| G-43 | 2-(2-chlorophényl)-1-(7-(9-(pyridine-4-yl)-3,9-diazaspiro[5.5]undécane-3-carbonyl)-3,4-dihydro-isoquinoléine-2(1H)-yl)éthanone |
| G-44 | (5-chlorothiophène-2-yl)(8-(9-(pyridine-4-yl)-3,9-diazaspiro[5.5]undécane-3-carbonyl)-3,4-dihydro-isoquinoléine-2(1H)-yl)méthanone |
| G-45 | N-(2-chlorobenzyl)-7-(9-(pyridine-4-yl)-3,9-diazaspiro[5.5]undécane-3-carbonyl)-3,4-dihydro-isoquinoléine-2(1H)-carboxamide |
| G-46 | N-(3,4-dichlorophényl)-3-(9-(pyridine-4-yl)-3,9-diazaspiro[5.5]undécane-3-carbonyl)-6,7-dihydro-isoxazolo[4,5-c]pyridine-5(4H)-carboxamide |
| G-47 | (2-(4-méthylnaphtalène-1-yl-sulfonyl)-1,2,3,4-tétrahydro-isoquinoléine-7-yl)(9-(pyridine-4-yl)-3,9-diazaspiro[5.5]undécane-3-yl)méthanone |
| G-48 | (2-(4-méthoxynaphtalène-1-ylsulfonyl)-1,2,3,4-tétrahydro-isoquinoléine-7-yl)(9-(pyridine-4-yl)-3,9-diazaspiro[5.5]undécane-3-yl)méthanone |
| G-49 | 2,2-diphényl-1-(7-(9-(pyridine-4-yl)-3,9-diazaspiro[5.5]undécane-3-carbonyl)-3,4-dihydro-isoquinoléine-2(1H)-yl)-éthanone |
| G-50 | (2-(4-chloronaphtalène-1-ylsulfonyl)-1,2,3,4-tétrahydro-isoquinoléine-7-yl)(9-(pyridine-4-yl)-3,9-diazaspiro[5.5]undécane-3-yl)méthanone |
| G-51 | 2-(1-(2-chloro-6-méthylphénylsulfonyl)-1,2,3,4-tétrahydroquinoléine-7-yl)-1-(8-(pyridine-4-yl)-2,8-diazaspiro[4.5]décane-2-yl)éthanone |
| G-52 | 2-(1-(naphtalène-1-ylsulfonyl)-1,2,3,4-tétrahydroquinoléine-7-yl)-1-(8-(pyridine-4-yl)-2,8-diazaspiro[4.5]décane-2-yl)éthanone |
| G-53 | 2-(1-(naphtalène-1-ylsulfonyl)-1,2,3,4-tétrahydroquinoléine-7-yl)-1-(7-(pyridine-4-yl)-2,7-diazaspiro[4.4]nonane-2-yl)éthanone |
| G-54 | 1- (9-(azétidine-1-yl)-3-azaspiro[5.5] undécane-3-yl)-2-(1-(naphtalène-1-ylsulfonyl)-1,2,3,4-tétrahydroquinoléine-7-yl)éthanone |
| G-55 | N-(3,4-dichlorophényl)-7-(9-(pyridine-4-yl)-3,9-diazaspiro[5.5]undécane-3-carbonyl)-3,4-dihydro-isoquinoléine-2(1H)-carboxamide |
| G-56 | (2-(4-fluoronaphtalène-1-ylsulfonyl)-1,2,3,4-tétrahydro-isoquinoléine-7-yl)(9-(pyridine-4-yl)-3,9-diazaspiro[5.5]undécane-3-yl)méthanone |
| G-57 | 1-(8-(pyridine-4-yl)-2,8-diazaspiro[4.5]-décane-2-yl)-2-(1-(2-(trifluorométhyl)phényl-sulfonyl)-1,2,3,4-tétrahydroquinoléine-7-yl)éthanone |
| G-58 | (5-méthylthiophène-2-yl)(7-(9-(pyridine-4-yl)-3,9-diazaspiro[5.5]undécane-3-carbonyl)-3,4-dihydro-isoquinoléine-2(1H)-yl)méthanone |
| G-59 | Benzo[b]thiophène-2-yl(7-(9-(pyridine-4-yl)-3,9-diazaspiro[5.5]undécane-3-carbonyl)-3,4-dihydro-isoquinoléine-2(1H)-yl)méthanone |
| G-60 | (5,6-dihydro-4H-cyclopenta[b]thiophéne-2-yl)(7-(9-(pyridine-4-yl)-3,9-diazaspiro-[5.5]undécane-3-carbonyl)-3,4-dihydro-isoquinoléine-2(1H)-yl)méthanone |
| G-61 | (3-chloro-6-méthoxybenzo[b]thiophène-2-yl)(7-(9-(pyridine-4-yl)-3,9-diazaspiro[5.5]undécane-3-carbonyl)-3,4-dihydro-isoquinoléine-2(1H)-yl)méthanone |
| G-62 | (2-(5-chloronaphtalène-1-ylsulfonyl)-1,2,3,4-tétrahydro-isoquinoléine-7-yl)(9-(pyridine-4-yl)-3,9-diazaspiro[5.5]undécane-3-yl)méthanone |
| G-63 | (5-tert-butylthiophène-2-yl)(7-(9-(pyridine-4-yl)-3,9-diazaspiro[5.5]undécane-3-carbonyl)-3,4-dihydro-isoquinoléine-2(1H)-yl)méthanone |
| G-64 | 2-[1-(2-chlorobenzoyl)-1,2,3,4-tétra-hydroquinoléine-7-yl]-1-(9-pyridine-4-yl-3,9-diazaspiro[5.5]undécane-3-yl)-éthanone |
| G-66 | 2-[1-[(2,6-dichloro-3-méthylphényl)sulfonyl]-1,2,3,4-tétrahydroquinoléine-7-yl]-1-(9-pyridine-4-yl-3,9-diazaspiro[5.5]undécane-3-yl)-éthanone |
| G-67 | [2-[(4-méthoxy-2,6-diméthylphényl)sulfonyl]-2,3-dihydro-1H-iso-indole-5-yl]-(9-pyridine-4-yl-3,9-diazaspiro[5.5]undécane-3-yl)-méthanone |
| G-68 | [2-[(2-chloro-6-méthylphényl)sulfonyl]-2,3-dihydro-1H-iso-indole-5-yl]-(9-pyridine-4-yl-3,9-diazaspiro[5.5]undécane-3-yl)-méthanone |
| G-69 | [2-(5-chloro-thiophène-2-carbonyl)-2,3-dihydro-1H-iso-indole-5-yl]-(9-pyridine-4-yl-3,9-diazaspiro[5.5]undécane-3-yl)-méthanone |
| G-70 | 2-[8-[(4-méthoxy-2,6-diméthylphényl)-sulfonyl]-5,6,7,8-tétrahydro-[1,8]naphtyridine-2-yl]-1-(9-pyridine-4-yl-3,9-diazaspiro[5.5]undécane-3-yl)-éthanone |
| G-71 | [7-[(5-chloro-thiophène-2-carbonyl]-5,6,7,8-tétrahydro-imidazo [1,2-a]pyrazine-2-yl](9-pyridine-4-yl-3,9-diazaspiro[5.5]undécane-3-yl)-méthanone |
| G-72 | [7-[(4-méthoxy-2,6-diméthylphényl)-sulfonyl]-5,6,7,8-tétrahydro-imidazo[1,5-a]pyrazine-1-yl]-(9-pyridine-4-yl-3,9-diazaspiro-[5.5]undécane-3'-yl)-méthanone |
| G-73 | [2-(4-méthoxy-2,6-diméthylbenzoyl)-2,3-dihydro-1H-iso-indole-5-yl]-(9-pyridine-4-yl-3,9-diazaspiro[5.5]undécane-3-yl)-méthanone |
| G_CC-006 | 2-(4-fluorobenzyl)-8-(1-(4-méthoxy-2,6-diméthylphénylsulfonyl)indoline-6-carbonyl]-2,8-diazaspiro[4.5]décan-1-one |
| G_CC-007 | (7-benzyl-2,7-diazaspiro[4.4]nonane-2-yl)(1-(4-méthoxy-2,6-diméthylphénylsulfonyl)-indoline-6-yl)-méthanone |
| G_CC-008 | (1-(4-méthoxy-2,6-diméthylphénylsulfonyl)-indoline-6-yl)(9-(pyridine-4-yl)-3,9-diazaspiro[5.5]undécane-3-yl)-méthanone |
| G_CC-009 | (1-(2-chlorobenzoyl)indoline-6-yl)(9-(pyridine-4-yl)-3,9-diazaspiro[5.5]undécane-3-yl)-méthanone |
| G_CC-013 | (1-(4-chloro-2,5-diméthylphénylsulfonyl)-indoline-6-yl)(9-(pyridine-4-yl)-3,9-diazaspiro[5.5]undécane-3-yl)-méthanone |
| G_CC-018 | 8-(2-(2-chlorobenzoyl)iso-indoline-5-carbonyl)-2-(4-fluorobenzyl)-2,8-diazaspiro[4.5]décane-1-one |
| G_CC-025 | 4-(4-fluorophényl)-8-(1-(4-méthoxy-2,6-diméthylphénylsulfonyl)-1,2,3,4-tétrahydroquinoléine-7-carbonyl)-2-méthyl-2,8-diazaspiro[4.5]décane-1-one |
| G_CC-026 | 2-benzyl-8-(1-(4-méthoxy-2,6-diméthylphénylsulfonyl)-1,2,3,4-tétrahydroquinoléine-7-carbonyl)-2,8-diazaspiro[4.5]décane-1-one |
| G_CC-027 | (7-benzyl-2,7-diazaspiro[4.4]nonane-2-yl)(1-(4-méthoxy-2,6-diméthylphénylsulfonyl)-1,2,3,4-tétrahydroquinoléine-7-yl)-méthanone |
| G_CC-039 | 2-(4-fluorobenzyl)-8-(2-(4-méthoxy-2,6-diméthylphénylsulfonyl)-1,2,3,4-tétrahydro-isoquinoléine-7-carbonyl)-2,8-diazaspiro-[4.5]décane-1-one |
| G_CC-040 | 2-benzyl-8-(2-(4-méthoxy-2,6-diméthylphényl-sulfonyl)-1,2,3,4-tétrahydro-isoquinoléine-7-carbonyl)-2,8-diazaspiro-[4.5]décane-1-one |
| G_CC-041 | (7-benzyl-2,7-diazaspiro[4.4]nonane-2-yl)(2-(4-méthoxy-2,6-diméthylphénylsulfonyl)-1,2,3,4-tétrahydro-isoquinoléine-7-yl)-méthanone |
| G_CC-043 | 8-(2-(4-méthoxy-2,6-diméthylphénylsulfonyl)-1,2,3,4-tétrahydro-isoquinoléine-7-carbonyl)-1-phényl-1,3,8-triazaspiro[4.5]décane-1-one |
| G_CC-045 | (2-(2-chlorobenzoyl)-1,2,3,4-tétrahydro-isoquinoléine-7-yl)(9-(pyridine-4-yl)-3,9-diazaspiro[5.5]undécane-3-yl)-méthanone |
| G_CC-053 | 2-benzyl-8-(2-(1-(4-méthoxy-2,6-diméthylphé-nylsulfonyl)-1,2,3,4-tétrahydroquinoléine-7-yl)-acétyl)-2,8-diazaspiro[4.5]décane-1-one |
| G_CC-054 | 1-(7-benzyl-2,7-diazaspiro[4.4]nonan-2-yl)-2-(1-(4-méthoxy-2,6-diméthylphénylsulfonyl)-1,2,3,4-tétrahydroquinoléine-7-yl)-éthanone |
| G_CC-055 | 2-[1-[(4-méthoxy-2,6-diméthylphényl)-sulfonyl]-1,2,3,4-tétrahydroquinoléine-7-yl]-1-(3-pyridine-4-yl)-3,9-diazaspiro[5.5]-undécane-9-yl)-éthanone |
| G_CC-055 | [1-[(4-méthoxy-2,6-diméthylphényl)-sulfonyl]-1,2,3,4-tétrahydroquinoléine-7-yl]-(3-pyridine-4-yl-3,9-diazaspiro[5.5]undécane-9-yl)-méthanone |
le cas échéant sous la forme d'un énantiomère individuel ou d'un diastéréomère individuel, du racémate, des énantiomères, des diastéréomères, des mélanges des énantiomères et/ou des diastéréomères, respectivement sous la forme de leurs bases et/ou de leurs sels physiologiquement acceptables, en particulier des sels de chlorhydrate.

13. Produit pharmaceutique contenant au moins un composé selon une ou plusieurs des revendications 1 à 12.

14. Utilisation d'au moins un composé selon une ou plusieurs des revendications 1 à 12 pour la préparation d'un produit pharmaceutique pour le traitement de la douleur, des douleurs aiguës, des douleurs viscérales, des douleurs neuropathiques, des douleurs chroniques et/ou des douleurs inflammatoires ; de la migraine ; du diabète ; des maladies des voies respiratoires ; des maladies intestinales inflammatoires ; des pathologies neurologiques ; des inflammations dermatologiques ; des pathologies rhumatismales ; du choc septique ; du syndrome de reperfusion ; de l'obésité et/ou comme inhibiteur de l'angiogenèse.
